# EUROPEAN PATENT SPECIFICATION

(11) **EP 3 095 056 B1**
(45) Date of publication and mention of the grant of the patent: **20.01.2021**
(21) Application number: 14863914.9
(22) Date of filing: 19.11.2014
(51) Int. Cl.: G16B 20/00

(54) **COMBINED CYTOLOGY AND MOLECULAR TESTING FOR EARLY DETECTION OF ESOPHAGEAL ADENOCARCINOMA**
KOMBINIERTE ZYTOLOGISCHE UND MOLEKULARE PRÜFUNG ZUR FRÜHERKENNUNG VON SPEISERÖHRENADENOKARZINOM
TEST CYTOLOGIQUE ET MOLÉCULAIRE COMBINÉ POUR LA DÉTECTION PRÉCOCE D'ADÉNOCARCINOME SOPHAGIEN

(30) Priority: 19.11.2013 US 201361906363 P
(43) Date of publication of application: 23.11.2016
(73) Proprietor: Zhou, Zhongren, Pittsford, NY 14534 (US)
(72) Inventor: ZHOU, Zhongren, Pittsford, NY 14534 (US); GODFREY, Tony, Edward, Chestnut Hill, MA 02467 (US)
(74) Representative: Richards, Michèle E.
(86) International application number: PCT/US2014/066476
(87) International publication number: WO 2015/077382

(56) References cited:
- WO-A1-01/75172
- WO-A1-2013/148984
- WO-A2-2012/031008
- US-A1- 2009 286 237
- US-A1- 2012 009 597
- US-A1- 2013 143 222
- US-A1- 2013 190 357
- AUSTIN M DULAK ET AL: "Exome and whole-genome sequencing of esophageal adenocarcinoma identifies recurrent driver events and mutational complexity", NATURE GENETICS., vol. 45, no. 5, 1 January 2013 (2013-01-01), pages 478-486, XP055405113, NEW YORK, US ISSN: 1061-4036, DOI: 10.1038/ng.2591
- BETTSTETTER MARCUS ET AL: "Epidermal growth factor receptor, phosphatidylinositol-3-kinase catalytic subunit/PTEN, and KRAS/NRAS/BRAF in primary resected esophageal adenocarcinomas: loss of PTEN is associated with worse clinical outcome", HUMAN PATHOLOGY, SAUNDERS, PHILADELPHIA, PA, US, vol. 44, no. 5, 14 November 2012 (2012-11-14), pages 829-836, XP028579318, ISSN: 0046-8177, DOI: 10.1016/J.HUMPATH.2012.08.005
- MARILANDA FERREIRA BELLINI ET AL: "Alterations of the TP53 Gene in Gastric and Esophageal Carcinogenesis", JOURNAL OF BIOMEDICINE AND BIOTECHNOLOGY, vol. 2012, 1 January 2012 (2012-01-01), pages 1-13, XP055405193, US ISSN: 1110-7243, DOI: 10.1155/2012/891961
- SAETTA A ET AL: "Mutational analysis of K-ras, B-raf, EGFR, PIK3CA, AKT-1 gene and pERK expression in carcinogenesis of esophagus", VIRCHOWS ARCHIV, vol. 457, no. 2, August 2010 (2010-08), page 228, XP002773646, & INTERCONGRESS MEETING OF THE EUROPEAN-SOCIETY-OF-PATHOLOGY; KRAKOW, POLAND; AUGUST 31 -SEPTEMBER 04, 2010 ISSN: 0945-6317
- WEAVER J M J ET AL: "DEFINING THE GENETIC LANDSCAPE OF OESOPHAGEAL ADENOCARCINOMA BY NEXT-GENERATION SEQUENCING", GUT, vol. 61, no. Suppl. 2, July 2012 (2012-07) , pages A3-A4, XP002773647, & DIGESTIVE DISORDERS FEDERATION MEETING; LIVERPOOL, UK; JUNE 17 -20, 2012
- BANDLA SANTHOSHI ET AL: "Targeted resequencing of genes frequently mutated in esophageal adenocarcinoma using highly multiplexed Ion Torrent AmpliSeq (TM) technology and semiconductor sequencing", CANCER RESEARCH, vol. 72, no. Suppl. 8, April 2012 (2012-04), pages LB-410, XP009500147, & 103RD ANNUAL MEETING OF THE AMERICAN-ASSOCIATION-FOR-CANCER-RESEARCH; CHICAGO, IL, USA; MARCH 31 -APRIL 04, 2012
- BANDLA S ET AL: "Analysis of Columnar Lined Esophagus Reveals Less Frequent Mutations In Non-Goblet Cell Metaplasia Than intestinal Metaplasia", LABORATORY INVESTIGATION, NATURE PUBLISHING GROUP, THE UNITED STATES AND CANADIAN ACADEMY OF PATHOLOGY, INC, vol. 93, no. Suppl. 1, 31 January 2013 (2013-01-31), page 145A, XP009500146, ISSN: 0023-6837
- STREPPEL ET AL.: 'Next-generation sequencing of endoscopic biopsies identifies ARID1A as a tumor-suppressor gene in Barrett's esophagus' ONCOGENE vol. 33, no. 3, 14 January 2014, pages 347 - 357, XP055237241
- DATABASE BIOSIS [Online] BIOSCIENCES INFORMATION SERVICE, PHILADELPHIA, PA, US; May 2012 (2012-05), KESSEL RACHEL M ET AL: "High Resolution Integrative Analysis Reveals Widespread Genetic and Epigenetic Alterations in Barrett's Epithelial Cells Exposed to Acid and Bile", XP009517163, Database accession no. PREV201200606445 & GASTROENTEROLOGY, vol. 142, no. 5, Suppl. 1, May 2012 (2012-05), page S444, DIGESTIVE DISEASE WEEK (DDW); SAN DIEGO, CA, USA; MAY 19 -22, 2012 ISSN: 0016-5085(print)
- S. R. Kadri ET AL: "Acceptability and accuracy of a non-endoscopic screening test for Barrett's oesophagus in primary care: cohort study", BMJ, vol. 341, no. sep10 1, 25 January 2010 (2010-01-25), pages c4372-c4372, XP055118821, ISSN: 0959-8138, DOI: 10.1136/bmj.c4372

## Description

### FIELD OF THE INVENTION

The invention features methods to determine a patient's risk of developing esophageal adenocarcinoma (EAC) and for early detection of esophageal adenocarcinoma.

### BACKGROUND

Esophageal adenocarcinoma (EAC) is one of the most common cancers worldwide and has increased 300-500% in the United States and other Western Countries over the past 30 to 40 years. In turn, death from EAC has also increased at an alarming rate. EAC is known to develop through a series of metaplastic, dysplastic, and neoplastic cellular changes that are largely initiated by chronic gastroesophageal reflux disease (GERD). GERD precipitates a metaplastic change of the esophageal epithelium from the normal squamous epithelium to a more columnar epithelium, which is known as Barrett's esophagus (BE). BE is estimated to be present in approximately 1.6% of adults in Western populations or up to 4 million Americans.

However, diagnosis of BE is controversial. In the United States, specialized intestinal metaplasia (IM) is defined by the presence of goblet cells and is required for a diagnosis of BE, while in the United Kingdom, the presence of globlet cells is not required for a diagnosis of BE. Recent studies have indicated that metaplasia without goblet cells (NGM) may also be pre-malignant and may carry a neoplastic progression risk similar to that of IM. Thus, patients with NGM may also be candidates for BE surveillance programs; however, the cancer-risk of NGM relative to IM remains largely unknown. Reducing the high mortality associated with EAC will require simplified means of diagnosing BE at an early stage in the progression to cancer. Therefore, there is an umet need for a reliable method to determine a subject's risk of developing EAC.

### SUMMARY OF THE INVENTION

The invention features methods for predicting the risk of a subject, e.g., a patient with esophageal columnar metaplasia (e.g., a subject with or without the present of goblet cells (NGH)), of developing EAC. Patients identified as at risk of developing EAC may then be treated, e.g., according to the methods known in the art for treating esophageal metaplasia or dysplasia. The methods and devices of the invention, e.g., microarrays, fluorescent probes, and sequencing technologies, can be used to detect and/or monitor the progression of metaplasia to dysplasia or EAC, e.g., using esophageal tissue samples from subjects in need thereof, for examples subjects at risk or having metaplasia.

The invention includes a method for determining the risk of, or detecting, EAC in a subject before or after one or more cancer treatments (e.g., surgery, radiation therapy, and/or chemotherapy) by determining a change in at least one biomarker (e.g., more than one biomarker, such as 2, 3, or 4 or more biomarkers), in which the biomarker has at least 85% (e.g., 85%, 90%, 95%, 97%, 99%, or 100%) sequence identity to the sequence of any one of the biomarkers from the group consisting of CDKN2A, ERBB2, SMARCA4, LRP1 B.

Also provided in this application are methods that include determining a change in pairwise combinations of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A (or a biomarker having at least 85%, 90%, 95%, 97%, 99%, or 100% sequence identity to the sequence of any one of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A). In another embodiment, the methods may include determining a change in triplet or quadruplet combinations of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A. (or a biomarker having at least 85%, 90%, 95%, 97%, 99%, or 100% sequence identity to the sequence of any one of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A). The methods of the invention may include determining a change in two or more biomarkers (e.g., 2, 3, or 4 biomarkers) simultaneously or in sequence.

A first aspect of the present invention relates to a method for determining a subject's risk for developing esophageal adenocarcinoma (EAC). The method involves (a) obtaining a biological sample from the esophagus of a subject having esophageal columnar metaplasia; (b) determining from the obtained biological sample the nucleic acid sequence of each gene in a panel of genes implicated in the development of EAC; (c) detecting the presence of any EAC-associated genetic mutations in the panel of genes sequenced from the obtained biological sample; and (d) determining the subject's risk for developing EAC based on the presence or absence of a threshold number of detected EAC-associated genetic mutations, where detecting the threshold number of EAC-associated mutations in the gene panel indicates high risk of developing EAC and detecting fewer than the threshold number of EAC-associated mutations in the gene panel indicates low risk of developing EAC.

A second aspect of the present invention is a method for determining a subject's risk for developing EAC. This method involves (a) obtaining a biological sample from the esophagus of a subject having esophageal columnar metaplasia; (b) contacting the biological sample with a reagent suitable to detect the presence or absence of an EAC-associated genetic mutations from any one or more members of a panel of genes implicated in the development of EAC; (c) identifying the presence of any EAC-associated genetic mutations in the panel of genes detected from the biological sample; (d) determining the subject's risk for developing EAC based on the presence or absence of a threshold number of detected EAC-associated genetic mutations, where detecting the threshold number of EAC-associated mutations in the gene panel indicates high risk of developing EAC and detecting fewer than the threshold number of EAC-associated mutations in the gene panel indicates low risk of developing EAC.

A third aspect of the present invention is a method for determining a subject's risk of developing EAC. The method includes detecting a change in one or more biomarkers in a biological sample including tissue from the esophagus of a subject, in which one or more biomarkers is selected from the group consisting of CDKN2A, ERBB2, SMARCA4, LRP1 B. The method further includes that detection of the change in the one or more biomarkers indicates the subject is at risk of developing EAC.

In some embodiments, the method includes detecting a change in one of the biomarkers. In particular embodiments, the method includes detecting a change in at least two of the biomarkers. In other embodiments, the method includes detecting a change in at least three of the biomarkers. In additional embodiments, the method includes detecting a change in at least four of the biomarkers. In further embodiments, the method includes detecting a change in at least five of the biomarkers.

In some embodiments, the subject has esophageal columnar metaplasia. In other embodiments, the change is a genetic mutation (e.g., a nonsynonymous mutation) in one or more of the biomarkers and/or a change in gene copy number of one or more of the biomarkers.

The method includes detecting a change in a biomarker, in which the biomarker is selected from the group consisting of CDKN2A, ERBB2, SMARCA4, LRP1 B.

In some embodiments, the biomarker is CDKN2A. In various embodiments, the method includes detecting a mutation in CDKN2A gene or a gene product thereof, in which preferably the gene product is a messenger ribonucleic acid (mRNA) or a complementary deoxyribonucleic acid (cDNA) thereof. In certain embodiments, the detecting includes contacting a probe capable of hybridizing to the CDKN2A gene or gene product thereof, in which preferably the probe includes a nucleic acid sequence including at least 15 contiguous nucleotides that are substantially identical or complementary to GenBank Accession No. NM_000077 (e.g, the sequence has at least 85%, 90%, 95%, 97%, or 100% sequence identity) and/or in which the probe includes a detectable label, such as a fluorophore. In further embodiments, the method further includes detecting a change in a second biomarker, in which the second biomarker is selected from the group consisting of ERBB2, SMARCA4, LRP1 B.

In some embodiments, the biomarker is ERBB2. In various embodiments, the method includes detecting a mutation in ERBB2 gene or a gene product thereof, in which preferably the gene product is a messenger ribonucleic acid (mRNA) or a complementary deoxyribonucleic acid (cDNA) thereof. In certain embodiments, the detecting includes contacting a probe capable of hybridizing to the ERBB2 gene or gene product thereof, in which preferably the probe includes a nucleic acid sequence including at least 15 contiguous nucleotides that are substantially identical or complementary to a sequence having at least 85% sequence identity to the sequence of GenBank Accession No. NM_001005862 (e.g, the sequence has at least 85%, 90%, 95%, 97%, or 100% sequence identity) and/or in which the probe includes a detectable label, such as a fluorophore. In further embodiments, the method further includes detecting a change in a second biomarker, in which the second biomarker is selected from the group consisting of CDKN2A, SMARCA4, LRP1 B.

In some embodiments, the biomarker is SMARCA4. In various embodiments, the method includes detecting a mutation in SMARCA4 gene or a gene product thereof, in which preferably the gene product is a messenger ribonucleic acid (mRNA) or a complementary deoxyribonucleic acid (cDNA) thereof. In certain embodiments, the detecting includes contacting a probe capable of hybridizing to the SMARCA4 gene or gene product thereof, in which preferably the probe includes a nucleic acid sequence including at least 15 contiguous nucleotides that are substantially identical or complementary to GenBank Accession No. NM_001128844 (e.g, the sequence has at least 85%, 90%, 95%, 97%, or 100% sequence identity) and/or in which the probe includes a detectable label, such as a fluorophore.

In further embodiments, the method further includes determining a change in a second biomarker, in which the second biomarker is selected from the group consisting of CDKN2A, ERBB2, LRP1 B.

In some embodiments, the biomarker is LRP1B. In various embodiments, the method includes detecting a mutation in LRP1B gene or a gene product thereof, in which preferably the gene product is a messenger ribonucleic acid (mRNA) or a complementary deoxyribonucleic acid (cDNA) thereof. In certain embodiments, the detecting including contacting a probe capable of hybridizing to the LRP1 B gene or gene product thereof, in which preferably the probe includes a nucleic acid sequence including at least 15 contiguous nucleotides that are substantially identical or complementary to GenBank Accession No. NM_018557 (e.g, the sequence has at least 85%, 90%, 95%, 97%, or 100% sequence identity) and/or in which the probe includes a detectable label, such as a fluorophore. In further embodiments, the method further includes determining a change in a second biomarker, in which the second biomarker is selected from the group consisting of CDKN2A, ERBB2, SMARC4.

In preferred embodiments, the biomarker is selected from the group consisting of CDKN2A, ERBB2, SMARCA4, and/or LRP1B and combinations of two, three, or four biomarkers.

In all embodiments, the method includes detecting a mutation in the group consisting of CDKN2A, ERBB2, SMARCA4, and/or LRP1B genes or gene products thereof, in which preferably the gene product is a messenger ribonucleic acid (mRNA) or a complementary deoxyribonucleic acid (cDNA) thereof.

In some embodiments, the biological sample is collected by brushing. In other embodiments, the biological sample is collected by use of a CYTOSPONGE® or CELL-MATE®. In alternative embodiments, the biological sample is collected by endoscopy.

Also provided is a method that further includes administering a therapy to the subject determined to be at risk for developing EAC. In certain embodiments, e.g., in which the subject is determined to have esophageal metaplasia, the therapy is selected from the group consisting of hyaluronic acid, nonsteroidal anti-inflammatory drugs, cyclooxygenase-1 (COX-1) inhibitors, cyclooxygenase-2 (COX-2) inhibitors, nonselective cyclooxygenase inhibitors, curcumin compositions, and endoscopy. In particular embodiments, e.g., in which the subject is determined to have dysplasia, the therapy is selected from radiation therapy, chemotherapy, immunomodulatory therapy, endoscopic mucosal resection (EMR), endoscopic submucosal dissection, radiofrequency ablation, cryoablation, photodynamic therapy, and combinations thereof.

In some embodiments, the change in one or more biomarkers is detected using a method selected from the group consisting of PCR, an array, a massarray, a beadarray, a microarray, a genechip, pyrosequencing, nanopore sequencing, nanoballs, sequencing by synthesis, sequencing by expansion, single molecule real time technology, true single molecule sequencing technology, sequencing by ligation, microfluidics, infrared fluorescence, next generation sequencing, and combinations thereof.

In some embodiments, the method further includes contacting the biological sample with a reagent capable of detecting the change in the one or more biomarkers. In various embodiments, the reagent includes a nucleic acid probe that is capable of specifically hybridizing to the one or more biomarkers. In particular embodiments, the nucleic acid probe includes a detectable label. In specific embodiments, the one or more single-stranded nucleic acid molecules have a length in the range of 10 to 100 nucleotides in length. In other embodiments, the one or more single-stranded nucleic acid molecules have a length in the range of 20 to 60 nucleotides. In preferred embodiments, the single-stranded nucleic acid molecules are at least 15 nucleotides long, preferably in which the single-stranded nucleic acid molecules are at least 25 nucleotides long, and/or in which the single-stranded nucleic acid molecules are deoxyribonucleic acid (DNA).

Also provided in here is a device including at least one single-stranded nucleic acid molecule having at least 15 contiguous nucleotides that are identical or complementary to a target nucleic acid molecule having at least 85% sequence identity to the nucleic acid sequence of one or more biomarkers selected from the group consisting of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A.

In some embodiments, at least one single stranded nucleic acid molecule is capable of hybridizing to the target nucleic acid molecule. In particular embodiments, the single-stranded nucleic acid molecule has 100% sequence identity to the target nucleic acid molecule.

In some embodiments, the device includes two or more of the different single-stranded nucleic acid molecules. In various embodiments, the device includes two or more different single stranded nucleic acid molecules, each of which is capable of hybridizing to a different target nucleic acid molecule. In other embodiments, the device includes three or more different single stranded nucleic acid molecules, each of which is capable of hybridizing to a nucleic acid molecule. In certain embodiments, the device includes four or more single stranded nucleic acid molecules capable of hybridizing to a different target nucleic acid molecule. In particular embodiments, the device includes five or more different single stranded nucleic acid molecules capable of hybridizing to a different target nucleic acid molecule.

In some embodiments, the biomarker is TP53. In other embodiments, the biomarker is CDKN2A. In certain embodiments, the biomarker is ERBB2. In alternate embodiments, the biomarker is SMARCA4. In particular embodiments, the biomarker is LRP1 B.

In some embodiments, the one or more single-stranded nucleic acid molecules have a length in the range of 10 to 100 nucleotides in length. In particular embodiments, the one or more single-stranded nucleic acid molecules have a length in the range of 20 to 60 nucleotides. In certain embodiments, the single-stranded nucleic acid molecules are at least 15 nucleotides long, preferably in which the single-stranded nucleic acid molecules are at least 25 nucleotides long, or in which the single-stranded nucleic acid molecules are deoxyribonucleic acid (DNA).

In some embodiments, the device allows a determination of a subject's risk of developing EAC by allowing detection of a change (e.g., a mutation, such as a nonsynonymous mutation or a change in gene copy number (e.g., a reduction)) in the one or more biomarkers.

Also provided in here
is a kit including the device and instructions for applying nucleic acid molecules collected from a biological sample including tissue from the esophagus of a subject to the device and/or instructions for determining a change in one or more biomarkers by detecting hybridization of the single-stranded nucleic acid molecules to the target nucleic acid molecules and/or instructions for determining a subject's risk of developing EAC, in which the one or more biomarkers is selected from the group consisting of TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A.

In some embodiments, the one or more single-stranded nucleic acid molecules have a length in the range of 10 to 100 nucleotides in length. In particular embodiments, one or more single-stranded nucleic acid molecules have a length in the range of 20 to 60 nucleotides. In certain embodiments, the single-stranded nucleic acid molecules are at least 15 nucleotides long, preferably in which the single-stranded nucleic acid molecules are at least 25 nucleotides long, or in which the single-stranded nucleic acid molecules are deoxyribonucleic acid (DNA).

In some embodiments of any of the above aspects, the subject is a human.

Monitoring a subject's determined risk for developing EAC is also contemplated. Such monitoring is carried out by repeating the methods according to the present invention after a period of time. The monitoring may include identifying an increase or decrease in risk by comparing the subject's determined risk to one or more previously determined risks. In one embodiment, the method according to the present invention involves repeating steps (a)-(d) noted above after a period of time for subjects determined to be at low risk for developing EAC. The period of time may be any suitable time period (e.g., approximately 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, or 24 months).

The panel of genes may include at least 4 from the group consisting of APC, ATM, CDKN2A, EGFR, ERBB2, KRAS, PIK3CA, PTEN, SMARCA4, TP53, AR, CDH11, CNTN6, CNTNAP5, COL11A1, CSMD3, CTNNB1, DPP10, ELTD1, LRP1B, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A.

The EAC-associated genetic mutations are described in the Examples herein or will be known to those of skill in the art. Dulak et al., "Exome and Whole-Genome Sequencing of Esophageal Adenocarcinoma Identifies Recurrent Driver Events and Mutational Complexity," Nature Genetics 45(5): 478-486 (2013).

In one embodiment the panel of genes includes at least 20 genes, and the threshold number of EAC-associated genetic mutations is at least one. In one embodiment, the panel of genes includes APC, ATM, CDKN2A, EGFR, ERBB2, KRAS, PIK3CA, PTEN, SMARCA4, TP53, AR, CDH11, CNTN6, CNTNAP5, COL11A1, CSMD3, CTNNB1, DPP10, ELTD1, and LRP1B. In one embodiment the panel of genes includes APC, ATM, CDKN2A, EGFR, ERBB2, KRAS, PIK3CA, PTEN, SMARCA4, TP53, AR, CDH11, CNTN6, CNTNAP5, COL11A1, CSMD3, CTNNB1, DPP10, ELTD1, and LRP1B, and the threshold number of EAC-associated genetic mutations is at least two.

The threshold number of EAC-associated genetic mutations may be at least one, at least two, at least three, at least four, at least five, at least six, at least seven, at least eight, at least nine, or at least ten. In one embodiment, the threshold number of EAC-associated genetic mutations is at least four and the subject is determined to have EAC.

Suitable subjects in accordance with the present invention include any mammal including, but not limited to, humans, bovines, primates, equines, porcines, caprines, ovines, felines, canines, and any rodent (e.g., rats, mice, hamsters, and guinea pigs). In one embodiment, the subject is a human. The subject may be one with no esophageal metaplasia. The subject may also be one with non-goblet cell metaplasia ("NGM"), intestinal metaplasia ("IM"), or Barrett's metaplasia ("BM") (also known as Barrett's Esophagus ("BE")).

As noted above, the methods according to the present invention may involve obtaining a biological sample from the esophagus of a subject having esophageal columnar metaplasia. Any appropriate method of obtaining such a sample is contemplated, including, but not limited to, collecting a cytological sample by brushing, collecting a cytological sample by use of a Cytosponge® or Cell-Mate®, collecting a cytological sample by endoscopy, or combinations of such collecting methods.

In one embodiment, the method also involves administering a therapy to those subjects determined to be at high risk for developing EAC. The therapy includes any suitable therapy for the treatment of such subjects, including, but not limited to, endoscopic mucosal resection ("EMR"), endoscopic submucosal dissection, a therapeutically effective amount of radiofrequency ablation, a therapeutically effective amount of cryoablation, a therapeutically effective amount of photodynamic therapy, and combinations thereof.

As noted above, the methods according to the present invention involve detecting the presence of EAC-associated genetic mutations. The methods according to the present invention may involve determining from the obtained biological sample the nucleic acid sequence of each gene in a panel of genes implicated in the development of EAC and detecting the presence of any EAC-associated genetic mutations in the panel of genes sequenced from the obtained biological sample. In one embodiment, next generation or whole-genome sequencing (e.g., ION Torrent Systems) is used to carry out sequencing according to the present invention.

The methods according to the present invention may involve contacting the biological sample with a reagent suitable to detect the presence or absence of an EAC-associated genetic mutations from any one or more members of a panel of genes implicated in the development of EAC and identifying the presence of any EAC-associated genetic mutations in the panel of genes detected from the biological sample. In one embodiment, the reagent suitable to detect the presence or absence of EAC-associated genetic mutations from any one or more members of a panel of genes implicated in the development of EAC may include a nucleic acid probe that is capable of specifically hybridizing to the EAC-associated genetic mutation. In one embodiment, the nucleic acid probe includes a detectable label. Such labels will be known to those of skill in the art and may include, for example, fluorophores.

Detecting the presence of EAC-associated genetic mutations according to the present invention may be carried out by using any genetic testing platform (such as those provided by Illumina, Sequenom, General Electric, Agilent, 454 Life Sciences, Pacific Biosciences, Complete Genomics, Helicos Biosciences, Intelligent Bio-Systems, IBM, ION Torrent Systems, Halcyon Molecular, GE Global, Stratos Genomics, Genome Corp., Genome Diagnostics, Agencourt Bioscience, Microchip Biotechnologies, or Affymetrix) and with any genetic testing methodology (such as arrays, massarrays, beadarrays, microarrays, genechips, PCR, partial or full exome sequencing, and partial or full genome sequencing, such as with pyrosequencing, nanopore, fluorophores, nanopore sequencing, nanoballs, sequencing by synthesis, sequencing by expansion, single molecule real time technology (SMRT)™, true single molecule sequencing technology (tSMS)™, or sequencing by ligation, microfluidics, infrared fluorescence, or other sequencing method or apparatus).

Also disclosed is a non-transitory computer readable medium having stored thereon instructions for determining patient-specific risk assessment of developing EAC, including machine executable code which when executed by at least one processor, causes the processor to perform steps. The steps include determining from the obtained biological sample a nucleic acid sequence of each gene in a panel of genes implicated in the development of EAC; detecting a presence of any EAC-associated genetic mutations in a panel of genes sequenced from the obtained biological sample; and determining the subject's risk of developing EAC based on the presence or absence of a threshold number of detected EAC-associated genetic mutations, where detecting the threshold number of EAC-associated mutations in the gene panel indicates high risk of developing EAC and detecting fewer than the threshold number of EAC-associated mutations in the gene panel indicates low risk of developing EAC.

The examples may also be embodied as a non-transitory computer readable medium having instructions stored thereon for one or more aspects of the present technology as described and illustrated by way of the examples herein, as described herein, which when executed by a processor, cause the processor to carry out the steps necessary to implement the methods of the examples, as described and illustrated herein.

Furthermore disclosed is a genetic mutation computing device. The device includes one or more processors and a memory, where the memory coupled to the one or more processors which are configured to execute programmed instructions stored in the memory. The instructions include determining from the obtained biological sample a nucleic acid sequence of each gene in a panel of genes implicated in the development of EAC; detecting a presence of any EAC-associated genetic mutations in a panel of genes sequenced from the obtained biological sample; and determining the subject's risk of developing EAC based on the presence or absence of a threshold number of detected EAC-associated genetic mutations, where detecting the threshold number of EAC-associated mutations in the gene panel indicates high risk of developing EAC and detecting fewer than the threshold number of EAC-associated mutations in the gene panel indicates low risk of developing EAC.

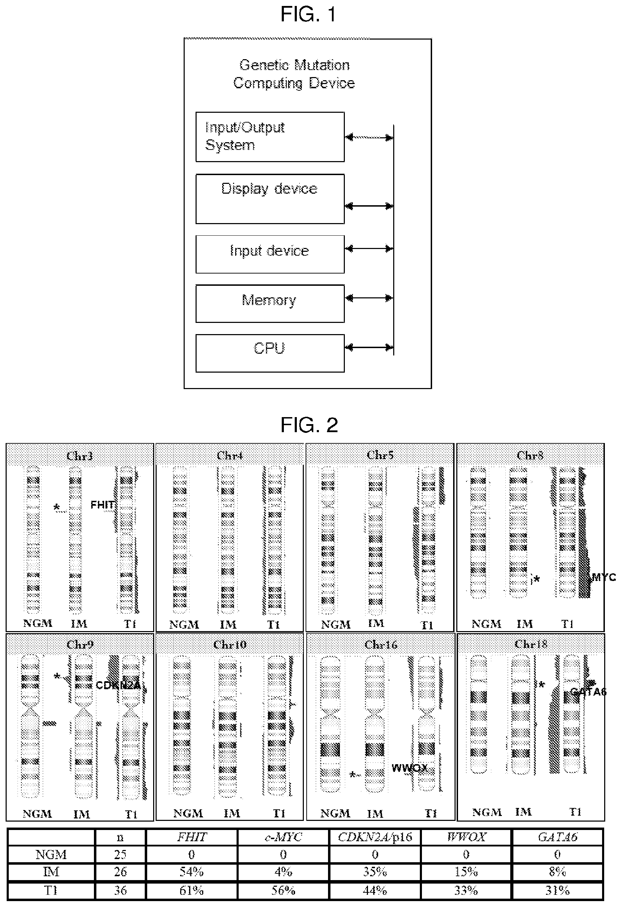

As illustrated in greater detail in FIG. 1, the genetic mutation computing device includes a central processing unit (CPU) or processor, a memory, a configurable logic device, an input device, a display device and an input/output system which are coupled together by a bus or other link, although other numbers and types of systems, devices, components, and elements in other configurations and locations can be used.

The processor in the genetic mutation computing device executes a program of stored instructions for one or more aspects of the present technology as described and illustrated by way of the examples herein, although other types and numbers of processing devices and logic could be used and the processor could execute other numbers and types of programmed instructions.

The memory in the genetic mutation computing device stores these programmed instructions for one or more aspects of the present technology as described and illustrated herein, although some or all of the programmed instructions could be stored and executed elsewhere. A variety of different types of memory storage devices, such as a random access memory (RAM) or a read only memory (ROM) in the system or a floppy disk, hard disk, CD ROM, DVD ROM, or other computer readable medium which is read from and written to by a magnetic, optical, or other reading and writing system that is coupled to the processor in the genetic mutation computing device, can be used for the memory in the genetic mutation computing device.

Input device enables a user, such as a programmer or a developer, to interact with the genetic mutation computing device, such as to input data and/or to configure, program and/or operate it by way of example only. By way of example only, input device may include one or more of a touch screen, keyboard and/or a computer mouse.

The display device enables a user, such as an administrator, to interact with the genetic mutation computing device, such as to view and/or input data and/or to configure, program and/or operate it by way of example only. By way of example only, the display device may include one or more of a touch screen display, a CRT, an LED monitor, or an LCD monitor, although other types and numbers of display devices could be used.

The input/output system in the genetic mutation computing device is used to operatively couple and communicate between the genetic mutation computing device, a second genetic mutation computing device, and/or any other database (e.g., reporting database or medical records database), which are all coupled together via the communications network, although other types and numbers of systems, devices, or elements and other types and numbers of communication networks or systems with other types and numbers of connections and configurations can be used. In this example, the communication network can be a Wide Area Network (WAN), or a Local Area Network (LAN), although the communication network can include other types of topologies supporting communication. By way of example only, the one or more the communications networks can use TCP/IP over Ethernet and industry-standard protocols, including NFS, CIFS, SOAP, XML, LDAP, and SNMP, although other types and numbers of communication networks, such as a direct connection, a local area network, a wide area network, modems and phone lines, e-mail, and wireless communication technology, each having their own communications protocols, can be used.

Although the invention has been described in detail for the purpose of illustration, it is understood that such detail is solely for that purpose, and variations can be made therein by those skilled in the art without departing from the spirit and scope of the invention which is defined in the following claims. Further, even though specific combinations may not be explicitly recited herein, it is to be understood by persons of skill in the art that such combinations of features are intended to be encompassed by the present disclosure for the recited products and methods.

### Definitions

"Metaplasia" as used herein is a form of controlled cell growth in which one type of adult or fully differentiated cell substitutes for another type of adult cell. Metaplastic disorders which can be diagnosed and/or treated by the methods of the invention include, but are not limited to epithelial metaplasia, esophageal metaplasia, and intestinal metaplasia.

"Dysplasia" as used herein refers to a form of cancer found mainly in the epithelia. Dysplasia is the most disorderly form of non-neoplastic cell growth, involving a loss in individual cell uniformity and in the architectural orientation of cells. Dysplastic cells often have abnormally large, deeply stained nuclei, and exhibit pleomorphism.

"Complement" of a nucleic acid sequence or a "complementary" nucleic acid sequence as used herein refers to an oligonucleotide which is in "antiparallel association" when it is aligned with the nucleic acid sequence such that the 5' end of one sequence is paired with the 3' end of the other. Nucleotides and other bases may have complements and may be present in complementary nucleic acids. Bases not commonly found in natural nucleic acids that may be included in the nucleic acids of the present invention include, for example, inosine and 7-deazaguanine. "Complementarity" may not be perfect; stable duplexes of complementary nucleic acids may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

When complementary nucleic acid sequences form a stable duplex, they are said to be "hybridized" or to "hybridize" to each other or it is said that "hybridization" has occurred. Nucleic acids are referred to as being "complementary" if they contain nucleotides or nucleotide homologues that can form hydrogen bonds according to Watson-Crick base-pairing rules (e.g., G with C, A with T or A with U) or other hydrogen bonding motifs such as for example diaminopurine with T, 5-methyl C with G, 2-thiothymidine with A, inosine with C, pseudoisocytosine with G, etc. Anti-sense RNA may be complementary to other oligonucleotides, e.g., mRNA.

"Gene" as used herein indicates a coding or noncoding gene whose activity can be determined by measuring a gene product, e.g. RNA or protein. Examples include protein coding genes, microRNAs, small nuclear RNAs and other RNAs with catalytic, regulatory or coding properties.

"Biomarker" as used herein indicates a gene or gene product (e.g., RNA or a protein) whose expression, or lack thereof, indicates risk of EAC. Two biomarkers may be expressed with one or more mutations (e.g., nonsynonymous mutation), the presence of which in a sample from a subject indicates an increased risk of EAC in the subject.

"Microarray" as used herein means a device employed by any method that quantifies one or more subject oligonucleotides, e.g., DNA or RNA, or analogues thereof, at a time. One exemplary class of microarrays consists of DNA probes attached to a glass or quartz surface. For example, many microarrays, including those made by Affymetrix, use several probes for determining the expression of a single gene. The DNA microarray may contain oligonucleotide probes that may be, e.g., full-length cDNAs complementary to an RNA or cDNA fragments that hybridize to part of an RNA. Exemplary RNAs include mRNA, miRNA, and miRNA precursors. Exemplary microarrays also include a "nucleic acid microarray" having a substrate-bound plurality of nucleic acids, hybridization to each of the plurality of bound nucleic acids being separately detectable. The substrate may be solid or porous, planar or nonplanar, unitary or distributed. Exemplary nucleic acid microarrays include all of the devices so called in Schena (ed.), DNA Microarrays: A Practical Approach (Practical Approach Series), Oxford University Press (1999); Nature Genet. 21 (1)(suppl.):1-60 (1999); Schena (ed.), Microarray Biochip: Tools and Technology, Eaton Publishing Company/BioTechniques Books Division (2000). Additionally, exemplary nucleic acid microarrays include substrate-bound plurality of nucleic acids in which the plurality of nucleic acids are disposed on a plurality of beads, rather than on a unitary planar substrate, as is described, inter alia, in Brenner et al., Proc. Natl. Acad. Sci. USA 97(4):1665-1670 (2000). Examples of nucleic acid microarrays may be found in U.S. Pat. Nos. 6,391,623, 6,383,754, 6,383,749, 6,380,377, 6,379,897, 6,376,191, 6,372,431, 6,351,712 6,344,316, 6,316,193, 6,312,906, 6,309,828, 6,309,824, 6,306,643, 6,300,063, 6,287,850, 6,284,497, 6,284,465, 6,280,954, 6,262,216, 6,251,601, 6,245,518, 6,263,287, 6,251,601, 6,238,866, 6,228,575, 6,214,587, 6,203,989, 6,171,797, 6,103,474, 6,083,726, 6,054,274, 6,040,138, 6,083,726, 6,004,755, 6,001,309, 5,958,342, 5,952,180, 5,936,731, 5,843,655, 5,814,454, 5,837,196, 5,436,327, 5,412,087, 5,405,783.

Exemplary microarrays may also include "peptide microarrays" or "protein microarrays" having a substrate-bound plurality of polypeptides, the binding of a oligonucleotide, a peptide, or a protein to each of the plurality of bound polypeptides being separately detectable. Alternatively, the peptide microarray, may have a plurality of binders, including but not limited to monoclonal antibodies, polyclonal antibodies, phage display binders, yeast 2 hybrid binders, aptamers, which can specifically detect the binding of specific oligonucleotides, peptides, or proteins. Examples of peptide arrays may be found in WO 02/31463, WO 02/25288, WO 01/94946, WO 01/88162, WO 01/68671, WO 01/57259, WO 00/61806, WO 00/54046, WO 00/47774, WO 99/40434, WO 99/39210, WO 97/42507 and U.S. Pat. Nos. 6,268,210, 5,766,960, 5,143,854.

As used herein, the term "percent (%) sequence identity" refers to the percentage of amino acid (or nucleic acid) residues of a candidate sequence, e.g., a probe or primer of the invention, that are identical to the amino acid (or nucleic acid) residues of a reference sequence, e.g., a biomarker sequence of the invention, after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared. In particular embodiments, a reference sequence aligned for comparison with a candidate sequence may show that the candidate sequence exhibits from 50% to 100% sequence identity across the full length of the candidate sequence or a selected portion of contiguous amino acid (or nucleic acid) residues of the candidate sequence. The length of the candidate sequence aligned for comparison purposes is at least 30%, e.g., at least 40%, e.g., at least 50%, 60%, 70%, 80%, 90%, or 100% of the length of the reference sequence. When a position in the candidate sequence is occupied by the same amino acid residue as the corresponding position in the reference sequence, then the molecules are identical at that position.

As used herein, the term "substantially identical" refers to a polypeptide or polynucleotide sequence that has the same polypeptide or polynucleotide sequence, respectively, as a reference sequence (e.g., a biomarker sequence of the invention), or has a specified percentage of amino acid residues or nucleotides, respectively, that are the same at the corresponding location within a reference sequence when the two sequences are optimally aligned. For example, an amino acid sequence that is "substantially identical" to a reference sequence has at least 50%, 60%, 70%, 75%, 80%, 85%, 90%, 95%, 96%, 97%, 98%, 99%, or 100% sequence identity to the reference amino acid sequence. For polypeptides, the length of comparison sequences will generally be at least 5, 6, 7, 8, 9, 10, 11, 12, 13, 14, 15, 16, 17, 18, 19, 20, 25, 50, 75, 90, 100, 150, 200, 250, 300, or 350 contiguous amino acids or more (e.g., the full-length sequence). For nucleic acids, the length of comparison sequences will generally be at least 5, 10, 11, 12, 13, 14, 15, 16, 17, 18, 19, 20, 21, 22, 23, 24, 25, 50, 75, 100, 200, 300, 400, 500, 1000, 2000, 3000, 4000, 5000 or more contiguous nucleotides (e.g., the full-length nucleotide sequence). Sequence identity may be measured using sequence analysis software on the default setting (e.g., Sequence Analysis Software Package of the Genetics Computer Group, University of Wisconsin Biotechnology Center, 1710 University Avenue, Madison, WI 53705). Such software may match similar sequences by assigning degrees of homology to various substitutions, insertions, deletions, and other modifications.

The term "sample," as used herein, refers to any specimen (e.g., tissue, fluid, and cells (e.g., a tissue sample obtained by biopsy) taken from a subject. Preferably, the sample is taken from a portion of the body affected by or at risk of a type of cancer (e.g., EAC). A sample of esophageal tissue may be obtained by endoscopy (e.g., by esophagogastroduodenoscopy (EGD)). The sample may be an esophageal culture. An esophageal sample may also be collected by CYTOSPONGE® or CELL-MATE®. Biopsy (e.g., cancer biopsy) may involve fine needle aspiration biopsy, core needle biopsy (e.g., stereotactic core needle biopsy, vacuum-assisted core biopsy, or magnetic resonance imaging (MRI) guided biopsy), or surgical biopsy (e.g., incisional biopsy or excisional biopsy). The sample may undergo additional purification and processing, for example, to remove cell debris and other unwanted molecules. Additional processing may further involve amplification, e.g., using PCR (RT-PCR). The standard methods of sample purification, such as removal of unwanted molecules, are known in the art.

The terms "patient" and "subject," as used interchangeably herein, refer to any animal (e.g., a mammal, e.g., a human). A subject to be treated or tested for risk of EAC according to the methods described herein may be one who has been determined to have a risk of EAC (such as GERD, BE, hiatal hernia, gastric reflux from the esophagus, heartburn, and/or regurgitation), may have a family history of EAC, or may have been diagnosed with a cancer (e.g., EAC or other cancer). Diagnosis may be performed by any method or technique known in the art, such as self-exam, endoscopy, EGD, esophageal pH monitoring, 48 hour reflux monitoring, endoscopic ultrasound, esophageal manometry, imaging (e.g., CT, MRI, or PET), x-ray (e.g., using a radiocontrast agent to observe reflux), or biopsy.

As used herein, "about" refers to an amount ± 10 of the recited value.

As used herein, "a" or "an" means "at least one" or "one or more" unless otherwise indicated. In addition, the singular forms "a", "an", and "the" include plural referents unless the context clearly dictates otherwise.

Other features and advantages of the invention will be apparent from the following Detailed Description, the drawings, and the claims.

### BRIEF DESCRIPTION OF THE DRAWINGS

**Figure 1** is an illustration of a genetic mutation computing device according to the present invention.
**Figure 2** is a diagram showing copy number alterations between 25 non-goblet metaplasia (NGM), 26 intestinal metaplasia (IM), and 36 T1 adenocarcinoma genomes. Copy number gains are represented in blue while losses are in red. Known cancer associated genes are indicated on the chromosomes for the T1 genomes and their alteration frequencies are indicated in the accompanying table. Presence of these changes in NGM and/or IM genomes is indicated by '*'.

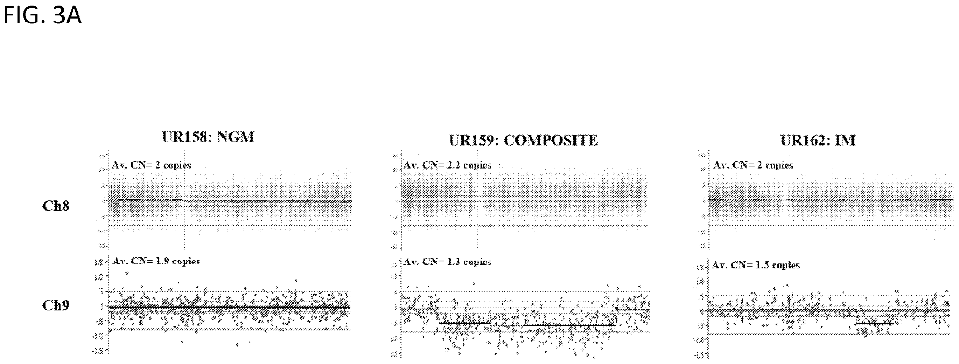

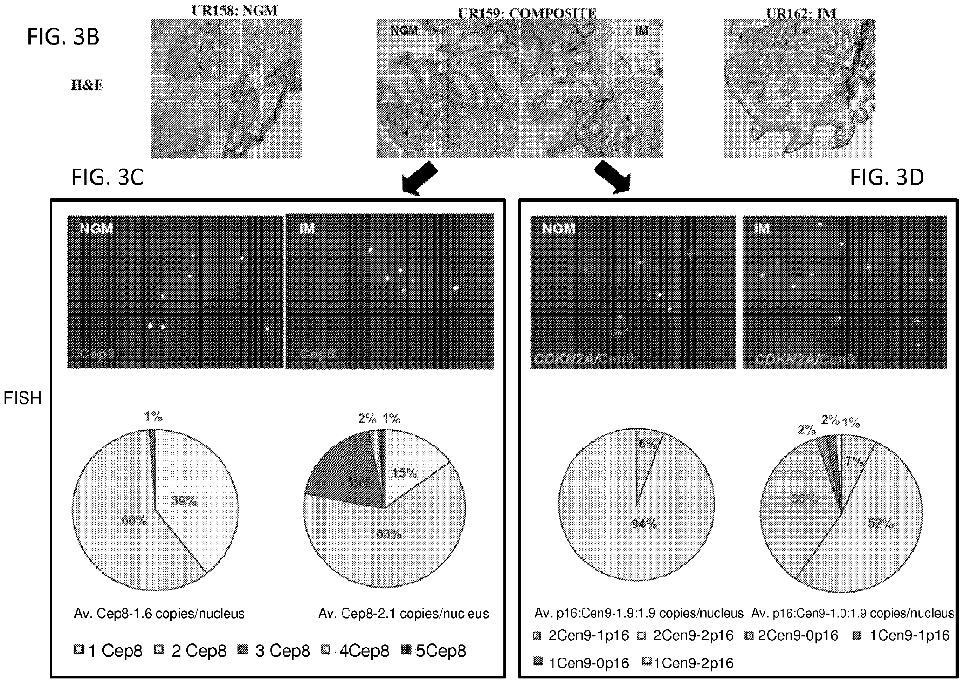

**Figure 3** is a diagram showing validation of genomic changes using fluorescence *in situ* hybridization (FISH) in composite sample (UR159) from subject 55. (A) Probe level copy number data showing the gain of entire chromosome 8 in the composite specimen (UR159) and loss of *CDK2NA* loci in composite (UR159) and intestinal metaplasia (IM; UR162) specimens compared to the NGM specimen (UR158). Y-axis represents the log₂ signal ratio where log₂0 = 2 copies (baseline). Average copy number per locus is indicated above each plot. The two green and red lines above and below the baseline respectively indicate the gain/high copy gain and loss/homozygous loss thresholds. (B) Representative H & E images for NGM (UR158), composite (UR159), and IM (UR162) taken at 20X magnification. (C) & (D) Representative fluorescence *in situ* hybridization (FISH) images of the NGM and IM portions of the composite tissue (UR159). Pie-charts summarize the result into different categories of normal (green) and abnormal signals (other colors as indicated) observed in each biopsy for gain of chromosome 8 (C) and loss of *CDKN2A* (D). Av.= average; CN= copy number; Cep8= centromere8; Cep9= centromere 9; p16= *CDKN2A*

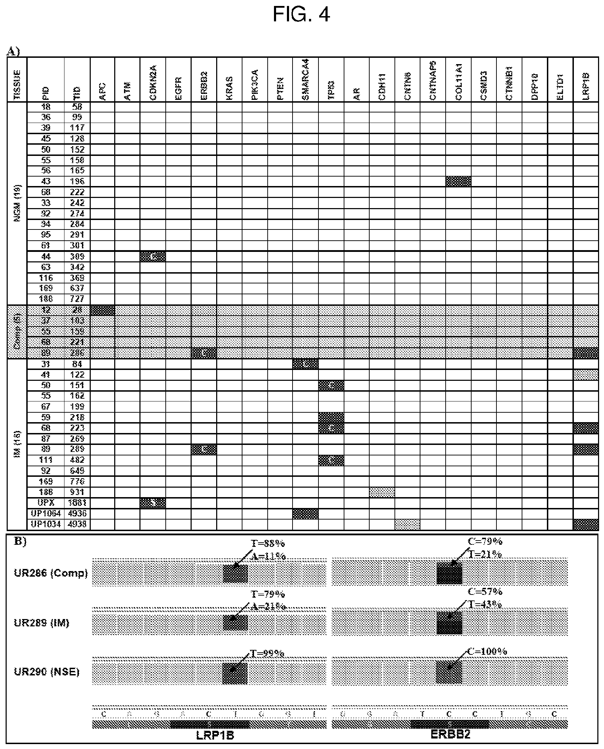

**Figure 4** is a graph showing targeted re-sequencing of 20 frequently mutated EAC genes from pre-neoplastic biopsies. Nineteen NGM, 5 composites, and 16 IM specimens from 36 subjects were analyzed along with matched normal squamous mucosa. Mutations in the coding regions within the specific genes are indicated in red (non-synonymous mutation) and green (silent substitutions) while 'C' indicates the mutation is reported in the COSMIC database and 'S' indicates the point mutation was identified by Sanger sequencing.

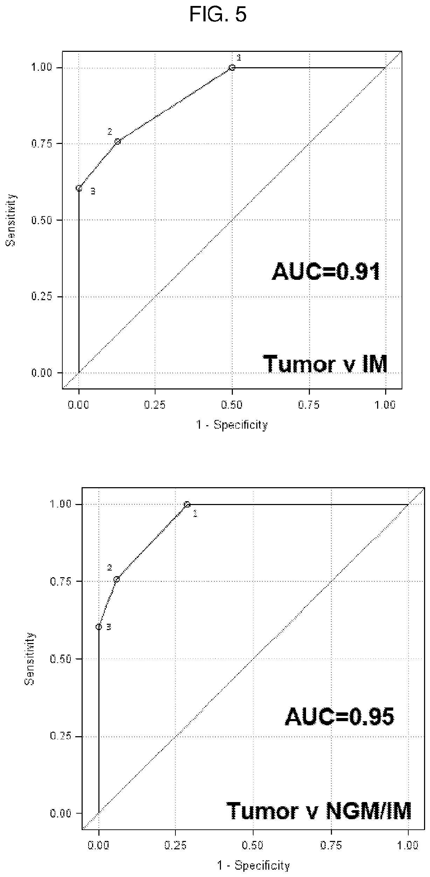

**Figure 5** is a diagram showing power of mutation load to discriminate between metaplasia and EAC. Pairwise Receiver-Operator Characteristics (ROC) curves analysis was performed for Tumor v IM and Tumor v any metaplasia (NGM/IM) using the 20-gene AmpliSeq mutation data from NGM and IM samples along with mutation data from 66 EAC tumor samples obtained from whole exome sequencing.

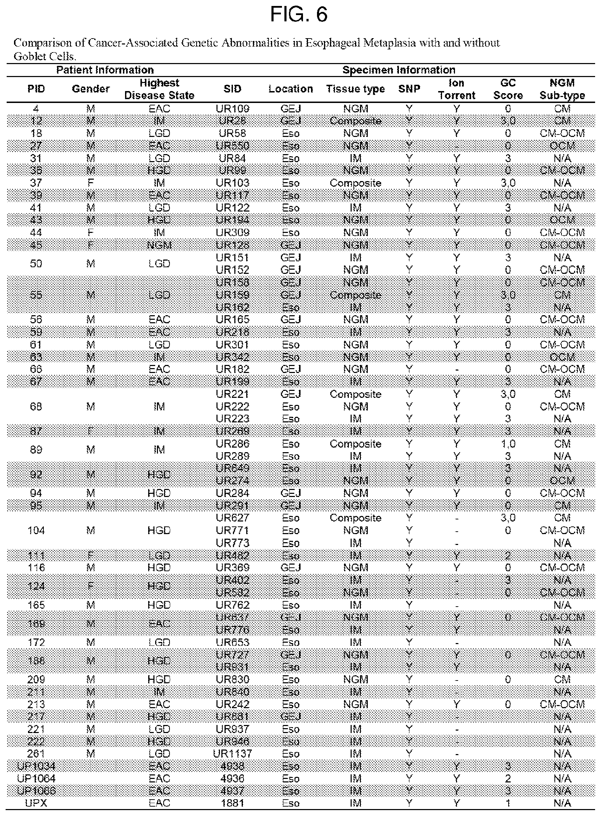

**Figure 6** is a graph showing patient and sample information.

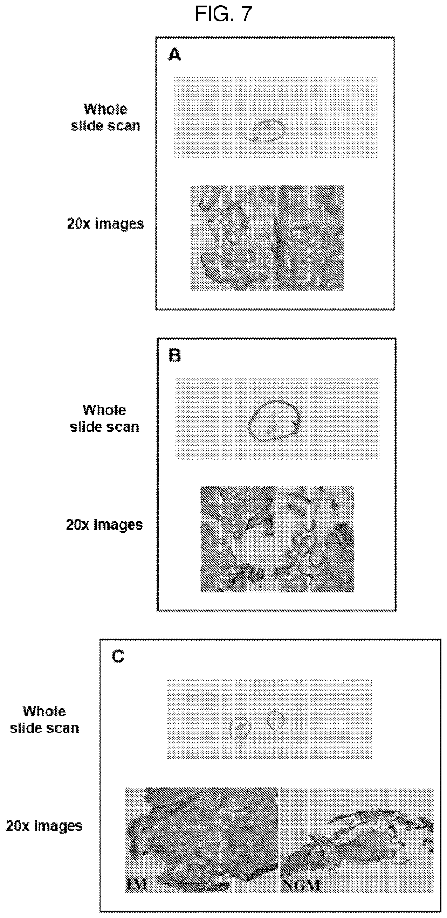

**Figure 7** is a diagram showing representative scans of various pre-neoplasia specimens. Whole slide scans and 20X magnification of (A) Intestinal metaplasia (IM) specimen (B) Non-goblet metaplasia (NGM) specimen; and (C) Composite specimen with IM in blue and NGM portion in red. Whole slide scans were obtained using Olympus VS-120 (Olympus, PA) and viewed in and exported from Visiopharm software (Visiopharm, Denmark). Representative H & E slides for each specimen taken using Olympus BX41 (Olympus, PA).

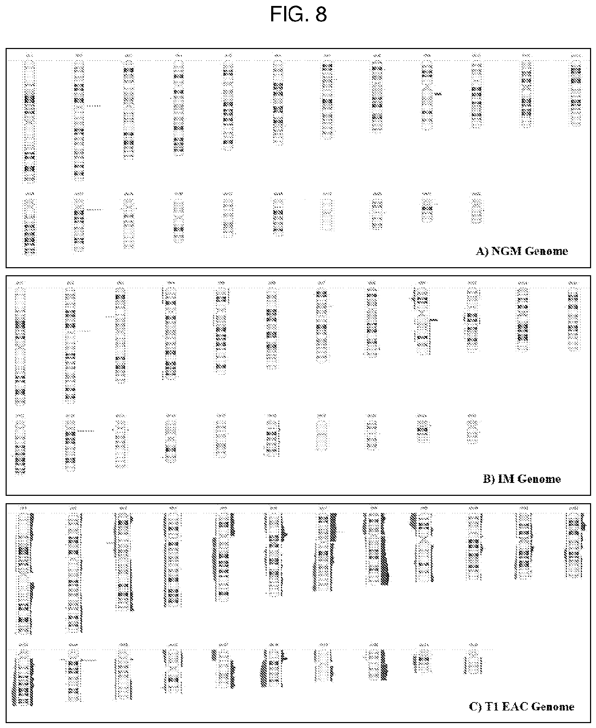

**Figure 8** is a diagram showing genome wide copy number changes observed in (A) 25 non-goblet metaplasia (NGM) samples (B) 26 Intestinal metaplasia (IM) samples, and (C) 36 T1 esophageal adenocarcinoma (EAC) samples. Blue indicates copy number gain while red indicates copy number loss.

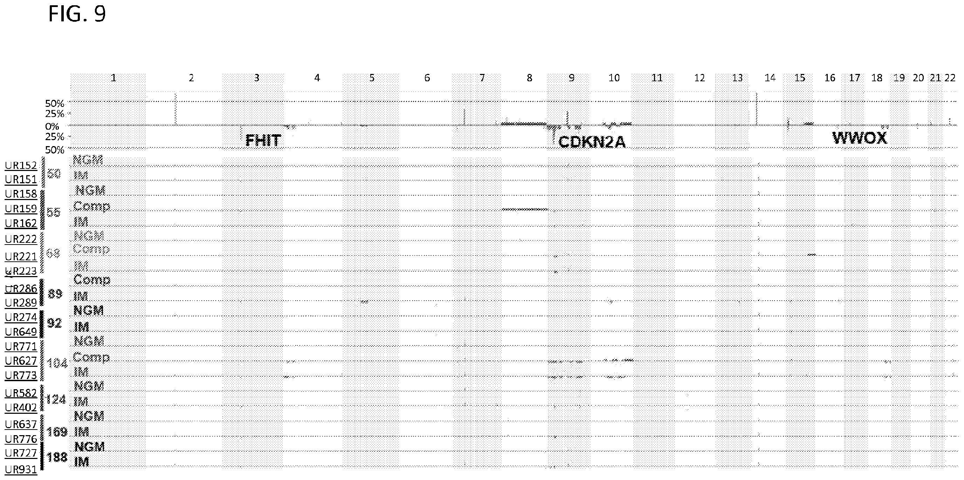

**Figure 9** is a diagram showing copy number comparison of specimens with different histologies from same patients. Overall genome view and frequencies of the 21 specimens from 9 patients being analyzed are shown in the top panel. Common cancer associated loci are indicated. Copy number data from non-goblet metaplasia (NGM), intestinal metaplasia (IM) and/or composite (Comp) samples from the same patients were compared. Patient IDs for multiple specimens from same patient and the individual specimen histologies are indicated along the specimen IDs.

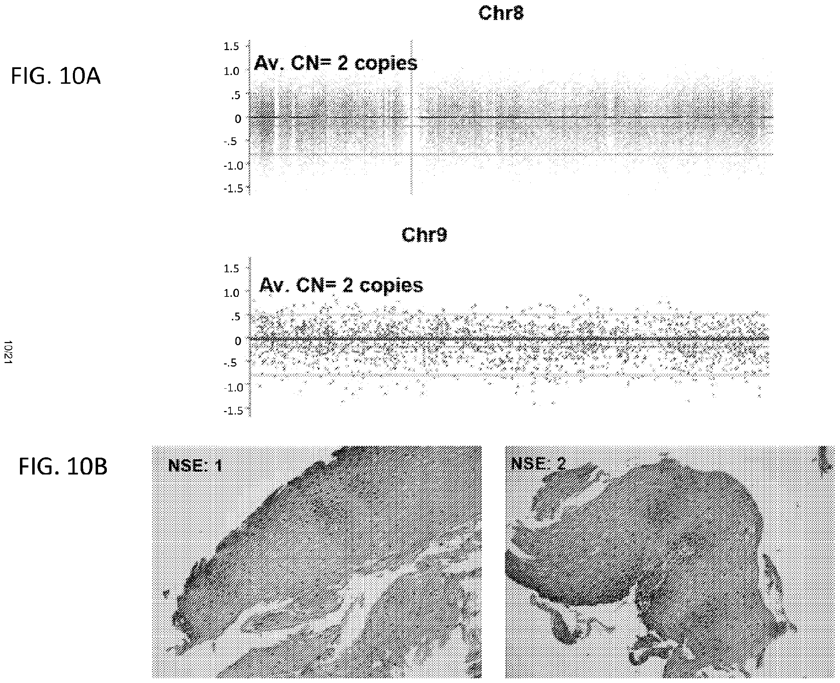

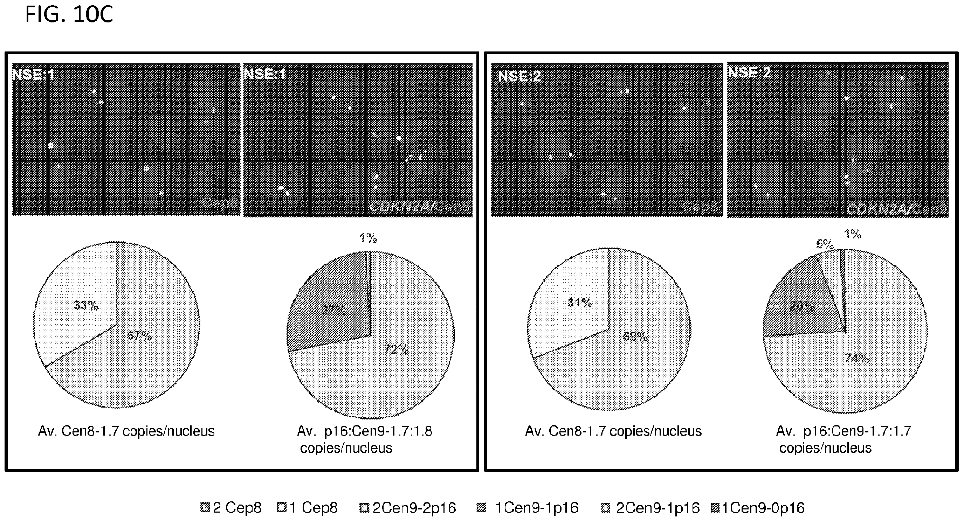

**Figure 10** is a diagram showing control Fluorescence in-situ hybridization (FISH) of normal squamous epithelium (NSE) (UR166) from patient 56. UR166 contained two biopsy tissues indicated as NSE:1 and NSE:2 (A) Probe level copy number data showing normal copy number on chromosome 8 and CDKN2A loci on chromosome 9. Y-axis represents the log2 signal ratio where log20= 2 copies (baseline). Average copy number per loci is indicated. The two green and red lines above and below the baseline respectively indicate the gain/high copy gain and loss/homozygous loss thresholds. (B) Representative H & E images of both tissue pieces (NSE:1 and NSE:2) taken at 20X magnification using Olympus BX41 (Olympus, PA). (C) Fluorescence in-situ hybridization (FISH) images of the both tissue pieces from UR166 showing 2 copies of centromere 8 and CDKN2A: centromere 9 loci. Also included are pie-charts summarizing the results into different categories signals observed in each tissue piece. Key for the piechart colors is included. Average signals per piece and legend are included below the image. Av.= average; CN= copy number; Cep8= centromere8; Cen9= centromere 9; p16= CDKN2A

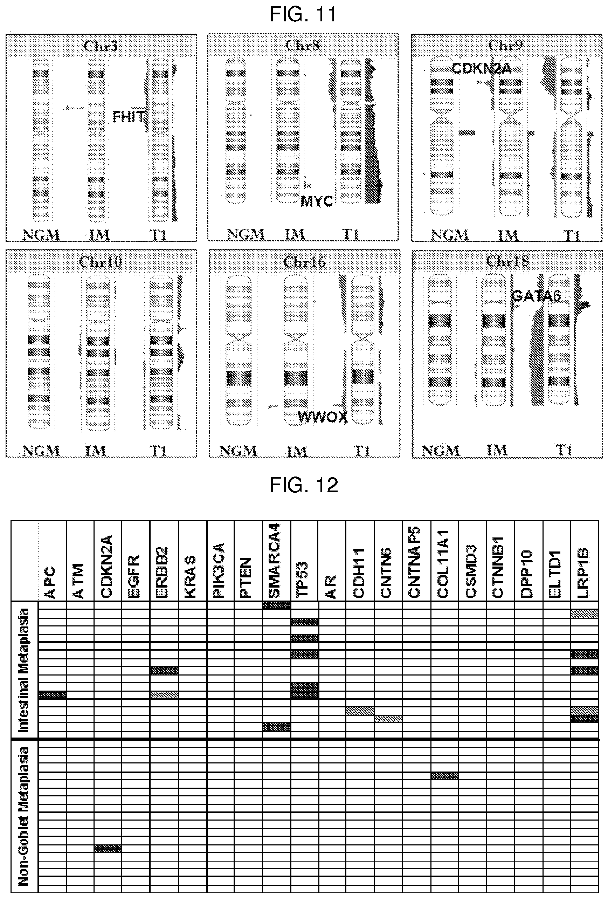

**Figure 11** is a diagram showing DNA copy number aberrations detected in non-goblet metaplasia (NGM), intestinal metaplasia (IM) and T1 tumor specimens. Red (left) indicates copy loss and blue (right) indicates copy gain. Common losses were observed on chromosome 3 (FHIT), 9 (CDKN2A) and 16 (WWOX) in both T1 tumors and IM but not in NGM. Similarly, amplifications of c-MYC (Chr8) and GATA6 Chr18) were common in tumors and were observed in some IM samples but not in NGM.
**Figure 12** is a graph showing mutations in a 20 gene panel detected in NGM (n=17) and IM (n=16) samples using AmpliSeq technology. Red indicates a non-synonymous (amino acid changed) mutation (APC, ERBB2, SMARCA4, TP53, COL11A1, and LRP1B) and blue indicates a synonymous mutation (ERBB2, CDH11, CNTN6, and LRP1B). Mutations are clearly much more frequent in IM than in NGM samples.

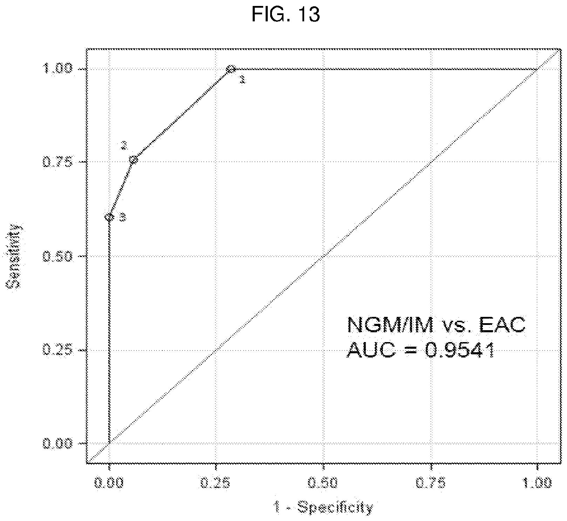

**Figure 13** is a graph showing ROC curve for NGM or IM versus EAC using number of mutations as the diagnostic criterion. AUC is 0.954 indicating strong discriminatory power of mutation load for identifying neoplastic status.

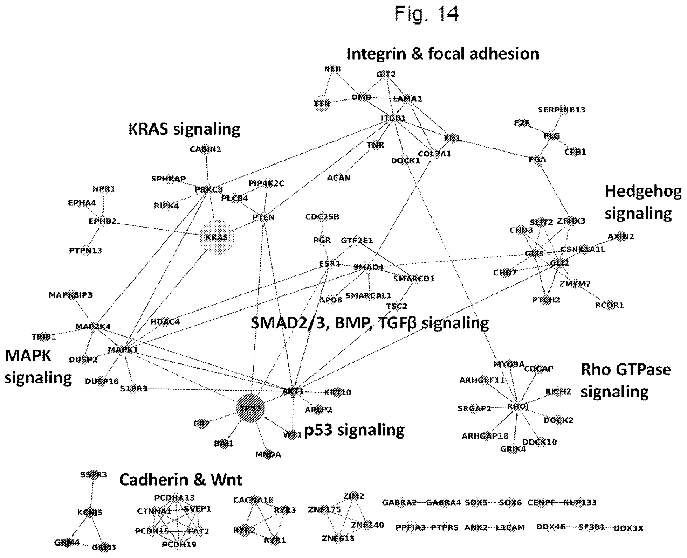

**Figure 14** is a diagram showing a gene module map for pancreatic cancer derived from exome sequencing of 44 pancreatic cancer specimens.

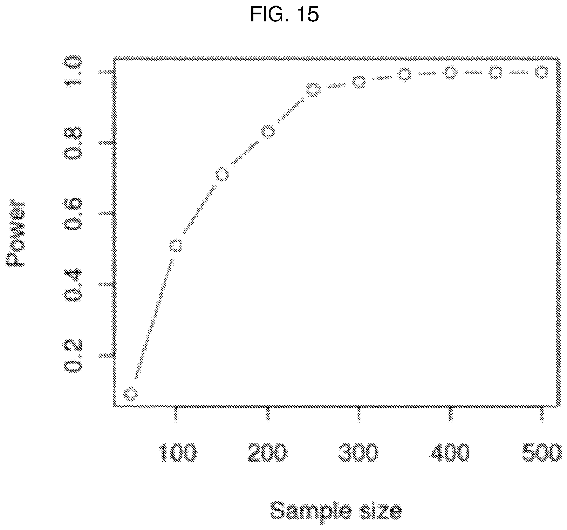

**Figure 15** is a graph showing power sample size against driver mutation detection power.

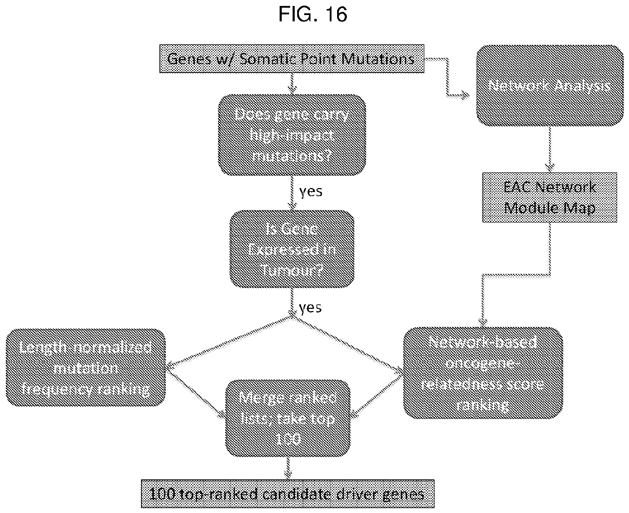

**Figure 16** is a diagram showing a flowchart for selecting candidate driver genes in EAC.

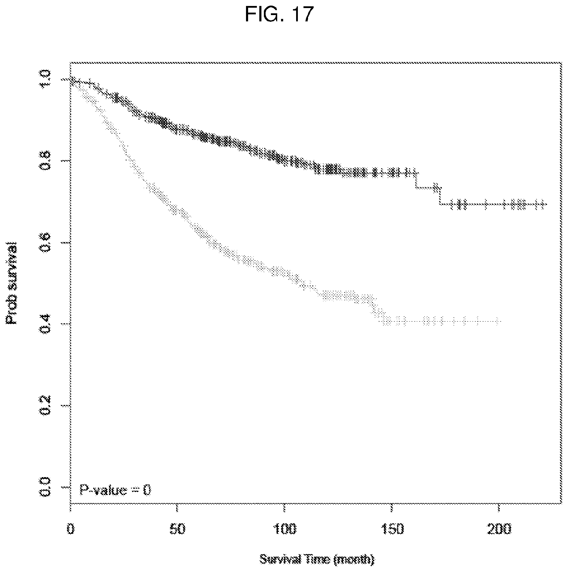

**Figure 17** is a graph showing the Kaplan-Meier survival curve for 1320 breast cancer patients. Red curve: patients with decreased expression of network-module based prognostic signature. Green curve: patients with increased expression of signature. Green curve: patients with decreased expression of signature.

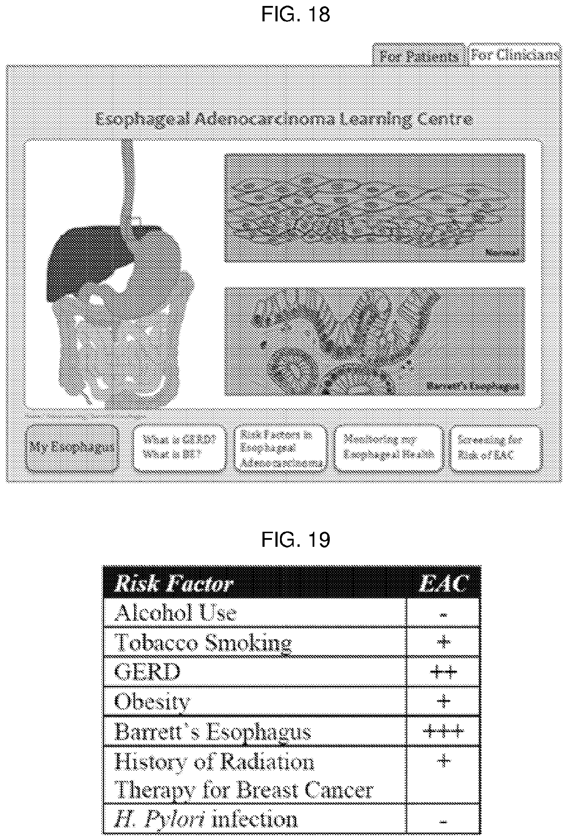

**Figure 18** is a diagram showing a proposed web-based educational tool for patients and clinicians.
**Figure 19** is a graph showing major risk factors for EAC.

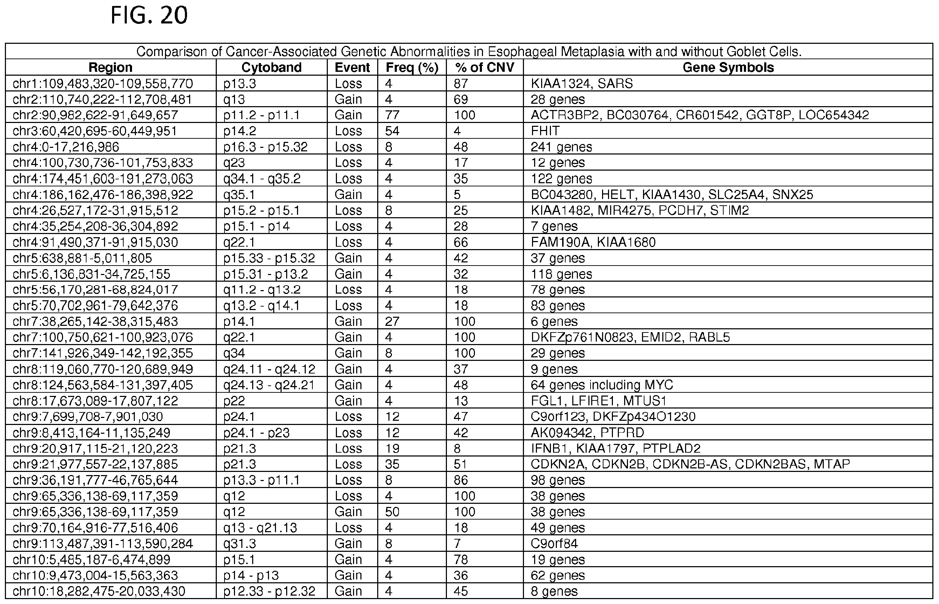

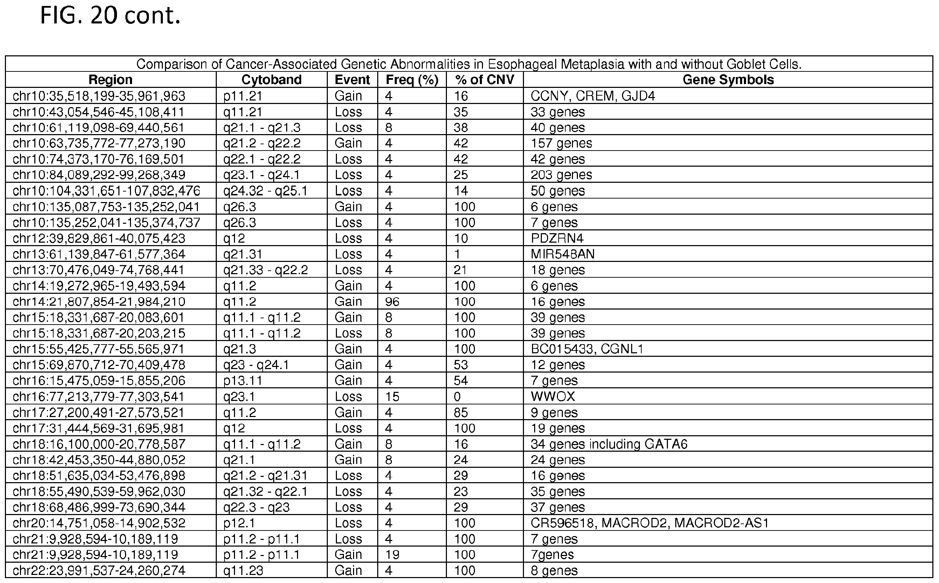

**Figure 20** is a graph showing copy number gains and losses observed in the genome of 26 intestinal metaplasia (IM) samples. The chromosomal region, cytoband, copy number event, event frequencies, % overlap with known copy number variations (CNV's), and genes within the region are indicated. Only regions encompassing genes are included in the table.

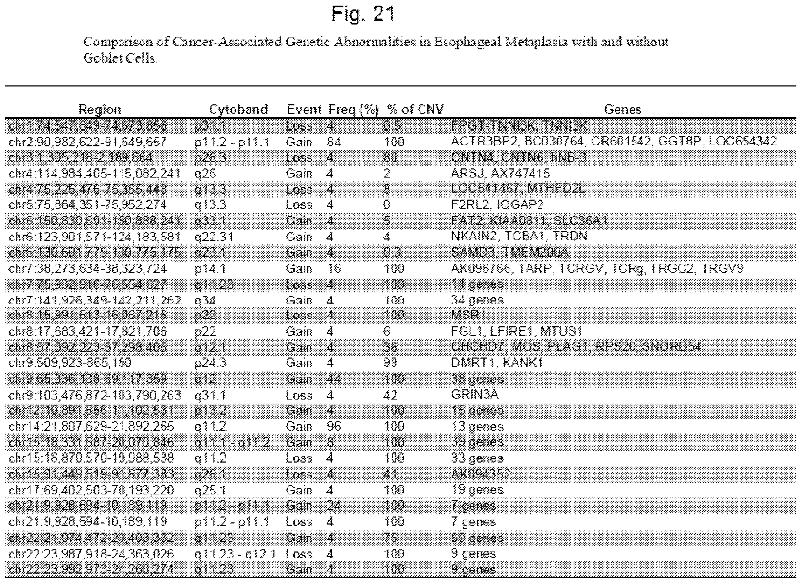

**Figure 21** is a graph showing copy number gains and losses observed in the genome of 25 non-goblet metaplasia (NGM) samples. The chromosomal region, cytoband, copy number event, event frequencies, % overlap with known copy number variations (CNV's), and genes within the region are indicated. Only regions encompassing genes are included in the table.

### DETAILED DESCRIPTION OF THE INVENTION

We have discovered that changes in certain biomarkers (e.g., the biomarkers listed in Table 1) may be used to determine a patient's risk of developing EAC, particularly in early stages of disease development, e.g., prior to the patient exhibiting the signs or symptoms of EAC. Patients may then be treated depending on the determined risk and stage of the disease (e.g., metaplasia, dysplasia, or EAC). The invention features devices and kits for determining changes in such biomarkers, e.g., arrays of oligonucleotide probes suitable for hybridization to and detection of the biomarkers. The devices and kits of the present invention may include an array (e.g., a microarray) with one or more single-stranded oligonucleotide probes that have substantial identity (e.g., at least 85%, 90%, 95%, 99%, or 100% sequence identity) to a target nucleic acid molecule having a sequence that is complementary or identical to the nucleic acid sequence of one or more biomarkers described herein. For example, the probes can be used to detect one or more (e.g., two, three, four, five, ten, twenty, or all) of the biomarkers listed in Table 1, such as CDKN2A, ERBB2, SMARCA4, and/or LRP1B. As such, the methods, devices, and kits described herein provide novel and effective means to, e.g., determine a patient's risk of developing EAC.

**Table 1. Biomarkers for determining a patient's risk of developing EAC.**

| **Biomarker** | **Gen Bank Accession Number(s)** |
|---|---|
| TP53 | NM_000546; NP_00537 |
| CDKN2A | NM_000077; NP_000068 |
| ERBB2 | NM_001005862; NP_001005862 |
| SMARCA4 | NM_001128844; NP_001122316 |
| LRP1 B | NM_018557; NP_061027 |
| APC | NM_000038; NP_000029 |
| ATM | NM_000051; NP_000042 |
| EGFR | NM_005228; NP_005219 |
| PIK3CA | NM_006218; NP_006209 |
| PTEN | NM_000314; NP_0000305 |
| AR | NM_000044; NP_000035 |
| CDH11 | NM_001797; NP_001788 |
| CNTN6 | NM_014461; NP_055276 |
| CNTNAP5 | NM_130773.3 |
| COL11A1 | NM_001190709; NP_001177638 |
| CSMD3 | NM_ 198124 |
| CTNNB1 | NM_001091679; NP_001159374 |
| DPP10 | NM_ 001004360; NP_ 001004360 |
| ELTD1 | NM_022159; NP _071442 |
| EYS | NM_ 001142800.1; NM_ 001292009.1 |
| ARID1A | NM_006015; NP 006006 |
| SMAD4 | NM_ 005359; NP_ 005350 |
| SLC39A12 | NM_001145195.1; NM_001282734.1 |
| SPG20 | NM_ 001142294; NP_ 001135766 |
| DOCK2 | NM_004946; NP_004937 |
| AKAP6 | NM_004274; NP_004265 |
| TLL1 | NM_001204760; NP_001191689 |
| TLR4 | NM_003266; NP_003257 |
| ARID2 | NM_152641; NP_689854 |
| HECW1 | NM_001287059; NP_001273988 |
| ELMO1 | NM_001039459; NP_001034548 |
| SYNE1 | NM_001099267; NP_149062 |
| AJAP1 | NM_001042478.1; NM_018836.3 |
| C6ORF118 | NM_144980.3 |
| ACTL7B | NM_006686.3 |
| F5 | NM_000130; NP _000121 |
| KCNU1 | NM_001031836; NP_001027006 |
| NUAK1 | NM_014840; NP_055655 |
| KAT6A | NM_001081149; NP_001092882 |
| SCN10A | NM_006514; NP_006505 |

### Methods for determining a patient's risk of developing EAC

Disclosed are methods useful for the detection and screening of patients that may be at risk of developing EAC using certain biomarkers, e.g., TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A. The methods, devices, and kits provided may be used for determining and monitoring a subject's disease progression (e.g., the progression from metaplasia to dysplasia or EAC), which may or may not present visual sign and symptoms (e.g., goblet cells). The biological sample can include, for example, cells, tissue (e.g., a tissue sample obtained by endoscopy or biopsy), blood, or serum. In some aspects, esophageal tissue samples may be collected that include, but are not limited to, non-goblet metaplasia, intestinal metaplasia, dysplasia, or tumor cells. Endoscopy or other diagnostic methods may be performed prior to sample collection. In certain embodiments, the biological sample is fresh frozen or formalin-fixed paraffin embedded (FFPE) tissue obtained from the subject, such as a tumor sample (e.g., a biopsy).

Numerous methods of determining changes in the sequence (e.g., nucleic acid, ribonucleic acid, or amino acid sequence) or reverse complement of certain biomarkers (e.g., listed in Table 1) are known in the art. Examples of sequencing based technologies include, but are not limited to, PCR, an array, a massarray, a beadarray, a microarray, a genechip, pyrosequencing, nanopore sequencing, nanoballs, sequencing by synthesis, sequencing by expansion, single molecule real time technology, true single molecule sequencing technology, sequencing by ligation, microfluidics, infrared fluorescence, next generation sequencing, and combinations thereof. Such techniques are not limited to nucleic acid molecules, but may also include ribonucleic acids (e.g., mRNA transcripts) and amino acids using proteomic techniques (e.g., mass spectrometry or protein arrays). In some embodiments, an mRNA corresponding to a biomarker can be detected in a biological sample by (a) producing cDNA from the sample by reverse transcription using at least one primer; (b) amplifying the cDNA so produced using a target polynucleotide as sense and antisense primers to amplify target cDNAs therein; and (c) detecting the presence of the amplified target cDNA using polynucleotide probes. In certain embodiments, primers and probes comprising the sequences described herein are used to detect changes in the biomarkers (e.g., Tables 1 and 2). Such changes in the nucleic acid sequence may include, but are not limited to, point mutations, nonsynonymous mutations, synonymous mutations, and changes in gene copy number (e.g., loss or gain of certain gene copies) of the biomarkers described further herein, e.g., TP53, CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A. In specific embodiments, biomarkers of the present invention may include, but are not limited to, CDKN2A, ERBB2, SMARCA4, and LRP1 B

### TP53

Biomarkers include TP53, which may be used to assess a patient's risk of developing EAC. The TP53 biomarker as described herein includes the nucleic acid and protein sequences of the molecule, which are available in GenBank under accession numbers NM_000546 and NP_00537, respectively (e.g., Table 1). Changes in the nucleic acid sequence of TP53 may be assessed using the paired forward and reverse primers (SEQ ID NOS: 475 and 476; 477 and 478; and 479 and 480, listed in Table 2). Exemplary changes of the TP53 biomarker signaling risk of EAC may include point mutations, such as a glycine to serine change at position 113 or an arginine to cysteine change at position 141 in the TP53 amino acid sequence. Such nonsynonymous mutations in the sequence of TP53 may result in an increased risk of developing EAC. The methods described above may be used to determine such changes in TP53, and the detection of such changes indicates the subject is at risk of developing EAC. TP53 may be used alone or in combination with one or more biomarkers of the present invention, e.g., CDKN2A, ERBB2, SMARCA4, LRP1B, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A.

### CDKN2A

Biomarkers of the present invention include CDKN2A, which may be used to assess a patient's risk of developing EAC. The CDKN2A biomarker as described herein includes the nucleic acid and protein sequences of the molecule, which are available in GenBank under accession numbers NM_000077 and NP_000068, respectively (e.g., Table 1). Changes in the nucleic acid sequence of CDKN2A may be assessed using the paired forward and reverse primers of the present invention (SEQ ID NOS: 201 and 202; 203 and 204; 205 and 206; and 209 and 201, listed in Table 2). Exemplary changes of the CDKN2A biomarker signaling risk of EAC may include a change in copy number, e.g., a loss, of the CDKN2A sequence on chromosome 8 in patients with intestinal metaplasia. Other changes, such as a mutation in the sequence of CDKN21 (e.g., a nonsynonymous mutation), may result in an increased risk of developing EAC. The methods of the invention described above may be used to determine such changes in CDKN2A, and the detection of such changes indicates the subject is at risk of developing EAC. CDKN2A may be used alone or in combination with one or more biomarkers of the present invention, e.g., ERBB2, SMARCA4, LRP1 B.

### SMARCA4

Biomarkers of the present invention include SMARCA4, which may be used to assess a patient's risk of developing EAC. The SMARCA4 biomarker as described herein includes the nucleic acid and protein sequences of the molecule, which are available in GenBank under accession numbers NM_001128844 and NP_001122316, respectively (e.g., Table 1). Changes in the nucleic acid sequence of SMARCA4 may be assessed using the paired forward and reverse primers of the present invention (SEQ ID NOS: 419 to 474, listed in Table 2). Exemplary changes of the SMARCA4 biomarker signaling risk of EAC may include point mutations, such as a threonine to methionine change at position 910 or a glycine to serine change at position 1162 in the SMARCA4 amino acid sequence. Such nonsynonymous mutations in the sequence of SMARCA4 may result in an increased risk of developing EAC. The methods of the invention as described above may be used to determine such changes in SMARCA4, and the detection of such changes indicates the subject is at risk of developing EAC. SMARCA4 may be used alone or in combination with other biomarkers of the present invention, e.g., ERBB2, CDKN2A, LRP1 B.

### LRP1B

Biomarkers of the present invention include LRP1B, which may be used to assess a patient's risk of developing EAC. The LRP1B biomarker as described herein includes the nucleic acid and protein sequence of the molecule, which are available in GenBank under accession numbers NM_018557 and NP_061027, respectively (e.g., Table 1). Changes in the nucleic acid sequence of LRP1B may be assessed using the paired forward and reverse primers of the present invention (SEQ ID NOS: 689 to 878, listed in Table 2). Exemplary changes of the LRP1B biomarker signaling risk of EAC may include point mutations, such as a valine to alanine change at position 2091, a serine to cysteine change at position 4003 or a threonine to alanine change at position 4410 in the LRP1B amino acid sequence. Such nonsynonymous mutations in the sequence of LRP1B may result in an increased risk of developing EAC. The methods of the invention as described above may be used to determine such changes in LRP1B, and the detection of such changes indicates the subject is at risk of developing EAC. LRP1 B may be used alone or in combination with one or more biomarkers of the present invention, e.g., ERBB2, CDKN2A, SMARC4.

### ERBB2

Biomarkers of the present invention include ERBB2, which may be used to assess a patient's risk of developing EAC. The ERBB2 biomarker as described herein includes the nucleic acid and protein sequence of the molecule, which are available in GenBank under accession numbers NM_001005862 and NP_001005862, respectively (e.g., Table 1). Changes in the nucleic acid sequence of ERBB2 may be assessed using the paired forward and reverse primers of the present invention (SEQ ID NOS: 1437 to 1488, listed in Table 2). Exemplary changes of the ERBB2 biomarker signaling risk of EAC may include point mutations, such as a serine to phenylalanine change at position 280 or a proline to leucine change at position 4410 in the ERBB2 amino acid sequence. Such nonsynonymous mutations in the sequence of ERBB2 may result in an increased risk of developing EAC. Methods of the invention as described above may be used to determine such changes in ERBB2. ERBB2 may be used in combination with other biomarkers of the present invention, e.g., LRP1B, CDKN2A, SMARCA4.

### Additional Biomarkers

Additional biomarkers used in the methods may include, but are not limited to, APC, ATM, EGFR, KRAS, PIK3CA, PTEN, AR, CDH11, CNTN6, CNTNAP5, COL11A1, CSMD3, CTNNB1, DPP10, ELTD1, EYS, ARID1A, SMAD4, SLC39A12, SPG20, DOCK2, AKAP6, TLL1, TLR4, ARID2, HECW1, ELMO1, SYNE1, AJAP1, C6ORF118, ACTL7B, F5, KCNU1, NUAK1, KAT6A, and SCN10A. GenBank accession numbers and paired forward and reverse primers for these biomarkers are listed in Tables 1-2. Exemplary changes in the nucleic acid or protein sequence of these biomarkers, which signal an increased risk of EAC, may include, but are not limited to, point mutations, nonsynomous mutations, synonymous mutations, and changes in gene copy number (e.g., loss or gain of certain gene copies). These biomarkers may further be used singularly or in combination (e.g., two, three, four, five, or more biomarkers) to determine a patient's risk of developing EAC.

### Methods of treatment

The current invention may be used to determine a subject's risk for developing EAC in a subject with one or more of the following risk factors: gastroesophageal reflux disease (GERD), goblet cells, and/or other metaplastic columnar cells. These risk factors characterize a patient in the metaplasia stage of disease progression, which may be used to diagnose the premalignant condition of Barrett's esophagus (BE). After the initial diagnosis of BE, patients may or may not progress to the development of dysplasia and ultimately to EAC. The methods and devices of the invention, e.g., cytology, molecular testing, biomarker gene arrays, hybridization probes, and sequencing technologies, can be used to detect and monitor the progression of metaplasia to dysplasia and/or EAC using esophageal tissue samples from patients with metaplasia or suspected to be at risk for EAC. Exemplary treatments administered after a diagnosis of metaplasia or risk of EAC using the methods of the invention, may include, but are not limited to, hyaluronic acid, nonsteroidal anti-inflammatory drugs, cyclooxygenase-1 (COX-1) inhibitors, cyclooxygenase-2 (COX-2) inhibitors, nonselective cyclooxygenase inhibitors, curcumin compositions, endoscopy, and combinations thereof. Exemplary treatments administered after a diagnosis of dysplasia or risk of EAC may include, but are not limited to, radiation therapy, chemotherapy, immunomodulatory therapy, endoscopic mucosal resection (EMR), endoscopic submucosal dissection, radiofrequency ablation, cryoablation, photodynamic therapy, and combinations thereof.

### Devices containing oligonucleotide probes of the invention

The devices (e.g., microarrays) of the invention can include one or more (e.g., two, three, four, five, ten, twenty, thirty, fifty, or all) oligonucleotide probes that have nucleotide sequences that are substantially identical (or share at least 85%, 90%, 95%, 99%, or 100 identity) to or are complementary to, e.g., at least 5, 8, 12, 20, 30, 40, 60, 80, 100, 150, or 200 consecutive nucleotides (or nucleotide analogues) of one or more of the biomarkers listed in Table 1. For example, a probe may have substantial sequence identity to all or a portion of a biomarker or its complement sequence, such as TP53 (GenBank Accession NOs: NM_000546; NP_00537), CDKN2A (GenBank Accession NOs: NM_000077; NP_000068), ERBB2 (GenBank Accession NOs: NM_001005862; NP_001005862), SMARCA4 (GenBank Accession NOs: NM_001128844; NP_001122316), LRP1 B (GenBank Accession NOs: NM_018557; NP_061027), APC (GenBank Accession NOs: NM_000038; NP_000029), ATM (GenBank Accession NOs: NM_000051; NP_000042), EGFR (GenBank Accession NOs: NM_005228; NP_005219), PIK3CA (GenBank Accession NOs: NM_006218; NP_006209), PTEN (GenBank Accession NOs: NM_000314; NP_0000305), AR (GenBank Accession NOs: NM_000044; NP_000035), CDH11 (GenBank Accession NOs: NM_001797; NP_001788), CNTN6 (GenBank Accession NOs: NM_014461; NP_055276), CNTNAP5 (GenBank Accession NO: NM_130773.3), COL11A1 (GenBank Accession NOs: NM_001190709; NP_001177638), CSMD3 (GenBank Accession NO: NM_198124), CTNNB1 (GenBank Accession NOs: NM_001091679; NP_001159374), DPP10 (GenBank Accession NOs: NM_001004360; NP_001004360), ELTD1 (GenBank Accession NOs: NM_022159; NP_071442), EYS (GenBank Accession NOs: NM_001142800.1; NM_001292009.1), ARID1A (GenBank Accession NOs: NM_006015; NP_006006), SMAD4 (GenBank Accession NOs: NM_005359; NP_005350), SLC39A12 (GenBank Accession NOs: NM_001145195.1; NM_001282734.1), SPG20 (GenBank Accession NOs: NM_001142294; NP_001135766), DOCK2 (GenBank Accession NOs: NM_004946; NP_004937), AKAP6 (GenBank Accession NOs: NM_004274; NP_004265), TLL1 (GenBank Accession NOs: NM_001204760; NP_001191689), TLR4 (GenBank Accession NOs: NM_003266; NP_003257), ARID2 (GenBank Accession NOs: NM_152641; NP_689854), HECW1 (GenBank Accession NOs: NM_001287059; NP_001273988), ELMO1 (GenBank Accession NOs: NM_001039459; NP_001034548), SYNE1 (GenBank Accession NOs: NM_001099267; NP_149062), AJAP1(GenBank Accession NOs: NM_001042478.1; NM_018836.3), C6ORF118 (GenBank Accession NOs: NM_144980.3), ACTL7B (GenBank Accession NOs: NM_006686.3), F5 (GenBank Accession NOs: NM_000130; NP_000121), KCNU1 (GenBank Accession NOs: NM_001031836; NP_001027006), NUAK1 (GenBank Accession NOs: NM_014840; NP_055655), KAT6A (GenBank Accession NOs: NM_001081149; NP_001092882), and SCN10A (GenBank Accession NOs: NM_006514; NP_006505). The oligonucleotide probes may be, e.g., 5-20, 25, 5-50, 50-100, or over 100 nucleotides long (e.g., at least 15 nucleotides long). The oligonucleotide probes may be deoxyribonucleic acids (DNA) or ribonucleic acids (RNA).

Probes that may be employed on devices (e.g., microarrays) of the invention include oligonucleotide probes having sequences complementary to or substantially identical to (or sharing at least 85%, 90%, 95%, 99%, or 100% identity to) any of the target biomarker sequences described herein (e.g., the biomarkers of Table 1). Additionally, probes employed on devices (e.g., microarrays) of the invention may also include proteins, peptides, or antibodies that selectively bind any of the oligonucleotide probe sequences or their complementary sequences or the biomarker protein sequences. Exemplary probes are listed in Table 2, which can be used to detect point mutations, nonsynomous mutations, synonymous mutations, and changes in gene copy number (e.g., loss or gain of certain gene copies) for the biomarkers described above.

### Kits:

Kits can also be used to determine a patient's risk of developing EAC. Kits include one or more of the following: a kit for DNA extraction (e.g., QiaAmp DNA Mini Kit from Qiagen Inc), a kit for RNA extraction (e.g., Trizol for mRNA, mirVana miRNA isolation kit from Ambion Inc), a kit for RNA labeling (e.g., MessageAmp from Ambion Inc., FlashTag from Genisphere Inc), a microarray for determining changes in biomarkers (e.g., SNP 6.0 GeneChip arrays from Affymetrix Inc), a kit of microarray reagents (e.g., Genome-Wide Human SNP Assay Kit from Affymetrix Inc), microarray hybridization station and scanner (e.g., GeneChip System 3000Dx from Affymetrix Inc), and/or software for analyzing changes in the biomarker genes or RNAs as described in herein (e.g., implemented in Nexus 6.0 Copy number analysis software from BioDiscovery).

The following examples are intended to illustrate, rather than limit, the invention.

### EXAMPLES

### Example 1. Comparison of Cancer-Associated Genetic Abnormalities in Columnar-Lined Esophagus tissues with and without Goblet Cells.

Intestinal metaplasia (IM) and non-goblet cell metaplasia (NGM) are implicated in EAC risk though little is known about genetic changes in NGM. This is the largest and most comprehensive comparison of DNA aberrations in both IM and NGM genomes. We report that IM tissues show more frequent cancer-associated mutations than NGM tissues.

To determine and compare the frequency of cancer-associated genetic abnormalities in esophageal metaplasia biopsies with and without goblet cells.

Barrett's esophagus (BE) is associated with increased risk of esophageal adenocarcinoma (EAC) but the appropriate histologic definition of BE is debated. Intestinal metaplasia (IM) is defined by the presence of goblet cells while non-goblet cell metaplasia (NGM) lacks goblet cells. Both have been implicated in EAC risk but this is controversial. While IM is known to harbor genetic changes associated with EAC, little is known about NGM. We hypothesized that if NGM and IM infer similar EAC risk then they would harbor similar genetic aberrations in genes associated with EAC. Ninety frozen NGM, IM, and normal tissues from 45 subjects were studied. DNA copy number abnormalities (CNA's) were identified using microarrays and fluorescence *in situ* hybridization (*FISH*). Targeted sequencing of all exons from twenty EAC-associated genes was performed on metaplasia biopsies using Ion AmpliSeq™ DNA sequencing.

Frequent CNA's targeting cancer-associated genes were found in IM whereas no such changes were observed in NGM. In one subject, *FISH* confirmed loss of *CDKN2A* and amplification of chromosome 8 in IM but not in a nearby NGM biopsy. Targeted sequencing revealed 11 non-synonymous mutations in 16 IM samples and 2 mutations in 19 NGM samples.

This study reports the largest and most comprehensive comparison of DNA aberrations in IM and NGM genomes. Our results show that IM has a much higher frequency of cancer-associated mutations than NGM.

Once a rare disease, the incidence of esophageal adenocarcinoma (EAC) has increased 300-500% in the United States and other Western Countries over the past 30-40 years^{1,2}. Paralleling the marked increase in incidence, death from EAC also continues to rise rapidly. EAC is known to develop through a series of metaplastic, dysplastic and neoplastic cellular changes that are largely initiated by chronic gastroesophageal reflux disease (GERD)³. GERD precipitates a metaplastic change of the esophageal epithelium from the normal squamous to a more columnar epithelium known as Barrett's esophagus (BE). BE is estimated to be present in approximately 1.6%⁴ of adults in Western populations (up to 4 million Americans)^{5,6} and progression to EAC is estimated to occur in 0.12-0.5% of patients per year^{5, 7-9}. Consequently, individuals with BE are recommended to undergo regular endoscopic surveillance with multiple biopsies to identify signs of dysplasia or EAC¹⁰. The appropriate definition of BE however is currently a matter of considerable debate. In the United States, specialized intestinal metaplasia (IM) containing histologically confirmed goblet cells is required for a diagnosis of BE¹¹ while in the United Kingdom, the presence of goblet cells is not currently required for the diagnosis of BE¹².

Historically, the presence of IM has been associated with EAC risk but recent studies have indicated that metaplasia without goblet cells (NGM) may also be pre-malignant and may carry a neoplastic progression risk similar to that of IM^{13, 14}. If correct patients with NGM would also be candidates for BE surveillance programs, the cost-effectiveness of which is often criticized given the low rate of progression from BE to cancer^{15, 16}. Unfortunately, the cancer-risk of NGM relative to IM remains controversial^{17, 18}. One thing that is known however, is that EAC arises through accumulation of multiple DNA alterations (mutation, DNA copy number abnormality, methylation), many of which are also observed in early dysplastic lesions¹⁹⁻²¹. Furthermore, IM contains frequent alterations in known cancer-associated genes (*TP53, CDKN2A, FHIT, WWOX*)^{20, 22, 23} and these DNA alterations are presumably the underlying cause for the increased cancer risk associated with IM²⁴. On the other hand, very little is known about DNA alterations in NGM with ploidy changes being the only possible aberrations noted to date²⁵. Thus, the goal of this study was to determine and compare the spectrum and frequency of DNA copy number alterations and cancer-associated point mutations in NGM and IM. This was performed on fresh-frozen esophageal biopsy specimens using a combination of Affymetrix SNP 6.0 microarrays, targeted re-sequencing of cancer-associated genes and fluorescence *in situ* hybridization.

### MATERIALS & METHODS:

**Subject cohort:** Fresh frozen esophageal mucosa specimens including non-goblet metaplasia (NGM), intestinal metaplasia (IM), and normal squamous epithelium (NSE) from 45 subjects were used in this study. Biopsies were collected from 41 subjects who underwent upper endoscopy at the University of Rochester Medical Center between 2004- 2010 and mucosal tissue pieces were obtained from four subjects who underwent esophagectomies at the University of Pittsburgh Medical Center between 2003-2008. For each subject, the most advanced disease state (EAC > dysplasia > IM > NGM) prior to, or at the time of tissue collection was noted (Figure 6). All subjects at both institutions provided informed consent and research was approved by appropriate IRB's. Additional information on subjects and specimens is provided in Figure 6.

**Specimen Screening:** The location of each biopsy/tissue piece was noted as either tubular esophagus or gastroesophageal junction (GEJ) at the time of endoscopy (or on the resection specimen). Specimens were immediately frozen in tissue cassettes containing optimal cutting temperature compound (OCT) to facilitate histologic review. Hematoxylin and eosin (H&E) stained sections of each specimen were independently screened by two pathologists (Z.Z, K.S). Tissues were classified as NSE, NGM or IM. IM was defined by the presence of any number of goblet cells (GCs) but goblet cell density was also recorded using a scale of 1-3 with 1 = rare GCs, 2= common GCs and 3 = numerous GCs (Figure 6). NGM was defined as columnar cell metaplasia without GCs in the entire specimen. NGM was further classified into two subgroups of mucosa: oxyntocardiac mucosa (OCM) and cardiac mucosa (CM). OCM consisted of both cardiac and oxyntic glands and CM consisted of only cardiac mucosa without parietal cells (Figure 6).

In some cases, more than one biopsy from the same esophageal level (distance from the incisors) was included in a single OCT block. When the histology of these two biopsies was different (one IM and one NGM), the tissue block was referred to as being a composite sample (Figure 7). It is important to note that composite samples consist of two separate biopsies that were typically separated by 1-2cm in esophageal location and are thus independent tissue pieces.

**Specimen Selection:** Specimens were selected for study only if diagnostic consensus was reached between the two pathologists and if the sample consisted of >70% tissue of interest with <30% NSE or submucosa. Furthermore, OCM samples were only included if the endoscopic location was tubular esophagus. OCM samples from the GEJ were excluded to avoid the possibility that they were actually oxyntic mucosa from the stomach. CM (and IM) samples were included from both GEJ and tubular esophagus as the histologic distinction between CM and oxyntic mucosa from the stomach is more easily made. In all cases, both pathologists were confident that the biopsies included for study represented esophageal metaplasia.

**Final Histologic Review and DNA isolation:** Selected specimens were cut on a cryostat (20-30 x 5µM sections) for DNA isolation. The first, middle and final sections were placed on slides, H&E stained and reviewed by both pathologists. Genomic DNA was isolated from the intervening tissue sections using the QiaAmp DNA Mini Kit (Qiagen, CA). DNA concentration and purity was assessed by UV absorbance (NanoDrop 1000, Thermo Fisher Scientific, Waltham MA) and the DNA quality was assessed by gel electrophoresis.

**Genome-wide Human SNP 6.0 Assay:** Fifty-eight specimens of NGM, IM, or composite histology were analyzed for DNA copy number abnormalities using the Affymetrix SNP 6.0 GeneChip arrays (Affymetrix Inc, CA). DNA labeling, array hybridization, washing and staining was carried out according to the standard Genome-Wide Human SNP Assay Kit instructions (Affymetrix Inc. CA) at the SUNY Upstate Medical University microarray core facility (Syracuse, NY).

**DNA Copy number analysis:** Data analysis was performed in Nexus 6.0 Copy number analysis software (BioDiscovery, CA). All samples were normalized to a baseline reference file created using DNA from blood of 15 individuals from a similar subject population. The data was pre-processed and the log2 DNA copy number ratios for each sample were generated for each probeset on the array using the reference files. The SNP-Rank Segmentation algorithm (implemented in Nexus 6.0) was used to segment the data with a minimum requirement of 8 probesets and a significance threshold p-value of 10⁻⁶. Log2 copy number thresholds for gains and losses were set at +0.15 (∼2.2 copies) and -0.2 (∼1.7 copies) respectively while high level gains and homozygous loss were set at +0.5 (∼2.8 copies) and -0.8 (∼1.1 copies) respectively.

**Fluorescence *in situ* hybridization (*FISH*):** For FISH analysis, frozen tissue samples were first formalin-fixed and paraffin-embedded and 4 µM sections were then cut and fixed on positively charged slides. H&E stained sections were reviewed by the pathologists to ensure that the reoriented tissue was the same histology as determined prior to the genomic studies.

FISH for *CDKN2A* (9p21.3) loss and chromosome 8 gain was performed in the Department of Cytogenetics FISH Core Facility (University of Rochester Medical Center, NY) using a VP2000 processor (Abbott Molecular, IL) and standard protocols. Probes for *CDKN2A* with Spectrum Orange label in combination with an internal control centromere 9 (CEP9) with Spectrum Green label (Cat. No: 05J51-001) and centromere 8 (CEP8) with Spectrum Green label (Cat. No: 06J37-018) were purchased from Abbott Molecular (Des Plaines, IL).

**FISH Analysis:** Manual enumeration of the FISH signals was performed using a Nikon eclipse 80i microscope (Nikon Instruments Inc., NY) and Cytovision software (Leica Microsystems, IL) under 60x oil immersion lens. Signals from overlapping nuclei were not included and at least 100 nuclei per biopsy were counted. The number of *CDKN2A* and CEP9 signals or the number of CEP8 signals were recorded for each cell nucleus.

**Targeted re-sequencing of genes frequently mutated in EAC:** A subset of metaplasia samples including 19 NGM, 16 IM and 5 composite tissues (Figure 6) along with matched NSE specimens were analyzed using targeted re-sequencing of 20 genes that are frequently somatically mutated in EAC. These 20 genes were identified from a preliminary analysis of whole exome sequencing data on 56 EAC tumor specimens (Dulak et al. Nature Genetics, 2013). Targeted re-sequencing libraries were generated using the Ion AmpliSeq™ Technology (Life Technologies, CA). Briefly, 1395 PCR amplicons (no longer than 140 bp) were designed to cover all exons from these 20 genes (>90kb of total DNA) using the Ion AmpliSeq Designer pipeline (Life Technologies, CA). Primers were pooled for two multiplex PCR pre-amplification reactions each with 10ng of input DNA using reagents provided by Ion Torrent. After 14-16 PCR cycles, the product was partially digested to polish the ends and adapter sequences were added for multiplexing. After an additional 5-cycle library amplification step, the libraries were combined with Ion Sphere™ particles for clonal amplification using emulsion PCR. The Ion Sphere™ particles, DNA polymerase and sequencing primers were then loaded into Ion AmpliSeq sequencing chips for sequencing on Ion Personal Genome Machine™ (PGM™) sequencer²⁶. Raw sequence reads were then processed using the Ion AmpliSeq Software Suite and mapped to the hg19 reference sequence. Sequence data from metaplasia samples was compared with subject-matched normal DNA in order to filter sequence polymorphisms and identify variants. Sequence variants were identified using Ion AmpliSeq Variant caller software followed by manual review using the Integrated Genome Viewer (IGV) (http://www.broadinstitute.org/igv/).

**PCR amplification and Sanger sequencing of *CDKN2A* exon 2:** Exon 2 from *CDKN2A* was PCR amplified using forward (5'-GACGACAGCTCCGCAGAAGTT-3') and reverse primers (5'-TCTGAGCTTTGGAAGCTCTCAGG-3') flanking the 5' and 3' ends of the exon 2. The PCR reaction consisted of 1X GC buffer, 0.25mM each of dNTP mix, 2µM each of forward and reverse primers, 1.25X of Advantage® GC polymerase mix (Cat. No: 639114, Clontech Laboratories Inc., CA), and 50ng genomic DNA in a final volume of 20ul. PCR conditions included an initial hot start at 94°C for 3 minutes followed by 40 cycles of denaturation at 94°C for 30 seconds and annealing/renaturation at 70°C for 1 minute and with a final hold at 25°C. Gel electrophoresis was performed on a 2% agarose gel to check for amplified DNA products. PCR products were then purified using ExoSAP-IT (Affymetrix, CA) and sequencing was performed with the PCR primers at Bio Basic Inc. (Ontario, Canada) on an ABI 3730XL sequencer (Life Technologies, CA). Chromatograms were assembled in DNA Baser Sequence Assembler v2 (Heracle BioSoft SRL, http://www.DnaBaser.com) followed by multiple sequence alignment in ClustalW 2.0 (http://www.ebi.ac.uk/Tools/msa/clustalw2/) using the RefSeq entry NM_000077.4 as the reference sequence.

### RESULTS:

**Tissue Pathology:** Ninety fresh frozen tissues (82 biopsies and 8 resection specimens) from 45 subjects met all criteria and were included in the study (73 from the tubular esophagus and 17 from the GEJ; Figure 6). Pathology review classified the samples as IM (n=26), NGM (n=25), composite (n=6) and NSE (n=33). Within this tissue set, multiple specimens (n= 21) were obtained from 9 subjects (indicated in Figure 6) while the remaining 69 samples were from independent subjects.

**Subject Cohort Characteristics:** The subject cohort was predominantly male (39 males vs 6 females) with an average age of 67 years. Prior to, or at the time of biopsy collection for this study, 13 subjects had a diagnosis of EAC, 23 had dysplasia, 9 had non-dysplastic IM and 1 subject had NGM only with no IM found (Figure 6).

**DNA Copy Number Aberrations in NGM and IM:** DNA copy number analysis was performed on 25 NGM, 26 IM and 6 composite specimens. For comparison, we also included previously analyzed data from 36 early stage (T1) esophageal adenocarcinoma specimens (Dulak et al. 2012²⁷; Figure 8). The 26 pure IM samples harbored a total of 73 regions of copy number aberrations. The median length of these regions was 667035bp (range 29256-28588324bp). Sixty-four out of 73 regions contain a total of 1935 unique genes (Figure 21). Consistent with previous reports^{20, 28} recurrent aberrations were observed that target well known cancer-associated genes (Figure 2). These include deletions at 3p14 (*FHIT;* 54% of samples), 9p21.3 (*CDKN2A;* 35%) and 16q23.1 (*WWOX*; 15%) and amplifications at 8q24 (c*-MYC*; 4%) and 18q11.2 (*GATA6*; 8%). In addition, other aberrations were observed in IM samples that appear to match those occurring in T1 EAC samples although the specific genes driving these events are not well defined. These include regional deletions on 4p and 4q, amplification on 5q and deletion on 5p, deletion on 9q and amplifications on both 10p and 10q (Figure 2).

In the 25 NGM samples, 40 regions of copy number aberrations were observed with a median length of 218086bp (range 23691-3781221bp). Twenty-nine regions contain a total of 297 unique genes (Figure 8) none of which appear to have any previous association with EAC. Six recurrent regions were identified but all have 100% overlap with regions of known copy number variation (CNV) and therefore likely represent copy number polymorphism²⁹ rather than true aberrations (Figure 22). All other changes observed occurred in only one sample each.

**Comparison of NGM and IM from the same subjects:** From 9 subjects, specimens with different histologies were available for study. This included 5 subjects (PID's: 50, 92, 124, 169, 188) with both IM and NGM, 1 subject (PID: 89) with both composite and IM and 3 subjects (PID's: 55, 68 and 104) with composite, IM and NGM. In the 5 subjects with IM and NGM specimens, DNA copy losses were identified at the *FHIT* locus in 4 cases, at the *CDKN2A* locus in 3 cases and at the *WWOX* locus in 1 case. In all 5 subjects, these losses were observed in the IM sample only and not in the matched NGM sample (Figure 9). In subject 89, losses at the *FHIT* and *WWOX* genes were observed in the IM sample in addition to losses on chromosomes 5 and 10, but no changes were seen in the composite sample (Figure 9).

In the 3 subjects with IM, NGM and composite samples multiple DNA copy losses and gains were identified in IM and composite samples but not in the NGM samples. For subject 68, loss was observed at the *CDKN2A* locus in the IM and composite samples but not in the NGM sample. Based on the boundaries of the deleted region, the loss in these two tissues appears to be clonally related. However, the composite sample also has an amplification event on chromosome 15 that is not observed in the IM or NGM samples. Similarly, for subject 104, clonally appearing losses of *FHIT,* chromosome 9 (including *CDKN2A* but also in other regions) and chromosome 18 were found in the IM and composite samples but not in the NGM sample. Chromosome 10 on the other hand had multiple regions of loss in the IM sample but regions of gain in the composite indicating some clonal divergence. Finally, subject 55 had a loss of *CDKN2A* in both IM and composite samples in addition to a gain of whole chromosome 8 and *FHIT* loss only in the composite sample (Figure 9).

For tissues from subject 55, we used fluorescence *in situ* hybridization (FISH) to determine if the alterations observed in the composite sample (UR159) were present in the IM or the NGM biopsy. The SNP array results from the composite specimen (UR159) of subject 55 indicates a gain of chromosome 8 and loss at the *CDKN2A* locus on chromosome 9 (Figure 2). The FISH results clearly show that both of these events are present only in the IM biopsy of the composite sample. For the chromosome 8 amplification, 60% of nuclei in the NGM portion had two copies of CEP8, 39% had 1 copy and 1% had 3 copies. This is almost identical to the control NSE sample (Figure 10). However, in the IM biopsy 63% of nuclei had two copies, 15% had one copy while 22% had three or more copies of CEP8. For *CDKN2A*/CEP9*,* 94% of nuclei in the NGM biopsy had two copies of both *CDKN2A* and CEP9 while 6% had two copies of CEP9 and one copy of *CDKN2A.* In the IM biopsy 52% of nuclei had two copies each of *CDKN2A* and CEP9, 7% had two copies of CEP9 and one copy of *CDKN2A* while 36% had two copies of CEP9 but no copies (homozygous deletion) of *CDKN2A* (Figure 3).

**Targeted re-sequencing of frequently mutated EAC genes in NGM and IM:** In order to identify cancer-associated point mutations in NGM and IM, we used a highly multiplexed PCR amplification approach to re-sequence 20 genes that are frequently mutated in EAC based upon an interim analysis of an emerging study of the spectrum of mutations in this disease. This focused sequencing was performed for 19 NGM, 16 IM, and 5 composite samples along with their matched normal samples. This analysis revealed a total of 16 non-synonymous (amino acid modifying) and 5 synonymous (no change to the amino acid) mutations (indicated in Figure 2). Two of these changes were observed in independent NGM samples (2/19; 10.5%) and both were non-synonymous mutations (in *CDKN2A* and *COL11A1*). A total of 14 mutations were observed in the IM samples. Eleven of these were non-synonymous changes that occurred in 9 (53%) independent samples with two samples each having mutations in two different genes. The genes with non-synonymous mutations in the IM samples were *TP53* (n=4; 25%), *SMARCA4* (n=2; 13%), *LRP1B* (n=3; 19%), *ERBB2* (n=1; 6%) and *CDKN2A* (n=1, identified by Sanger sequencing; 6%). Finally, three non-synonymous mutations were identified in the composite samples including a mutation in *APC* in one sample and mutations in *ERBB2* and *LRPB1* in another sample (sample UR286 from subject 89) (Figure 2). Interestingly, the IM sample (UR289) from the same subject had identical *ERBB2* and *LRPB1* mutations indicating clonality. Furthermore, the mutant fraction observed in the IM sample was almost exactly double that seen in the composite for both mutations.

**Mutation Load Discriminates Between Metaplasia and EAC.** Detecting progression of metaplasia to EAC (or dysplasia) is the goal of surveillance in subjects with BE. Therefore, we evaluated the discriminatory capacity of mutation load (number of mutations per sample in the 20-gene panel) to detect this event. Data from whole exome sequencing of 66 EAC samples was used for this analysis along with the targeted re-sequencing data from metaplasia samples. A pairwise Receiver-Operator Characteristic (ROC) analysis resulted in an area under the curve (AUC) of 0.954 for any metaplasia (NGM or IM) versus EAC and 0.915 for IM alone versus EAC (Figure 5). Thus, mutation load appears to have good potential for detecting disease progression in subjects with BE and this approach may be applied to esophageal cytology samples as well as biopsies.

**DISCUSSION:** These data report the largest and most comprehensive comparison of cancer-associated genetic changes in NGM and IM. We have carefully controlled the collection, preparation and histologic characterization of the specimens. The location of biopsies with respect to the GEJ was carefully noted, tissues were snap frozen in OCT to facilitate sectioning and histologic analysis and multiple sections of the same specimen used for DNA isolation were examined independently by two pathologists. Using high resolution DNA copy number analysis of the whole genome we identified numerous genetic changes in IM epithelium. These findings are in agreement with previous studies and reveal that the changes frequently target well known cancer-associated genes and regions which are similarly altered in early stage EAC. In contrast, we found far fewer DNA copy number changes in NGM. When present in NGM, abnormalities were commonly in regions with established copy number polymorphisms, were smaller than those in IM and often did not contain any known genes. Furthermore, when analyzing multiple tissues from the same subject, IM samples were found to have copy number changes while the matched NGM samples did not. This was true for at least one subject where NGM and IM biopsies from the same level (distance from the incisors) were analyzed using FISH. In these tissues, chromosome 8 gain and *CDKN2A* loss were found only in the IM biopsy and not in the adjacent NGM biopsy.

We further refined the findings with targeted re-sequencing of the entire coding region of 20 cancer-associated genes frequently mutated in EAC. This was achieved using novel technology (AmpliSeq™) capable of assembling customized next-generation sequencing libraries starting with very low DNA input. Eleven non-synonymous mutations in 16 IM samples were identified including mutations in the *TP53* and *CDKN2A* genes (Figure 4). We also identified non-synonymous mutations in other cancer-associated genes, including *ERBB2, SMARCA4* and *LRP1B.* Many of the observed mutations in IM tissues are present in the COSMIC database, indicating that they may be drivers of cancer risk. As with DNA copy number changes, point mutations in NGM were much less common than in IM with only 2/19 samples each having a single mutation. Interestingly one of these mutations was in the *CDKN2A* gene and this specific mutation is also in the COSMIC database. Taken together these data show that NGM harbors far fewer cancer-associated genetic aberrations than IM. This was true even though the NGM samples were collected from an extremely "high-risk" cohort of subjects with most having coincident dysplasia or EAC.

Since the recognition of the columnar lined esophagus (CLE) in the 1950's and subsequent studies drawing a link between metaplastic esophageal epithelium and EAC, the diagnosis of BE has become synonymous with risk of developing EAC. It is now recognized that esophageal columnar metaplasia is not a simple or single entity but can be classified into a variety of sub-types depending on the type and distribution of specific cell types (e.g. parietal cells, chief cells, mucous cells, goblet cells, Paneth cells and pancreatic acinar cells) and/or the presence and structure of sub-mucosal glands. Among these, the presence or absence of goblet cells has emerged as the most clinically relevant as it identifies intestinal metaplasia (IM), currently required for the definition of BE in the United States and many other countries. The diagnosis of BE triggers recommendations to enter endoscopic surveillance programs aimed at detecting progression to dysplasia or EAC. Of significance, the requirement for presence of goblet cells in the definition has recently been challenged as a result of studies implicating non-goblet cell metaplasia (NGM) in the development of EAC (reviewed in Riddell 2009¹⁸). The requirement of the goblet cell to define BE, and its inferred cancer risk, remains a highly controversial topic and one that carries major clinical and economic implications.

Cancer develops as a result of genetic abnormalities that drive transformation and progression. Numerous studies have also shown the presence of genetic changes (mutation, DNA copy number changes, methylation, etc.) in samples of IM²⁰_{'} ^{22, 28}. These include changes in well-known cancer genes including *TP53, CDKN2A*/*p16, APC, SMAD4, FHIT* and *WWOX.* Many of these changes have also been associated with a markedly increased risk of progression to dysplasia or EAC^{19,30,31}. Specifically, loss or mutation of *TP53* and *CDKN2A,* aneuploidy and the extent of clonal diversity observed in IM are all generally accepted biomarkers for increased risk of progression^{3,19,24}. In contrast, only one study has specifically analyzed DNA changes in NGM²⁵. DNA content abnormalities, DNA heterogeneity index, DNA exceeding 5N and aneuploidy were reported to occur with equal frequency and extent in NGM and BE. In another study, Chaves and colleagues reported that gains of chromosome 7 and 18 are more common in goblet cells than in columnar cells³². Although this study has been quoted as evidence for genetic abnormalities in NGM¹⁸, it should be noted that all specimens used in the Chaves study were clearly described as being IM. As such this study does not globally identify changes in NGM but rather shows that at the cellular level within intestinalized epithelia (IM), the frequency of genetic changes is lower in the goblet cells than in the surrounding columnar cells. These reports represent what is very limited data on the genetic abnormalities present in NGM epithelia. Genomic analyses of the scale performed here are currently not economically feasible for clinical testing, particularly considering the need for multiple biopsies performed every one-two years, nor do they address the issue of sampling error. It is feasible however that adding genetic analysis to the recently reported use of a Cytosponge^{33, 34} to collect esophageal cytology specimens may prove clinically useful. For example, given the identification of IM via a screening cytology analysis, a paired mutation analysis could provide a baseline estimate of progression risk (regardless of IM or NGM status). Abnormal cytology or a high incidence of mutations could also trigger endoscopy. Repeat cytology samples could be collected and mutation analysis used to detect/monitor (not predict) progression from baseline and triage subjects to endoscopy. Even with the limited set of 20 genes analyzed, our data clearly show that mutation load can differentiate metaplasia from EAC. Additional studies would be required to identify an optimal gene panel, evaluate mutation load in dysplasia and develop appropriate classification algorithms. Next generation sequencing technologies are perfect for this type of test as they are capable of detecting very rare mutation events in the large background of normal cell DNA present in cytology specimens. Furthermore, the 20-gene assay presented here was designed (and has been tested) to work on highly fragmented DNA such as that obtained from routinely fixed and processed clinical specimens. Perhaps the question to ask when determining effective management strategies for subjects with esophageal columnar metaplasia should no longer be the presence or absence of goblet cells but rather the spectrum and frequency of mutations present in each individual case.

### References:

1. Pohl H, Welch HG. The role of overdiagnosis and reclassification in the marked increase of esophageal adenocarcinoma incidence. J Natl Cancer Inst 2005; 97(2) :142-6.
2. Brown LM, Devesa SS, Chow WH. Incidence of adenocarcinoma of the esophagus among white Americans by sex, stage, and age. J Natl Cancer Inst 2008; 100(16):1184-7.
3. Reid BJ, Li X, Galipeau PC, et al. Barrett's oesophagus and oesophageal adenocarcinoma: time for a new synthesis. Nat Rev Cancer 2010; 10(2):87-101.
4. Ronkainen J, Arc P, Storskrubb T, et al. Prevalence of Barrett's esophagus in the general population: an endoscopic study. Gastroenterology 2005; 129(6):1825-31.
5. Jankowski J, Barr H, Wang K, et al. Diagnosis and management of Barrett's oesophagus. BMJ 2010; 341:c4551.
6. Jankowski JA, Harrison RF, Perry I, et al. Barrett's metaplasia. Lancet 2000; 356(9247):2079-85.
7. Hvid-Jensen F, Pedersen L, Drewes AM, et al. Incidence of adenocarcinoma among patients with Barrett's esophagus. N Engl J Med 2011 ; 365(15):1375-83.
8. Bhat S, Coleman HG, Yousef F, et al. Risk of malignant progression in Barrett's esophagus patients: results from a large population-based study. J Natl Cancer Inst 2011; 103(13):1049-57.
9. Desai TK, Krishnan K, Samala N, et al. The incidence of oesophageal adenocarcinoma in non-dysplastic Barrett's oesophagus: a meta-analysis. Gut 2012; 61(7):970-6.
10. American Gastroenterological A, Spechler SJ, Sharma P, et al. American Gastroenterological Association medical position statement on the management of Barrett's esophagus. Gastroenterology 2011; 140(3):1084-91.
11. Wang KK, Sampliner RE, Practice Parameters Committee of the American College of G. Updated guidelines 2008 for the diagnosis, surveillance and therapy of Barrett's esophagus. Am J Gastroenterol2008; 103(3):788-97.
12. Playford RJ. New British Society of Gastroenterology (BSG) guidelines for the diagnosis and management of Barrett's oesophagus. Gut 2006; 55(4):442.
13. Kelty CJ, Gough MD, Van Wyk Q, et al. Barrett's oesophagus: intestinal metaplasia is not essential for cancer risk. Scand J Gastroenterol2007; 42(11):1271-4.
14. Gatenby PA, Ramus JR, Caygill CP, et al. Relevance of the detection of intestinal metaplasia in non-dysplastic columnar-lined oesophagus. Scand J Gastroenterol2008; 43(5):524-30.
15. Inadomi JM. Surveillance in Barrett's esophagus: a failed premise. Keio J Med 2009; 58(1):12-8.
16. Inadomi JM, Sampliner R, Lagergren J, et al. Screening and surveillance for Barrett esophagus in high-risk groups: a cost-utility analysis. Ann Intern Med 2003; 138(3):176-86.
17. Chandrasoma P, Wickramasinghe K, Ma Y, et al. Is intestinal metaplasia a necessary precursor lesion for adenocarcinomas of the distal esophagus, gastroesophageal junction and gastric cardia? Dis Esophagus 2007; 20(1):36-41.
18. Riddell RH, Odze RD. Definition of Barrett's esophagus: time for a rethink--is intestinal metaplasia dead? Am J Gastroenterol 2009; 104(10):2588-94.
19. Ong CA, Lao-Sirieix P, Fitzgerald RC. Biomarkers in Barrett's esophagus and esophageal adenocarcinoma: predictors of progression and prognosis. World J Gastroenterol 2010; 16(45):5669-81.
20. Li X, Galipeau PC, Sanchez CA, et al. Single nucleotide polymorphism-based genome-wide chromosome copy change, loss of heterozygosity, and aneuploidy in Barrett's esophagus neoplastic progression. Cancer Prev Res (Phila) 2008; 1(6):413-23.
21. Lai LA, Paulson TG, Li X, et al. Increasing genomic instability during premalignant neoplastic progression revealed through high resolution array-CGH. Genes Chromosomes Cancer2007; 46(6) :532-42.
22. Paulson TG, Maley CC, Li X, et al. Chromosomal instability and copy number alterations in Barrett's esophagus and esophageal adenocarcinoma. Clin Cancer Res 2009; 15(10):3305-14.
23. Paulson TG, Galipeau PC, Xu L, et al. p16 mutation spectrum in the premalignant condition Barrett's esophagus. PLoS One 2008; 3(11):e3809.
24. Reid BJ. Early events during neoplastic progression in Barrett's esophagus. Cancer Biomark 2010; 9(1-6):307-24.
25. Liu W, Hahn H, Odze RD, et al. Metaplastic esophageal columnar epithelium without goblet cells shows DNA content abnormalities similar to goblet cell-containing epithelium. Am J Gastroenterol 2009; 104(4):816-24.
26. Rothberg JM, Hinz W, Rearick TM, et al. An integrated semiconductor device enabling non-optical genome sequencing. Nature 2011; 475(7356):348-52.
27. Dulak AM, Schumacher S, van Lieshout J, et al. Gastrointestinal adenocarcinomas of the esophagus, stomach and colon exhibit distinct patterns of genome instability and oncogenesis. Cancer Res 2012.
28. Lai LA, Kostadinov R, Barrett MT, et al. Deletion at fragile sites is a common and early event in Barrett's esophagus. Mol Cancer Res 2010; 8(8):1084-94.
29. Sebat J, Lakshmi B, Troge J, et al. Large-scale copy number polymorphism in the human genome. Science 2004; 305(5683):525-8.
30. Merlo LM, Shah NA, Li X, et al. A comprehensive survey of clonal diversity measures in Barrett's esophagus as biomarkers of progression to esophageal adenocarcinoma. Cancer Prev Res (Phila) 2010; 3(11):1388-97.
31. Maley CC, Galipeau PC, Finley JC, et al. Genetic clonal diversity predicts progression to esophageal adenocarcinoma. Nat Genet2006; 38(4):468-73.
32. Chaves P, Crespo M, Ribeiro C, et al. Chromosomal analysis of Barrett's cells: demonstration of instability and detection of the metaplastic lineage involved. Mod Pathol 2007; 20(7):788-96.
33. Kadri S, Lao-Sirieix P, Fitzgerald RC. Developing a nonendoscopic screening test for Barrett's esophagus. Biomark Med 2011; 5(3):397-404.
34. Kadri SR, Lao-Sirieix P, O'Donovan M, et al. Acceptability and accuracy of a non-endoscopic screening test for Barrett's oesophagus in primary care: cohort study. BMJ2010; 341 :c4372.

### Example 2. Genomic mutation of esophageal columnar cell metaplasia reveals less frequent changes in non-goblet cell metaplasia than intestinal metaplasia by next generation sequencing.

The requirement of goblet cells (intestinal metaplasia; IM) to define Barrett's esophagus (BE) remains controversial as some studies have shown that the risk of progression to esophageal adenocarcinoma (EAC) is similar for non-goblet cell metaplasia (NGM) and IM. While the presence of goblet cells is required in the United States for diagnosis of BE and follow up surveillance, this is not the case in Japan and United Kingdom. BE/IM reportedly harbors frequent cancer-associated genetic mutations, but NGM is not studied. The aim 1 of this study is to investigate the genomic mutation in NGM compared to IM and to assess if NGM is at direct risk for progression to cancer. The aim 2 is to develop a new, molecular test to help clinical BE surveillance.

The samples including 19 NGM, 16 IM and 5 composite tissues with both NGM and IM along with matched normal squamous specimens were analyzed using targeted re-sequencing of 20 genes (IonAmpliSeq™ Technology) that are frequently somatically mutated in EAC from a preliminary analysis of whole exome sequencing data on 56 EAC tumor specimens. Sequence data from metaplasia samples was compared with patient-matched normal DNA in order to filter sequence polymorphisms and identify variants. In addition, Genetic mutation load from 56 EAC samples with whole exome sequencing was compared with that from metaplasia samples with targeted gene sequencing. A pairwise Receiver-Operator Characteristic (ROC) analysis was used.

This analysis revealed a total of 16 non-synonymous (amino acid modifying) and 5 synonymous (no change to the amino acid) mutations. Two of these changes were observed in independent NGM samples (2/19; 10.5%) in *CDKN2A* and *COL11A.* A total of 14 mutations were observed in the IM samples including non-synonymous mutations *TP53* (n=4; 24%), *SMARCA4* (n=2; 12%), *LRP1B* (n=3; 18%), *ERBB2* (n=1; 6%) and *CDKN2A* (n=1; 6%) and synonymous mutations *APC, ERBB2* and *LRPB1* (n=1/each mutation).

Genetic mutation load from 56 EAC samples with whole exome sequencing was compared with those from metaplasia samples with targeted sequencing. A pairwise ROC analysis resulted in an area under the curve (AUC) of 0.954 for any metaplasia (NGM or IM) versus EAC and 0.915 for IM alone versus EAC.

Our results show that IM has a much higher frequency of cancer-associated mutations than NGM and is therefore likely to pose a higher risk for development of EAC. In addition, the mutation load appears to have good potential for detecting disease progression in patients with BE.

### Example 3. Genomic analysis of esophageal columnar cell metaplasia reveals less frequent changes in non-goblet cell metaplasia than intestinal metaplasia.

Gastroesophageal reflux disease (GERD) results in metaplastic changes in the esophagus from normal squamous epithelium to a columnar epithelium with (IM) or without goblet cells (NGM). While the presence of goblet cells is required in the United States for diagnosis of Barrett's esophagus (BE) and follow up surveillance, this is not the case in Japan and United Kingdom. BE/IM reportedly harbors frequent genetic alterations which are often observed in adenocarcinoma. The aim of this study is to investigate the genomic changes in NGM compared to IM and to assess if NGM is at direct risk for progression to cancer.

Genomic DNA from 46 biopsies (36 patients) including 23 pure non-goblet metaplasia (NGM), 16 pure intestinal metaplasia (IM) samples, and 7 composite samples (one tissue piece is NGM and the other is IM from the same patient) were analyzed using Affymetrix SNP 6.0 arrays. Normal samples from the same patient population were used as the baseline reference. Analysis was performed with Nexus 5.0 Copy number software using SNPRank segmentation algorithm with log2 copy number thresholds for gains and losses set at +0.15 and -0.2 respectively while high level gains and homozygous loss were set at +0.5 and -0.8 respectively.

IM samples display cancer associated changes including high frequency loss of *FHIT* (44%), *CDKN2A* (25%), and WWOX (19%) in addition to a low frequency gains at c-*MYC* (6%) and *GATA6* (6%). In contrast, one sample in the NGM samples displayed changes at *KRAS* (4%) and *CDKN2A* (4%). Genomic comparisons of the composite samples with available pure NGM and/or IM samples from same patients suggest that changes at *CDKN2A, FHIT,* and *WWOX* occur in the IM portion. Results from validation studies using FISH will also be reported.

Results from this study so far indicate that the NGM harbors genetic changes but at a much lower frequency than that observed in IM. These findings would therefore argue that NGM is at a much lesser risk for progression to cancer compared to IM, thereby supporting the US definition of BE.

### Example 4: Early detection of patients at high risk of esophageal adenocarcinoma.

Cancer of the esophagus is a rare but devastating disease. Most patients first learn they have the disease when they experience discomfort on swallowing, by which time it is usually too late for a cure. One particular type of this disease, esophageal adenocarcinoma, is rising rapidly in Canada for reasons that are not entirely clear. Since the 1980's, the rate has increased 3-6 fold and now affects roughly 1,500 new patients per year. This is double the number of annual drowning deaths, and about half the number of motor vehicle fatalities.

The single major risk factor for development of esophageal adenocarcinoma is chronic heartburn which eventually leads to a change in the lining of the esophagus called Barrett's Esophagus. Patients with Barrett's Esophagus have an 11-40 times increased risk of developing esophageal cancer, roughly the same increased risk as long-term cigarette smokers have for developing lung cancer. An estimated 3 million Canadians have chronic heartburn, and of these roughly 800,000 have Barrett's Esophagus. If the presence of Barrett's were known, the cancer could be detected and treated early, but few patients are aware that they have this risk factor. The diagnosis of Barrett's requires endoscopy, an uncomfortable procedure that incurs loss of a half day of work and few primary physicians are willing to recommend this procedure to their patients with heartburn.

Using genomics, this research seeks to build upon the recent development of a quick and painless diagnostic test for Barrett's Esophagus that can be applied in the family doctor's office. By adding genomic technologies to this test, will enable to follow Barrett's patients at periodic intervals in order to identify and treat those who are progressing to cancer. In Canada alone, if deployed widely, and under optimistic projections, the test would be able to detect, and potentially prevent, cancer in 800 patients per year, and save the Canadian health care system $300M/year in endoscopies and terminal care. Global impact would be proportionately greater.

Roughly 800,000 Canadians have Barrett's Esophagus (BE); a metaplastic change of the esophageal lining in which the normal squamous mucosa is replaced by a glandular epithelium similar to that of the small intestine (1). BE is the single major risk factor for development of esophageal adenocarcinoma (EAC), a devastating disease with rising incidence and a 5-year survival of only 15% (2,3). BE, in turn, is a consequence of chronic gastroesophageal reflux disease (GERD), a condition present in over 3 million Canadian adults (4).

Patients with BE have between a 0.12 and 0.5% annual rate of progression to EAC, and an 11.3 to 40-fold increase in their lifetime risk of developing EAC (5-7). Because BE is such a strong risk factor, both Canadian and U.S. guidelines recommend that all patients with chronic GERD obtain a baseline endoscopy to diagnose BE (8,9). Patients with BE are then recommended to undergo periodic surveillance endoscopies with biopsy to detect progression to dysplasia and EAC (10). In practice, endoscopic surveillance of patients with BE is expensive, uncomfortable and not cost-effective (11-13). Surveillance programs currently cost the Canadian health care system $400MCDN per year (8,14,15). Furthermore, endoscopy involves sedation, loss of a partial work day, and appointment wait times in excess of 200 days in Canada (16). For these reasons, many patients and their providers are reluctant to commit to endoscopy. Consequently, just 5% of the population that is thought to have BE is under surveillance (17) and of the estimated 1,500 new cases of EAC diagnosed in Canada per year, only a fraction are identified by surveillance. Most cases of EAC are diagnosed in patients who present with the symptoms of late-stage EAC (18) at which time treatment options are limited and expensive.

This project will lay the foundations for a combined genetic and cytological test that can be used by primary care physicians to screen GERD patients for BE and to monitor those with confirmed BE for genetic changes that signal progression to dysplasia and EAC. The project builds on the recently-developed Cytosponge system, a method for obtaining esophageal cytology specimens in an office or clinic visit with minimal discomfort to the patient. This will help overcome barriers associated with patient/physician compliance with baseline screening recommendations. However, while cytology is effective for the initial identification of BE in GERD patients, it cannot currently replace endoscopy for routine surveillance of patients with known BE. This project will use genomics to identify the genetic markers of progression in BE, and use these markers to develop and evaluate a targeted sequencing-based assay to detect early signs of progression in esophageal cytology specimens. It is anticipated that this will replace the need for repeated surveillance endoscopies, improve compliance, reduce surveillance costs and increase early detection of EAC.

The ultimate deliverable of the project is a practical genetic assay for determination of cancer progression markers in esophageal cytology specimens that meets or exceeds the minimal criteria laid out by the socio-economic model for a socially and economically beneficial diagnostic test. If successful, this assay can be used as a clinical test for BE surveillance.

The overall goal of this project is to develop a molecular cytology test that can be used as a cost-effective approach to surveillance of patients with intestinal metaplasia (IM) of the esophagus, otherwise known as Barrett's Esophagus (BE). We collected esophageal cytology specimens using the Cytosponge and a combination of cytology, immunohistochemistry and DNA mutation markers using next-generation sequencing.

We first identified a set of genes that have recurrent point mutations in EAC using a combination of exome and RNA sequencing of matched tumour/normal resection specimens. A targeted sequencing assay can be used for the identified genes to characterize their mutation frequency in premalignant lesions obtained by endoscopic biopsy. From this information we will derive a molecular biomarker for progression, and adapt the targeted sequencing assay for use on cytology specimens. Finally, we will evaluate the performance of the progression biomarker against concurrent cytology and biopsy specimens.

### Background

Chronic gastroesophageal reflux disease (GERD) damages the normal esophageal squamous epithelium, leading to development of columnar metaplasia in the lower esophagus. Two major types of metaplasia exist: non-goblet cell metaplasia (NGM) and intestinal metaplasia (IM). The presence of IM defines Barrett's esophagus (BE), which affects some 800,000 Canadian adults (1).

BE is by far the greatest risk factor for esophageal adenocarcinoma (EAC). The presence of BE increases the lifetime risk of developing EAC by 11 - 40 fold (6), which is roughly the same increased risk as cigarette smoking bears to lung cancer (26 fold (19)). Whether NGM constitutes the same, lower or no risk for EAC is currently controversial (20) and it is not included in the current definition of BE. Canadian and American clinical guidelines both call for endoscopic biopsies in patients with GERD to identify BE (8,9), followed by periodic endoscopic surveillance of BE patients to detect early progression to EAC (10). However, compliance with these guidelines is low and it is estimated that 5% of patients with BE are identified and placed in surveillance (17). Therefore, despite an estimated annual cost for surveillance of $400 M (1) in Canada the vast majority of patients with EAC still present with symptoms of advanced disease and an expected 5 year survival of just 15% (18).

Several studies have reported better survival in patients whose EAC was detected and treated at an early stage (21,22). For example, in one study comparing tumors that arose during surveillance versus those that were present at first endoscopy, 80% were operable versus 50% and 70% of patients were disease-free at 2-years (versus 8%) (22). Furthermore, esophagectomy itself has a significant mortality rate and is a highly morbid procedure. For patients diagnosed with dysplasia or superficial EAC, endoscopic mucosal resection (EMR) is rapidly becoming the preferred treatment approach due to its low morbidity rate and excellent outcomes. For example, in one large study of 100 such cases, the 5-year survival was 98%³ (23). Thus there is every reason to believe that early detection of BE progression will result in the reduction of morbidity and mortality from EAC.

Two leading approaches to non-endoscopic screening are esophageal balloon cytology (EBrC) (18,24) and the Cytosponge (25-27). The Cytosponge is particularly promising as it is administered in the form of a sponge-containing capsule that can be swallowed in a primary physician's office. The sponge expands in the patient's stomach, and is retrieved via a string after a short interval. Adherent esophageal mucosa cells can then be examined histologically and subjected to immunohistochemical and molecular characterization. Currently available only in the UK, the Cytosponge is expected to be available for use in America and Canada by 2014.

Fitzgerald's group in the UK recently reported a trial for BE detection using the Cytosponge with a combination of cytology and immunohistochemistry for trefoil factor 3 (TFF3). Sensitivity and specificity reported were 90.0% and 93.5% for IM of 2 cm or more (26), which compares favorably to the Pap smear screen for cervical dysplasia (70% and 95% respectively (28)). If validated in an ongoing clinical trial, this leads to the possibility of detecting the majority of patients with BE with an inexpensive office test. However, TFF3 positivity only identifies BE, and esophageal balloon cytology (and presumably the cytosponge also) has poor sensitivity for detecting dysplasia. Thus, neither assay provides for risk stratification or monitoring of progression.

Cancers are the result of an accumulation of somatic mutations involving key growth, differentiation and cell communication pathways (29). Point mutations, copy number alterations (CNAs), epigenetic modifications and general genome instability occur in nearly all tumour types studied and are frequently associated with disease progression (30). We hypothesize that complementing esophageal cytology and TFF3 staining with molecular biomarkers for premalignant genetic changes will allow us to identify patients who are at greatest risk for, or who have already progressed to dysplasia/EAC. Furthermore, for a clinically-practical assay to work in the mixed cellular environment of cytology specimens, we believe that sensitive detection of point mutations using next-generation sequencing provides the best approach. Our assumption (supported by literature and data cited below) is that some of the genes that are mutated in EAC will harbor similar mutations in dysplasia and high-risk IM.

### Preliminary Studies

The preliminary studies described in this section demonstrate the presence of mutations shared among esophageal adenocarcinoma and its precursor lesions. We describe the expertise of the two institutions in next generation sequencing and analysis, and describe our background in the identification of biomarkers.

*Presence of somatic mutations in EAC and precursor lesions.* There have been relatively few genome-wide studies of DNA alterations in EAC and its precursor lesions, but these studies have revealed many DNA abnormalities in both dysplasia and EAC (31-33). IM has also been found to harbor DNA alterations, most notably DNA copy number abnormalities, TP53 and CDKN2A mutations and alterations in methylation (31,34). DNA content abnormalities, methylation, mutation, loss of heterozygosity (LOH) and clonal diversity have all been proposed as biomarkers to predict BE progression (reviewed in (32)) but none are currently being used in clinical practice. One reason for this is that BE is highly heterogeneous with a complex pattern of clonal evolution (27,28). Even with rigorous biopsy protocols, there is still considerable sampling error and important biomarkers (and dysplasia) may be missed, even if present.

Ploidy abnormalities have also been identified in one study of NGM (35) but no detailed examination of specific DNA alterations has been reported in these tissues. Using high resolution DNA microarrays, we have found a high incidence of well- known cancer-associated DNA copy number aberrations in IM (n=26) including loss of *FHIT*(54%), *CDKN2A* (35%) and *WWOX* (15%) and gain of MYC (4%) and GATA6 (8%) while no such changes were observed in NGM (n=25; Figure 11). Furthermore, our data show that DNA copy number changes increase in frequency from IM to early stage (T1) EAC. Thus, the frequency of DNA copy number abnormalities increase during the development of EAC and could be used as a biomarker for progression. However, array-based DNA copy number analysis is not a viable approach for cytology specimens, where the cells of interest may be very few in a large background of normal squamous esophageal cells. Fluorescence *in situ* hybridization (*FISH*) may be an option but the majority of early events appear to be DNA copy number losses. In this scenario, FISH would not be reliable as a small but significant number of normal nuclei typically appear to have only one copy of tested loci due to technical artifact.

**Identification of Genes that are Frequently Mutated in EAC.** In collaboration with Dr. Adam Bass from the Dana Farber Cancer Institute, our research group has recently completed whole exome sequencing of 150 EAC samples and we have therefore been able to identify a set of genes that are frequently mutated in EAC (Dulak et al., Nature Genetics, in review). Based on an early analysis of 56 samples, we selected a set of 20 frequently mutated genes to use in preliminary studies evaluating mutation load in premalignant lesions. An average of 3.2 mutations (range 1-8) in these 20 genes is observed per EAC sample.

**Targeted re-Sequencing of Mutated Genes in pre-Neoplastic Samples.** We developed a highly multiplexed PCR amplification approach to generate targeted sequencing libraries covering all coding regions of the selected 20 genes. This approach requires as little as 5ng of input DNA and is designed to work with highly degraded DNA such as that from formalin-fixed tissues. Thus, this method is a perfect adjunct to cytology and TFF3 staining. Furthermore, the technology utilizes sequencing chips and the Personal Genome Machine (PGM) sequencer to dramatically decrease the cost and complexity of individual DNA sequencing tests.
Briefly, the AmpliSeq™ custom primer design pipeline was used to design PCR amplicons of no larger than 140bp that provide approximately 2x overlapping coverage of the coding regions of the 20 genes. This resulted in 1,395 PCR primer pairs covering >90kb of total DNA. The target DNA was then PCR amplified, and the amplification products are barcoded, ligated to adapters, and then further amplified for sequencing on the Ion Torrent PGM (36). We mapped raw reads to the hg19 human genome reference sequence and called somatic against matched normal patient DNA using Ion Torrent's variant caller software.

Using this methodology, we first sequenced our 20 gene panel in 10 EAC specimens in order to confirm mutations identified in the exome sequencing. On average, we obtained >2 million mapped reads for each sample resulting in an average base sequence coverage of 1890x with >99% of the target bases covered at >100X and >90% covered at >500X. Based on our Illumina exome sequence data, these 10 tumors harbor 42 mutations in the gene panel and all 42 (100%) were also identified by Ion Torrent re-sequencing. These data demonstrate that the Ampliseq™ technology combined with Ion Torrent sequencing can accurately identify mutations in our 20-gene panel starting with very small amounts of target DNA.

Next we analyzed DNA from biopsy specimens of NGM and IM using matched squamous epithelium biopsies for controls. Biopsies were taken from patients being evaluated for GERD or who were undergoing BE surveillance. In many cases both IM and NGM were present and evaluated from the same patient. A total of 19 NGM/Normal and 17 IM/Normal pairs were sequenced and any variants identified by the Ion Reporter software were manually reviewed for accuracy using the Integrated Genome Viewer (http://www.broadinstitute.org/igv/). Results for verified mutations are shown in Figure 12. Ten of 16 (63%) of IM samples harbored at least one mutation while only 2 of 17 (12%) of NGM samples had mutations. Furthermore, 3 of 17 IM samples had 2 or more mutations while this did not occur in any NGM samples. This data, and that from our copy number studies, clearly indicate that DNA abnormalities increase in frequency during progression from normal to IM and EAC. Since progression from NGM or IM to EAC (or dysplasia) is clinically actionable, we next decided to evaluate the discriminatory capacity of mutation load to identify this event. Specifically, we performed a Receiver-Operator Characteristic (ROC) analysis using the 20-gene AmpiiSeq mutation data from NGM and IM samples along with data for the same 20 genes obtained from whole exome sequencing of 66 EAC samples. Area under the ROC curve for NGM or IM versus EAC was 0.954 (Figure 13).

This data clearly support the underlying hypothesis for this proposal: that mutation load can be used as a biomarker to determine neoplastic status in the development of EAC. Since next-generation sequencing technologies can detect mutations with 1% or less mutation detection sensitivity and can now be performed on small amounts of DNA derived from FFPE specimens, this technology is perfect for detection of mutations in cytology specimens where many cells are actually normal.

In this project, we propose to develop a test designed to detect BE progression without the need for endoscopy or biopsies and that may therefore prove more acceptable and cost-effective than the current surveillance model.

*Identification of Biomarkers via Network Analysis.* Our group has developed several forms of gene network analysis that draw heavily on curated pathway databases, and which can be used to reduce a large list of genes that are altered in a disease to a concise small set of disease gene modules (38), which together form a disease-specific gene module map (Figure 14). This technique can readily identify tumour subtypes based on the pattern of altered modules in each subtype and the module-defined subtypes are often correlated with clinically-relevant disease characteristics such as survival or progression.

The network analysis techniques start with curated pathway data from several curated open source databases, primarily our own Reactome database. Curated pathway data is highly accurate, but has low coverage of the genome. For example, the combination of pathways derived from Reactome, KEGG, NCI PID and Panther covers roughly 25% of known human genes. In contrast, molecular and genetic interaction data derived from high throughput studies, such as proteomics, covers a much large proportion of the genome, but has a high rate of false positives. We use an automated technique to transform curated pathways into bimolecular interactions, and then use this as a training set to train a machine learning system for predicting true biologically functional interactions from multiple orthogonal high throughput data sets. The predicted and curated functional interactions are then combined to create the Reactome Functional Interaction (FI) network, an assemblage of ∼11,000 genes and ∼200,000 interactions among them. Various assessments described in Wu, *et al.* (38) suggests that the FI network has a high degree of accuracy.

To identify putative disease modules, we take a list of genes of interest, for instance those that carry somatic mutations in a cancer genome, and extract those genes and their interactions from the Reactome FI network to give a disease-specific gene interaction subnetwork. Next, we test whether the subnetwork appears to be biologically significant by comparing it to networks of equal size created by selecting genes at random. Provided that this check succeeds, we use network clustering algorithms to partition the subnetwork into a series of highly-interacting gene modules and annotate those modules using over-representation analysis approaches (39) tests, to produce networks similar to that shown in Figure 15.

We have also employed the Reactome FI network to develop a metric for the potential oncogenicity of a novel cancer causing candidate, based on the amount of interconnection of the candidate gene to known cancer-related genes (40). This approach is described in more detail in the next section.

### Approach

This section describes the experimental approach for discovering the majority of driver mutations in EAC.

*Power calculations.* Based on our preliminary work, we expect the spectrum of mutations in EAC to be complex. In order to maximize our chances for identifying the majority of driver mutations that are present in precursor lesions, we aim to capture roughly 90% of the common driver point mutations in this disease. We need to distinguish a passenger gene in which mutations occur at the background level and a driver gene in which somatic mutations occur in at least 5% of samples.

To estimate the number of samples required to reach this goal, we have performed power calculations based on those used for the ICGC (41) (Figure 15). We assume that a background mutation rate is 1.5x10⁻⁶ non-synonymous mutations per base. If we assume that all genes have a coding region of 1,500 bases, this gives us a background rate of 2.25x10⁻³ somatic mutations per gene. Given ∼20,000 protein-coding genes in human genome, some genes may exceed the expectation by random chance. So we need to set up a threshold of somatic mutations across all cancer samples that will be rarely exceeded by passenger genes and frequently exceeded by drivers. For example, a sample of 500 tumors provides 99.99% power to detect a gene mutated in 5% of samples, with a 10% false discovery rate and a missing data rate of ∼10% due to incomplete coverage (provided by Cancer Genomics platform in OICR). This model predicts that the current set of 150 EAC exome pair samples is sufficient to identify fewer than 75% of the driver mutations that are present in at least 5% of tumours. To capture roughly 94% of common drivers, we must increase the total sample size to 250 (Figure. 16).

***Nucleotide Sequencing.*** We will assemble a total of 100 tumour/normal specimen pairs from esophageal resection patients with EAC, using the extensive collections of consented specimens at Universities of Michigan and Rochester. When combined with the 150 specimen pairs previously analyzed by whole-exome sequencing, this brings the total to the 250 pairs called for by the power analysis. We will perform exome sequencing on all 100 tumours and their matched normal tissue, and RNA sequencing on a subset of 50 tumour samples selected to maximize the number of potential driver mutations. This will enable us to determine whether putative driver genes are being expressed, and if so, whether the expressed transcripts contained the altered allele.

The DNA and RNA extraction will be performed at the sample collection centers, and sent to OICR. At OICR, we will assess the quantity and quality of the DNA using the Qubit Double Stranded Broad Range Assay, the Nanodrop Assay, and agarose gel. We will perform short tandem repeat (STR) fingerprinting to ensure that tumour and matched normal are from the same individual and are distinct from other collected samples. This step will catch sample mixups and some forms of contamination.

For whole-exome sequencing, we will enrich exonic regions using the Agilent SureSelect 50 Mb exome enrichment kit, and generate Illumina libraries using the NEBNext™ DNA Sample Prep Kit. To allow for multiplexing, we will barcode tumour and normal samples using Integrated DNA Technologies (IDT) oligos. We will multiplex samples at a level of 2 tumours per lane and 3 matched normals per lane on the Illumina HiSeq 2000 platform using paired end 101 bp reads. This will give roughly 100 fold coverage of exome sequence for normal, and 100-200 fold coverage of tumour, depending on its cellularity.

To aid in the identification of potential drivers, we will also subject a subset of EAC samples to RNA sequencing to identify expression of mutated forms. We will perform directional RNA sequencing (RNA-seq) on 50 selected EAC samples. Ribo-depleted directional RNAseq barcoded libraries will be prepared using the Nugen Encore Complete library preparation kit, and subsequently sequenced on the Illumina HiSeq 2000 platform using paired-end 101 bp reads and two samples per lane, for 3 billion reads/sample. Assuming a median transcript length of 3 kb and 1 million transcripts per cell, this will provide a nominal 12.5x coverage of the transcriptome, sufficient to identify most rare transcripts.

***Sequence Analysis.*** For somatic mutation calling, we will use our standard pipeline, which consists of aligning sequencing reads to the reference human genome using Novoalign (Novocraft, Technologies), filtering unmapped and poorly mapped reads with Samtools (42), collapsing PCR duplicated reads with Picard (43), recalibrating the quality scores and performing local realignment with GATK (44), calling somatic and germline mutations using an in-house algorithm, and performing a variety of filtering and QC steps. This protocol typically yields 85-95% validation rates on called somatic point mutations.

For RNA-seq, we will align reads to the genome with Bowtie (45), map splice sites with TopHat (45), and use Trans-ABYSS (46) to identify putative fusion transcripts. This will yield quantitative expression information on both mutant and normal transcripts, and allow us to identify structural changes involving altered splicing and/or gene fusions.

*Validation of Somatic Mutation Calling.* To assess the accuracy of the somatic mutation calling pipeline on the esophageal tumour/normal pairs, we will perform four validation exercises on mutations identified over the course of the two years of this phase of the project. During each validation exercise, we will randomly select 96 mutations identified from tumour/normal pairs analyzed during the previous quarter, design PCR amplification systems for them, amplify isolated DNA from the pair, and sequence the amplification products on the MiSeq platform using paired-end 150 bp reads. We will map reads from the amplification products onto the appropriate region of the genome using Novalign, and manually review them with IGV (47) to score the presence of the predicted somatic mutation in tumour vs normal tissue.

For validation of putative fusion transcripts, we will design primer pairs that span the predicted transcript junction, subject mRNA from the appropriate sample to RT-PCR, and sequence the amplification products on the MiSeq platform.

Somatic mutation calls that are implicated as candidate drivers using the techniques described in the next section will be individually validated during the development and application of the AmpliSeq assay for driver genes.

*Development of an EAC gene module map.* Using the network analysis techniques described under Preliminary Results, we will create a gene module map for the somatic mutations identified among the full set of 250 tumour/normal pairs. This map will reflect common pathways of genomic alteration in esophageal adenocarcinoma. By comparing patterns of module alteration that are altered in individual tumours, we may be able to discern different subtypes of the disease and potentially relate these to patient survival and other clinical features. More to the main goal of this project, however, we will use the gene module map to determine sets of genes that identify progression in biopsied pre-neoplastic lesions.

*Identification of Candidate Drivers.* We will identify common candidate driver mutations using techniques that take into account the biological impact of the mutation, whether the mutated gene is expressed, the frequency with which the gene in which the mutation occurs is altered in the patient population, and network-based metrics of the oncogenicity of the mutation (Figure 16). First, we will grade each mutation for the severity of its effect on the associated protein coding gene using MutationAssessor (48). This will deprioritize mutations that are predicted to cause synonymous changes to the amino acid sequence, and increase the priority of those that introduce frameshifts, introduce alterations to known functional protein domains or are likely produce other high impact events on the associated proteins. Each gene will then be ranked according to a weighted average of the severity of the mutations that have occurred within it in the specimen population.

We will incorporate RNA sequencing into the mutation severity assessment ranking by selecting for RNA sequencing those sample pairs which carry genes with high impact mutations. From the RNA-seq data we will assess whether the mutant gene is expressed at all in the tumour tissue, and/or whether there is a significant (greater than 2-fold) change in expression level of the gene between tumour and control normal, which might indicate an alteration in transcript stability or splicing. We will deprioritize genes which are not expressed in either normal or tumour tissue, and increase the priority of genes that seem to be differentially expressed. Because of the rarity of gene fusion events and their high likelihood of significance, any fusion events that we detect during RNA sequencing will be added as high-ranking potential markers.

For frequency-based driver gene inference, we will calculate the overall frequency of somatic mutations in the adenocarcinoma exon set as a whole using both synonymous and nonsynonymous mutations. From this we will calculate a background mutation rate and apply this to each candidate gene, taking into account both the gene's exome length and the number of potential non-synonymous mutation sites in the gene, to derive the expected number of mutations that would occur by chance. Using a binomial distribution to simulate sampling error, we will compare the expected and observed numbers of non-synonymous mutations occurring in each candidate gene across all patient samples sequenced and derive an uncorrected P-value for the presence of driver mutations within each gene. To prioritize genes based on gene network properties, we will draw on earlier work that we performed on hepatocellular carcinoma (HCC) (40), in which we were able to accurately predict novel human HCC oncogenes that emerged during a mouse hepatoblast screening experiment. In this technique, we take a ranked list of genes that have been annotated by the community as being cancer oncogenes or suppressors; in the case of the HCC work, we used the Memorial Sloan Kettering Gene Ranker lists (http://cbio.mskcc.org/tcga-generanker/). Using the Reactome FI network, we count the number of interactions between the gene of interest and cancer-related genes on the gene list, taking the ranking of the cancer-related genes into account. This metric was strongly predictive of oncogenicity in the hepatoblast model, with a cross-validation accuracy of roughly 80%.
For this project, we will calculate a similar metric for each of the EAC candidate driver genes, using an updated version of the Reactome FI network, and the current MSKCC gene ranker lists, producing an interaction-based ranking of each candidate.

These techniques will produce two independent rankings of the candidate genes based on frequency of mutations and relatedness of the genes to known cancer-related genes. Because we do not have a gold standard set on which to train a classifier to intelligently combine these rankings, we will compute a simple combined ranking score by taking the maximum rank on any of the gene lists for each gene. The top 100 ranked genes will be used for assay development.

In parallel with this effort, we will assess alternative methods for ranking potential early driver genes, including Oncodrive FM (49), mutationassessor (48), CHASM (50) and MuSiC (51). In addition, if esophageal adenocarcinoma mutation data emerges from the TCGA or other groups during this phase of the project, we will use this information to inform the search for potential progression markers. *Development of prognostic signatures from molecular alteration data.* Although it is not a formal deliverable, and is outside of the project main flowchart, we note that it will be possible to mine the EAC data for signatures based on mRNA expression and/or somatic mutation that are prognostic of patient survival time. This will take advantage of the fact that patient vital status and disease-free survival time will be available in the clinical data that we collect. For this purpose, we will use the Reactome FI network-based prognostic signature discovery protocol described under Activity 3, Preliminary Studies. Any molecular signature we identify will be combined with clinical and histopathological characteristics under a Cox multivariate proportional hazard model (52) to identify subpopulations of patients, if any, who have distinct risks of recurrence.

Similarly, we also note that the genetic alteration information resulting from this part of the study may well provide new insights into pathways involved in EAC and suggest new genes for targeted drug therapies.

In this phase of the project we will characterize the point mutations identified during the discovery phase in a variety of pre-neoplastic lesions. The challenge for this phase is that both biopsy and cytology samples tend to be small and/or formalin-fixed and paraffin-embedded (FFPE). Thus, DNA yield from these samples can be both scant and of low quality.

*Preliminary Studies.* To overcome this limitation, we have adopted a new technology called AmpliSeq™ for generation of targeted resequencing libraries using highly multiplexed PCR (53). The advantages of this library generation approach include its low DNA input requirements (<10ng) and its applicability to FFPE specimens. We demonstrated the feasibility of this approach by resequencing a set of 20 genes in EAC and preneoplastic biopsies. Briefly, custom primers were designed to cover the >90kb of target DNA with amplicons <140 bp. We then analyzed the products on the Personal Genome Machine (36)using the Ion Torrent 316 chip. 100% concordance was found between mutations identified by Illumina exome sequencing and Ampliseq™ targeted resequencing in 10 EAC samples.

*Approach.* We will design AmpliSeq™ PCR primer panels to the developed gene set using the web-based Ion AmpiiSeq Designer tool. Optimal PCR amplicon size for FFPE tissue DNA is 150-160bp (Ion Torrent Personal communication) and thus we anticipate that approximately 5000 primer pairs will be required. As with our 20 gene panel, these primer pairs will be divided into two pools for PCR amplification to minimize unwanted amplification products generated from forward and reverse primers of neighbouring amplicons.

Next we will test the primer panel by generating sequencing libraries starting with high quality human genomic DNA (n=3). These libraries will be prepared using AmliSeq library construction kits and sequencing will be performed on Ion 318 chips on the Ion Torrent PGM sequencer. This will provide more rapid turnaround and flexibility than Illumina Hi Seq for the iterative process of primer testing and optimization described below. For each of the three replicates we will evaluate sequence coverage and depth. Regions with no read coverage will be evaluated manually to determine the reason for failure. This may include lack of coverage in the primer design (gaps) due to sequence constraints not permitting primer design parameters to be met or failure of specific amplicons in the multiplex PCR. Similarly, some regions will have low sequence depth (<20% of the mean depth) due to inefficient amplification of some amplicons. In all cases, we will attempt to fill the gaps or increase sequence depth with manual primer designs. The additional primers will then be added to the appropriate multiplex primer pools and sequence coverage with these new pools will be re-evaluated in the same manner. In our preliminary studies, one iteration of this process was sufficient to provide >500X sequence depth for >90% of bases and >100X coverage for 99% of the bases. We anticipate being able to achieve similar results with the expanded gene set although additional iterations will be considered if necessary.

Once the primers designs are optimized, we will confirm that similar sequence metrics can be obtained using DNA from 3 separate FFPE samples. We anticipate no problems at this step as the fragmented DNA from FFPE tissues seems to perform at least as well as high quality DNA in the multiplex amplification reactions (assuming amplicons sizes are 150-160bp). In fact, sequence coverage and depth is often improved as DNA fragmentation reduces the chance of crosstalk between primers from nearby amplicons. However, any major gaps or low depth regions will be addressed as above.
Finally, we will adapt the library construction to facilitate sequencing using the Illumina HiSeq 2000 platform. The higher throughput and multiplexing capacity of the HiSeq instrument will greatly reduce the cost for Activity 3 compared to sequencing on the Ion Torrent PGM platform. This will be achieved by interrupting the AmpliSeq protocol after the PCR amplification step and replacing the AmpiiSeq sequencing adapters with barcoded Illumina adapters. Library construction and analysis on the Illumina HiSeq 2000 will then be performed using standard protocols.

Pilot studies on sample multiplexing at OICR indicate that we can easily multiplex 24 samples per Illumina lane, with the strong possibility of scaling up to 96 samples/lane. We conservatively assume that by year 3 of this project we will be able to routinely multiplex 48 samples/lane. Given a total target region of 500 kb, 75 GB of 101 bp paired read data per lane, a barcode length of 8, and a multiplex level of 48, we will achieve ∼2900x coverage of the target region per multiplexed sample. Conservatively assuming that we will need to see a mutation at least 10 times in order to call it and that most mutant alleles are present in a heterozygous state in a diploid region of the genome, this will allow us to detect a mutation if it is present in roughly 0.5% of nuclei. In the case of a homozygous mutation, or one that is present in a region of amplification or loss of heterozygosity, detection sensitivity will be improved.

To simultaneously test the AmpliSeq/Illumina protocol and confirm the somatic mutations identified during Activity 1, we will subject the DNA previously isolated from all 150 of the tumour specimens and matched normal samples to the amplification protocol and analyze them in a total of 7 Illumina lanes. The deep sequencing that will result from this exercise will also give us a window into the heterogeneity of the sampled areas of the EAC tumours by allowing us to assess the number and nature of subclones within these primaries using tumour clonality assessment algorithms. Our current clonality inference algorithm of choice is pyClone (54), which models clonal progression as a Dirichlet process. In recent months we have been working collaboratively with the authors to increase pyClone's accuracy and sensitivity by adding constraints based on the assumption that all subclones in a single tumour mass share a single common ancestor.

### Characterize normal, metaplastic, and dysplastic lesions to identify signature(s) of progression.

We will use the targeted sequencing assay to identify the patterns of point mutations in BE and dysplastic samples. From this information, we will build signature(s) that discriminate dysplasia from normal mucosa and IM.

Preliminary studies. We have recently developed a variation on the gene network analysis to identify prognostic signatures in a variety of cancers (Wu and Stein 2012, manuscript in review). We took published mRNA expression array data from the tumors of patients with breast ductal adenocarcinoma (five independent studies, 1320 patients in total). We formed network clusters from the expression data by weighting the Reactome FI graph with coexpression correlation coefficients followed by Markov clustering (55), and then used a form of supervised principal component analysis called SuperPC (56) to group the resulting modules into a small series of principal components involving one to several of the modules. We then tested whether any of the principal components was related to patient survival singly or in combination.

For breast cancer, we discovered a novel prognostic signature involving 31 genes highly enriched for the aurora kinase A and kinetochore maintenance pathways. As measured by its ability to validate across multiple independent studies, the breast cancer signature outperformed 48 previously published signatures and has the greatest prognostic power in ER positive tumours (Figure 17). Extending the technique to somatic genomic mutations identified in high grade serous adenocarcinoma of the ovary (four studies, patients), we found a novel 75-gene signature highly enriched in immune system function, the cell cycle and cell adhesion (manuscript in review). This signature outperformed a recently published signature obtained by combining somatic mutations with expression, methylation, and copy number changes (57).

*Approach.* We will draw on the Michigan and Rochester repositories to obtain samples of IM (200), low + high grade dysplasia (200), and NGM (150), along with matched normal mucosa from the same patients. All tissues will be evaluated by two study pathologists to ensure that they meet study requirements and achieve diagnostic consensus. Further details on the patient cohort, tissue processing and quality control are provided in Activity 7. Since most patients with dysplasia also have concurrent IM, we can make inferences on the temporal and causal relationships among these pre-neoplastic lesions. Our primary source will be archived (frozen or FFPE) tissue from patients who underwent esophagectomy for HGD, supplemented as necessary with FFPE specimens from surveillance endoscopy and endoscopic mucosal resection (EMR) procedures at both institutions.

Overall, we will have between 750 and 1100 biopsy samples taken from 200 to 550 patients. The range comes from the number of patients who have concurrent IM, dysplasia and/or NGM biopsies, which enables the same matched normal mucosa to be used as a control for multiple samples. In our plan, we make the conservative estimate that 1000 samples (550 lesions and 450 normal tissues) will need to be processed. Using the same power estimation methodology, we estimate that we will be powered to detect 92-95% of the genes that discriminate among the lesion types.

**Mutation profiling of the BE and dysplastic samples.** Each lesion/normal pair will be interrogated for point mutations in the identified gene panel identified using the designed AmpliSeq/Illumina HiSeq assay. DNA from each specimen will be isolated and amplified via the AmpliSeq panel. As before, we will ship amplification samples to OICR for barcoding, pooling, library preparation and Illumina HiSeq 2000 sequencing. Allowing for multiplexing of 48 samples per lane by the year 2015, the experiment will require 21 Illumina lanes or three full runs of the machine after accounting for failed lanes and libraries.

*Assessment of BE Heterogeneity.* We will then test whether the signature developed here can be applied to cytology specimens from patients undergoing concurrent endoscopic surveillance of BE and/or dysplasia. Because cytology collects cells from a wide surface area, the extent and heterogeneity of the lesional tissue become key factors in determining the sensitivity of the test. To give an example, if 10% of the nuclei sampled by cytology are from BE cells and there is no genomic heterogeneity, then the test needs only sufficient sensitivity to detect diagnostic mutations in 10% of cells. However, if the lesion is highly heterogeneous and the diagnostic mutations are only present in 10% of the BE tissue, then the test must have 1% sensitivity.

To measure and minimize the effect of heterogeneity on the signature, we are proposing a heterogeneity assessment side-project within the companion NIH R24 grant that provides the bulk of project co-funding. In this side-project, we will directly measure the heterogeneity of BE tissue by prospectively collecting samples at the University of Rochester from 12 patients with EAC undergoing surgical resection without neoadjuvant therapy. After resection, "biopsies" will be performed by the project pathologist by systematic collection collection of approximately 15 biopsies of BE tissue radiating out from the tumor at 1-2 cm intervals, for a total of 128 samples including normal control mucosa.

During the third year of the project, and following the first round of progression signature discovery, we will subject the heterogeneity samples to targeted sequencing of the genes contained within in the signature using the protocol described earlier. We will assign each gene a heterogeneity score based on the frequency with which samples taken from the same patient show mutations in that gene, and incorporate the score information into the classifier. We anticipate that signals from genes with the lowest levels of heterogeneity will be most informative for the identification of progression.

**Progression Signature Discovery.** After demultiplexing the samples, we will compare tumour to normal using the OICR somatic mutation analysis pipeline to identify the presence and frequency of point mutations in the premalignant and control lesions. We will then mine this data set for gene signatures that distinguish NGM from IM from dysplasia, using the EAC gene module map. For each sample, we will project its detected mutations onto the module map to create a vector of affected modules. We will then develop a classifier for progression by comparing the vectors of NGM, IM and dysplasia in order to discover patterns of change that are diagnostic of each. The idea behind this is that the gene module map will reflect a small number of common pathways of tumorigenesis; a signature based on the alteration patterns of pathways will likely be more robust and sensitive than signatures derived from a larger and more diverse set of genes. To improve the performance of the classifier, we will incorporate information from the heterogeneity ascertainment described in the previous section.

In addition to the network-based algorithm, we will apply more traditional gene-at-a-time signature discovery methods, including those based on logistic regression, naive Bayes classifiers, Bayesian networks, and support vector machines (for review, see (58)). The predictive accuracy of various algorithms will be evaluated via ten-fold cross-validation.

### Develop a clinically practicable Ion Torrent-based test for the identified signature for use in cytology specimens.

In this activity we translate the developed progression biomarker into an assay that can be used on patient cytology specimens to identify disease progression.

We have chosen the combination of AmpiiSeq and Ion Torrent as the platform on which to develop this assay because it is compatible with either of two alternative marketing models for the commercial diagnostic test that we hope to enable with this work. However, we recognize that sequencing technologies evolve rapidly, and are prepared to change the plan to take advantage of the best current technologies when the time comes.

*Approach.* Based on the pre-identified signature(s), we will select a subset of the genes for assay development using AmpiiSeq library construction and PGM sequencing. Starting with the designed PCR amplicons, we will refine the design to improve sequence coverage and depth across the sites of identified mutation hot-spots. Since new primer pools will need to be synthesized for this activity, we will initially have the opportunity not only to add primer sets but also to drop out primer sets from problem regions and replace them with new, manual designs.

At this point, testing and iterative optimization of the primer pools will be achieved. Goals for sequence coverage and depth are difficult to specify 4-5 years prior to actually performing these studies as sequencing capacity on the PGM chips (or a suitable replacement) will certainly increase dramatically over this period. However, at a minimum, we would expect to cover >95% of bases at >20% of the mean sequence depth and to cover all mutations with a sequence depth of no less than 50% of the overall mean sequence depth achieved. We assume that by the year 2016 mean sequence depth will be much greater than that achieved in our preliminary studies (1890X) and our target depth (2900X), ensuring sensitive detection of mutants.

Once optimized, the revised assay will be validated on a subset of the samples characterized. The deliverable from this activity is an assay for BE progression that is ready for evaluation on clinically-obtained cytology specimens.

### Evaluate Ion Torrent test on clinical cytology specimens.

In this activity, we will evaluate the developed progression assay on prospectively-gathered esophageal cytology specimens, in order to evaluate the performance of the assay on a clinically realistic patient population.

*Approach.* Over the first 4 years of the project, we will prospectively collect Cytosponge samples from approximately 350 patients undergoing endoscopy for surveillance of BE, for confirmation of dysplasia or EAC, or for EMR of dysplasia or early stage EAC lesions. Blood will also be collected as a source of matched normal DNA for each patient. Samples will be collected from patients being followed for a previous diagnosis of dysplasia at the University of Rochester Medical Center and St. Michael's Hospital in Toronto, using protocols approved by the research ethics boards at each institution. All patients will be required to provide informed consent to participate in the study. All patients will be required to provide informed consent to participate in the study and will be directed to the educational website to gain awareness and information to make such consent.

Samples will be collected while the patients are unsedated, prior to endopcsopy and will be processed for formalin fixation and paraffin. Based on the histology of endoscopic biopsies taken during the visit, we will classify the cytology specimens into three groups: IM, dysplasia and EAC. (There may also be a small number of normal samples from patients from whom BE is not endoscopically confirmed.) We will process the cytology samples on a microtome and H&E stain them for evaluation by the study pathologist and a cytotology technician. Sections will also be immunostained to detect expression of the intestinal metaplasia biomarker TFF3. We will use additional cytology block sections for DNA isolation and sequencing with the AmpliSeq/lon Torrent test, multiplexing each cytology specimen library with its matched normal DNA isolated from the patient's blood sample.

At the time this was written, it was uncertain when the Cytosponge would be available use outside of the UK, but approval was expected to come during 2013. Should the Cytosponge not be available during a portion of the project period, we will collect cytology specimens using an alternative encapsulated sponge device, the Cell-Mate™ (Hydromer, Inc. Branchburg, NJ), or else obtain standard esophageal brushing cytology samples during the endoscopy.

*Analysis*/*Identification of mutations.* We will test the ability of the classifier developed to accurately classify cytology samples. In the case of NGM vs. IM, we will evaluate the accuracy of TFF3 staining for identification of metaplasia both alone and in combination with the mutation data. For IM vs. dysplasia/cancer, the molecular biomarker will be evaluated alone and in combination with cytopathology diagnosis. With ∼100 samples per group the precision of our accuracy estimates will be adequate to evaluate the feasibility of the biomarker test for accurate detection and classification of pre-neoplastic lesions, and to assess the test against the cost and performance metrics identified in the GE³LS component.

The deliverable from this activity will be a measure of the accuracy of the candidate progression test, and estimates of its technical success rate, turnaround time and potential cost.

### Develop an economic model for BE screening that will set performance parameters on the progression test developed by earlier activities.

*Aim*/*Research Question:* The purpose of this activity is to evaluate the costs and benefits of an innovative screening and surveillance protocol for BE progression in chronic GERD patients. The protocol will be executed by primary care physicians as an integral part of patient management. Our core question is whether the new intervention yields good value for money, which is cost-effective or economically attractive relative to the current standard of care.

*Preliminary Work:*We have performed a review of the literature related to cost-effectiveness analyses of BE screening and surveillance in recent years (11,13,59-61). We found that there is substantial heterogeneity in modeling assumptions, no randomized trial evidence available to inform model construction, and inadequate incorporation of emerging evidence on novel screening methods, risk stratification, and treatments. Therefore, we find that currently available health economics evidence on BE screening and surveillance is insufficient to support the introduction of BE screening programs among chronic GERD patients. In addition, we have developed a survey of patient perceptions/attitudes regarding the care of chronic GERD, BE, and quality of life (see details below).

**Review of literature and data synthesis.** We will first conduct a systematic literature search and collection of all available data on the prevalence of BE, dysplasia and cancer in BE in Canada and the USA, death related to EAC, and the estimated annual rate of disease progression and regression. Similarly, we will collect relevant information on progression tests and treatments, including endoscopic and biopsy characteristics, screening uptake, accuracy of diagnostic tests, treatment of HGD/EAC and cure rates, complications of endoscopy and treatment, and health state utilities (to adjust the outcomes for decrements in quality of life associated with the stages of disease and treatment). Lastly, we will survey the costs of screening, surveillance and treatment in Canada and USA. These data gathering activities will ensure that the most up-to-date data are available to assess, collate, and synthesize in order to produce estimates for each model input parameter (see below).

**Patient attitude study recruitment and surveys.** Because much of the modeling depends on patient acceptance and uptake of the test, we will conduct a survey of patient perceptions/attitudes regarding the care of chronic GERD, BE, and quality of life. This test will be conducted as a sub-study under the Ontario Health Study (OHS), a large population cohort study being undertaken by the OICR in which baseline health and demographic information is gathered from Ontario adults via a self-administered electronic questionnaire. With over 225,000 enrollees in just one year of operation and growing at a rapid pace, the OHS (https://ontariohealthstudy.ca/en/about-study) provides the infrastructure to rapidly identify a subpopulation of interest, re-contact those individuals, and solicit their participation in follow-up studies. We will use this infrastructure to recruit approximately 11,350 participants (i.e., 5% of OHS participants), based on the 0.5% level of precision, 8.5% chronic GERD prevalence, population size of 225,000 and 95% confidence level. They will complete a follow-up electronic health questionnaire study investigating their attitudes towards chronic GERD/BE, their evaluation of the risks of developing EAC, and their knowledge of, and attitudes towards, endoscopic screening and surveillance. Participants who complete the questionnaire will be redirected to the web-based educational tools for interactive and visual information on GERD, risks of developing EAC and screening options. A post-questionnaire will be used to reassess participant attitudes to learn if attitudes changed following exposure to the educational tools.
While the OHS-based study will capture the attitudes and perceptions of the general population with GERD/BE, it cannot adequately sample the smaller population of patients with known BE who are undergoing endoscopic surveillance. Understanding this patient population is important because they have direct experience with the potential anxiety of having a condition that predisposes to cancer, as well as with the inconvenience of the current endoscopy-based standard of care for screening and surveillance. For this reason, we propose to perform a discrete choice experiment (DCE) on a population of patients currently undergoing BE surveillance or follow-up for previously-diagnosed esophageal dysplasia.

The DCE technique is an attribute-based measure of benefit based on the assumptions that health-care interventions, services, or policies can be described by their attributes and that an individual's valuation depends upon the levels of these attributes (62,63). DCEs that include a cost attribute also allow for estimations of willingness-to-pay (WTP). Since patients are the ultimate consumers of health services, it is important for the cost-benefit model to include patient WTP for various attributes associated with different intervention scenarios and health outcomes in order to quantify in monetary terms the aspects of scenarios that patients value the most (64) (e.g., medication costs, side effects, anxiety, participation in daily activities, days missed from work, out-of-pocket cost). WTP also provides a basis on which to estimate an acceptable price point for a commercialized version of the proposed diagnostic test, under the assumption that in some markets patients may have to bear all or a portion of the cost of the test.

To design the DCE, we will perform a literature review followed by discussions with project End User gastroenterologists to define a list of attributes and levels that are clinically relevant and appropriate for eliciting the preferences of patients with GERD, BE, dysplasia, and EAC. From this we will develop a DCE questionnaire and test it on a small sample of eligible patients (n=10) in order to adjust the labeling and framing of attributes. For the main part of the DCE study, we will recruit 300 patients undergoing endoscopic surveillance or follow-up for dysplasia who are 18 years or older and undergoing endoscopy at the Gastrointestinal Diseases Unit of St. Michael's Hospital, Toronto. These patients will complete a paper questionnaire under the guidance of a research nurse. Non-English speaking patients will be excluded.

**Markov model development and decision analysis.** From the information gathered from the literature and patient surveys, we will develop a Markov model to perform cost-effectiveness analysis and cost-benefit analysis to compare lifetime costs, life expectancy, and quality-adjusted life years (QALYs) of a hypothetical cohort of 50-year olds with chronic GERD, who are followed until death with a variety of screening and surveillance methods. The model will define health states for patients without BE, patients with BE and no dysplasia, low and high grade dysplasia, and EAC, which will be distributed among the model population according to estimated prevalence in North America and will then transition through the different health states, screening, surveillance and treatment on an annual basis. The hypothetical population will be stratified by sex and disease risk on the basis of both clinical and diagnostic factors. The analysis will be performed according to guidelines by the Canadian Agency for Drugs and Technologies in Health (65) and the Panel on Cost Effectiveness in Health and Medicine (66), and will factor in the benefits, risks and costs from the perspectives of both the health sector and society (67). The model will incorporate both direct medical costs and indirect costs of lost productivity. All future costs and benefits will be discounted at 5% per year (65).

The screening modalities considered under the model will include standard endoscopy, several endoscopy variants such as ultrathin endoscopy, cytology with histology only, and cytology with histology plus molecular biomarkers (developed in the current project). The model input parameter ranges will be based on available Canadian data as well as from a systematic review of published literature, the Canadian Institute for Health Information (CIHI), Canadian Classification of Health Interventions (CCI), Canadian Classification of Diagnostic, Therapeutic, and Surgical Procedures (CCP) and Ontario Health Insurance Plan databases, and from the Ontario Case Costing Initiative database. Indirect costs of lost productivity attributable to GERD and associated sequelae will be estimated using both the human capital approach (68), and the friction cost method (69). Data related to EAC prevalence, absenteeism from work, inability to perform housekeeping activities, travel and waiting times for medical care, premature death from cancer, age and sex-specific average annual earnings, and the friction period (the period needed to replace a sick worker by another worker) will be abstracted from the Canadian Cancer Statistics, Canadian Community Health Survey, and Labour Force Survey, Statistics Canada.
Finally, uncertainty and sensitivity analyses will be conducted to determine plausible bounds on outcomes, allow greater insight into trade-offs between intervention strategies, and determine which input parameters have the greatest influence on the key outcomes.

*Preliminary Studies:* This research is enabled by tissue collections built at the Universities of Michigan and Rochester. The Michigan collection is rich in surgical resections, while the Rochester collection has extensive endoscopic biopsy samples. Both collections have matched normal tissue samples with representation of multiple states along the progression to EAC from the same patient. At Michigan, fresh-frozen surgical samples have been obtained from >700 patients with EAC, HGD and IM. The Rochester repository contains both esophagectomy specimens from patients with EAC and endoscopic biopsies from patients with GERD and/or a prior diagnosis of BE. The Rochester collection currently has over 100 EAC samples and 2000 biopsy specimens from almost 300 patients. These are two of the largest collections of esophageal tissue specimens in North America.
For the prospective collection of cytology specimens, we will be leveraging the endoscopy practices at St. Michael's Hospital in Toronto, and University of Rochester, both of which are major regional referral centers for BE surveillance and follow-up.

*Approach.* After obtaining ethics board approval, we will collect appropriately consented samples from the Rochester and Michigan biobanks, and the endoscopy units at St. Michael's Hospital and University of Rochester. Samples will be reviewed by a project pathologist to confirm the diagnosis and presence of tumour in the specimen (where appropriate) and then processed in a uniform manner to extract nucleic acids for sequencing.

**Collection of EAC tumour/normal pairs.** We have already collected and obtained appropriate consent for the 100 EAC and matched normal specimens required. We will use approximately 50 EAC and matched normal tissue pairs each from the Michigan and Rochester repositories. All tissues will be subject to histologic review in order to confirm the tissue diagnosis, tissue quality and cellularity. All samples will be subjected to nucleic acid extraction and additional pathology review to ensure that the lesion of interest was sampled. Our standard protocol typically results in yields of 5-40µg of total RNA/DNA.

**Collection of IM/dysplasia biopsies.** Frozen and fixed biopsy samples will be obtained from archived material at the biobanks of the Universities of Rochester and Michigan. The archived material will have been taken from patients who are undergoing surveillance for BE or who were referred for verification of dysplasia or EAC, and who consented to broad research use of their biopsy specimens. All specimens will be reviewed by the project pathologist, and processed in a uniform manner for DNA extraction.

**Collection of cytology/biopsy sets.** Cytology specimens and matched blood samples will be obtained from patients who are undergoing surveillance or who are referred for verification of dysplasia or EAC at the University of Rochester and St. Michael's Hospital in Toronto. For efficiency and cross-comparison purposes, many of the cytology specimens obtained at Rochester will be obtained concurrently with biopsy specimens. Cytology specimens will be obtained using Cytosponge kits provided by Dr. Rebecca Fitzgerald, of the University of Cambridge. Information about the Cytosponge technique and surveillance capacity will be available for clinicians and patients alike through the web-based educational tools.

Cytosponge specimens will be routinely processed as ThinPrep smears and paraffin embedded cell blocks for cytological diagnosis. The cytology specimens will be screened by a cytotechnician with final diagnosis by at least two experienced cytopathologists. TFF3 immunohistochemistry will be performed using standard techniques and scored by Dr. Zhongren Zhou at University of Rochester, and Dr. Tom Giordano at University of Michigan. Additional cell block sections will be cut for DNA isolation and targeted re-sequencing.

In the case of the Cytosponge not being available for a portion of the study period, we will fall back on an alternative commercially available encapsulated sponge sampler called the Cell-Mate™ (Hydromer, Inc. Branchburg, NJ), or else obtain cytology samples by esophageal brushings performed during the endoscopy.

**Recruitment of patients for discrete choice experiment.** 300 patients undergoing baseline evaluation of BE or surveillance will be recruited. These patients will complete an online discrete choice questionnaire as described in that activity. Use of the interactive and visual web-based educational tools will aid recruitment efforts. Use of the interactive and visual web-based educational tools will aid recruitment efforts.

All portions of the study will be reviewed by the institutional REBs/IRBs, and patients will be appropriately consented.

### Stakeholder Engagement

Understanding the health risks associated with GERD and comprehending the need for continual monitoring of BE is also an important component in reducing the incidence of late-stage EAC diagnosis. The purpose of this activity is to improve patient understanding of their condition and EAC risks, to educate and decrease patient anxiety on esophageal monitoring procedures, to increase patient participation and monitoring of their esophageal health and to provide training support to clinicians. *Preliminary Studies:* We have previously produced numerous knowledge translation projects for both the research and public communities. Among others, we have developed and offered training programs in bioinformatics tools and techniques to advance cancer research (www.bioinjormatics.ca). and have enhanced end-user understanding of biological pathways by converting text content into pictorial displays (www.reactome.org). We bring over 8 years of experience in post-secondary education, knowledge translation and educational outreach to both public and scientific audiences (Michelle Brazas, PhD), as well as extensive artistic and visual communication expertise (Heeyeon Song). As our medical illustrator, Song is also obtaining a Masters in Biomedical Communication from the Biomedical Communications program at the University of Toronto, a relationship that further strengthens our capacity to provide end-user training using current and effective communication techniques.
*Approach:* To complement the proposed research activities, we will develop an interactive and visual web-based education tool. Whereas current patient awareness programs rely heavily on text-rich content (Canadian Digestive Health Foundation (CDHF), http://www.cdhf.ca/diaestive-disorders/aerd.shtml), our plan is to guide patient awareness, education and participation through video vignettes and interactive visual aids (Figure 18).

The educational tool will be actively developed over the duration of the project building from basic GERD, BE and EAC awareness material through to detailed information on current (e.g. endoscopy) and new esophageal monitoring procedures (e.g. Cytosponge), and will thus act as a resource during patient sample acquisition. The tool will also act as a knowledge resource for the patient in support of the socio-economic evaluation. Through the Canadian Digestive Health Foundation and its Canadian gastroenterologist community, feedback from the patient end-user on the usefulness and comprehension of the web-based material will be gathered through anonymous pop-up surveys linked to the web site. This feedback as well as guidance from the Biomedical Communications program will be key to producing an effective educational tool for improving end-user awareness of EAC risks, decreasing patient anxiety of monitoring techniques and increasing patient participation in monitoring their esophageal health.

The tool will be further developed to include information and clinician training modules on the cytological biomarker assay. Detailed clinical information and guidelines for use and evaluation will be incorporated into the tool for the clinician, using similar web-based interactive and visual knowledge translation strategies. Feedback from the clinical community on the effectiveness of the training modules will also be gathered and used to modify the tool to improve its utility as a resource. At the conclusion of project, the tool will remain as an invaluable resource for the patient and clinician, and will be shared with the CDHF for permanent dissemination and marketing among the gastroenterology community in Canada.

### Reference List

1. CDHF (2012) http://www.cdhf.ca/digestive-disorders/barret-esophagus.shtml.
2. Pohl, H. and Welch, H. G. (2005) J. Natl. Cancer Inst. 97, 142-146
3. Reynolds, J. V., Donohoe, C. L., McGillycuddy, E., Ravi, N., O'Toole, D., O'Byrne, K., and Hollywood, D. (2012) J. Thorac. Cardiovasc. Surg. 143, 1130-1137
4. CDHF (2012) http://www.cdhf.ca/digestive-disorders/gerd.shtml.
5. Hvid-Jensen, F., Pedersen, L., Drewes, A. M., Sorensen, H. T., and Funch-Jensen, P. (2011) N. Engl. J. Med. 365, 1375-1383
6. Jankowski, J., Barr, H., Wang, K., and Delaney, B. (2010) BMJ 341, c4551
7. Bhat, S., Coleman, H. G., Yousef, F., Johnston, B. T., McManus, D. T., Gavin, A. T., and Murray, L. J. (2011) J. Natl. Cancer Inst. 103, 1049-1057
8. Armstrong, D., Marshall, J. K., Chiba, N., Enns, R., Fallone, C. A., Fass, R., Hollingworth, R., Hunt, R. H., Kahrilas, P. J., Mayrand, S., Moayyedi, P., Paterson, W. G., Sadowski, D., and van Zanten, S. J. (2005) Can. J. Gastroenterol. 19, 15-35
9. Kahrilas, P. J., Shaheen, N. J., Vaezi, M. F., Hiltz, S. W., Black, E., Modlin, I. M., Johnson, S. P., Allen, J., and Brill, J. V. (2008) Gastroenterology 135, 1383-1391, 1391
10. Spechler, S. J., Sharma, P., Souza, R. F., Inadomi, J. M., and Shaheen, N. J. (2011) Gastroenterology 140, 1084-1091
11. Inadomi, J. M., Sampliner, R., Lagergren, J., Lieberman, D., Fendrick, A. M., and Vakil, N. (2003) Ann. Intern. Med. 138, 176-186
12. Inadomi, J. M. (2009) Keio J. Med. 58, 12-18
13. Gerson, L. B., Groeneveld, P. W., and Triadafilopoulos, G. (2004) Clin. Gastroenterol. Hepatol. 2, 868-879
14. Veldhuyzen van Zanten, S. J., Thomson, A. B., Barkun, A. N., Armstrong, D., Chiba, N., White, R. J., Escobedo, S., and Sinclair, P. (2006) Aliment. Pharmacol. Ther. 23, 595-599
15. Statistics Canada. (2008) http://www.statcan.gc.ca/daily-quotidien/090115/t090115c1-eng.htm. Population estimates by sex and age group*,*
16. Yu, D., Hopman, W. M., and Paterson, W. G. (2008) Can. J. Gastroenterol. 22, 621-626
17. Spechler, S. J. (1997) Gastroenterol. Clin. North Am. 26, 455-466
18. Jemal, A., Siegel, R., Xu, J., and Ward, E. (2010) CA Cancer J. Clin. 60, 277-300
19. DOLL, R. and HILL, A. B. (1950) Br. Med. J. 2, 739-748
20. Riddell, R. H. and Odze, R. D. (2009) Am. J. Gastroenterol. 104, 2588-2594
21. Corley, D. A., Levin, T. R., Habel, L. A., Weiss, N. S., and Buffler, P. A. (2002) Gastroenterology 122, 633-640
22. Aldulaimi, D. M., Cox, M., Nwokolo, C. U., and Loft, D. E. (2005) Eur. J. Gastroenterol. Hepatol. 17, 943-950
23. Ell, C., May, A., Pech, O., Gossner, L., Guenter, E., Behrens, A., Nachbar, L., Huijsmans, J., Vieth, M., and Stolte, M. (2007) Gastrointest. Endosc. 65, 3-10
24. Adams, L., Roth, M. J., Abnet, C. C., Dawsey, S. P., Qiao, Y. L., Wang, G. Q., Wei, W. Q., Lu, N., Dawsey, S. M., and Woodson, K. (2008) Cancer Prev. Res. (Phila) 1, 357-361
25. Kadri, S., Lao-Sirieix, P., and Fitzgerald, R. C. (2011) Biomark. Med. 5, 397-404
26. Kadri, S. R., Lao-Sirieix, P., O'Donovan, M., Debiram, I., Das, M., Blazeby, J. M., Emery, J., Boussioutas, A., Morris, H., Walter, F. M., Pharoah, P., Hardwick, R. H., and Fitzgerald, R. C. (2010) BMJ 341, c4372
27. Lao-Sirieix, P. and Fitzgerald, R. C. (2012) Nat. Rev. Clin. Oncol. 9, 278-287
28. Nanda, K., McCrory, D. C., Myers, E. R., Bastian, L. A., Hasselblad, V., Hickey, J. D., and Matchar, D. B. (2000) Ann. Intern. Med. 132, 810-819
29. Hanahan, D. and Weinberg, R. A. (2011) Cell 144, 646-674
30. Herceg, Z. and Hainaut, P. (2007) Mol. Oncol. 1, 26-41
31. Paulson, T. G., Maley, C. C., Li, X., Li, H., Sanchez, C. A., Chao, D. L., Odze, R. D., Vaughan, T. L., Blount, P. L., and Reid, B. J. (2009) Clin. Cancer Res. 15, 3305-3314
32. Ong, C. A., Lao-Sirieix, P., and Fitzgerald, R. C. (2010) World J. Gastroenterol. 16, 5669-5681
33. Goh, X. Y., Rees, J. R., Paterson, A. L., Chin, S. F., Marioni, J. C., Save, V., O'Donovan, M., Eijk, P. P., Alderson, D., Ylstra, B., Caldas, C., and Fitzgerald, R. C. (2011) Gut60, 1317-1326
34. Lai, L. A., Kostadinov, R., Barrett, M. T., Peiffer, D. A., Pokholok, D., Odze, R., Sanchez, C. A., Maley, C. C., Reid, B. J., Gunderson, K. L., and Rabinovitch, P. S. (2010) Mol. Cancer Res. 8, 1084-1094
35. Liu, W., Hahn, H., Odze, R. D., and Goyal, R. K. (2009) Am. J. Gastroenterol. 104, 816-824
36. Rothberg, J. M., Hinz, W., Rearick, T. M., Schultz, J., Mileski, W., Davey, M., Leamon, J. H., Johnson, K., Milgrew, M. J., Edwards, M., Hoon, J., Simons, J. F., Marran, D., Myers, J. W., Davidson, J. F., Branting, A., Nobile, J. R., Puc, B. P., Light, D., Clark, T. A., Huber, M., Branciforte, J. T., Stoner, I. B., Cawley, S. E., Lyons, M., Fu, Y., Homer, N., Sedova, M., Miao, X., Reed, B., Sabina, J., Feierstein, E., Schorn, M., Alanjary, M., Dimalanta, E., Dressman, D., Kasinskas, R., Sokolsky, T., Fidanza, J. A., Namsaraev, E., McKernan, K. J., Williams, A., Roth, G. T., and Bustillo, J. (2011) Nature 475, 348-352
37. Hudson, T. J., Anderson, W., Artez, A., Barker, A. D., Bell, C., Bernabe, R. R., Bhan, M. K., Calvo, F., Eerola, I., Gerhard, D. S., Guttmacher, A., Guyer, M., Hemsley, F. M., Jennings, J. L., Kerr, D., Klatt, P., Kolar, P., Kusada, J., Lane, D. P., Laplace, F., Youyong, L., Nettekoven, G., Ozenberger, B., Peterson, J., Rao, T. S., Remade, J., Schafer, A. J., Shibata, T., Stratton, M. R., Vockley, J. G., Watanabe, K., Yang, H., Yuen, M. M., Knoppers, B. M., Bobrow, M., Cambon-Thomsen, A., Dressler, L. G., Dyke, S. O., Joly, Y., Kato, K., Kennedy, K. L., Nicolas, P., Parker, M. J., Rial-Sebbag, E., Romeo-Casabona, C. M., Shaw, K. M., Wallace, S., Wiesner, G. L., Zeps, N., Lichter, P., Biankin, A. V., Chabannon, C., Chin, L., Clement, B., de, A. E., Degos, F., Ferguson, M. L., Geary, P., Hayes, D. N., Hudson, T. J., Johns, A. L., Kasprzyk, A., Nakagawa, H., Penny, R., Piris, M. A., Sarin, R., Scarpa, A., Shibata, T., van, d., V, Futreal, P. A., Aburatani, H., Bayes, M., Botwell, D. D., Campbell, P. J., Estivill, X., Gerhard, D. S., Grimmond, S. M., Gut, I., Hirst, M., Lopez-Otin, C., Majumder, P., Marra, M., McPherson, J. D., Nakagawa, H., Ning, Z., Puente, X. S., Ruan, Y., Shibata, T., Stratton, M. R., Stunnenberg, H. G., Swerdlow, H., Velculescu, V. E., Wilson, R. K., Xue, H. H., Yang, L., Spellman, P. T., Bader, G. D., Boutros, P. C., Campbell, P. J., Flicek, P., Getz, G., Guigo, R., Guo, G., Haussler, D., Heath, S., Hubbard, T. J., Jiang, T., Jones, S. M., Li, Q., Lopez-Bigas, N., Luo, R., Muthuswamy, L., Ouellette, B. F., Pearson, J. V., Puente, X. S., Quesada, V., Raphael, B. J., Sander, C., Shibata, T., Speed, T. P., Stein, L. D., Stuart, J. M., Teague, J. W., Totoki, Y., Tsunoda, T., Valencia, A., Wheeler, D. A., Wu, H., Zhao, S., Zhou, G., Stein, L. D., Guigo, R., Hubbard, T. J., Joly, Y., Jones, S. M., Kasprzyk, A., Lathrop, M., Lopez-Bigas, N., Ouellette, B. F., Spellman, P. T., Teague, J. W., Thomas, G., Valencia, A., Yoshida, T., Kennedy, K. L., Axton, M., Dyke, S. 0., Futreal, P. A., Gerhard, D. S., Gunter, C., Guyer, M., Hudson, T. J., McPherson, J. D., Miller, L. J., Ozenberger, B., Shaw, K. M., Kasprzyk, A., Stein, L. D., Zhang, J., Haider, S. A., Wang, J., Yung, C. K., Cros, A., Liang, Y., Gnaneshan, S., Guberman, J., Hsu, J., Bobrow, M., Chalmers, D. R., Hasel, K. W., Joly, Y., Kaan, T. S., Kennedy, K. L., Knoppers, B. M., Lowrance, W. W., Masui, T., Nicolas, P., Rial-Sebbag, E., Rodriguez, L. L., Vergely, C., Yoshida, T., Grimmond, S. M., Biankin, A. V., Bowtell, D. D., Cloonan, N., deFazio, A., Eshleman, J. R., Etemadmoghadam, D., Gardiner, B. B., Kench, J. G., Scarpa, A., Sutherland, R. L., Tempero, M. A., Waddell, N. J., Wilson, P. J., McPherson, J. D., Gallinger, S., Tsao, M. S., Shaw, P. A., Petersen, G. M., Mukhopadhyay, D., Chin, L., DePinho, R. A., Thayer, S., Muthuswamy, L., Shazand, K., Beck, T., Sam, M., Timms, L., Ballin, V., Lu, Y., Ji, J., Zhang, X., Chen, F., Hu, X., Zhou, G., Yang, Q., Tian, G., Zhang, L., Xing, X., Li, X., Zhu, Z., Yu, Y., Yu, J., Yang, H., Lathrop, M., Tost, J., Brennan, P., Holcatova, I., Zaridze, D., Brazma, A., Egevard, L., Prokhortchouk, E., Banks, R. E., Uhlen, M., Cambon-Thomsen, A., Viksna, J., Ponten, F., Skryabin, K., Stratton, M. R., Futreal, P. A., Birney, E., Borg, A., Borresen-Dale, A. L., Caldas, C., Foekens, J. A., Martin, S., Reis-Filho, J. S., Richardson, A. L., Sotiriou, C., Stunnenberg, H. G., Thoms, G., van, d., V, van't Veer, L., and Calvo, F. (2010) Nature 464, 993-998
38. Wu, G., Feng, X., and Stein, L. (2010) Genome Biol. 11, R53
39. Khatri, P., Sirota, M., and Butte, A. J. (2012) PLoS. Comput. Biol. 8, e1002375
40. Sawey, E. T., Chanrion, M., Cai, C., Wu, G., Zhang, J., Zender, L., Zhao, A., Busuttil, R. W., Yee, H., Stein, L., French, D. M., Finn, R. S., Lowe, S. W., and Powers, S. (2011) Cancer Cell 19, 347-358
41. ICGC (2008) http://icac.org/files/ICGC April 29 2008.pdf.
42. Li, H., Handsaker, B., Wysoker, A., Fennell, T., Ruan, J., Homer, N., Marth, G., Abecasis, G., and Durbin, R. (2009) Bioinformatics. 25, 2078-2079
43. PICARD (2012) http://picard.sourceforae.net/.
44. DePristo, M. A., Banks, E., Poplin, R., Garimella, K. V., Maguire, J. R., Hartl, C., Philippakis, A. A., del, A. G., Rivas, M. A., Hanna, M., McKenna, A., Fennell, T. J., Kernytsky, A. M., Sivachenko, A. Y., Cibulskis, K., Gabriel, S. B., Altshuler, D., and Daly, M. J. (2011) Nat. Genet. 43, 491-498
45. Langmead, B., Trapnell, C., Pop, M., and Salzberg, S. L. (2009) Genome Biol. 10, R25
46. Trans-ABySS (2012) http://www.bcgsc.ca/platform/bioinfo/software/trans-abyss.
47. Robinson, J. T., Thorvaldsdottir, H., Winckler, W., Guttman, M., Lander, E. S., Getz, G., and Mesirov, J. P. (2011) Nat. Biotechnol. 29, 24-26
48. Reva, B., Antipin, Y., and Sander, C. (2011) Nucleic Acids Res. 39, e118
49. Gonzalez-Perez, A. and Lopez-Bigas, N. (2012) Nucleic Acids Res.
50. Carter, H., Samayoa, J., Hruban, R. H., and Karchin, R. (2010) Cancer Biol. Ther. 10, 582-587
51. Dees, N. D., Zhang, Q., Kandoth, C., Wendl, M. C., Schierding, W., Koboldt, D. C., Mooney, T. B., Callaway, M. B., Dooling, D., Mardis, E. R., Wilson, R. K., and Ding, L. (2012) Genome Res. 22, 1589-1598
52. Cox, D. R. (2012) Regression models and life-tables.
53. AmpiiSeq (2012) https://www.ampliseg.com/browse.action.
54. Shah, S. P., Roth, A., Goya, R., Oloumi, A., Ha, G., Zhao, Y., Turashvili, G., Ding, J., Tse, K., Haffari, G., Bashashati, A., Prentice, L. M., Khattra, J., Burleigh, A., Yap, D., Bernard, V., McPherson, A., Shumansky, K., Crisan, A., Giuliany, R., Heravi-Moussavi, A., Rosner, J., Lai, D., Birol, I., Varhol, R., Tam, A., Dhalla, N., Zeng, T., Ma, K., Chan, S. K., Griffith, M., Moradian, A., Cheng, S. W., Morin, G. B., Watson, P., Gelmon, K., Chia, S., Chin, S. F., Curtis, C., Rueda, O. M., Pharoah, P. D., Damaraju, S., Mackey, J., Hoon, K., Harkins, T., Tadigotla, V., Sigaroudinia, M., Gascard, P., Tlsty, T., Costello, J. F., Meyer, I. M., Eaves, C. J., Wasserman, W. W., Jones, S., Huntsman, D., Hirst, M., Caldas, C., Marra, M. A., and Aparicio, S. (2012) Nature 486, 395-399
55. van Dongen, S. (2000) Graph Clustering by Flow Simulation. PhD thesis, University of Utrecht.
56. Bair, E. and Tibshirani, R. (2004) PLoS. Biol. 2, E108
57. Mankoo, P. K., Shen, R., Schultz, N., Levine, D. A., and Sander, C. (2011) PLoS. One. 6, e24709
58. Ressom, H. W., Varghese, R. S., Zhang, Z., Xuan, J., and Clarke, R. (2008) Front Biosci. 13, 691-708
59. Nietert, P. J., Silverstein, M. D., Mokhashi, M. S., Kim, C. Y., Glenn, T. F., Marsi, V. A., Hawes, R. H., and Wallace, M. B. (2003) Gastrointest. Endosc. 57, 311-318
60. Gerson, L. and Lin, O. S. (2007) Clin. Gastroenterol. Hepatol. 5, 319-325
61. Hur, C., Nishioka, N. S., and Gazelle, G. S. (2004) J. Natl. Cancer Inst. 96, 316-325
62. de Bekker-Grob, E. W., Ryan, M., and Gerard, K. (2012) Health Econ. 21, 145-172
63. Ryan, M., Bate, A., Eastmond, C. J., and Ludbrook, A. (2001) Qual. Health Care 10 Suppl 1, i55-i60
64. Kleinman, L., Mclntosh, E., Ryan, M., Schmier, J., Crawley, J., Locke, G. R., III, and De, L. G. (2002) Arch. Intern. Med. 162, 1361-1366
65. Canadian Agency for Drugs and Technologies in Health (2006) Guidelines for the economic evaluation of health technologies: Canada [3rd Edition].
66. Weinstein, M. C., Siegel, J. E., Gold, M. R., Kamlet, M. S., and Russell, L. B. (1996) JAMA 276, 1253-1258
67. Gold, M. R., Siegel, J. E., Russell, L. B., and Weinstein, M. C. (1996) Cost-Effectiveness in Health and Medicine, New York
68. Hodgson, T. A. (1983) Adv. Health Econ. Health Serv. Res. 4,129-164
69. Koopmanschap, M. A., Rutten, F. F., van Ineveld, B. M., and van, R. L. (1995) J. Health Econ. 14, 171-189

### EXPECTED SOCIO-ECONOMIC BENEFITS

Esophageal adenocarcinoma (EAC) is a rare but devastating cancer with a five year post-diagnosis survival of just 15%. Barrett's esophagus (BE), a frequent consequence of chronic gastrointestinal reflux disease (GERD), is the overwhelming risk factor for developing EAC. Both GERD and BE are extremely common in the Canadian population, and most individuals who have the conditions are unaware of it.

Currently, the options for the early detection and treatment of patients with EAC are unsatisfactory. The Canadian Association of Gastroenterology recommends that patients with long-standing GERD undergo a screening endoscopy to identify BE, and that those with BE undergo periodic endoscopy with biopsy to detect early EAC and the dysplasias that precede it (1). However, this is neither effective nor cost-efficient. The annual cost of endoscopic surveillance of BE in Canada is in excess of $400M (2), but only an estimated 75-100 patients with early EAC are identified, at a cost of roughly $4-5M/cancer detected.

This project seeks to develop a cost-effective alternative to endoscopy screening and surveillance that uses a combination of cytology and genetic markers to identify patients who are at high risk for developing EAC. The envisioned test can be administered in a primary care physician's office, will cause minimal patient discomfort or inconvenience, and is targeted to a price point that allows it to be applied as a screening test to all chronic GERD sufferers.

In this section, we first describe the epidemiology of EAC, BE and GERD as it relates to Canadian health and its health economics. We then describe how the proposed diagnostic test could change the way that EAC surveillance is conducted in this country, and estimate the economic impact this would have. Finally, we review the steps that will be needed to translate our research work into a commercially viable test that can enter clinical practice and describe our plan for achieving this goal.

**Background.** The incidence of esophageal adenocarcinoma (EAC) has increased 300 - 600% in the US and other Western Countries over the last 20-30 years (3) and continues to increase. In Canada, the number of reported cases of EAC increased from 1,066 in 1992 to 1,550 in 2007, a 50% increase over 15 years (http://www.cancer.ca/statistics). EAC tends to be diagnosed late and most patients present with locally advanced or metastatic disease. Consequently, the overall prognosis for patients with EAC is very poor with approximately 15% 5-year overall survival (4). When diagnosed early, EAC has a much better prognosis. Patients who are treated while the EAC is confined to the mucosa have a 5-year survival of 80% (5), while patients who receive ablative therapy for high grade dysplasia are likely to see a complete cure (6).

The strongest known risk factor for EAC is Barrett's esophagus (BE); a metaplastic change of the esophageal epithelium that occurs as a consequence of chronic gastroesophageal reflux disease (GERD). Tobacco smoking is also associated with EAC but alcohol consumption and bacterial infection (such as *Helicobactor pylori*) are **not** (Figure 19) (7,8).

GERD is associated with an 8-fold increased for development of EAC and BE is associated with an 11.3 to 40-fold increased risk (9,10), which is comparable to the roughly 20-fold increase in lung cancer risk in cigarette smokers. Because of this, both Canadian and U.S. guidelines recommend that patients with chronic GERD and other risk factors (including age 50 years or older, male sex, white race, hiatal hernia, elevated body mass index, and intra-abdominal distribution of body fat) undergo an endoscopy to diagnose BE histologically (10,11). Those with BE are further recommended to undergo periodic (annual or biennial) surveillance endoscopies to detect progression to dysplasia and EAC (12). However, these recommendations were graded as "weak" based on moderate quality evidence, and both the cost-effectiveness of BE screening and its risk/benefit ratio is controversial (13).

In practice, compliance with these recommendations is low and relatively few patients with chronic GERD undergo a screening endoscopy. In part this is because the procedure is uncomfortable, requires sedation, engenders the loss of a partial day of work, and necessitates a visit to a clinical specialist. Moreover, it is unlikely that there are sufficient trained endoscopists in Canada to perform baseline screening of the GERD population or to perform periodic surveillance on the entire population of patients with BE. As a result, just 5% of patients with BE are actually identified by health care providers (13), and only a portion of these undergo endoscopic surveillance. Not surprisingly therefore, >90% of EAC cases still occur in patients with no prior diagnosis of BE. Despite an estimated expenditure of over $400 M annually on endoscopic surveillance of BE in Canada (1,2,14,15), at most a hundred patients with EAC arising from BE are diagnosed at an early stage of disease subsequent to endoscopic surveillance. **Health and Economic Benefits.** We are aiming to improve the current system of endoscopic screening and surveillance by introducing a diagnostic assay that can detect patients at high risk of developing esophageal adenocarcinoma. A primary physician who has a patient with long-standing symptoms of GERD (10 years or more) will offer to screen the patient for Barrett's Esophagus. The patient will swallow a Cytosponge, a gelatin capsule containing a compressed sponge, and after a short period the physician will retrieve the sponge via an attached string. The sponge will then be sent to a central facility for cytological screening for BE.

Ten to 15% of the patients screened in this way will test positive for BE. These patients will be enrolled in an annual surveillance programme in which the Cytosponge test is repeated, but this time with the addition of genetic testing for mutational markers of premalignant changes that warn of progression towards EAC. These patients will be referred for endoscopic biopsy and more aggressive follow-up. Patients who test negative during the initial BE screening will be offered follow-up screening at ten year intervals.

The overall effect of this strategy would be to greatly reduce the number of patients undergoing negative endoscopies. Patients would only undergo endoscopy and biopsy if they had an elevated risk of a positive result. The strategy is similar to that used by the Pap test, under which patients with an abnormal Pap result are referred to cervical colposcopy and biopsy for definitive diagnosis.

There are two potential benefits of this paradigm change. The first is to increase screening of patients who are at elevated risk of EAC, thereby allowing them to be treated at an early stage and reducing the number of fatalities from this disease. The second is to reduce the personal and systemwide costs of performing endoscopies on patients who will never develop EAC.

We present here a preliminary framework for the cost benefit analysis in order to illustrate the magnitude of the potential socio-economic benefits. To estimate the effect of morbidity and mortality reduction, we consider that in the year 2007, 1,550 Canadian adults were diagnosed with EAC (http://www,cancer.ca). To add context, during the same year there were 590 drowning deaths and 2800 fatal motor vehicle accidents. Of these, 85% will be dead within 5 years, giving a steady state mortality rate of roughly 1,300 fatalities/year. Assuming a BE prevalence of 450,000 in the Canadian population and an annual progression rate from BE to EAC of 0.3%, we can estimate that 85% of annual cases are attributable to GERD/BE, and so up to 1,100 Canadian EAC fatalities/year would be potentially preventable if screening and surveillance were universal, and the proposed diagnostic test was 100% sensitive.

To estimate the potential economic benefits, we first look at the cost of EAC and its surveillance under current clinical practice. The cost for tertiary care of a patient with EAC, including surgeries, radiological studies, hospital stays and hospice care is roughly $50,000 per fatal case, and $20,000 per non-fatal case when summed over 5 years from the time of diagnosis. However, the loss of economic activity resulting from premature fatality is a more significant cost. The median age at diagnosis for patients with EAC is 68, and a healthy Canadian of that age is expected to live an additional 20 years, so we can estimated that a typical patient diagnosed with EAC will lose 15 years of life expectancy. Since the median household income for single seniors is $23,000 (http://www.cra-arc.gc.ca/) we estimate that the lost income due to a potentially preventable EAC cases is $345,000 per patient. Adding in the cost of cancer care for these patients gives a cost of $395,000 /patient for potentially preventable cases who die of their disease, and $20,000/patient who survive.

Endoscopic screening and surveillance for patients with GERD and/or BE together cost Canadians over $400M in 2006 (1,2,14,15). Only about 5% of patients with BE are under surveillance, so assuming 100% accuracy, endoscopic surveillance can potentially catch 35 progressing patients, of whom 30 would otherwise die of their disease. It results in a savings of 30 x 345,000 for patients saved, minus 30 x 20,000 for the cost of their treatment, for a total of $10M. Thus the $420M annual cost of endoscopic screening results in roughly $10M in economic savings for EAC cases prevented. In contrast, screening of GERD patients by cytology should cost roughly the same as a Pap test, or $50/patient screened. If we screen 80% of the GERD population of 3M patients once every 10 years, the annual cost of this procedure is $12M. Roughly 15% of the screened population, or 360,000 adults are carrying BE; and assuming that the cytological test for BE is 90% sensitive, 324,000 patients become eligible for annual surveillance by a combination of cytology and genetics.

The total cost of surveillance under optimistic projections for the cost and sensitivity of the proposed test is 12+97+0.43 = $109M/year. It would catch 870 patients at an early stage of disease, resulting in a net savings of 870 x (345,000-20,000) = $282M in economic activity gains due to reduced fatality. In summary this exercise suggests that if we were successful in developing the diagnostic test proposed in this study, and if it were commercialized and widely adapted by the Canadian health care system, $109M/year in annual screening and surveillance costs could result in economic gains of $282M/year, as opposed to conventional endoscopic screening, which costs more than 40 times what it gains in purely economic terms. In addition to saving 800 additional lives per year over the current screening system, the envisioned tiered screening system would avoid the discomfort and inconvenience of endoscopy for over 100,000 adults annually.

The above preliminary analysis will ultimately be superseded by the full GE³LS economic model, which will take into account the sensitivity and specificity of the test, its fully loaded cost, and the degree of its uptake by primary care physicians. Such factors can make a significant difference. For example, if the test has a false positive rate of 5%, then the annual costs increase by roughly $8M to pay for 16,000 endoscopic biopsies on patients who are not in fact progressing. If the price point for the genetic test were $1000, then the overall cost of surveillance would rise from $109M to $324M/annum. Nevertheless, this exercise illustrates, within a few fold, the potential effect of the diagnostic test envisioned here, and illustrates some of the parameters that will go into making the economic model that will guide our work.

### Reference List

1. Armstrong, D., Marshall, J. K., Chiba, N., Enns, R., Fallone, C. A., Fass, R., Hollingworth, R., Hunt, R. H., Kahrilas, P. J., Mayrand, S., Moayyedi, P., Paterson, W. G., Sadowski, D., and van Zanten, S. J. (2005) Can. J. Gastroenterol. 19, 15-35
2. CDHF (2012) http://www.cdhf.ca/digestive-disorders/statistics.shtml.
3. Pohl, H. and Welch, H. G. (2005) J. Natl. Cancer Inst. 97,142-146
4. Jemal, A., Siegel, R., Xu, J., and Ward, E. (2010) CA Cancer J. Clin. 60, 277-300
5. Rice, T. W., Blackstone, E. H., and Rusch, V. W. (2010) Ann. Surg. Oncol. 17, 1721-1724
6. Ell, C., May, A., Pech, O., Gossner, L., Guenter, E., Behrens, A., Nachbar, L., Huijsmans, J., Vieth, M., and Stolte, M. (2007) Gastrointest. Endosc. 65, 3-10
7. Enzinger, P. C. and Mayer, R. J. (2003) N. Engl. J. Med. 349, 2241-2252
8. Badreddine, R. J. and Wang, K. K. (2010) Nat. Rev. Gastroenterol. Hepatol. 7, 369-378
9. Hvid-Jensen, F., Pedersen, L., Drewes, A. M., Sorensen, H. T., and Funch-Jensen, P. (2011) N. Engl. J. Med. 365, 1375-1383
10. Jankowski, J., Barr, H., Wang, K., and Delaney, B. (2010) BMJ 341, c4551
11. Kahrilas, P. J., Shaheen, N. J., Vaezi, M. F., Hiltz, S. W., Black, E., Modlin, I. M., Johnson, S. P., Allen, J., and Brill, J. V. (2008) Gastroenterology 135, 1383-1391, 1391
12. Spechler, S. J., Sharma, P., Souza, R. F., Inadomi, J. M., and Shaheen, N. J. (2011) Gastroenterology 140, 1084-1091
13. Inadomi, J. M. (2009) Keio J. Med. 58, 12-18
14. Veldhuyzen van Zanten, S. J., Thomson, A. B., Barkun, A. N., Armstrong, D., Chiba, N., White, R. J., Escobedo, S., and Sinclair, P. (2006) Aliment. Pharmacol. Ther. 23, 595-599
15. Statistics Canada. (2008) http://www.statcan.ac.ca/daily-quotidien/090115/t090115c1-eng.htm. Population estimates by sex and age group*,*
16. Kadri, S. R., Lao-Sirieix, P., O'Donovan, M., Debiram, I., Das, M., Blazeby, J. M., Emery, J., Boussioutas, A., Morris, H., Walter, F. M., Pharoah, P., Hardwick, R. H., and Fitzgerald, R. C. (2010) BMJ 341, c4372
17. Goh, X. Y., Rees, J. R., Paterson, A. L., Chin, S. F., Marioni, J. C., Save, V., O'Donovan, M., Eijk, P. P., Alderson, D., Ylstra, B., Caldas, C., and Fitzgerald, R. C. (2011) Gut 60, 1317-1326
18. Peters, C. J., Rees, J. R., Hardwick, R. H., Hardwick, J. S., Vowler, S. L., Ong, C. A., Zhang, C., Save, V., O'Donovan, M., Rassl, D., Alderson, D., Caldas, C., and Fitzgerald, R. C. (2010) Gastroenterology 139, 1995-2004
19. Kadri, S., Lao-Sirieix, P., and Fitzgerald, R. C. (2011) Biomark. Med. 5, 397-404
20. Sepehr, A., Razavi, P., Saidi, F., Salehian, P., Rahmani, M., and Shamshiri, A. (2000) Acta Cytol. 44, 797-804

**Table 2. Paired forward and reverse primers of biomarkrs.**

| **SEQ ID NO** | **Description** | **Sequence** | **SEQ ID NO** | **Description** | **Se**q**uence** |
|---|---|---|---|---|---|
| 1 | APC for primer | CCTTTTTAACCTTATAGGTCCAAGGGT | 2 | APC rev primer | CTTCTAGCTCTTGTCGAAGATTTGAGT |
| 3 | APC for primer | CTTAAACAACTACAAGGAAGTATTGAAGATGA | 4 | APC rev primer | CACCTAAAGATGACAATTTGAGCTTAAAA |
| 5 | APC for primer | AGAATATTTTAGACTGCTTAAAGCAATTGTTG | 6 | APC rev primer | CCGCAGTTTTACTCCAGGGAAATTA |
| 7 | APC for primer | CGGTCAAAAATGTCCCTCCGTT | 8 | APC rev primer | AATATCCAGTACTTTCTCTGCTTCCATTTAC |
| 9 | APC for primer | TCTTCTGCAGTCTTTATTAGCATTGT | 10 | APC rev primer | TTTTAGTGAGATTCTGAAGTTGAGCGT |
| 11 | APC for primer | AGTTTTCCTTACAAACAGATATGACCAGAAG | 12 | APC rev primer | AACAAGTAACTTACCTGTGCTCGTT |
| 13 | APC for primer | GCGAAGAATAGCCAGAATTCAGC | 14 | APC rev primer | AAAGGCAATTTACTAACCTCTGCTTCT |
| 15 | APC for primer | CCTTGGGCTAAGAAAGCCTAC | 16 | APC rev primer | GATTTCTCCCACTCCTTGACCTT |
| 17 | APC for primer | ACCATCTATAATGTGCTTAATTTTTAGGGT | 18 | APC rev primer | TTACCTTGGTTCCCAGATGACTTG |
| 19 | APC for primer | TGGTTTTTGGCTTTTGGATATTAAAGTC | 20 | APC rev primer | AGCTAGCAAAGTTCGCGACATA |
| 21 | APC for primer | AGTCTGGATGTCTTCCTCTCCTC | 22 | APC rev primer | AGGCTGTGAGTGAATGATGTTGT |
| 23 | APC for primer | CATCAGATCTGTCCTGCTGTGT | 24 | APC rev primer | ACCATGATTTAAAAATCCACCAGTAATTG |
| 25 | APC for primer | CAGAATTATTGCAAGTGGACTGTGA | 26 | APC rev primer | TGTTATAAAAACATACCTTGTTGGCTACATCT |
| 27 | APC for primer | AATAAAGCTTGGCTTCAAGTTGTCT | 28 | APC rev primer | CCTGCTGTAAGTCTTCACTTTCAGATT |
| 29 | APC for primer | ATCTAAAATTGATTAATTTGCAGGTTATTGCGA | 30 | APC rev primer | CAATGCTTTCACACTTCCAACTTCT |
| 31 | APC for primer | GAAGTTAATGAGAGACAAATTCCAACTCT | 32 | APC rev primer | CACATATATCAGCTTTATTCTCAGTGCAATG |
| 33 | APC for primer | CTTGCATTTTTGGTTGGCACTCT | 34 | APC rev primer | ATAAAACTCTATATATACCTGTGGTCCTCAT |
| 35 | APC for primer | TTTGTTGTTACTGCATACACATTGTGA | 36 | APC rev primer | ACTGACTATTGTCAAACTATGAGATTTTAAGT |
| 37 | APC for primer | CTTTGTGGAATCTCTCAGCAAGAAATC | 38 | APC rev primer | GAGATTCCTTAAAGCTGCAGCAC |
| 39 | APC for primer | GCCAATATTATGTCTCCTGGCTCA | 40 | APC rev primer | CGATGAGATGCCTTGGGACT |
| 41 | APC for primer | GACACAAGCAAAGTCTCTATGGTGA | 42 | APC rev primer | TGAAGAGGAGCTGGGTAACACT |
| 43 | APC for primer | GGAAGCTTAGATAGTTCTCGTTCTGAAA | 44 | APC rev primer | GCTGCAGTGGTGGAGATCTG |
| 45 | APC for primer | TCAGCCATTCATACCTCTCAGGAA | 46 | APC rev primer | TCCGACTTAGTGAAATTGTAAGTGTTTGA |
| 47 | APC for primer | AGTTCATTATCATCTTTGTCATCAGCTGAA | 48 | APC rev primer | CAGCTGACCTAGTTCCAATCTTTTCT |
| 49 | APC for primer | CACCCTAGAACCAAATCCAGCA | 50 | APC rev primer | TTCAGGTGGACTTTTGGGTGT |
| 51 | APC for primer | CTGTCAGTTCACTTGATAGTTTTGAGAGT | 52 | APC rev primer | TCTGCTTGGTGGCATGGTT |
| 53 | APC for primer | TGAGAGAGTTTTCTCAGACAACAAAGATT | 54 | APC rev primer | GAGGTGAATCAAAAGCAAAACTTCCT |
| 55 | ATM for primer | TTTTACAGACAGTGATGTGTGTTCTGA | 56 | ATM rev primer | GTAATTTACTACCTTTCGTTCTGTAGCTCT |
| 57 | ATM for primer | ATTTAAGTATTCAACGAGTTTCTGAAATTGCAT | 58 | ATM rev primer | GCTGATACATTTGGTTTTGCTATTCTCA |
| 59 | ATM for primer | TGCCATTCCAAGTGTCTTATTTTTGTT | 60 | ATM rev primer | CCATTAGATGAATCTTTCACTGTATCCATGA |
| 61 | ATM for primer | CTGATTGTAGCAACATACTACTCAAAGACA | 62 | ATM rev primer | CTTTGGTGAAGTTTCATTTCATGAGGAA |
| 63 | ATM for primer | AGTGGCTAGAATAATTCATGCTGTTACC | 64 | ATM rev primer | ACAAAAGAAAAAGAGATTAGATTACCTCGCA |
| 65 | ATM for primer | TGTTTCTTCACAGACAAGAAAAGAGCT | 66 | ATM rev primer | GCAAAGTGGGAAGAATTTCATCTCCTA |
| 67 | ATM for primer | GGTGCTTATGAATCAACAAAATGGAGAA | 68 | ATM rev primer | CAATCAAATTTTCTTTGACGGCAATATTACG |
| 69 | ATM for primer | TTTACAGGTTTTTAATGAAGATACCAGATCCT | 70 | ATM rev primer | GTGATCTTTTATTACTTCCCAGCCTAGT |
| 71 | ATM for primer | CACCTTCAGAAGTCACAGAATGATTTTG | 72 | ATM rev primer | CCACTGAAAGTTTTCTGAAAAATAACATACA |
| 73 | ATM for primer | ACCCAATTAATATCAAAGTATCCTGCAAGT | 74 | ATM rev primer | GCAACTTCCGTAAGGCATCGT |
| 75 | ATM for primer | GTGTCAAGACAAGAGGTCAAACCTA | 76 | ATM rev primer | TGGCTCCAAGTAAGCCAAAGTT |
| 77 | ATM for primer | CTGATAGATAAAGTCTTTGCCCCTC | 78 | ATM rev primer | GCTCTATTCCCATTTTTACCGTTCCT |
| 79 | ATM for primer | TAAAGTATTCTTTACATGGCTTTTGGTCTT | 80 | ATM rev primer | GAGACTCACAAGAATTTTCTCCAGTACA |
| 81 | ATM for primer | AAGGCAAAGCATTAGGTACTTGGT | 82 | ATM rev primer | AGTCCATCTTGTCAAAAGTTGTCTGAA |
| 83 | ATM for primer | ATTGTGAGAGAATGTGGTATAGAAAAGCA | 84 | ATM rev primer | AATCTCACCTCAGATGAGTAATTATTCAGAAG |
| 85 | ATM for primer | CTTTAAGATTACAAATTCAGAAACTCTTGTCC | 86 | ATM rev primer | CCTTGGCTTTCTGGAATAATTCTGACTTA |
| 87 | ATM for primer | TAATTTTAACTGGAATTTGCATTTTTCCTTCTA | 88 | ATM rev primer | CTGCATCATATTTCTCAAGGAACCAATT |
| 89 | ATM for primer | ACTGTCTGCCAAGAATAATTGTTTTTATTT | 90 | ATM rev primer | CTGCAATGTCATTCATTAGCTTTGATGT |
| 91 | ATM for primer | AAATTTTGACTACAGCATGCTCCT | 92 | ATM rev primer | GTATCATCTTCCATTGATTCTACTTCTCCAC |
| 93 | ATM for primer | AATCTAATGGAGGTGGAGGATCAGT | 94 | ATM rev primer | TCCCAAGTAGTAAATATGTATTTACCTATGGT |
| 95 | ATM for primer | GGTGCCATTAATCCTTTAGCTGAAGA | 96 | ATM rev primer | CCTCCGAATATCAGCTGCCC |
| 97 | ATM for primer | GTTAATTGATTCTAGCACGCTAGAACCT | 98 | ATM rev primer | CAACTCATTACATTTAGTCAGCAACATC |
| 99 | ATM for primer | ACTGATGTGTTCTGTTAAGCTTATAAAGTT | 100 | ATM rev primer | GGTTTTTCACTACATGAAGGACATGGT |
| 101 | ATM for primer | AGCAATATGGACTCTGAGAACACAAG | 102 | ATM rev primer | ACAAAACTTGCATTCGTATCCACA |
| 103 | ATM for primer | CAGGCATCTAACAAAGGAGAGGAAATA | 104 | ATM rev primer | GACTGCTTTAAAATTTTCAATGGAGATCTT |
| 105 | ATM for primer | AATGGGCCATTCTTAATGTAATGGGA | 106 | ATM rev primer | TTTGACCCATTACCTATTGATTGACTCTG |
| 107 | ATM for primer | AGCTTCAGCAAACAGCTTTTGAAA | 108 | ATM rev primer | TGAAGTAATTTATGGGATATTCATAGCAAGC |
| 109 | ATM for primer | CTGAGAACCCTGAAACTTTGGATG | 110 | ATM rev primer | TCCATTCTCTTTCACAGATTTACACAGG |
| 111 | ATM for primer | CATTTTTCTTAACACATTGACTTTTTGGTTCG | 112 | ATM rev primer | TGAAGATTTAGCCATTCCAAAACCAGAT |
| 113 | ATM for primer | CAGATTCAAGAGGACTGGAAAAGTCT | 114 | ATM rev primer | TCATAGACCTTGGTAGCAGTCTCTC |
| 115 | ATM for primer | AGGTAATATATGCCTTTTGAGCTGTCTTG | 116 | ATM rev primer | ACGTCATCAATAACTCCACCACAAT |
| 117 | ATM for primer | GACGTTACATGAGCCAGCAAATT | 118 | ATM rev primer | CAACCAATAAACTTCTTTAAAAACTTGTGAAG |
| 119 | ATM for primer | CTGGTCTATGAACAAAACTTTTTAAAACGA | 120 | ATM rev primer | CAATTGCTGATATAGGCAAATGTTGCTT |
| 121 | ATM for primer | CCTATCAGAAAATTCTTCTTGCCATATGTGA | 122 | ATM rev primer | GCTCCTCCTAAGCCACTTTTTATATCT |
| 123 | ATM for primer | TTTCAGAGTAATTTTCCAGAACTTACTGGTT | 124 | ATM rev primer | GCTGTCTGGCAAACCTGACT |
| 125 | ATM for primer | atttaGGTATTGGACTTGTTGAAATACTTAGT | 126 | ATM rev primer | ACAACATGGTCAGGAAAAGGATCTAAAA |
| 127 | ATM for primer | AAGTTTTATTTCACAGGCTTAACCAATAC | 128 | ATM rev primer | GATCCTTTAGTCCTTCAAGTCTTGTCA |
| 129 | ATM for primer | TGGAACTACATAAAGATCAGATGGTGGA | 130 | ATM rev primer | TCTGTGACAATGAAACCAAGAGCAA |
| 131 | ATM for primer | TCCGCAAGATGGGATTATGGTG | 132 | ATM rev primer | cgcatgactgTAGTTTACCTAGAACT |
| 133 | ATM for primer | GGGAGAAGTGGGTCCTATAGATTTCT | 134 | ATM rev primer | GTGTGTTATTCAGGTAGGTCAGCA |
| 135 | ATM for primer | ACATTCATCAAGATTAATAACTGGTGTACT | 136 | ATM rev primer | ATCTCCCAGAAACTATGTCCAGTCT |
| 137 | ATM for primer | GGGAGATTTATAAGATGACAACAGATCCAA | 138 | ATM rev primer | CCCACAGCAAACAGAACTGTTTTA |
| 139 | ATM for primer | GGTACAATGATTTCCACTTCTCTTATTTACAT | 140 | ATM rev primer | GATTTTCACTTAGAGGAATCCACAGATTTATA |
| 141 | ATM for primer | AGACTGTACTTCCATACTTGATTCATGATATT | 142 | ATM rev primer | GGTTGTGGATCGGCTCGT |
| 143 | ATM for primer | aAGAAGGAAGAAGGTGTGTAAGCAA | 144 | ATM rev primer | ACAACAGCAAGCATTGTTCTTTGT |
| 145 | ATM for primer | TGACATTATATCTCATTTTTCTTTAGACCTTCT | 146 | ATM rev primer | AAAGTGAGCAGCACAAGACTGA |
| 147 | ATM for primer | ATTCTGTTTATGAAGGAGTTATGTGTGT | 148 | ATM rev primer | GGGATTGCTGTTTCGAGGT |
| 149 | ATM for primer | CTCTGGTTTTCTGTTGATATCTTTGATTACT | 150 | ATM rev primer | GACACCAGTCTTTATTTTCATAATCCAATCC |
| 151 | ATM for primer | AGAAGAACTTCATTACCAAGCAGCA | 152 | ATM rev primer | AGAAAAACAGTTGTTGTTTAGAATGAGGA |
| 153 | ATM for primer | AATGCTCTACAATCTCTAAGAGACAGAGAA | 154 | ATM rev primer | CAAGTTTTCAGAAAAGAAGCCATGAC |
| 155 | ATM for primer | TCTGTGTATTCGCTCTATCCCACA | 156 | ATM rev primer | TCACTATTGGTAACAGAAAAGCTGCA |
| 157 | ATM for primer | GCAGAAACACTCCCAGCTTC | 158 | ATM rev primer | ATGTCCTTAATACATTCTCTTTGTGAGTTGT |
| 159 | ATM for primer | GGACATTCTCACCAAACACCTTGT | 160 | ATM rev primer | AAATAATAAAAGGAAAGTCAAGAGGTAAGATGA |
| 161 | ATM for primer | ATTAAACAGTACAATTCAGTTAGCTGTGGA | 162 | ATM rev primer | GCACAGCTGGCATCCAACT |
| 163 | ATM for primer | CATACACAGAATGTCTGAGGGTTTGT | 164 | ATM rev primer | TCATGCTAAGTAACTATCTTAAGGGTTGC |
| 165 | ATM for primer | GAGCTAAGAAATGGAAAAATGAAGGCA | 166 | ATM rev primer | ACTTCCTCTTTGGCTCTTTTCAGG |
| 167 | ATM for primer | TGTGTGATTTTGTAGTTCTGTTAAAGTTCA | 168 | ATM rev primer | TGCACGCAGGGCTAATTCA |
| 169 | ATM for primer | CGCTTCTTATGTAAAGCAGTTGAAAATTAT | 170 | ATM rev primer | CTTGCCTTCATCATGCCATTGAC |
| 171 | ATM for primer | TGCATAAATCTAATAGTTCTTTTCTTACAGCTAACAGCTA | 172 | ATM rev primer | TCTGGCTACCTCTGGTTTAGTCA |
| 173 | ATM for primer | TCTAACTCTGAGAAGTTTAAATGTTGGG | 174 | ATM rev primer | CAACACTTCTGACCATCTGAGGT |
| 175 | ATM for primer | TGTTGAGGCACTTTGTGATGCTTA | 176 | ATM rev primer | AACTAATATATCTCTACAGAGAGTAACACAGC |
| 177 | ATM for primer | TTAAATACAGAAGGCATAAATATTCCAGCAGA | 178 | ATM rev primer | TCAAGAGTTAATTGCAAATTACCTTAATTTCCA |
| 179 | ATM for primer | AAATCTGGTGACTATACAGTCATTTAAAGCA | 180 | ATM rev primer | AGAGAAGGGAAGGCTCACCTTA |
| 181 | ATM for primer | GCAACAGGTCTTCCAGATGTGT | 182 | ATM rev primer | ccAAATGGCATCTTTTATATGTTTTTGGT |
| 183 | ATM for primer | TCTTTTGAAGAGAAATATGAAGTCTTCATGGA | 184 | ATM rev primer | ACACTGCGCGTATAAGCCAAT |
| 185 | ATM for primer | GGCTATATATTAGAAAGAGATGGAATCAGTGAT | 186 | ATM rev primer | GTTCTGCTGACTGCTCATTTATCAAG |
| 187 | ATM for primer | ATTCTAACTGGAAAGAAAGTAAATTAGCTGT | 188 | ATM rev primer | CACAATATCTCTGGTGAGTCTAAAAGGA |
| 189 | ATM for primer | AGATAAAGATACGTTGACAACATTGGT | 190 | ATM rev primer | ACTTTACCTCTACAATGGTTAACAGABTTTC |
| 191 | ATM for primer | TTTTAATACATATGTTCTCTCTGTTTAAGGTCC | 192 | ATM rev primer | TCAGAGTAGGGTGAAGCTCAGTTT |
| 193 | ATM for primer | CTTATTCCCAAGGCCTTTAAACTGT | 194 | ATM rev primer | CTTCTTCCACTCCTTTCAGTTTCTCT |
| 195 | ATM for primer | CTGTGCTCAGTGTTGGTGGA | 196 | ATM rev primer | AGGCTGAATGAAAGGGTAATTCATATACTG |
| 197 | ATM for primer | CATTAAAAGAGGTGTTCTTGTGACAAACA | 198 | ATM rev primer | GAGCCTCTAA TATTTACCTGTAAACTTATTCC |
| 199 | ATM for primer | TTTGTTGTTTCCATGTTTTCAGGATCTTC | 200 | ATM rev primer | TACCGTTGTTTAGAAAAATGCAATTTACCT |
| 201 | CDKN2A for primer | TCCTCATTCCTCTTCCTTGGCT | 202 | CDKN2A rev primer | GGAATTGGAATCAGGTAGCGCTT |
| 203 | CDKN2A for primer | CGGCCTCCGACCGTAACTA | 204 | CDKN2A rev primer | ATGGAGCCTTCGGCTGAC |
| 205 | CDKN2A for primer | GGCAGTTGTGGCCCTGTAG | 206 | CDKN2A rev primer | CAGGTTTCTAACGCTGTTTTCTTT |
| 207 | CDKN2A for primer | CCAAACAAAACAAGTGCCGAATG | 208 | CDKN2A rev primer | CCCTCGTGCTGATGCTACT |
| 209 | CDKN2A for primer | GAATCCGGAGGGTCACCAA | 210 | CDKN2A rev primer | TGGGTCCCAGTCTGCAGTTA |
| 211 | CTNNB1 for primer | CATTTCCAATCTACTAATGCTAATACTGTTTC | 212 | CTNNB1 rev primer | AGAATGGATTCCAGAGTCCAGGTA |
| 213 | CTNNB1 for primer | AGAGGGTACGAGCTGCTATGT | 214 | CTNNB1 rev primer | GTTTACAACTGCATGTTTCAGCATCT |
| 215 | CTNNB1 for primer | ACTATCAAGATGATGCAGAACTTGCC | 216 | CTNNB1 rev primer | ACTACTCCCCTTGAGCATTTACT |
| 217 | CTNNB1 for primer | CAGTTATGGTCCATCAGCTTTCTAAAAA | 218 | CTNNB1 rev primer | CAAGGTCCCAGCGGTACAA |
| 219 | CTNNB1 for primer | AGTATACTCACAATATTTCTGATGAGGCT | 220 | CTNNB1 rev primer | CACTGCCATTTTAGCTCCTTCTTG |
| 221 | CTNNB1 for primer | GCCTTGCTCAACAAAACAAATGTTAAA | 222 | CTNNB1 rev primer | CACTGGAGGTGTCCAATGCT |
| 223 | CTNNB1 for primer | GTGGTGGACCCCAAGCTTTA | 224 | CTNNB1 rev primer | CAGCTTCTACAATAGCCGGCTT |
| 225 | CTNNB1 for primer | CTGACAGATCCAAGTCAACGTCT | 226 | CTNNB1 rev primer | CAGTACAAGCACATACTCATCTTGACTC |
| 227 | CTNNB1 for primer | CTTGTTCAGCTTCTGGGTTCAGA | 228 | CTNNB1 rev primer | CAGTACGCACAAGAGCCTCTA |
| 229 | CTNNB1 for primer | CTGCCATCTGTGCTCTTCGT | 230 | CTNNB1 rev primer | CTTTATCAGAGGCCAGTGGGA |
| 231 | CTNNB1 for primer | GTAAGAAAATGATTTTGTTGAGTTGTATGCC | 232 | CTNNB1 rev primer | GTGCATGATTTGCGGGACAA |
| 233 | CTNNB1 for primer | CAGTTGCTTGTTCGTGCACAT | 234 | CTNNB1 rev primer | GATCCTTTGGATTTATGCATTCCTTTTAG |
| 235 | CTNNB1 for primer | TTGTACCGGAGCCCTTCAC | 236 | CTNNB1 rev primer | ΓΓΓΓATGGACATAAAACCTAGAACACΠCA |
| 237 | CTNNB1 for primer | TGAAAACATCCAAAGAGTAGCTGCA | 238 | CTNNB1 rev primer | ACTTACCCACACCTTCATTCCTAGA |
| 239 | CTNNB1 for primer | AAGGAACCAAAGCCTTTAGCAGAT | 240 | CTNNB1 rev primer | TTGTAATCTTGTGGCTTGTCCTCA |
| 241 | CTNNB1 for primer | AACAGAGCCAATGGCTTGGA | 242 | CTNNB1 rev primer | GCCAGGGAAACATCAATGCAAATG |
| 243 | CTNNB1 for primer | TCTCTCCTCTCTCTTTTGCCTT | 244 | CTNNB1 rev primer | GGCATCCTGGCCATATCCAC |
| 245 | CTNNB1 for primer | CCACCCTGGTGCTGACTATC | 246 | CTNNB1 rev primer | TCTTACCTAAAGGATGATTTACAGGTCAGTA |
| 247 | EGFR for primer | AGTTTTTCTGCATTTCTCAGTATTTCATGT | 248 | EGFR rev primer | GTTATTGAACATCCTCTGGAGGCT |
| 249 | EGFR for primer | GACCTTGAGTTCTTGAGTTCCTCA | 250 | EGFR rev primer | ATGATCTGCAGGTTTTCCAAAGGA |
| 251 | EGFR for primer | CCTATGCCTTAGCAGTCTTATCTAACTATGAT | 252 | EGFR rev primer | AGCCATCGGAACTGCTGTC |
| 253 | EGFR for primer | CGTGCGGTTCAGCAACAAC | 254 | EGFR rev primer | GTGTGTATGCGACACTTACAGC |
| 255 | EGFR for primer | CCTGGGAAATGATCCTACCCTCA | 256 | EGFR rev primer | ACTGGTTGTGGCAGCAGTC |
| 257 | EGFR for primer | GTGTGGCGCTGAGTGTACTTA | 258 | EGFR rev primer | GCAGGTGTCCTTGCACGT |
| 259 | EGFR for primer | CATGCTCTACAACCCCACCA | 260 | EGFR rev primer | GACAAGGATGCCTGACCAGTTA |
| 261 | EGFR for primer | CCCCAGGTAATTATGTGGTGACAG | 262 | EGFR rev primer | GGCGGGCTTCCTACCTTTG |
| 263 | EGFR for primer | AATAGGTATTGGTGAATTTAAAGACTCACTCT | 264 | EGFR rev primer | AAGCAACTGAACCTGTGACTCA |
| 265 | EGFR for primer | CTCTGGATCCACAGGAACTGG | 266 | EGFR rev primer | CAAGGGAACAGGAAATATGTCGAAAAG |
| 267 | EGFR for primer | CAGGGATACATTGTTTTTATAATTTTTCACCA | 268 | EGFR rev primer | CGTAATCCCAAGGATGTTATGTTCAG |
| 269 | EGFR for primer | CAGGTCTGCCATGCCTTGT | 270 | EGFR rev primer | AAAGAAATAACCTCCTACCCCTCCA |
| 271 | EGFR for primer | AGGGAGTTTGTGGAGAACTCTGA | 272 | EGFR rev primer | GTGAAGGCCCTTCCCATGA |
| 273 | EGFR for primer | TCCCCAGGCCTCTCACATA | 274 | EGFR rev primer | GGGACACGCTGCCATCATTA |
| 275 | EGFR for primer | CTTAGGATGGATCCCTTCTCTTCTG | 276 | EGFR rev primer | AGACACGTGAAGGCATGAGG |
| 277 | EGFR for primer | TGCCAAAGAAGTAGAATGAGAAAAATGTAT | 278 | EGFR rev primer | CTTGACGAGGTCCATGTGACA |
| 279 | EGFR for primer | TCCACCTCATTCCAGGCCTA | 280 | EGFR rev primer | GTGCGCTTCCGAACGAT |
| 281 | EGFR for primer | GCCTCTTACACCCAGTGGAGA | 282 | EGFR rev primer | CCTGTGCCAGGGACCTTAC |
| 283 | EGFR for primer | TCCCAGAAGGTGAGAAAGTTAAAATTCC | 284 | EGFR rev primer | GCCTGAGGTTCAGAGCCATG |
| 285 | EGFR for primer | AGCTCATCACGCAGCTCAT | 2B6 | EGFR rev primer | CCCGTATCTCCCTTCCCTGAT |
| 287 | EGFR for primer | GAGGACCGTCGCTTGGT | 288 | EGFR rev primer | TGCCTCCTTCTGCATGGTATTC |
| 289 | EGFR for primer | AGGGATTGTGATTGTTCATTCATGAT | 290 | EGFR rev primer | AGGATGGAGGAGATCTCGCT |
| 291 | EGFR for primer | GCCATCTTTATCATTTCTTCCAGTGT | 292 | EGFR rev primer | GGTCTCGGGCCATTTTGGAG |
| 293 | EGFR for primer | GCCAAGAAGTGGAATAGCATCT | 294 | EGFR rev primer | CGTCCATGTCTTCTTCA |
| 295 | EGFR for primer | ATGGACGACGTGGTGGATG | 296 | EGFR rev primer | AGTAGACACAGCTTGAGAGAGAGAG |
| 297 | EGFR for primer | gccctgACCGGAGTAACC | 298 | EGFR rev primer | GGAGGAAGGTGTCGTCTATGC |
| 299 | EGFR for primer | cactttcagaATACATAAACCAGTCCGT | 300 | EGFR rev primer | GGGTCCTGGTAGTGTGGGT |
| 301 | ERBB2 for primer | GCAGGGAAACCTGGAACTCAC | 302 | ERBB2 rev primer | TCTTCTGCCACCCACCTGTA |
| 303 | ERBB2 for primer | GGCTACGTGCTCATCGCT | 304 | ERBB2 rev primer | GTGGTATTGTTCAGCGGGTCT |
| 305 | ERBB2 for primer | GGGAAAGGGTCCTCTGATCAT | 306 | ERBB2 rev primer | TGAGAGCCAGCTGGTTGTTC |
| 307 | ERBB2 for primer | ACCCGTCCTCTCGCTGTTA | 308 | ERBB2 rev primer | CCTGAGACTCACGGCTCTGA |
| 309 | ERBB2 for primer | CCTTTGCCCAACAGTGACG | 310 | ERBB2 rev primer | CACATACCAGGCAGTCAGAGT |
| 311 | ERBB2 for primer | CCTGCCTCCACTTCAACCAC | 312 | ERBB2 rev primer | AGCTGGCGCCGAATGTATA |
| 313 | ERBB2 for primer | GCTCATGGTGGTGCACGAA | 314 | ERBB2 rev primer | GTTCCATCCTCTGCTGTCACC |
| 315 | ERBB2 for primer | TTGACCTGTCCCGGTATGAA | 316 | ERBB2 rev primer | CAGCCAGCAAACTCCTGGATA |
| 317 | ERBB2 for primer | TGGGAGTGATGTCCACCCT | 318 | ERBB2 rev primer | CACCTGTGATCTCTTCCAGAGTC |
| 319 | ERBB2 for primer | CTGGGAGTCCTTGTCCTGTC | 320 | ERBB2 rev primer | GCAGAATTCGTCCCCGGATTA |
| 321 | ERBB2 for primer | GTGGTAAGGTGCCCACCTTT | 322 | ERBB2 rev primer | AGAGGTGGGTGTTATGGTGGA |
| 323 | ERBB2 for primer | GGGCCTCCCCTAAAAGTCC | 324 | ERBB2 rev primer | AGGAACTGGCTGCAGTTGAC |
| 325 | ERBB2 for primer | GCTCCCCAGGGAGTATGTGAA | 326 | ERBB2 rev primer | GCAGCTCACCGGTCCAAA |
| 327 | ERBB2 for primer | CCTGACCTCTCCTACATGCC | 328 | ERBB2 rev primer | AGACCCCTGAAAGGAAAGTCCT |
| 329 | ERBB2 for primer | CCTCCCACCCCAAACTAGC | 330 | ERBB2 rev primer | GTCGCTTGATGAGGATCCCAAA |
| 331 | ERBB2 for primer | TGGAGCCGCTGACACCTA | 332 | ERBB2 rev primer | CTGACCTTGTAGACTGTGCCAAA |
| 333 | ERBB2 for primer | GGTTTGTGATGOTTGGOAGG | 334 | ERBB2 rev primer | ACCAGCCATCACGTATGCTT |
| 335 | ERBB2 for primer | GCTCGGAACGTGCTGGTC | 336 | ERBB2 rev primer | TGCTCCTTGGTCCTTCACCTA |
| 337 | ERBB2 for primer | CTCCCCACAACACACAGTTG | 338 | ERBB2 rev primer | CCCCCATCACACACCATAACT |
| 339 | ERBB2 for primer | CTGTGTGGGAGCTGATGACT | 340 | ERBB2 rev primer | ATGATCATGTAGACATCAATGGTGCA |
| 341 | ERBB2 for primer | TCTGAATGTCGGCCAAGATTCC | 342 | ERBB2 rev primer | TCTGCAGGAGGGTGCTCTTA |
| 343 | ERBB2 for primer | CCCATCCCAGATCCGTGAGT | 344 | ERBB2 rev primer | CTCCTCTTCAGAGGGCTCCA |
| 345 | ERBB2 for primer | GGGCTGGCTCCGATGTATT | 346 | ERBB2 rev primer | GTCCTCACTGTACCGCTGT |
| 347 | ERBB2 for primer | CCTTCCCCTAATGGGTCACCT | 348 | ERBB2 rev primer | GTCTTGGGCCTTTCCAGAGT |
| 349 | ERBB2 for primer | GGGTCGTCAAAGACGTTTTTGC | 350 | ERBB2 rev primer | GTCCCAGTAATAGAGGTTGTCGAA |
| 351 | KRAS for primer | TTGTTACCTTTAAAAGACATCTGCTTTCT | 352 | KRAS rev primer | GAAAAGACTCCTGGCTGTGTGA |
| 353 | KRAS for primer | GACATAACAGTTATGATTTTGCAGAAAACA | 354 | KRAS rev primer | GACACAAAACAGGCTCAGGACTTA |
| 355 | KRAS for primer | GTCTACTGTTCTAGAAGGCAAATCACAT | 356 | KRAS rev primer | ATTTGTGTTACTAATGACTGTGCTATAACT |
| 357 | KRAS for primer | GTACTGGTCCCTCATTGCACT | 358 | KRAS rev primer | GCACTGTAATAATCCAGACTGTGTTTC |
| 359 | KRAS for primer | ACATAATTACACACTTTGTCTTTGACTTCT | 360 | KRAS rev primer | TACTTTTTATGTATTTCAGGGTGTTGATGA |
| 361 | PIK3CA for primer | GGTTTCTGCTTTGGGACAACC | 362 | PIK3CA rev primer | AGTGGATGCCCCACAGTTC |
| 363 | PIK3CA for primer | AAACATGTTCATGCTGTGTATGTAATAGA | 364 | PIK3CA rev primer | AGTCCTGTACTTCTGGATCTTTAACCA |
| 365 | PIK3CA for primer | TCCCTTGAAAAATGAAAGAGAGATGGT | 366 | PIK3CA rev primer | ACACAGTCATGGTTGATTTTCAGAGT |
| 367 | PIK3CA for primer | CCAGAACAAGTAATTGCTGAAGCAATC | 368 | PIK3CA rev primer | TCTAGGAAGTATTCATCACATCCACACA |
| 369 | PIK3CA for primer | GCCCAATTTGATGTTGATGGCT | 370 | PIK3CA rev primer | AAGGGATTTTGTAGATGTTTCTCCATTCA |
| 371 | PIK3CA for primer | CCTTTGGGTTATAAATAGTGCACTCAGA | 372 | PIK3CA rev primer | ATAAAATAATAAGCATCAGCATTTGACTTTACC |
| 373 | PIK3CA for primer | GTTCGAACAGGTATCTACCATGGA | 374 | PIK3CA rev primer | GGATCATACTGCTAAACACTAATATAACCTT |
| 375 | PIK3CA for primer | ATTCCTGATCTTCCTCGTGCTG | 376 | PIK3CA rev primer | TCAGCGGTATAATCAGGAGTTTTTAAAG |
| 377 | PIK3CA for primer | TTTGAATCTTTGGCCAGTACCTCAT | 378 | PIK3CA rev primer | ATAAGAGAGAAGGTTTGACTGCCA |
| 379 | PIK3CA for primer | GACTGGTTCAGCAGTGTGGTA | 380 | PIK3CA rev primer | TGATACTTAATAAACTCAGTGATTTGCCTT |
| 381 | PIK3CA for primer | GTCAACCTTTTGAACAGCATGCA | 382 | PIK3CA rev primer | CTGGGCTACTTCATCTCTAGAATTCC |
| 383 | PIK3CA for primer | CGGCCATGCAGAAACTGAC | 384 | PIK3CA rev primer | GCTTTCTTCAGTAAAAATCTCACAAGCA |
| 385 | PIK3CA for primer | TCGTGCATGTGGGATGTATTTGAA | 386 | PIK3CA rev primer | TCTCAAACACAAACTAGAGTCACACAC |
| 387 | PIK3CA for primer | GGTACAGATGAAGTTTTTAGTTGAGCAAAT | 388 | PIK3CA rev primer | CAATGAAACCCCCAAGAAAGTACCT |
| 389 | PIK3CA for primer | CACTGTGGTTGAATTGGGAGAAC | 390 | PIK3CA rev primer | ATAAAAAGATAGCTAAATTCATGCATC ATAAG C |
| 391 | PIK3CA for primer | TTCAGATTTACGGCAAGATATGCTAACA | 392 | PIK3CA rev primer | TAGTTCACTTTTTCAGGATAACTTTCAACA |
| 393 | PIK3CA for primer | GGTGGTGCGAAATTCTCACACT | 394 | PIK3CA rev primer | GAAAGAATAATACAACTCACATTTCTCCTTTG |
| 395 | PIK3CA for primer | TCTCAAGTTGGCCTGAATCAC | 396 | PIK3CA rev primer | CCAATTCCCAAAATGAAGGTAGCTACA |
| 397 | PIK3CA for primer | GAGATCGTCACAATAGTAACATCATGGT | 398 | PIK3CA rev primer | TACAAAATGTCTTGAATAAATTAAGAACACCAA |
| 399 | PIK3CA for primer | GGACACTTTTTGGATCACAAGAAGAAA | 400 | PIK3CA rev primer | GCTCACCTCTCAAATTCTCTTGTCT |
| 401 | PIK3CA for primer | AGACCTGAAGGTATTAACATCATTTGCT | 402 | PIK3CA rev primer | CATCATTGAGAAAAGATTTATGAAGAGATTGG |
| 403 | PIK3CA for primer | GATGCTTGGCTCTGGAATGC | 404 | PIK3CA rev primer | AGCCACCATGATGTGCATCATT |
| 405 | PTEN for primer | GCCATCTCTCTCCTCCTTTTTCTT | 406 | PTEN rev primer | GCCGCAGAAATGGATACAGGTC |
| 407 | PTEN for primer | TGCTGCATATTTCAGATATTTCTTTCCTTA | 408 | PTEN rev primer | GCATTCTTACCTTACTACATCATCAATATTGT |
| 409 | PTEN for primer | TCTTTTTACCACAGTTGCACAATATCCT | 410 | PTEN rev primer | GCTTTACAGTGAATTGCTGCAACA |
| 411 | PTEN for primer | GGATTATAGACCAGTGGCACTGTT | 412 | PTEN rev primer | CTAGATATGGTTAAGAAAACTGTTCCAATACA |
| 413 | PTEN for primer | ACCATGCAGATCCTCAGTTTGTG | 414 | PTEN rev primer | ACTTTGATATCACCACACACAGGTAAC |
| 415 | PTEN for primer | GCAAATAAAGACAAAGCCAACCGATA | 416 | PTEN rev primer | TTAAACACACATCACATACATACAAGTCA |
| 417 | PTEN for primer | AAATTTTCTTTCTCTAGGTGAAGCTGTACTT | 418 | PTEN rev primer | AGTGGTGTCAGAATATCTATAATGATCAGGTT |
| 419 | SMARCA4 for primer | CATGAGAAGGGCATGTCGGA | 420 | SMARCA4 rev primer | AGGAACGCAGTTCTGTACCTTG |
| 421 | SMARCA4 for primer | ACAGACATATGCTGCCGAGTG | 422 | SMARCA4 rev primer | CTGGCCCGGAAGACATCTG |
| 423 | SMARCA4 for primer | TCAGAGCTCAGATCATGGCCTA | 424 | SMARCA4 rev primer | GTGGAGGTAGCGTTGGCAT |
| 425 | SMARCA4 for primer | GTATGGGAGGGCCCAACATG | 426 | SMARCA4 rev primer | CGGGTGCACCACCATAGAA |
| 427 | SMARCA4 for primer | GCATCGCACACCGAATTCA | 428 | SMARCA4 rev primer | CACCTGCCTCTGGAAGTTCA |
| 429 | SMARCA4 for primer | GGCTTGTCCTCTTCCCTCCTA | 430 | SMARCA4 rev primer | GCTGCGCTTGTAGGCCTTA |
| 431 | SMARCA4 for primer | GCATGCCAAGGATTTCAAGGAAT | 432 | SMARCA4 rev primer | GCATGCGCTCCTTCTCGA |
| 433 | SMARCA4 for primer | GGTGCTCACAGACATGCACAT | 434 | SMARCA4 rev primer | GTCTGTCTGCTGCAAGAGGT |
| 435 | SMARCA4 for primer | CTGACCGTGTCTCTCTCTATTTCC | 436 | SMARCA4 rev primer | GACCAACGCCTTAGGGCTA |
| 437 | SMARCA4 for primer | CGGTGAAGGTGATCCACGT | 438 | SMARCA4 rev primer | GACTTGGATCCCCACTGCAT |
| 439 | SMARCA4 for primer | GGGATGAACTGAGGTGACATG | 440 | SMARCA4 rev primer | CGTCATCGCTGTCTGGATCT |
| 441 | SMARCA4 for primer | CAGCACATTGTCACAGATAGGAA | 442 | SMARCA4 rev primer | CCACGGCATAGTAGGACTGC |
| 443 | SMARCA4 for primer | GGGATGTGGCTTAGGCAGAA | 444 | SMARCA4 rev primer | CCAGCCACTCCAAACCTTTGA |
| 445 | SMARCA4 for primer | GCTCCAAAAGCCGAGCTGT | 446 | SMARCA4 rev primer | TGCTTGTCTTTGATGATGTACTCGT |
| 447 | SMARCA4 for primer | CCGCTGCAGAACAAGCTTC | 448 | SMARCA4 rev primer | ATGGCAAAGGGTGCGTTAAAC |
| 449 | SMARCA4 for primer | CCTCTGTCGCCCTCCTTTG | 450 | SMARCA4 rev primer | AGACGCCGGATGATGAGAATG |
| 451 | SMARCA4 for primer | CTGCAGGTGGAGTACGTCAT | 452 | SMARCA4 rev primer | GGCCCACCTTCTTGTCCTTC |
| 453 | SMARCA4 for primer | CCAAGACCCTGATGAACACCATC | 454 | SMARCA4 rev primer | GCCTAGGAGCTCAGCAGACA |
| 455 | SMARCA4 for primer | CCTGCAGGGTTCCAGGTTTA | 456 | SMARCA4 rev primer | TTGTGGTTGGTTGCTCGGA |
| 457 | SMARCA4 for primer | GTCCCCACTCTACCCCTGA | 458 | SMARCA4 rev primer | TGAGCAGGAAGATGAAGTACTCAGA |
| 459 | SMARCA4 for primer | GTCGGCAGACACTGTGATCAT | 460 | SMARCA4 rev primer | CCTCGAGGTTTTGCAGGCA |
| 461 | SMARCA4 for primer | GGCATGTTCGACCAGAAGTC | 462 | SMARCA4 rev primer | GCTCACGCGTCCACCATT |
| 463 | SMARCA4 for primer | GTTTGCAATCTTATAGGAGGAAGACGA | 464 | SMARCA4 rev primer | GCAGAGTGGGATGCCTGAAC |
| 465 | SMARCA4 for primer | CTCGTGGATCATCAAGGACGA | 466 | SMARCA4 rev primer | CCTTGAGCCACTGCTTCTCC |
| 467 | SMARCA4 for primer | GGGTCCTGAGGTAAGACCTG | 468 | SMARCA4 rev primer | CACAGGAGCATGACGTCCTT |
| 469 | SMARCA4 for primer | TGCCCAGATCTATGAAGACTCCA | 470 | SMARCA4 rev primer | ATTCGGATTCGGAGCcttcc |
| 471 | SMARCA4 for primer | CCAGCTCGGTCCGTCAAA | 472 | SMARCA4 rev primer | TCCTCCTCACTGTCATCGTCA |
| 473 | SMARCA4 for primer | CGCACACTCTCTCCTCCTGT | 474 | SMARCA4 rev primer | CTGCTAAGGCCTATGCCATCTC |
| 475 | TP53 for primer | GGCTCCTGACCTGGAGTCTT | 476 | TP53 rev primer | GGGCCTGTGTTATCTCCTAGGT |
| 477 | TP53 for primer | ACCTATTGCAAGCAAGGGTTCAA | 478 | TP53 rev primer | GTTCAAGACAGAAGGGCCTGA |
| 479 | TP53 for primer | CTTCCAATGGATCCACTCACAGT | 480 | TP53 rev primer | CCACTTTTCCTCTTGCAGCAG |
| 481 | CNTNAP5 for primer | CGGCTGGCTACTGCGAATT | 482 | CNTNAP5 rev primer | CAGTTTGTCGCTGTTAATCCTAAATGC |
| 483 | CNTNAP5 for primer | ATTTCTCAACTTCTCTTCTTATGGAATGT | 484 | CNTNAP5 rev primer | CTGAGGAACTGGAAAAAGCCATTG |
| 485 | CNTNAP5 for primer | GTTCAGTGACACAGGACGCA | 486 | CNTNAP5 rev primer | TTGGAAAATGACGGAAATATACTCTGGTT |
| 487 | CNTNAP5 for primer | TTCGTGGAGTAACAAATATGGTTTTCC | 488 | CNTNAP5 rev primer | ACCACGCTGTCAGCATTCA |
| 489 | CNTNAP5 for primer | TTTGTGCCCCTGGAATGGAAT | 490 | CNTNAP5 rev primer | GTTACGTAGGCGGTGCCA |
| 491 | CNTNAP5 for primer | CCCAGAATCAGATGTTGCTGACT | 492 | CNTNAP5 rev primer | CCATGGAACAGGACCCCATC |
| 493 | CNTNAP5 for primer | CACATCACCTTGGAACTCCAGAA | 494 | CNTNAP5 rev primer | ACTGCAAGTGTCAGACACTG |
| 495 | CNTNAP5 for primer | CCTCCTGGATGACCAGCAC | 496 | CNTNAP5 rev primer | GTCAATGTCTAAGGCATCCGTCT |
| 497 | CNTNAP5 for primer | CCAGGTGGTTGTTTATATCAGCTGT | 498 | CNTNAP5 rev primer | TTGACAAATGTGATGGGCACAATC |
| 499 | CNTNAP5 for primer | CCCCAAATTGATGGGCTCTCA | 500 | CNTNAP5 rev primer | TGAATCACGAGTCTCAGGATTCCA |
| 501 | CNTNAP5 for primer | GGGAAATGAGCCCCCTCTCT | 502 | CNTNAP5 rev primer | GGTTCCTCCTGGCGTTGATG |
| 503 | CNTNAP5 for primer | GGAACCGCATCACGCTCA | 504 | CNTNAP5 rev primer | TCCCTCTTAAAGACTGGAGAATTTACCT |
| 505 | CNTNAP5 for primer | GGATATGAGACATTTTTGCTTTCCCCTT | 506 | CNTNAP5 rev primer | GGAGCAGCTTCCTCCATGTT |
| 507 | CNTNAP5 for primer | CCAGTCCTGGACTACCTTCTATTGTA | 508 | CNTNAP5 rev primer | CATCCTGAAGTTAACAATGGTGTTCC |
| 509 | CNTNAP5 for primer | AGAGGACAAGATCTGGACATCAGT | 510 | CNTNAP5 rev primer | GGCCTCCAGCTGTTCCATG |
| 511 | CNTNAP5 for primer | CCTACCACTGCAGGAGGTC | 512 | CNTNAP5 rev primer | AAATTTTCCTTTGCCTAATACTCCAGGT |
| 513 | CNTNAP5 for primer | ACCATTTACCTGGTGGATTGGG | 514 | CNTNAP5 rev primer | TCAGCGTCGCAATTGCAAA |
| 515 | CNTNAP5 for primer | CACTGATACCGACAGATCAAACTCA | 516 | CNTNAP5 rev primer | AGGGATAAGGGAGTAAGAACATCTTGT |
| 517 | CNTNAP5 for primer | GAAAATTATCCACATGTCCAGTGCT | 518 | CNTNAP5 rev primer | AGGTAGGAAAGTGGAGGTAAGAGG |
| 519 | CNTNAP5 for primer | CCTACCTTCCATGCGGAATTCAG | 520 | CNTNAP5 rev primer | TTCATAATTGAGGGCACTTACAGCTT |
| 521 | CNTNAP5 for primer | CCTTTGCCATCGATGTTGGG | 522 | CNTNAP5 rev primer | CCTTGGAAGGTTGTCCACCTG |
| 523 | CNTNAP5 for primer | CTTACCCATGCCAACATTAAACCT | 524 | CNTNAP5 rev primer | TCAAGTGTAAGGAGCGAATGCA |
| 525 | CNTNAP5 for primer | cgAGAAAGCAGCTAAGGTTAGCTT | 526 | CNTNAP5 rev primer | ATGTAAGTAACCGACGTGCCA |
| 527 | CNTNAP5 for primer | ACCTAAACAAGGAAGAAACCCATGT | 528 | CNTNAP5 rev primer | GGCTGAAGCAGGAGAAAGTAAGG |
| 529 | CNTNAP5 for primer | ACTACTCAAAGAGGACCCAGCATA | 530 | CNTNAP5 rev primer | CCATGGCATTTGCTTTAGCAACT |
| 531 | CNTNAP5 for primer | CAGGTGGGCCACACTGTAG | 532 | CNTNAP5 rev primer | ACTGCCGAATCACTTCGAACA |
| 533 | CNTNAP5 for primer | CTCATCTGGAGGGAATCCACT | 534 | CNTNAP5 rev primer | GGAACCGGGTCATGATGCC |
| 535 | CSMD3 for primer | GTGACCCAGCAAGTCCTGA | 536 | CSMD3 rev primer | ACTTGAACACGGTTTGCACAATG |
| 537 | CSMD3 for primer | TCCACTGACTTTGCGTTGGT | 538 | CSMD3 rev primer | ACACATAGCTAATCAATGTTCTCATCAACA |
| 539 | CSMD3 for primer | GATGTCTGGGATAATAACAGAAGTTCGT | 540 | CSMD3 rev primer | GGTTTCTGCTGAGCCTGATGG |
| 541 | CSMD3 for primer | tgtcTGGAGCAAAGGAAAGCAATA | 542 | CSMD3 rev primer | GCAGGTGGAACAGAACATAGAGT |
| 543 | CSMD3 for primer | GCCAGGCTGACAGGTATAAATCA | 544 | CSMD3 rev primer | TTTACATTGTTCTCTTTTCTTZZTATAGCCCAC |
| 545 | CSMD3 for primer | CCATAATACTTACGTATGCATTCAGGCT | 546 | CSMD3 rev primer | TGCTATAGGTAGGAAGTGTTGTACAGT |
| 547 | CSMD3 for primer | TATGCATATGAACCAAAGACGCA | 548 | CSMD3 rev primer | CCTACCCAAACCTCTTGTGAAAAC |
| 549 | CSMD3 for primer | AAATGTCACCCCTATTAATTACACAGTT | 550 | CSMD3 rev primer | TCCTTTCATATTAGTGGGATCAAGCAC |
| 551 | CSMD3 for primer | CTTTTGCCTTCTCTTTTTCCTTGGG | 552 | CSMD3 rev primer | TTGATTGTCACCATTTTTCCAAACTG |
| 553 | CSMD3 for primer | ACTTCACCACTCCAGGTACCA | 554 | CSMD3 rev primer | CCCCAGATCTCTAATGGAAGGC |
| 555 | CSMD3 for primer | atatGTACTGGTTACCATTTTTAAATGTTGAAG | 556 | CSMD3 rev primer | TCAGGACTTGTACAATTAATGGCACAT |
| 557 | CSMD3 for primer | GGCTGGCACATGTAAGAAACATT | 558 | CSMD3 rev primer | ACATGCCTCATTAACCATCTGCTC |
| 559 | CSMD3 for primer | CTTGTACAGTTAGGTAAAGTTCCAGACC | 560 | CSMD3 rev primer | AGTCTTCCGAATTGATGGCACA |
| 561 | CSMD3 for primer | GATTTAGATACACACTAAAATCCAGTAGTTGC | 562 | CSMD3 rev primer | GGTTACATCCTTCATGGCTCAGAA |
| 563 | CSMD3 for primer | AAATTGCTTTCCTGTCTAGAGCCAT | 564 | CSMD3 rev primer | GGCCCTTGGACTAACTGGTAAA |
| 565 | CSMD3 for primer | GTTGTGATCCACTCCAATGGC | 566 | CSMD3 rev primer | TCCTCCTAATGCAGTCCTGTCT |
| 567 | CSMD3 for primer | GTTTCCATGACATGTTCTCAAGAAACA | 568 | CSMD3 rev primer | TGGATCTTCAGTTTTGATATGTCAACCA |
| 569 | CSMD3 for primer | AGCTGTTTTAAGTGTGTCACTGATGT | 570 | CSMD3 rev primer | CCAACAGACAGTGGAGCCATC |
| 571 | CSMD3 for primer | CACACAGGATGGGAGCTGAC | 572 | CSMD3 rev primer | ATGGTCAAGTCATTGGAGAAAATTATGGA |
| 573 | CSMD3 for primer | GAGATGCCTCTTTTCAAGGGTGA | 574 | CSMD3 rev primer | GCTTGTGGGCTCTGCTGTA |
| 575 | CSMD3 for primer | TGAGCCATATGAAGTTTGAGTTCCAA | 576 | CSMD3 rev primer | AGCACAGTTTTGTTACAAGTGCAG |
| 577 | CSMD3 for primer | TCTCCAACTCCAAGTACCATTAGGA | 578 | CSMD3 rev primer | TGGAACATGGAAGATGGCGAA |
| 579 | CSMD3 for primer | AAAATACCAAGAGAAAAACAAGTGATGAAA | 580 | CSMD3 rev primer | TCGATTGTCATCCAAAGAACTCACTAC |
| 581 | CSMD3forprimer | GCGTTCCTACCAAATAGTCTGTTGTTA | 582 | CSMD3 rev primer | ACATTTTTCTGTTTATAGCTTGTGT |
| 583 | CSMD3 for primer | GCATGATACCCATAGGAAGACTTTCT | 584 | CSMD3 rev primer | AATCCCCACCTCATGGATATATTATCAGT |
| 585 | CSMD3 for primer | TTGATGAATATCCAATAAATATAACCCTTGGTT | 586 | CSMD3 rev primer | TGTGTGCATGGAGCATTTCAGT |
| 587 | CSMD3 for primer | TGTCAGGATATCCAGGGCTCAA | 588 | CSMD3 rev primer | ATACAGTTTTCACTGAGGCTCTAAAAG |
| 589 | CSMD3 for primer | GTTCTCCTAATCTGCACGTCAGA | 590 | CSMD3 rev primer | TTTGTTTATTTTTATCATCACCTTGTTGCT |
| 591 | CSMD3 for primer | CATCATCTTCCGTCAAAATTTCAGCAT | 592 | CSMD3 rev primer | TGCATATGTGAATACATGTAGCTGCA |
| 593 | CSMD3 for primer | CCAACCGTGATAACTGAGCACA | 594 | CSMD3 rev primer | ACCAAAATTCACCTCAGATCGGT |
| 595 | CSMD3 for primer | TCCAACCTTGTTTTACTGTGTATATAATTGA | 596 | CSMD3 rev primer | CCCCTGGGAATGGCATCTAC |
| 597 | CSMD3 for primer | GGGTACTCTTACAAGCCAAAAACAAT | 598 | CSMD3 rev primer | TTTGTTTTGTGACTTTTTCCGTAGCTC |
| 599 | CSMD3 for primer | GGAAGACAAGTAAAAAGTGCCAAAAC | 600 | CSMD3 rev primer | TCACATGCCTTCACGGAGTC |
| 601 | CSMD3 for primer | TGTGTATCCTGGGAAACATTCAAATGA | 602 | CSMD3 rev primer | TCTTTCTTTTTCCTCTTTCCAGCCT |
| 603 | CSMD3 for primer | GCTGAATAAGGAAGATGGTATTTGAGGA | 604 | CSMD3 rev primer | CTTCAGGTGTACATCTCCAGTTTGT |
| 605 | CSMD3 for primer | TGTCCATGTGCAATCTAAACTGCT | 606 | CSMD3 rev primer | TCCTCTACTCTGGTTTACTTTGGATTTCT |
| 607 | CSMD3 for primer | GGCCATGAAAATCAAAACCACAAC | 608 | CSMD3 rev primer | TCCTGAACCACAAACTCCTAGC |
| 609 | CSMD3 for primer | ATCTGTATTTTAATTAAGCTCAGCTCTCTT | 610 | CSMD3 rev primer | CACAGGTGCAAGTTGTTAGCTTT |
| 611 | CSMD3 for primer | CTTCCACACACAATTCAGATTGTTGT | 612 | CSMD3 rev primer | TTGCATTAGAATTAACTCCCTCTCTTGG |
| 613 | CSMD3 for primer | TGGATCCATGGAGAATATATCCTGGATT | 614 | CSMD3 rev primer | GAACAAGTTCTACACAATGCAGTTCT |
| 615 | CSMD3 for primer | AGCTGTTATTGGTCCAACTGAAGTAAAT | 616 | CSMD3 rev primer | TATGATATCTTAGTGTTTGTTTTGTTGAGTAGG |
| 617 | CSMD3 for primer | AATGGGATCCTGAGAGGGAAGA | 618 | CSMD3 rev primer | GTTTGCTTCTCCGGGTTCCT |
| 619 | CSMD3 for primer | TGATACAGGTGAGTGTTGAATAACCTTG | 620 | CSMD3 rev primer | ACTGCGAGAGTCCTGCTTTG |
| 621 | CSMD3 for primer | ATGGGCCATACCAAATTATTCTCC | 622 | CSMD3 rev primer | TTGGCTTTTCGGAGTGATGGAT |
| 623 | CSMD3 for primer | GTACAAATGCTAAAGCAAAATGGCCTA | 624 | CSMD3 rev primer | CATAGCAGAGACTGTGACTGGA |
| 625 | CSMD3 for primer | GCTGGGCTGCCAGAAGTAC | 626 | CSMD3 rev primer | GGGACACTGTTGTTTTTCAATGTGA |
| 627 | CSMD3 for primer | CATCACCTTCCTATTTTGCATTGAAGAA | 628 | CSMD3 rev primer | CTGAAGGAATTCATAGCACCCTCA |
| 629 | CSMD3 for primer | ACCTGACAATATTTCCAGGATCTACCT | 630 | CSMD3 rev primer | CAGCTGAATGTGGAGGTCGT |
| 631 | CSMD3 for primer | CCTGTCATGCACTTGAGAAGG | 632 | CSMD3 rev primer | CACACTGTGAAGATCCTGGCAT |
| 633 | CSMD3 for primer | TTATTTAATATACATGCAGCACCCCAGT | 634 | CSMD3 rev primer | GAATTTAATTCCGATACTGAAGGGACAGA |
| 635 | CSMD3 for primer | AAGTGTCAGTCCGCGCAT | 636 | CSMD3 rev primer | ACATGCTCTGAATTGATTATTATTGGCA |
| 637 | CSMD3 for primer | CTTGTGCTAAATGAAATGTTCTGGCT | 638 | CSMD3 rev primer | TGCAACGAATAATGAAGGAATTTTGCT |
| 639 | CSMD3 for primer | ACGAAGGGTGTAACCATGCAA | 640 | CSMD3 rev primer | CAGAGATCATCTGTCTTGGTGGTG |
| 641 | CSMD3 for primer | TCTCTGATGTTCCTTCCAGTCGAT | 642 | CSMD3 rev primer | AGAATATAACCTTGAACCTTGTGAAGATCC |
| 643 | CSMD3 for primer | GCCCTGAAATTTCCATAGAGACCA | 644 | CSMD3 rev primer | TCCACACTTTTCATTTGGAAGACCA |
| 645 | CSMD3 for primer | ACTACATTGATTTTTCTGTTTATAAATCAGCAT | 646 | CSMD3 rev primer | GAAGAGCCCCTTCTATGCGAAA |
| 647 | CSMD3 for primer | GGCAAACAGAATGAGGAAATCAAAAAGTT | 648 | CSMD3 rev primer | GTGCCCCAGTTTCTATTTAGTAGCA |
| 649 | CSMD3 for primer | AAATGTAATTTTGATAGAGTGTCCAGGTTCA | 650 | CSMD3 rev primer | TCCCAGTGGAGTGATTCTCTCA |
| 651 | CSMD3 for primer | TTGTGGTTTCTTGTACATAATGATGATGA | 652 | CSMD3 rev primer | CCCAGGGAACAGAAACAATTACATGTAT |
| 653 | CSMD3 for primer | ATGTTAAAGCCACGTCCTGACA | 654 | CSMD3 rev primer | GATGGTGACTCTCCAGAATCCC |
| 655 | CSMD3 for primer | TCCAGGTCAAAGTCATTGAAAGAAAGA | 656 | CSMD3 rev primer | TCTAACTTTACTGCACCAATGGGAA |
| 657 | CSMD3 for primer | AGATGGGTATGTTTGCAGACCATT | 658 | CSMD3 rev primer | CTTATATGGAATTAGAGAAGGCGATGGA |
| 659 | CSMD3 for primer | ATCATTACCTTTGTAGTTAACCTTGAAACCA | 660 | CSMD3 rev primer | GCTGACTGGAAGCTTTGTACCA |
| 661 | CSMD3 for primer | AAAATTTTATTAGTACCCGATAGGCTAAGG | 662 | CSMD3 rev primer | TCTGGAGATTGGCTATGATACCTTGA |
| 663 | CSMD3 for primer | CTGCATGTTCCGTATTATTTCTTTGC | 664 | CSMD3 rev primer | GACAGCAATGCACAATGTGTCT |
| 665 | CSMD3 for primer | GTTAAACTATTAAAATGTGTTCAGAGGGCAT | 666 | CSMD3 rev primer | CCTGTCAACGGATAGCTGAAGTTTT |
| 667 | CSMD3 for primer | ACACTCACAGATAGAAAACTTGTTGCT | 668 | CSMD3 rev primer | TCCTGATACATGTTTTTGTTTGTTTTCTTAACT |
| 669 | CSMD3 for primer | AGTAACAACTGCAAGCTCTTTAGTTCT | 670 | CSMD3 rev primer | CGAGTCTCAGAAATTCAGGTCTGG |
| 671 | CSMD3 for primer | GGGAGCACTAAATCCACGGTAT | 672 | CSMD3 rev primer | TGTTGTCTTTTGTAATTCAGGTTATCTGGA |
| 673 | CSMD3 for primer | TGGTAGGTGGCTCAGAACCT | 674 | CSMD3 rev primer | TGAGTACCATAACAATGCTGATTGCA |
| 675 | CSMD3 for primer | GCTGGATATGATGCCACTGGAT | 676 | CSMD3 rev primer | TTGCTTTACTTACAACTGAACAATTGCA |
| 677 | CSMD3 for primer | AAATCCCACGAAGCTGTATTAACTGAA | 678 | CSMD3 rev primer | CCACCCAAAGGTGTATTATATGGCA |
| 679 | CSMD3 for primer | CATGATGTTGTCTCAAATGTTGATTCTT | 680 | CSMD3 rev primer | TGCAGTTAGTGCTCATGGATTTAAGG |
| 681 | CSMD3 for primer | CTAAAGTTTGTAGGATGAGGATGTCCAT | 682 | CSMD3 rev primer | CCAAATGGTGCAAACTGCACA |
| 683 | CSMD3 for primer | GAGGCTGTCTGTCGGTCAA | 684 | CSMD3 rev primer | TCTTCCAGATCATCCGGGAAAG |
| 685 | CSMD3 for primer | CAGACCTACCTTTCACACAAGACA | 686 | CSMD3 rev primer | CTGGCAAGCGAAGATGCG |
| 687 | CSMD3 for primer | GGCTTTCCCCTTTGCGGAT | 688 | CSMD3 rev primer | TGCTGTGGTATCCCAGAAACAC |
| 689 | LRP1 B for primer | TACTGTTGGAACATACAAAAGTAATCCTTATAT | 690 | LRF1B rev primer | GTGTTGATGAAAGGAAAGAACTGCTT |
| 691 | LRP1B for primer | TGGGTCTATCATAAAGCCAGGATCTAAA | 692 | LRP1B rev primer | CAATGGAGGAATAAATGTAGAAATTGGCAA |
| 693 | LRP1B for primer | ACTGACTGCTAATCTAGAACATTGCAT | 694 | LRP1B rev primer | tcatttatttTCCAGGAAGCATTGCC |
| 695 | LRF1B for primer | TGATATGATCAGACTTGCTGCTCTTTG | 696 | LRP1B rev primer | CTACATTAGCTGACTTCTGGGCTT |
| 697 | LRF1B for primer | AGATGCCCATGACTTGATTAAATACTCAC | 698 | LRP1B rev primer | GCAGCTGCACTAATGGAAAGA |
| 699 | LRF1B for primer | ggaaggagtgGATACAGGAAATCAG | 700 | LRP1B rev primer | CCACTGCCAGCCGGAATA |
| 701 | LRF1B for primer | GGCAGTAAGGCTGGTTTCCA | 702 | LRP1B rev primer | CTTATTGTTTCCAATAGGGAGACCCA |
| 703 | LRP1 B for primer | CCTCCATTCTGGCAGTAGTTGC | 704 | LRP1B rev primer | AGTTAACTTGTGAAAATGGAGGAAGAT |
| 705 | LRP1B for primer | CCCACCTAACAGGCTGTCAT | 706 | LRP1B rev primer | TCACCAGTACCCAATCCATGC |
| 707 | LRF1B for primer | GTACTCTACTGAACCATGGCCAAAT | 708 | LRP1B rev primer | TTTCATAACTGCCTTTTTAAAATTCCAGGTT |
| 709 | LRP1B for primer | CCTTGTTTGCTGCTGACAGAG | 710 | LRP1B rev primer | CTTGTGTGTGTTGATTTTGCAGGT |
| 711 | LRF1B for primer | ATCATACCTGTGGGTCTCTGTAAGT | 712 | LRP1B rev primer | CCTCTCTGACCCGCTCTCTT |
| 713 | LRP1B for primer | GCCACCCAGTCAACTGCAA | 714 | LRP1B rev primer | CAGAAAACTCCGCTAATTTCCTGT |
| 715 | LRP1B for primer | AAAATTCCGCCTGGATTAAACTGAGTA | 716 | LRP1B rev primer | TCCATTCAACTACAGTGGCGATC |
| 717 | LRP1B for primer | ACACACACATTTATATGATCCTTCCACAT | 718 | LRP1B rev primer | GTGGTAAGAATAGGAGTCTGAATTGAGG |
| 719 | LRP1B for primer | TCCAGAACATTTTAGTCATGTACAAGCA | 720 | LRP1B rev primer | CTCCAGTGTGATCGACTTGATGA |
| 721 | LRF1B for primer | ACTGGAGATCCATAGGGATGCATT | 722 | LRP1B rev primer | CAATAAGAGTGTTTTGATTCTGTGAAGCA |
| 723 | LRF1B for primer | CCACCACAGTGATCTTCATCTGA | 724 | LRP1B rev primer | TCTTCAGTCAAATTTCTTTGTCCATCCA |
| 725 | LRF1B for primer | CGTGAAATGCCATACCACACATATC | 726 | LRP1B rev primer | AGGAGGAAATATATGTAGAGCTGATGAGTT |
| 727 | LRF1B for primer | AGCATACACTGTATATTCTGCACAGAAC | 728 | LRP1B rev primer | GGCTGTGTGATGGAATTCATGAC |
| 729 | LRP1B for primer | GTGGGCTTTATTTTTGCACCGA | 730 | LRP1B rev primer | TTACAAGCTGCATGTTTATTTAGCC |
| 731 | LRF1B for primer | ttaCCTCATCTGAACCATCAGCAC | 732 | LRP1B rev primer | ATTGTATTCAGCTTCTCCTACTTGCTC |
| 733 | LRF1B for primer | CTTTTACCTGGCTCACAGCTTTTC | 734 | LRP1B rev primer | AGAGAAATTGTGAGACAAGTTGTTCCAA |
| 735 | LRP1 B for primer | AATTACAAATGTGGTTCTAAAGCGCTG | 736 | LRP1B rev primer | TTGGTGTGATGGAGATTTTGACTGT |
| 737 | LRF1B for primer | TTTCTTCATCTGAATTATCACTGCAGTCA | 738 | LRP1B rev primer | TGCAAACTCTGGTTCTTTCCATTACA |
| 739 | LRP1B for primer | AAATTTGTTTAACTGTGGTTGTCGCA | 740 | LRP1B rev primer | GGGTTTGTGACGAGGATCCA |
| 741 | LRP1B for primer | GGAGTCAAAGTTAAGACAAGACAGAGT | 742 | LRP1B rev primer | CAAATGTACCAAGAACCAGAAATGTATCC |
| 743 | LRF1B for primer | AGTTCATCAGAATTGTCTCCACAATCA | 744 | LRF1B rev primer | TCCACAATATCTATGCTTTCTCCCCTA |
| 745 | LRF1B for primer | TTAGGTGTAGATTCAATTTTCCAGGTC | 746 | LRF1B rev primer | TGGAAATGTGACACCGTGGAT |
| 747 | LRF1B for primer | TCAATGTTATGCTAAACAAAAGAGCTGATG | 748 | LRF1B rev primer | CACTAACTTCTATCTGGCAGCTGAT |
| 749 | LRP1B for primer | GACATGCACAAGTGTGGGTTT | 750 | LRP1B rev primer | GTCTTGATTATCCTGTTTTGTTGTTGTTGT |
| 751 | LRF1B for primer | GTCTGTCTGCTCCCGATGT | 752 | LRF1B rev primer | CTTACCATCTCCTATTACAGTCCCTAATCA |
| 753 | LRF1B for primer | TTTATGTTACTGGACCAACCTTTGTGT | 754 | LRF1B rev primer | GAAACCAAGATTTCTAGACCTATGGCA |
| 755 | LRF1B for primer | ACAACACTCTGATTGGTTCCATCC | 756 | LRP1B rev primer | TGTTTGTTGTTTTGCTCCCAGA |
| 757 | LRF1B for primer | ACTTCAGCCTTTTGCTAACGAGT | 758 | LRF1B rev primer | ACACAGCGGTCCCCAATG |
| 759 | LRF1B for primer | ATCATACTGGTATAATATTCAACAATCCCATCA | 760 | LRF1B rev primer | GATCGATTCTAGCCGACCCAAT |
| 761 | LRF1B for primer | ATTGCCAGGTAGTTCTACAGCTC | 762 | LRF1B rev primer | GAGATAAGGAAAATTAGCACTGATGGCT |
| 763 | LRF1B for primer | AATAAGAACGGCTATAAAATTCAGGATCCA | 764 | LRF1B rev primer | CTGTACAGATGGGTATGAAATACAACCT |
| 765 | LRF1B for primer | GTATTGATGCATTGCTGGCTACAG | 766 | LRF1B rev primer | TTGATTATGCTCATTTTTCTTTTCCCAGTG |
| 767 | LRP1B for primer | CCGGAAGGTCTTGACAGTCTTG | 768 | LRP1B rev primer | CAGGTGCATTCCCAGTGGA |
| 769 | LRF1B for primer | GCACCTGCCATTTTTGCACATAAA | 770 | LRF1B rev primer | CTGTAGTATAAAACAATGGCATGCCC |
| 771 | LRF1B for primer | TGGGTTTAAAGGTGCTTCATCAGAAT | 772 | LRP1B rev primer | TTCTGCGTCTCTAGGATATCGACA |
| 773 | LRF1B for primer | ACTAGTΓTΓGAAAGACTCTΓAGCCTAGGA | 774 | LRP1B rev primer | TGCATTCCCAAGCAATTTGTTTGT |
| 775 | LRP1B for primer | CATCGCTTCCATCTGGACAGT | 776 | LRP1B rev primer | AATGGCATTATGTTTTTGTGTTTCAGGT |
| 777 | LRF1B for primer | AGGTTAACAAAAACTAAGCTAAATACAATGTCT | 778 | LRF1B rev primer | ATTTTGAATACCTGGATATGCGATGGT |
| 779 | LRP1B for primer | aaaattCACTCTATACAACCAGGTAGCC | 780 | LRP1B rev primer | GGATATGCGACGGGTCTAATGA |
| 781 | LRF1B for primer | CAGTAAAATCTTGCACAGTGTTCAAT | 782 | LRF1B rev primer | GATCAGCACGATGCAACCAG |
| 783 | LRP1B for primer | AGACAAGCAAACCAAAAACTGTAAAGAATC | 784 | LRF1B rev primer | AGCTCACCTGTGATGGCATT |
| 785 | LRF1B for primer | CACAATTTCAGTGATGTGTGTGGAA | 786 | LRF1B rev primer | CCAATGGGAGAGTGAATTGTTCCT |
| 787 | LRF1B for primer | TTGGGAGTTAAAAGGCACAAGTCA | 788 | LRF1B rev primer | TCTAACATAGAACAGGAATTCGATATAAAATGT |
| 789 | LRP1B for primer | TTCTGACATGGTGATGACAGCTT | 790 | LRP1B rev primer | GTGGAAGGACTTGCCTATCACA |
| 791 | LRF1B for primer | TCTAGTAATCTAAATGATACTCATTTCCACCTA | 792 | LRF1B rev primer | GCTTATTAAAGACAACTGGGAAGACAGA |
| 793 | LRF1B for primer | TGTATTCTTTCAAGAGTGCTGACCTTT | 794 | LRF1B rev primer | ACAAGAATGATGCCACAGAAACGATA |
| 795 | LRP1B for primer | CCTGTCAGACCAGTAGATGTAAGC | 796 | LRF1B rev primer | GGTGTGATGCTCGCACAGA |
| 797 | LRF1B for primer | AATACTGAAAAGAAATCCAACAGACCTCA | 798 | LRF1B rev primer | GGGACAAATGCCCTGTATTGGAA |
| 799 | LRP1B for primer | CTTTCTCTGGGTGCACAGCTA | 800 | LRP1B rev primer | CCTAGGTAACATATATTGGACAGATCATGGT |
| 801 | LRF1B for primer | TTTTATGAAGCAGTTTGAGCCCCTA | 802 | LRF1B rev primer | GAAGATATCATTACCAATGGCTTGGGA |
| 803 | LRP1B for primer | CTTCTTTCCAAGTCTGATCTCTTTTAGCT | 804 | LRF1B rev primer | CCGATAΠGATACCTTTTACAGTGTGT |
| 805 | LRF1B for primer | TTGGTTGCCTAACATCTGCAT | 806 | LRP1B rev primer | AGGACTTGTATGTGTACAGTGGGA |
| 807 | LRP1B for primer | AAGTCCTTGTAGITTCAGATGTTGGT | 808 | LRF1B rev primer | ATTTGTTTCATTΓΓCATTCTATGCAGGCA |
| 809 | LRF1B for primer | GGTTAGGGCTGTAGCTTTTGTTAATTC | 810 | LRP1B rev primer | TATCGATAGACTATGTGGAAAACAAGC |
| 811 | LRF1B for primer | GGTATATTGTACAAGTACAGGTTTGTCACA | 812 | LRF1B rev primer | GGATGGCAGTAATAGCAAGATTCTGTT |
| 813 | LRP1B for primer | ACTGGGTGAGCTGCAAGAC | 814 | LRP1B rev primer | GATTGGGTGTCACGTAATTTATACTGGA |
| 815 | LRP1B for primer | AAACACTTTTAAAGTTGTAACATGTACGAATCT | 816 | LRF1B rev primer | CGATGCATCTGAGGAACGTTTATACTG |
| 817 | LRP1B for primer | GATTAGAACTGTAAGAAATTTACCATAGCAGGT | 818 | LRF1B rev primer | CTGCTCTCACATGTGTCTAATCAATCA |
| 819 | LRP1B for primer | GGGCCTTTGCCATCATTAGC | 820 | LRP1B rev primer | TGAATAACCCCTGTCAACCTCAGA |
| 821 | LRF1B for primer | GTTTGTTCCATCATAGAGGGCTGAA | 822 | LRP1B rev primer | AGCTGAGTTACATGATTATGTCTCTGGA |
| 823 | LRF1B for primer | GTCTCTCCACACTATCCTTTTCTCA | 824 | LRF1B rev primer | GGGATGCAAATTTTCCTCGCA |
| 825 | LRP1B for primer | GTGTTCCATGGCTCCTGCTAT | 826 | LRP1B rev primer | GGCTACTCCAGAAGGCCTGA |
| 827 | LRF1B for primer | AAAGCTTTCCCCGGTATATTCTGT | 828 | LRF1B rev primer | ACTATAGTCTACTTGTTCCTGGATTGA |
| 829 | LRF1B for primer | AGTCTCTTTTGTGAAGATCAATCCTTCTG | 830 | LRF1B rev primer | ATCCCAAGCATTTCATTACCAGAGT |
| 831 | LRP1B for primer | GTGCTTGTGCTGCTCACATAC | 832 | LRP1B rev primer | GTTCTGTTGTTCCTGGAAGAGGA |
| 833 | LRF1B for primer | GCTACTATATTAAGTAACAGCAACTGAACT | 834 | LRF1B rev primer | GGATTGTCCTGATGGCTCTGAT |
| 835 | LRF1B for primer | CCATTGCAGAGTTTCTCTGGCT | 836 | LRP1B rev primer | GCATGGGTGTGTGATGGAGAT |
| 837 | LRF1B for primer | TGGTACCATTGCAACCTTTTTCA | 838 | LRF1B rev primer | GAGATTCATTCTCCTGCTGGTTGT |
| 839 | LRP1B for primer | GCATCTGCCACTGGAACATC | 840 | LRF1B rev primer | AATTTCTTCATGGΠCAGATTTCACTGC |
| 841 | LRF1B for primer | GCCAAGCCCCCGATCAAATTA | 842 | LRP1B rev primer | CTTTCACAGAATTCCCAACTTGTGAG |
| 843 | LRP1B for primer | TCACAACTTACCACAAGATGCCA | 844 | LRP1B rev primer | TGATCATCATAGCCAGAACATGCC |
| 845 | LRP1B for primer | GACTAACTTTGGAGGAGATTTACGTGT | 846 | LRP1B rev primer | GCCAGAATAATCGCTGCATCC |
| 847 | LRF1B for primer | TGTCACAAACTGCCTTGATAATTCA | 848 | LRF1B rev primer | GAAATGTGATGGCGACGATGAC |
| 849 | LRP1B for primer | CGAGCTTGGATACAGTGTCTGT | 850 | LRP1B rev primer | AATTTTAATGGACTGACTGTAGCTAGCA |
| 851 | LRF1B for primer | GTTGTCCCATTTTCATCCAAAAGTTGA | 852 | LRF1B rev primer | CACATTTGCTTTCTTCCTCTTGCA |
| 853 | LRF1B for primer | ACCTTGTTGCTTTCGTGGATCATAA | 854 | LRF1B rev primer | TGTTCTGGACTGATTATATGAATGGTTCC |
| 855 | LRF1B for primer | CATAATTTCCATGATGCGACAGTCC | 856 | LRP1B rev primer | GGTCAGTTCTGATTTTCACAATGTGT |
| 857 | LRF1B for primer | TATCCTTTTAAAGTGCTTCATCTCTGG | 858 | LRF1B rev primer | CTACTTTGCTGACACCACCAGT |
| 859 | LRP1B for primer | GGGTAGCTGCTGGCAAATGT | 860 | LRP1B rev primer | GGGCTGTTCACACATCTGTCT |
| 861 | LRP1B for primer | CCCCATTCAATCTACCATTAGGAAGG | 862 | LRF1B rev primer | CCGATTTAATGGGACTGATATTCACTCA |
| 863 | LRP1 B for primer | AAAGAATTTCTTACCCTGCTATTGGATCTAC | 864 | LRP1 B rev primer | GTCGGTGACCGGATCTTTGTT |
| 865 | LRF1B for primer | AAACACTTACTGTGGAAGGATTGAAGAA | 866 | LRF1B rev primer | GTTGGATTGAATCAAGAGAATCTTCAAATCA |
| 867 | LRP1B for primer | GTATGAATTTCATTTCCATTGACTGAGCTT | 868 | LRF1B rev primer | CCAGAACCTACAGATAGACCACCTA |
| 869 | LRF1B for primer | AGCCTTCCACACAACTGCAA | 870 | LRP1B rev primer | AAGTGCCCAAGATAAAGCTGCTTA |
| 871 | LRF1B for primer | CTTACCTTTACAGCTTCTCCCATCTT | 872 | LRP1B rev primer | TCTCTACAGAACTGTTATCCAATTGCC |
| 873 | LRF1B for primer | CAATGTACTCCTTCGTCATACCCA | 874 | LRF1B rev primer | TTTTCAGGTCCCGAGGAGGTA |
| 875 | LRP1B for primer | ACTGACATTTAGTTTCACATGTAAGGT | 876 | LRF1B rev primer | CGTGACTTGTGTCTCCCAGA |
| 877 | LRP1B for primer | GGGAGGTGTTCTCCTTACCT | 878 | LRF1B rev primer | GCGACCACAATGTCCGAGTTT |
| 879 | CNTN6 for primer | TCATGACAATTTTGTCTTTTTCAGACTCTTG | 880 | CNTN6 rev primer | CAGAATTACCAGTTTCCATAGCAACCT |
| 881 | CNTN6 for primer | TTCTTTTCTTTCCCAGGTGATGGT | 882 | CNTN6 rev primer | CCATTAGCAGCACAATTCAGGATG |
| 883 | CNTN6 for primer | AAGATTGATTGACTCTGTATATTATAAGCACTT | 884 | CNTN6 rev primer | CCATCCAACCTGTAGTGATAACTCATAG |
| 885 | CNTN6 for primer | CAAGAAGCACAGTATCTGTCCGA | 886 | CNTN6 rev primer | GTGCTCACATCACTCTCCAAG |
| 887 | CNTN6 for primer | AAACTTCTATTCTCCAGAAATGCTGCT | 888 | CNTN6 rev primer | GTTTTACAGATGAATCCTTTGCAGCTT |
| 889 | CNTN6 for primer | AGCATAATGTCTTGGATAACTGCCT | 890 | CNTN6 rev primer | AGCTTTACATTCCCAGAGCAAGTT |
| 891 | CNTN6 for primer | GTAAAGCTAGTGGAAAGCCAAACC | 892 | CNTN6 rev primer | AGTAGGTAAACTTTTAATATCAACATAGTTGCT |
| 893 | CNTN6 for primer | GGAGAGAATTCAAATAGAAAATGGGACACT | 894 | CNTN6 rev primer | CCTAAAACTCTCAATTCAGCATTTGCAT |
| 895 | CNTN6 for primer | CTCCAGATTTCTCCAAAAGTCCAGT | 896 | CNTN6 rev primer | GCTTTGTCTAAGGGTCTCCGT |
| 897 | CNTN6 for primer | GAGGATGGCAGCCTCAAGAT | 898 | CNTN6 rev primer | CTGCCGTGTTCTTTGCAGT |
| 899 | CNTN6 for primer | ACAGTTGGCGAGAGTATAGTGCTA | 900 | CNTN6 rev primer | CTCCTCCAATCCTTTCAAAATGAGC |
| 901 | CNTN6 for primer | AGTAGGACTTGTTATATGGGCTTACGT | 902 | CNTN6 rev primer | |
| 903 | CNTN6 for primer | GAATGTAAGTATCTCCAATGGAGTCATGAT | 904 | CNTN6 rev primer | GGGCCTGCTCTCCAACTTAG |
| 905 | CNTN6 for primer | GGCCCAGATAATAACAGTCCCATT | 906 | CNTN6 rev primer | GTGACCCAAAACACTTTTGTCACT |
| 907 | CNTN6 for primer | TCAATGGTAAGACATACAATGCAACAGT | 908 | CNTN6 rev primer | ACCTGATGCTTTAGTTCTTAACAATTCTGA |
| 909 | CNTN6 for primer | TCCTGATGAAGACGTACTTTAATAATTGCC | 910 | CNTN6 rev primer | GTAATGACGAGTTCAGACCGACTT |
| 911 | CNTN6 for primer | TTGTTCCATTTTCAATGAACCACTCT | 912 | CNTN6 rev primer | GGCAATAGCATTCCATGAAACCTC |
| 913 | CNTN6for primer | GGGAACAATAAATTGCTTGTTTTGGT | 914 | CNTN6 rev primer | AGCTTTCAGCCCCGTGATG |
| 915 | CNTN6 for primer | TTGCTTCCGTAAGAGCTTACAACA | 916 | CNTN6 rev primer | GCAAAGGAATAGGTTGCTCTTCA |
| 917 | CNTN6 for primer | GTTGTGTGAACCTTAGAAAAGCAAC | 918 | CNTN6 rev primer | GGAACCAGAAGCTCAGCTGAT |
| 919 | CNTN6 for primer | GAAATAAGAACAGTCAGTGATGGTGGA | 920 | CNTN6 rev primer | TCATGCTGCAGCTGTCTAGG |
| 921 | CDH11 for primer | GCCTGTTTTAAATGAGCCTTTCCTT | 922 | CDH11 rev primer | CCAAAGACACTTTTGATGACGATTCTT |
| 923 | CDH11 for primer | AGGTCCCCAGTTCTGTAGATAATCA | 924 | CDH11 rev primer | CCCCACGGCTCCTCCTTAT |
| 925 | CDH11 for primer | GGCCGGCGTTCAGAATGTA | 926 | CDH11 rev primer | CCTTCTGCCCATAGTGATCAGC |
| 927 | CDH11 for primer | AAAACACACTCTATTTCCAAGTGCA | 928 | CDH11 rev primer | ACGATAATGCTCCCAAGTTTGCT |
| 929 | CDH11 for primer | TCTGCTGCAAAGACAGTGATGT | 930 | CDH11 rev primer | CTGCTTAGGTATTCCATCGATCGT |
| 931 | CDH11 for primer | GTCTGTACCTTATCGGGCTGT | 932 | CDH11 rev primer | AGATGCTGATGAGCCCCCTA |
| 933 | CDH11 for primer | CTTGAAAGGGCCATTGCTGAT | 934 | CDH11 rev primer | TCTTTCAAGGAATTGGTTGTCAATCTTCT |
| 935 | CDH11 for primer | GCCTCCCTGTTTCTTGCAGT | 936 | CDH11 rev primer | GGAATCGTTTGAAATCACAACGGA |
| 937 | CDH11 for primer | CGATTCCATACCATCTCCATCAACAA | 938 | CDH11 rev primer | CTAGGCGTATACCAGATGTCTGTG |
| 939 | CDH11 for primer | AAAAGTGAAAGCCAAGTCCACCTA | 940 | CDH11 rev primer | TGATCCAGGCCAAGGACATG |
| 941 | CDH11 for primer | CCTCCCTGTCCATGTTGGGTA | 942 | CDH11 rev primer | TGCTTCCTCAGTGCTCAGAA |
| 943 | CDH11 for primer | CAGTACCTGTCTGTGCTTCCA | 944 | CDH11 rev primer | TTTCACTTTTAAGGAACGTCAGTAATCCA |
| 945 | CDH11 for primer | GGTTGTCATTAATGTCCTGGACCT | 946 | CDH11 rev primer | CATTCATGCCACCAAGACGTTG |
| 947 | CDH11 for primer | GCTCCTTCCCCTGAGAGAATGT | 948 | CDH11 rev primer | TTGTTGCAATCACTTGTAAGTGAGTTTC |
| 949 | CDH11 for primer | GGAAAGCTCAGGATTGAGGGAA | 950 | CDH11 rev primer | GGTGCTACAGCGCTCCAA |
| 951 | CDH11 for primer | TTCTCATGGTGCCCATGGAAG | 952 | CDH11 rev primer | CCTACCACGTAACCAAAAATGAAGGA |
| 953 | AR for primer | GAAGATTCAGCCAAGCTCAAGGA | 954 | AR rev primer | CCCGGGTTCTGGATCACTT |
| 955 | AR for primer | ATGAGGAACAGCAACCTTCACA | 956 | AR rev primer | TCATCCTCGTCCGGAGGT |
| 957 | AR for primer | CCCACTTTCCCCGGCTTAA | 958 | AR rev primer | CTGCTGCTGCCTTCGGATA |
| 959 | AR for primer | CCCACTTCCTCCAAGGACAATTA | 960 | AR rev primer | CGAAGCTGTTCCCCTGGAC |
| 961 | AR for primer | CCGAAGAAGGCCAGTTGTATG | 962 | AR rev primer | CCGAGTGTAGCCGTAGGG |
| 963 | AR for primer | CGACTTCACCGCACCTGA | 964 | AR rev primer | CTTACCGCATGTCCCCGTA |
| 965 | AR for primer | CACTTGCCTATTTCTGCCATTCA | 966 | AR rev primer | TCTCCACAGATCAGGCAGGT |
| 967 | AR for primer | GAGATGAAGCTTCTGGGTGTCA | 968 | AR rev primer | TGTCTCTCTCTGGAAGGTAAAGG |
| 969 | AR for primer | CTGTGCGCCAGCAGAAATG | 970 | AR rev primer | agaaagaaaagTATCTTACCTCCCAGAGT |
| 971 | AR for primer | CCAATAGCCCGGAAGCTGA | 972 | AR rev primer | GGGCTGACATTCATAGCCTTCAA |
| 973 | AR for primer | AGACTTAGCTCAACCCGTCA | 974 | AR rev primer | ATGAGCCCCATCCAGGAGTA |
| 975 | AR for primer | GCTGGCGATCCTTCACCAA | 976 | AR rev primer | GTCTGGCCAAGCTGCTGTAT |
| 977 | AR for primer | CCAGTGGATGGGCTGAAAAATC | 978 | AR rev primer | AGGAGCTTGGTGAGCTGGTA |
| 979 | AR for primer | GAGGCCACCTCCTTGTCAA | 980 | AR rev primer | CGCTCACCATGTGTGACTTGAT |
| 981 | AR for primer | GCCCAAGATCCTTTCTGGGA | 982 | AR rev primer | TGCAGAGGAGTAGTGCAGAGTTA |
| 983 | ELTD1 for primer | TGCAGTTGTAATTATCCACCATTCTCT | 984 | ELTD1 rev primer | ATTGTTATGATTAACATTTCTTCCTTTCCTTTT |
| 985 | ELTD1 for primer | AGTGAACTTAAAGACGCATTTACCTTATGT | 986 | ELTD1 rev primer | TCCAGGTTAATCTCTTGGCTTTTGG |
| 987 | ELTD1 for primer | TTTAAGCACCCTATGATCATCACTTTT | 988 | ELTD1 rev primer | CAGATGTTGGCTTAGCACCGA |
| **989** | ELTD1 for primer | CCTAGTGCTGCCGAAAATCCAA | 990 | ELTD1 rev primer | GCATGGATGTGCATTGAAGGC |
| 991 | ELTD1 for primer | ACATTTAAGAAAGTACACATATCATAGGACAA | 992 | ELTD1 rev primer | GCTGTAGCCTATTTCTTGCTGAACT |
| 993 | ELTD1 for primer | GGGAAAATTATCTTACAATGGAAGGACCA | 994 | ELTD1 rev primer | AGAGGGCTGTGAGCTGACATA |
| 995 | ELTD1 for primer | ACCAGAAAAACAGCTTACCTTTCGAT | 996 | ELTD1 rev primer | TGATAATTCTGAAGAGGAGGAAAGAGTCA |
| 997 | ELTD1 for primer | TCCTACCATTTGAATCATATGCAGCTT | 998 | ELTD1 rev primer | CATGGTTTTGTTTCAGCTCTCAAAGT |
| 999 | ELTD1 for primer | GGTCTTTTGGAAGCTCTGGGATA | 1000 | ELTD1 rev primer | CCGTGAATAATTTTGTTCAAAGGGATACA |
| 1001 | ELTD1 for primer | TGGTTTGAATATGTTTAACCTCTGGAGGTA | 1002 | ELTD1 rev primer | CTTCAGATCCAGCAGTAACCAAGA |
| 1003 | ELTD1 for primer | CTGAAGCCAGGTACACACATACAATAAT | 1004 | ELTD1 rev primer | TCTTTTAAATTCATATGCCAACAGATGATAATG |
| 1005 | ELTD1 for primer | CACAAATTGTGACACCATTTCCTGAA | 1006 | ELTD1 rev primer | TTCCACTTTGTTGAATTGTTCCTATACTCA |
| 1007 | DPP10 for primer | CATGCACTGAATCATTCCTTTATGAATAC | 1008 | DPP10 rev primer | AGCAATAGCAATTCCCTTCCAGTT |
| 1009 | DPP10 for primer | TGGTGATTTTAGTTGTATGCTCACTCAT | 1010 | DPP10 rev primer | TGCTACCCTCTTAAGGCATTTGA |
| 1011 | DPP10 for primer | AAATTCGTCAGAAACCAGATTGTCTTTG | 1012 | DPP10 rev primer | AGAACTCAAAATGAATACAATGTTTAAAAAGGT |
| 1013 | DPP10 for primer | AATTGTGAATGTCTTTGTTGTTGCAGAT | 1014 | DPP10 rev primer | GTTGTGTTTTCCAATAATAATGTGGTAGCA |
| 1015 | DPP10 for primer | AAAGCATCAAGACATTCAGTTTCACC | 1016 | DPP10 rev primer | TGAGTAAAATTATGCATTGTCACCCCAA |
| 1017 | DPP10 for primer | GGAAGTTTGGGAGTTAAATCCTCCA | 1018 | DPP10 rev primer | ACATCTTCCATAAAACCATGATTGGCA |
| 1019 | DPP10 for primer | AGTTTCAAAAGGCATCCTAACGTGT | 1020 | DPP10 rev primer | TTTTCCAGAAGATGTCAGTCGCA |
| 1021 | DPP10 for primer | CCAATATATTCTAATGCTTAATGTACTTGCAAT | 1022 | DPP10 rev primer | GTGAGTTGGTCCATACAGGTTTACAA |
| 1023 | DPP10 for primer | GCTCATTTTGGTCTTTAGATGCTTCAT | 1024 | DPP10 rev primer | GAACAGGATGGTCACAATGGC |
| 1025 | DPP10 for primer | GTATTTTAGAATGAGGAGCCCGTGT | 1026 | DPP10 rev primer | AAAAAGCCAGCACTTACCTGGA |
| 1027 | DPP10 for primer | CAAGCAGTTGGTACATTCGTGT | 1028 | DPP10 rev primer | CTTTATCACTTCCCAGTTTCCTGATGT |
| 1029 | DPP10 for primer | ACACCACACATTCAAGTGCCT | 1030 | DPP10 rev primer | TGAAATTACATGAAATGCATTGGCGAT |
| 1031 | DPP10 for primer | ACATATTTTGATGCCAGTTTTAGTCCCA | 1032 | DPP10 rev primer | TTGCTGATACCAACAGATATTGACCAA |
| 1033 | DPP10 for primer | TCCCAGTGGTCAGCCTACA | 1034 | DPP10 rev primer | GATATGACTCAGAATAGATAACACGATGTC |
| 1035 | DPP10 for primer | ACACTGATTCTGTTTAATGCATAGGG | 1036 | DPP10 rev primer | CTGGCTTTCCTATCTTCTTCTTCAGG |
| 1037 | DPP10 for primer | CAGCTGGTTACAGATAAGTTCCATATTGA | 1038 | DPP10 rev primer | CCTAATCTTCGATGAATCTCCTGCAA |
| 1039 | DPP10 for primer | GCAGATTTTTGCTGAAACTGCCT | 1040 | DPP10 rev primer | TGGCCTCCTGTGAATGTGTA |
| 1041 | DPP10 for primer | GCTACATAATGTTCATGGCTTGAAAGAA | 1042 | DPP10 rev primer | CTGTAAGCAAGTCCTTAGATGTATCCC |
| 1043 | DPP10 for primer | ACTCAGCAGAATTAATCAAGCACCT | 1044 | DPP10 rev primer | AATAATAGAGAAGCTGTGTTGCGT |
| 1045 | DPP10 for primer | CGGACTCTGCGGGAAGTTA | 1046 | DPP10 rev primer | GGGCACCTTCAGCCTTTAC |
| 1047 | COL11A1 for primer | GGTGGCACCAAGGTATATTTTCTGT | 1048 | COL11A1 rev primer | TGATGTCATGATCAATGACTTTGGTGA |
| 1049 | COL11 A1 for primer | TCTGGCTAAGGACCGACTGA | 1050 | COL11A1 rev primer | ATGAGGAGATGTCCTATGACAATAATCCT |
| 1051 | COL11A1 for primer | AATCAGAATGTCACTTCTTGAGTTCGT | 1052 | COL11A1 rev primer | TTTGCAACAGGTAAGAATTTCATCATGG |
| 1053 | COL11A1 for primer | GTTCAATATTGCAAAACATGGCAGGA | 1054 | COL11A1 rev primer | CTGTAATTTCACATCTGGTGGTGAGA |
| 1055 | COL11A1 for primer | ACTAGAATGAATGAGCTGCCAATG | 1056 | COL11A1 rev primer | ACCAATCCAGCCCGAACTTG |
| 1057 | COL11A1 for primer | ATTGTCAAAGGGAAAAGTACTTACAGTAGAG | 1058 | COL11A1 rev primer | ATATAACAGAGACACAAATGCTCCAGAT |
| 1059 | COL11A1 for primer | AAGGTTATTTTGTCTTGTACTTACTGGTAA | 1060 | COL11A1 rev primer | CCCTTTAAATTGGCTGATTCTCTCTCT |
| 1061 | COL11A1 for primer | TCCCCTTTTGCTCCTGGAGA | 1062 | COL11A1 rev primer | CTGCCTTTCAAAGGGACATCCT |
| 1063 | COL11A1 for primer | CCTTTTCACCCTTGGAGCCA | 1064 | COL11A1 rev primer | AGGTCTGTGACTAGTTATAGGATTCTC |
| 1065 | COL11A1 for primer | CCCCGAAGACCTTCTGGAC | 1066 | COL11A1 rev primer | GTATTTTCTCATCACTAATGTGCTCCCA |
| 1067 | COL11 A1 for primer | AAAATGCCTTGAGACTATGTAAAAACTTAAAA | 1068 | COL11A1 rev primer | TGCCTCTTATAGGGTCAAGATGGT |
| 1069 | COL11A1 for primer | GCTGTTTCAAACTGTGTGTCACTAAC | 1070 | COL11A1 rev primer | TGGATAATTACATTCCTTTTTCTACACTGCA |
| 1071 | COL11A1 for primer | AAACCAGTGACTGCCATGCATA | 1072 | COL11A1 rev primer | CCCACCTGGTGAAAAAGGTGA |
| 1073 | COL11A1 for primer | CCAGAAAACCTGGTGAGAAGTAGG | 1074 | COL11A1 rev primer | ATGTTTGGGCAAAAAGGTGATGAG |
| 1075 | COL11A1 for primer | GGATTTAGATTTGCTGAACAATGTGGAA | 1076 | COL11A1 rev primer | GCTCTTCTGGCTTCACTTTAAAAATTACTTA |
| 1077 | COL11A1 for primer | GCTTTTTCACAGGATCTACTAACACGA | 1078 | COL11A1 rev primer | CCTGCAGGGAGAGATGGAGT |
| 1079 | COL11A1 for primer | AAAAAGTAATAACGTGTACAGGGTAGCA | 1080 | COL11A1 rev primer | ACAGCTGGCCCAATTGGTT |
| 1081 | COL11A1 for primer | AATCACAGCATGAGAGTAATGAAAGAA | 1082 | COL11A1 rev primer | GGGTGCACCTGGACTGAAAG |
| 1083 | COL11A1 for primer | GCTTCTAATAAACAATTGGAATCACTTGCT | 1084 | COL11A1 rev primer | GGTCCTCAAGGTATCTCAGGGAAA |
| 1085 | COL11A1 for primer | AAAAAGTTTTCTGAAGCATGTTGTACCTTT | 1086 | COL11A1 rev primer | CCGGTGAGACTGGTCCAAT |
| 1087 | COL11A1 for primer | ATGAAGAAAGAGAAAGAGAAGCTCTGAC | 1088 | COL11A1 rev primer | TTCTCCCCACTGCTTTTTATTTCCA |
| 1089 | COL11A1 for primer | ACATCCAAACAGAAAAAGTACAGGTGATA | 1090 | COL11A1 rev primer | GATTTGTCTGGCATTAGTTTGGGTTC |
| 1091 | COL11A1 for primer | CAAGGCACCTACCGTTGGA | 1092 | COL11A1 rev primer | GCATGACTACTCAGTCTCAGCA |
| 1093 | COL11A1 for primer | CATACCCGTGCACCTTTCTCT | 1094 | COL11A1 rev primer | CATCTTGTGACTGAAAATGTTAATTGCC |
| 1095 | COL11A1 for primer | TCATTTACCAACCTTTTCTCCTGCT | 1096 | COL11A1 rev primer | GTTGTTATTCTGTGCCTCAGGGA |
| 1097 | COL11A1 for primer | CTCTGTCACCTTTTAGACCCATGT | 1098 | COL11A1 rev primer | ATTTTAGGTTACTCCAAAGGACTGATTAAA |
| 1099 | COL11A1 for primer | GACCAAATTAAGGCATTTTCTCTTGGAA | 1100 | COL11A1 rev primer | TGCTAAATATAGGGTCATCCTGGGAA |
| 1101 | COL11A1 for primer | GGCAGACCTTATACCTGCCTAAGA | 1102 | COL11A1 rev primer | TTTTAAAATAGGGTCTTCCTGGTCCAC |
| 1103 | COL11A1 for primer | GGTCCTGGATTCCCTTGTTGAC | 1104 | COL11A1 rev primer | AGAACAAAAATCTATTGTGTAATATAGCCCAT |
| 1105 | COL11A1 for primer | TGGTTTCTTAGGGCTTATATTCAAATATGAGTT | 1106 | COL11A1 rev primer | GCAGGGTATGGCAGGTGTA |
| 1107 | COL11A1 for primer | TGTGTAACACCAGTCACTAGTCCT | 1108 | COL11A1 rev primer | TGTCAATGACAGGGAGAAGATGGA |
| 1109 | COL11A1 for primer | CATACCCTCATTCCATCATCACCA | 1110 | COL11A1 rev primer | TTGGTTTGGATAGCTACCTGATAGA |
| 1111 | COL11A1 for primer | GATGTATCTTACCCTGTGACCTTTGT | 1112 | COL11A1 rev primer | TGCAGTACATATCTAGACTGGAATTGtga |
| 1113 | COL11A1 for primer | AATGATCATGGCAGATGCCTTCA | 1114 | COL11A1 rev primer | ATGTCTTGATATAGGGCCCTCGA |
| 1115 | COL11A1 for primer | ACCTGAGGACCTGGATCACC | 1116 | COL11A1 rev primer | TCAGGTGCAATTGGCAGGAT |
| 1117 | COL11A1 for primer | GAAGAATAGCTTGAGCCTGAGCTT | 1118 | COL11A1 rev primer | CAAAGCACGTAATTCAACATGTTTGA |
| 1119 | COL11A1 for primer | TGAGATATCATGTCCATTCACTTACCCT | 1120 | COL11A1 rev primer | GGAATCTGATTAAGATATGGTGTGTTTCTCT |
| 1121 | COL11A1 for primer | ATAATCGTGGCTCATAAGCAATTTTACC | 1122 | COL11A1 rev primer | ACAGATAAATGGCCATGGTGCAT |
| 1123 | COL11A1 for primer | TGTATCCTCAGAATTTTTCCTCTGGGA | 1124 | COL11A1 rev primer | CTTCATGTTCCTCAATTCCTTGCAAT |
| 1125 | COL11A1 for primer | TGGGACTGTGATTTAATACTGTCTATACGT | 1126 | COL11A1 rev primer | ACCATTAGGCAAACATCGTTGATGA |
| 1127 | COL11A1 for primer | TTTGAGGAACAGTCAACATAAGGAAC | 1128 | COL11A1 rev primer | GGCTGAAAGTGTAACAGAGGGA |
| 1129 | COL11A1 for primer | GTTCAAAAACTGCACTGCGATGT | 1130 | COL11A1 rev primer | GCATTATAGTCCAGACTGTGACTCTTC |
| 1131 | COL11A1 for primer | TGATTTGTCAATATTCAGCTTAGAGTTGGA | 1132 | COL11A1 rev primer | AAACCACGAAACCACTTGATAGAAGT |
| 1133 | COL11A1 for primer | GAAAAACAGGTGATCTCCCAACCT | 1134 | COL11A1 rev primer | AGGTGGAACTTTCCCAGAAGAC |
| 1135 | COL11A1 for primer | ACTCTGTAAGCAGTATCTGAGCCT | 1136 | COL11A1 rev primer | GTTTGCTTATTTTTCAGCTGCTCCA |
| 1137 | COL11A1 for primer | AATGCGAGGGTTGTTACGGT | 1138 | COL11A1 rev primer | GGACGAAGTTGGTCTGCAGT |
| 1139 | APC for primer | AGCAAGTTGAGGCACTGAAGAT | 1140 | APC rev primer | TGGATAAACTACAATTAAAAGTCACAGTCT |
| 1141 | APC for primer | GCTATGGCTTCTTCTGGACAGAT | 1142 | APC rev primer | ACACCTAAAGATGACAATTTGAGCT |
| 1143 | APC for primer | GACTGCTTAAAGCAATTGTTGTATAAAAAC | 1144 | APC rev primer | GATCCTTCCCGGCTTCCATAA |
| 1145 | APC for primer | CTGTTCCTATGGGTTCATTTCCAAGA | 1146 | APC rev primer | AACAATAAACTGGAGTACACAAGGCA |
| 1147 | APC for primer | TCTTGCTGATCTTGACAAAGAAGAA | 1148 | APC rev primer | TAAAGTTTCAAATAAGTTGTACTGCCAAGT |
| 1149 | APC for primer | CAAATCAGAGTTGCGATGGAAGAA | 1150 | APC rev primer | TTCATTTTATTCCTAATAGCTCTTCGCTGT |
| 1151 | APC for primer | CAGAATTCAGCAAATCGAAAAGGACATA | 1152 | APC rev primer | CTCAGAATAACTACCTATTTTTATACCCACAAA |
| 1153 | APC for primer | ACTCCATCTTAACAGAGGTCATCTCA | 1154 | APC rev primer | aatttatttaCCTGACCATTACCAGAAGT |
| 1155 | APC for primer | AACAGCCAGTGTTTTGAGTTCTAGT | 1156 | APC rev primer | cAAGAATGTCTTAGCAAAGTAGTCATGG |
| 1157 | APC for primer | GTTGTCAATGCTTGGTACTCATGATAAG | 1158 | APC rev primer | TGTCATTGCCATGTAAAAGCTGGA |
| 1159 | APC for primer | ATTGCTCTTCAAATAACAAAGCATTATGGT | 1160 | APC rev primer | TCATTGCATGTCTATGCTCTTCATCAA |
| 1161 | APC for primer | TGGTACCAGTTTGTTTTATTTTAGATGATTG | 1162 | APC rev primer | ACTGTAGTGGTCATTAGTAAGCCCATA |
| 1163 | APC for primer | CAGTATTACACTAAGACGATATGCTGGAA | 1164 | APC rev primer | ATGAAAGTAAATTAACTCATACCTGAGCTATC |
| 1165 | APC for primer | ACAGGCTACGCTATGCTCTATGA | 1166 | APC rev primer | GGTGAAATTCTAAATAGTACCTGCTGTAAGT |
| 1167 | APC for primer | GATTAATTTGCAGGTTATTGCGAGTGTT | 1168 | APC rev primer | AGGTACCTTTTTAACTTCTAAAGCACA |
| 1169 | APC for primer | CCTCAAAAGCGTATTGAGTGCCTTAT | 1170 | APC rev primer | TGGCTAAAGTGTTTGTCTGGCT |
| 1171 | APC for primer | GATATTACGGAATGTGTCCAGCTTGA | 1172 | APC rev primer | TTAGGTCTTTTTGAGAGTATGAATTCTGTAC |
| 1173 | APC for primer | AAGAGAGAACAACTGTCTACAAACTTTATTAC | 1174 | APC rev primer | CCCCCATGTCCCATAATGCT |
| 1175 | APC for primer | CATTCAAAGCACAAAATGATTGCTATGG | 1176 | APC rev primer | TTGTTTCCTAACATGAAGAGATGGCA |
| 1177 | APC for primer | CTCAGCACTTATCAGAAACTTTTGACAA | 1178 | APC rev primer | TGACCTATTATCATCATGTCGATTGGTG |
| 1179 | APC for primer | ATGACTGTCCTTTCACCATATTTGAATACT | 1180 | APC rev primer | GGATGGTAGTTGCCTAGACCAATT |
| 1181 | APC for primer | AGGAACTTCTTCAAAGCGAGGTTT | 1182 | APC rev primer | CTGTCACACAATGTAATTCAGTGGTAGA |
| 1183 | APC for primer | TGTGACAGATGAGAGAAATGCACTT | 1184 | APC rev primer | TTGAAGATCTCTTGTATTCTAATTTGGCAT |
| 1185 | APC for primer | TGCTAATACCCTGCAAATAGCAGAAA | 1186 | APC rev primer | CTGAAGATAAACTAGAACCCTGCAGT |
| 1187 | APC for primer | CCCTCCAAAAGTGGTGCTCA | 1188 | APC rev primer | CTCTGAACGGAGCTGGCAA |
| 1189 | APC for primer | GATCTTCCAGATAGCCCTGGAC | 1190 | APC rev primer | TCCACTCTCTCTCTTTTCAGCAGTA |
| 1191 | APC for primer | GATAAGCTCCCAAATAATGAAGATAGAGTCA | 1192 | APC rev primer | TCATCATCATCAAAATCTAGAGAACTCAAAGA |
| 1193 | ATM for primer | AGAAGTTGAGAAATTTAAGCGCCTGA | 1194 | ATM rev primer | TTGAATAGAATACCTAAAAACAGCATCCCA |
| 1195 | ATM for primer | TCAACGAGTTTCTGAAATTGCATTTTGT | 1196 | ATM rev primer | CCTGCATCTTTTTCTGCCTGGA |
| 1197 | ATM for primer | ACCT AGGCT AAAATGTCAAGAACTCTT AAAT | 1198 | ATM rev primer | TTTCACACCAGTATTTTCTCACAGAAAGA |
| 1199 | ATM for primer | aGAATTGTTCTCTGTGTACTTCAGGC | 1200 | ATM rev primer | GTCAGTCTGAGAACAGCATCCT |
| 1201 | ATM for primer | AACTGTTCAGTTTGTACAGTTTGTTCC | 1202 | ATM rev primer | TTGAGGAAGATAGTAAGAGCTGCTAAGA |
| 1203 | ATM for primer | TGTGGGAGCTAGCAGTGTAA | 1204 | ATM rev primer | ATGACTTATCTCATTCACTAGCAGATCATA |
| 1205 | ATM for primer | AAGTCATATAGGAAGTAGAGGAAAGTATTCTT | 1206 | ATM rev primer | AACAGGAAATTTCTAAATGTGACATGACCTA |
| 1207 | ATM for primer | GTGTCCCTTGCAAAAGGAAGAAAA | 1208 | ATM rev primer | ACAAGAGATTAAAATGACACTGAATGAGAA |
| 1209 | ATM for primer | CCTTTTAGTTTGTTAATGTGATGGAATAGTTT | 1210 | ATM rev primer | TGGAGACAGCTCACAGTTAGGTA |
| 1211 | ATM for primer | ACTATCTCAGCTTCTACCCCAACA | 1212 | ATM rev primer | CCAGAGTTTTAATAAATCTGACTTTTGTGAGC |
| 1213 | ATM for primer | CCTTTCGTGGTATAAGTTCTGAGCAA | 1214 | ATM rev primer | CATGAAGGTCTGCAGGCTGA |
| 1215 | ATM for primer | CTTTGGCACTGACCACCAGTA | 1216 | ATM rev primer | AGTCACCCTCTAACTGATAGAATAAGAGC |
| 1217 | ATM for primer | TGGTCTTCTAAGTGAAGCTTTTTGTTTTTC | 1218 | ATM rev primer | AACATACCATTCTGGCACGCT |
| 1219 | ATM for primer | AAAGAAGAACTTTCATTCTCAGAAGTAGAA | 1220 | ATM rev primer | TCTGGTGGACAGAGAAGCCAATA |
| 1221 | ATM for primer | CAAGGAATCACTGGATCGCTGT | 1222 | ATM rev primer | CTCCTTCCTAACAGTTTACCAAAGTT |
| 1223 | ATM for primer | GGTGTCCTTGGCTGCTACT | 1224 | ATM rev primer | CACAGGAATACATTTCATTCAAATTTATCCGA |
| 1225 | ATM for primer | GCAATGTGCAGGAGAAAGTATCACT | 1226 | ATM rev primer | agaagaagaaaaTCTTACCTTGGTACAGTTG |
| 1227 | ATM for primer | TGCAGAAGAGTCCAAATAAGATTGCAT | 1228 | ATM rev primer | tcaaaatatgATAGCAAAACAGGAAGCATA |
| 1229 | ATM for primer | GCATCCTTCATCAAAAAGCCATTTGAC | 1230 | ATM rev primer | GCATCACTAACACTACTATCAGGGTAATC |
| 1231 | ATM for primer | ATGACTATATATGGCTGTTGTGCCC | 1232 | ATM rev primer | ACACAAGAACTTGAGCATGTCTAAGAAA |
| 1233 | ATM for primer | GCTCAGACCAATACTGTGTCCT | 1234 | ATM rev primer | ATCCAAGAGCTTCTTCATTTAACGTAACT |
| 1235 | ATM for primer | CCCTCCTACCATCTTAGTATCTAATGCT | 1236 | ATM rev primer | AGGTTTTAATTTCTTACGATAGTGGTTTCAGA |
| 1237 | ATM for primer | GTCGTGACCAAGATGTTTGTAAAACT | 1238 | ATM rev primer | CTACCAAAATGCTCCAATTACTGTAAGAAAC |
| 1239 | ATM for primer | CAGTGAGTTTTCTGAGTGCTTTTATCA | 1240 | ATM rev primer | AGTTTTAAGGCAATTTACTAGGGCCA |
| 1241 | ATM for primer | CTCTATTTCATATTTAACCACAGTTCTTTTCC | 1242 | ATM rev primer | GAACTTGGTGATGATTGTCAGCA |
| 1243 | ATM for primer | ttgcttgtttTAAGATTGTTCCAGGAC | 1244 | ATM rev primer | CATTTCTCTCATTCCTTCCTGAGCTT |
| 1245 | ATM for primer | AATTTCTTTTTAAGTCCCATAGTGCTGAGA | 1246 | ATM rev primer | GGCTACAGGATAAAACCACAGCTA |
| 1247 | ATM for primer | CCTATCTGCGAAAAACAGGCTTTG | 1248 | ATM rev primer | TTAAATTATTTCACAGTGACCTAAGGAAGCT |
| 1249 | ATM for primer | AGATCTTGTTATAAGGTTTTGATYCCACA | 1250 | ATM rev primer | CAAGAATCTTTGGAAAGCAGTCTGTT |
| 1251 | ATM for primer | TGCCTATGAGGGTACCAGAGAC | 1252 | ATM rev primer | GGGAATGAAAAGTACATAAAAATTGAAGCCA |
| 1253 | ATM for primer | GCCCTTGCAGATTGATCACTT | 1254 | ATM rev primer | AATGTACATACCCTGAAAAGTCACAGAG |
| 1255 | ATM for primer | AGTTCACTGGTCTATGAACAAAACTT | 1256 | ATM rev primer | GCAAATGTTGCTTTAATCACATGCGA |
| 1257 | ATM for primer | AAAGTGTATTTATTGTAGCCGAGTATCTAATT | 1258 | ATM rev primer | AACATTATTTGTTTCAGCTGCTTGCT |
| 1259 | ATM for primer | GATATAAAAAGTGGCTTAGGAGGAGCT | 1260 | ATM rev primer | AGGAAGAACAGGATAGAAAGACTGCT |
| 1261 | ATM for primer | GTAGCTTCTCCCTTTGTTGTGACT | 1262 | ATM rev primer | CTGAACCTCCACCTGCTCAT |
| 1263 | ATM for primer | AGTGATAGATAACAAGGATAATGAAAACCTCT | 1264 | ATM rev primer | GTGAAAAGGGTCCTCTACTGTATTTGA |
| 1265 | ATM for primer | TCAGTAAGTGTTTATGATGCACTTCCA | 1266 | ATM rev primer | TCATTTAAAATTAGCACCCTGAGAAGCT |
| 1267 | ATM for primer | TGCAATTATAAACAAAAGTGTTGTCTTCA | 1268 | ATM rev primer | GTTTATTGCCATCTTGGATAACTGCAA |
| 1269 | ATM for primer | TTATGTATGATCTCTTACCTATGACTCTACTG | 1270 | ATM rev primer | GATGCATCTTTACTATGTTGTATAGCTATGG |
| 1271 | ATM for primer | GAAGATAAAGAACTTCAGTGGACCTTCATA | 1272 | ATM rev primer | TGTGTGAAGTATCATTCTCCATGAATGT |
| 1273 | ATM for primer | TCAAAGTTCGATCAGCAGCTGTT | 1274 | ATM rev primer | TTTTCTTGATGTTCTAAAAGGCTGTAGATA |
| 1275 | ATM for primer | TTGTGTAGGAAAGGTACAATGATTTCCA | 1276 | ATM rev primer | ACAGATTTATATCATCCAGGCCTTCAAAA |
| 1277 | ATM for primer | ACTCATTTTTACTCAAACTATTFGGGTGGAT | 1278 | ATM rev primer | GAAAGCAGATTTCTCCATGATTCATTTGT |
| 1279 | ATM for primer | CACCAGCTGTCTTCGACACT | 1280 | ATM rev primer | CCATCTTAAATCCATCTTTCTCTAGAACTG |
| 1281 | ATM for primer | CACTTTTTCCGATGCTGTTTGGA | 1282 | ATM rev primer | CGATAGTTAACAAGTTACACTCTAGATCCT |
| 1283 | ATM for primer | TGCTTTCTGGCTGGATTTAAATTATCTAGAA | 1284 | ATM rev primer | AATTCCATTACCTTTTCTCTTGATCATCCAT |
| 1285 | ATM for primer | AACATATGAACACGAAGCAATGTGG | 1286 | ATM rev primer | CTACACTAGTGATGGCTTTACCAAATCT |
| 1287 | ATM for primer | GACTCTGCCATATTCTTTCCGTCTA | 1288 | ATM rev primer | TCTTACCTGACGGAAGTGCAATG |
| 1289 | ATM for primer | CTTATCTCACAGCAAAGAAGTAGAAGGA | 1290 | ATM rev primer | GTAACTTTGTCTTTTCATAATACCTGGCATAT |
| 1291 | ATM for primer | TGAAAACCTCTTCTTTATTTTCAGAGTGTCT | 1292 | ATM rev primer | AATGGCCTGCAACCTGCTA |
| 1293 | ATM for primer | TTCATTTCTCTTGCTTACATGAACTCTATGT | 1294 | ATM rev primer | GGCTCCTGAAAACTAAAATCACTGTC |
| 1295 | ATM for primer | ATTTTGGAGATCCTGATGGAAAAGGAA | 1296 | ATM rev primer | AGTTTTAAATTGTATTTACCTGAGTGTTCTTG |
| 1297 | ATM for primer | CCCATATGTCATTTTCATTTCAGCTCC | 1298 | ATM rev primer | TTGCCCAGAATACTTGTGCTTCT |
| 1299 | ATM for primer | ATGAAGGGCAGTTGGGTACA | 1300 | ATM rev primer | AGCACGTTTCTGCTAACCAGT |
| 1301 | ATM for primer | ATTTAAATTGGTTGTGTTTTCTTGAAGGC | 1302 | ATM rev primer | GTATTGAGTATCTGAAAACCGGGCTAA |
| 1303 | ATM for primer | TGAAATCATCGGAATTTGAAAACAAGCA | 1304 | ATM rev primer | AGTTAACTGGTGAACATAAAATTGTCACT |
| 1305 | ATM for primer | GTTCAGCGAGAGCTGGAGTT | 1306 | ATM rev primer | GTCGGAATACCCACATATCATGTTCT |
| 1307 | ATM for primer | ACTTACTTGCTTAGATGTGAGAATATTTGAAAT | 1308 | ATM rev primer | AGCCAATTGGTACATAAGAGGCAAAA |
| 1309 | ATM for primer | CCTTAGCAAATGCAAACAGAGATGA | 1310 | ATM rev primer | ACACTTCTAAAAGGTACGTATGTTTAATCCA |
| 1311 | ATM for primer | AACAGAGGCTGCAAATAGAATAATATGTACTA | 1312 | ATM rev primer | ACATACTTCTCTGAGTCTTCCACTGA |
| 1313 | ATM for primer | TTCCTGCTTGACCTTCAATGCT | 1314 | ATM rev primer | CCATAGTAGGGACAACAACATCTTCTAAA |
| 1315 | ATM for primer | TTTCTTTAAGTGCAAATAGTGTATCTGACC | 1316 | ATM rev primer | TTTACACCTCCTGCTAAGCGAAATT |
| 1317 | ATM for primer | ACTGTACTTGTTTATTCATGCTTAATTATTCTG | 1318 | ATM rev primer | TTTCCGTGTTTCTCTGCAGTAATGTAT |
| 1319 | ATM for primer | TGAATGATCATCAAATGCTCTTTAATGGC | 1320 | ATM rev primer | AGTTCCTGTGCACCATTCAAGAA |
| 1321 | ATM for primer | GAAAAATTCTTGGATCCAGCTATTTGGT | 1322 | ATM rev primer | ACAAAAATAAAACCTGCCAAACAACA |
| 1323 | ATM for primer | GGACTTGGTGATAGACATGTACAGAAT | 1324 | ATM rev primer | GCAAACAACATTCCATGATGACC |
| 1325 | ATM for primer | TTTGAACAGGGCAAAATCCTTCCTA | 1326 | ATM rev primer | ATTATTTCCCTCCTTTACTTCATATCACTTAC |
| 1327 | ATM for primer | ACTCTAGATGCTGTGAGAAAACCATG | 1328 | ATM rev primer | ACTTCCTGATGAGATACACAGTCT |
| 1329 | ATM for primer | GAAAGCTTTGTATTTACAGCAGAGGC | 1330 | ATM rev primer | ATCTGAAAAACTGACAACAGGACCTT |
| 1331 | ATM for primer | GTAGCTGAACGTGTCTTAATGAGACT | 1332 | ATM rev primer | CCTGGGAAAAGTCGGCTGA |
| 1333 | ATM for primer | GTATTCAGGAGCTTCCAAATAGTATGTT | 1334 | ATM rev primer | GCTAGAAATAGTTGTATTCTGGCTTCCT |
| 1335 | ATM for primer | TTCCTTCTTCAATTTTTGTTGTTTCCATG | 1336 | ATM rev primer | AAATGCAATTTACCTAGTAATGGGTTGTAA |
| 1337 | CDKN2A for primer | CCCCTTCAGATCTTCTCAGCA | 1338 | CDKN2A rev primer | GGGTTTGTAATCACAGACCTCCT |
| 1339 | CDKN2A for primer | CCAGGAAGCCTCCCCTTTTTC | 1340 | CDKN2A rev primer | GATCCAGGTGGGTAGAGGGT |
| 1341 | CDKN2A for primer | TCCGACCCTGTCCCTCAAAT | 1342 | CDKN2A rev primer | GGGTGCCACATTCGCTAAG |
| 1343 | CDKN2A for primer | AATCACACCAAACAAAACAAGTGC | 1344 | CDKN2A rev primer | ACTGAGGAGCCAGCGTCTA |
| 1345 | CDKN2A for primer | CCTCAGTAGCATCAGCACGA | 1346 | CDKN2A rev primer | TCTTGGTGACCCTCCGGAT |
| 1347 | CTNNB1 for primer | TAGGGTATTTGAAGTATACCATACAACTGTTTT | 1348 | CTNNB1 rev primer | CAGAGCCCCAATTCAGTAACTAAAGATT |
| 1349 | CTNNB1 for primer | AGCAAATACTTAGGTAAATGCTGAACT | 1350 | CTNNB1 rev primer | CATGCCCTCATCTAATGTCTCAGG |
| 1351 | CTNNB1 for primer | CAGCGTTTGGCTGAACCAT | 1352 | CTNNB1 rev primer | CATTGCTTACCTGGTCCTCGT |
| 1353 | CTNNB1 for primer | GAATGTCTACCCAATACCAGTACTTG | 1354 | CTNNB1 rev primer | AGAACGCATGATAGCGTGTCT |
| 1355 | CTNNB1 for primer | TGTACGTACCATGCAGAATACAAATGA | 1356 | CTNNB1 rev primer | CATTTTCACCAGGGCAGGAATG |
| 1357 | CTNNB1 for primer | CCATTACAACTCTCCACAACCTTTTATT | 1358 | CTNNB1 rev primer | AAGGCAGTCTGTCGTAATAGCC |
| 1359 | CTNNB1 for primer | CTTCTCAGACATGTGATCAAGATTCC | 1360 | CTNNB1 rev primer | GTCCACAGTAGTTTTTCGTAAGTATAGGT |
| 1361 | CTNNB1 for primer | AGGATTGATAGGCACTTCTAGCT | 1362 | CTNNB1 rev primer | GATTCCTGAGAGTCCAAAGACAGTTC |
| 1363 | CTNNB1 for primer | CATGGGAATAGAGTCAAGATGAGTATG | 1364 | CTNNB1 rev primer | GCTGCACAGGTGACCACATT |
| 1365 | CTNNB1 for primer | GATGGTCTGCCAAGTGGGT | 1366 | CTNNB1 rev primer | GCCATCTCTGCTTCTTGGTGTC |
| 1367 | CTNNB1 for primer | CAGTTCGCCTTCACTATGGACT | 1368 | CTNNB1 rev primer | CAGCTAGAGATGCTTCATTTTCAATTC |
| 1369 | CTNNB1 for primer | GCTACTGTTGGATTGATTCGAAATCTT | 1370 | CTNNB1 rev primer | CCACCCATGGACGTACGG |
| 1371 | CTNNB1 for primer | TTGTTCCTCAAACTTTACAGAGGAG | 1372 | CTNNB1 rev primer | TGTGAACATCCCGAGCTAGGA |
| 1373 | CTNNB1 for primer | GGGCTTGCCATGTTTTAGCT | 1374 | CTNNB1 rev primer | CAGCTTCCTTGTCCTGAGCAA |
| 1375 | CTNNB1 for primer | CTATTGAAGCTGAGGGAGCCA | 1376 | CTNNB1 rev primer | GGCCCTTGAGGAAAAACTGA |
| 1377 | CTNNB1 for primer | CAGCTGCTGTTTTGTTCCGAAT | 1378 | CTNNB1 rev primer | GATAAATAACTGCTCACATTTCCCTACCT |
| 1379 | CTNNB1 for primer | TTTCATTTTGCTTTCTATTCTTCCTTGCTT | 1380 | CTNNB1 rev primer | CTGGAGTTAATCGAGAAATATGAGACACA |
| 1381 | CTNNB1 for primer | TCTTCTAGATCCTAGCTATCGTTCTTTTCA | 1382 | CTNNB1 rev primer | CAGATCTGGCAGCCCATCA |
| 1383 | CTNNB1 for primer | CCAGGTGACAGCAATCAGCT | 1384 | CTNNB1 rev primer | CCACCCTACCAACCAAGTCT |
| 1385 | EGFR for primer | CAGTTGGGCACTTTTGAAGATCATT | 1386 | EGFR rev primer | ATCAAAGTCACCAACCTTTAAGAAGGAA |
| 1387 | EGFR for primer | CCTCATTGCCCTCAACACAGT | 1388 | EGFR rev primer | CAGCTCCTTCAGTCCGGTT |
| 1389 | EGFR for primer | CCTTCATGGGAATTTAAAGGAGCT | 1390 | EGFR rev primer | ACTATGTCCCGCCACTGGAT |
| 1391 | EGFR for primer | CATCATTTCACTGAGATATGCATCTATTACTT | 1392 | EGFR rev primer | GTCTGAGGCTGTTCACTGACTTAC |
| 1393 | EGFR for primer | CAAAATCATCTGTGCCCAGCA | 1394 | EGFR rev primer | GGGCATCTTACCAGGCAGTC |
| 1395 | EGFR for primer | CTCCATAGGTCTGCCGCAAA | 1396 | EGFR rev primer | AGGTGGCACCAAAGCTGTA |
| 1397 | EGFR for primer | GTGCCACCGTCATCACCTT | 1398 | EGFR rev primer | GCCGTCTTCCTCCATCTCATA |
| 1399 | EGFR for primer | CCCTAGCTATTCTTAATCCAACAAATGT | 1400 | EGFR rev primer | GCAGTTTTTGAAGTGTTTAATATTCGTAGCA |
| 1401 | EGFR for primer | AATCTGCCTTTTTAACTGGTAGAGATTG | 1402 | EGFR rev primer | CCTGTGATTTCCTTTACGGTTTTCAG |
| 1403 | EGFR for primer | GTCTGAAGTCTTTCATCTGCCTTACA | 1404 | EGFR rev primer | GCTTTGGCTGTGGTCAACTTAC |
| 1405 | EGFR for primer | CCTGCTCTGTCACTGACTGC | 1406 | EGFR rev primer | GGCTGACATTCCGGCAAGA |
| 1407 | EGFR for primer | TTGTGTTCCTGCAATAATGTCTCA | 1408 | EGFR rev primer | CACTCTGGGTGGCACTGTAT |
| 1409 | EGFR for primer | AAAAATGTTAGTGGTCATTTTTCTAATGTCTT | 1410 | EGFR rev primer | GGAAATGCAGTTTTAGTGGGAGACT |
| 1411 | EGFR for primer | TTCTGCCTTTTTAAACTATCATCCTGTAAT | 1412 | EGFR rev primer | CCCCAACCCAGCTGAAACTC |
| 1413 | EGFR for primer | GTGATCATCACGGCCTCCTC | 1414 | EGFR rev primer | GAGTGGAAGGAAGGAAATGGGT |
| 1415 | EGFR for primer | GCCATGGAATCTGTCAGCAAC | 1416 | EGFR rev primer | GCATGAAGAGGCCGATCCC |
| 1417 | EGFR for primer | CTGCTTTCCAGCATGGTGAG | 1418 | EGFR rev primer | TCAGTTTCCTTCAAGATCCTCAAGAGA |
| 1419 | EGFR for primer | CAGATCACTGGGCAGCATGT | 1420 | EGFR rev primer | GTTGCTTCTCTTAATTCCTTGATAGCG |
| 1421 | EGFR for primer | CCCTCCAGGAAGCCTACGT | 1422 | EGFR rev primer | GTGTTCCCGGACATAGTCCAG |
| 1423 | EGFR for primer | CGGATGCAGAGCTTCTTCCC | 1424 | EGFR rev primer | GCTGCGGTGTTTTCACCAGTA |
| 1425 | EGFR for primer | CCTCATCTCTCACCATCCCAA | 1426 | EGFR rev primer | CTCAGTACAAACTCATTAGCATCAGGATTAT |
| 1427 | EGFR for primer | CAAGCCATATGACGGAATCCCT | 1428 | EGFR rev primer | GTGGACAGACCCACCAGTC |
| 1429 | EGFR for primer | GCCCAAAGTTCCGTGAGTTGAT | 1430 | EGFR rev primer | GTGACATGAGATGCTCATTAGAGG |
| 1431 | EGFR for primer | GGGATGAAAGAATGCATTTGCCA | 1432 | EGFR rev primer | CCCTGCTGTGGGATGAGGTA |
| 1433 | EGFR for primer | GATGAGATGTGGTACAAGCATTCCA | 1434 | EGFR rev primer | GTAAATTCTGGCTTATAAGGTGTTCATACATA |
| 1435 | EGFR for primer | GTCCCATCAAGGAAGACAGCT | 1436 | EGFR rev primer | AGGTTGAGGAGCAGGACTGT |
| 1437 | ERBB2 for primer | GTGCAGGGAAACCTGGAACT | 1438 | ERBB2 rev primer | CTTCTGCCACCCACCTGTAAA |
| 1439 | ERBB2 for primer | GGGTGGCAGTGTTCCTATTTCA | 1440 | ERBB2 rev primer | GACCTGCCTCACTTGGTTGT |
| 1441 | ERBE2 for primer | CCTGGCCGTGCTAGACAAT | 1442 | ERBB2 rev primer | GAATAACCAAGAGAAGGTTTCAATGACG |
| 1443 | ERBB2 for primer | TGCTACCAGGACACGATTTTGT | 1444 | ERBB2 rev primer | GGGTCAGGCTGTCTGAGAGA |
| 1445 | ERBB2 for primer | CCGTCCTCTCGCTGTTAGAC | 1446 | ERBB2 rev primer | CCCTGAGACTCACGGCTCT |
| 1447 | ERBB2 for primer | CCACTGACTGCTGCCATGAG | 1448 | ERBB2 rev primer | GGTAGAGCACATTGGGCACAA |
| 1449 | ERBB2 for primer | TGGTCACCTACAACACAGACAC | 1450 | ERBB2 rev primer | ACAAACCTCCCCACCAAAATGA |
| 1451 | ERBB2 for primer | GACAACTACCTTTCTACGGACGT | 1452 | ERBB2 rev primer | GGGCAGTGAGTGGGTACCT |
| 1453 | ERBB2 for primer | AGGTGAGGGCAGTTACCAGT | 1454 | ERBB2 rev primer | TCTGCCCTGACACTGTCTCA |
| 1455 | ERBB2 for primer | AGTGATGTCCACCCTGTTCCT | 1456 | ERBB2 rev primer | GACAGAGCCCACCTGTGATC |
| 1457 | ERBB2 for primer | TGCAGGTTACCTATACATCTCAGCA | 1458 | ERBB2 rev primer | CTGGGACTCCTCCCCATCA |
| 1459 | ERBB2 for primer | GTGCCCACCTTTCTCCCATA | 1460 | ERBB2 rev primer | CGAAGCAGAGGTGGGTGTTA |
| 1461 | ERBB2 for primer | CCCTCACCACTGTCCCTTCT | 1462 | ERBB2 rev primer | CAAAACAGGTCACTGAGCCATTC |
| 1463 | ERBB2 for primer | GTGGCCTGTGCCCACTATA | 1464 | ERBB2 rev primer | CGCCCTCCTCATCTGGAAAC |
| 1465 | ERBB2 for primer | GGGTGGTTCCCAGAATTGTTGA | 1466 | ERBB2 rev primer | GGGCAATGAAGGGTACATCCTG |
| 1467 | ERBB2 for primer | CATTCTGCTGGTCGTGGTCTT | 1468 | ERBB2 rev primer | CTCACCTCCGTTTCCTGCA |
| 1469 | ERBB2 for primer | ACACCTAGCGGAGCGATG | 1470 | ERBB2 rev primer | CCTGGCCCTGACCTTGTAGA |
| 1471 | ERBB2 for primer | GGCCCTCCCAGAAGGTCTA | 1472 | ERBB2 rev primer | TCTGTAATTTTGACATGGTTGGGACT |
| 1473 | ERBB2 for primer | CTGGACATTGACGAGACAGAGTA | 1474 | ERBB2 rev primer | TCTCCCATGGGCTAGACAC |
| 1475 | ERBB2 for primer | GCTCAGTACACTAAAGCTCCCT | 1476 | ERBB2 rev primer | GGGATCCCATCGTAAGGTTTG |
| 1477 | ERBB2 for primer | CCTGGGACTAGCATGCTGAC | 1478 | ERBB2 rev primer | GGCCATGCGGGAGAATTCA |
| 1479 | ERBB2 for primer | GCCTCTCCTTCCTCCACAGA | 1480 | ERBB2 rev primer | GGGTACCAGATACTCCTCAGC |
| 1481 | ERBB2for primer | GACTTTCTTTCTTGTCCCCAGAGT | 1482 | ERBB2 rev primer | CCCATTCCCAGGTCACCATC |
| 1483 | ERBB2 for primer | CAAAGCCTCCCCACACATGA | 1484 | ERBB2 rev primer | TGCTTAGACTGGACTCCATACCA |
| 1485 | ERBB2 for primer | AGAATATGTGAACCAGCCAGATGTT | 1486 | ERBB2 rev primer | CGTCTTTGACGACCCCATTCTT |
| 1487 | ERBB2 for primer | GTGGAGAACCCCGAGTACTT | 1488 | ERBB2 rev primer | GTGGGTCCTGGTCCCAGTA |
| 1489 | KRAS for primer | ATGTGCTGAACTTAAACTTACCAGATTACA | 1490 | KRAS rev primer | ATGCTTTTTATACATTGGTGAGGGAGAT |
| 1491 | KRAS for primer | TGCTGATGTTTCAATAAAAGGAATTCCATA | 1492 | KRAS rev primer | AAGAGTTAAGGACTCTGAAGATGTACCT |
| 1493 | KRAS for primer | ATTCAATTTAAACCCACCTATAATGGTGAA | 1494 | KRAS rev primer | ACACAGCAGGTCAAGAGGAGTA |
| 1495 | KRAS for primer | TCTGTATCAAAGAATGGTCCTGCAC | 1496 | KRAS rev primer | GGCAAGAGTGCCTTGACGATA |
| 1497 | KRAS for primer | TCTTCTTTTTACCATCTTTGCTCATCTTTT | 1498 | KRAS rev primer | CCTGTACACATGAAGCCATCGT |
| 1499 | PIK3CA for primer | AATGCCTCCACGACCATCAT | 1500 | PIK3CA rev primer | GCTTTATGGTTATTAATGTAGCCTCACG |
| 1501 | PIK3CA for primer | GGCATGCCAGTGTGTGAATT | 1502 | PIK3CA rev primer | GGATAGACATACATTGCTCTACTATGAGGT |
| 1503 | PIK3CA for primer | AGTTTCTCCAAATAATGACAAGCAGAAGT | 1504 | PIK3CA rev primer | AAACACAGAGTTTTAGTTGTTCAGAGGAT |
| 1505 | PIK3CA for primer | TTAAAATGAAAAACCTTACAGGAAATGGCT | 1506 | PIK3CA rev primer | AGTCCATTGGCAGTTGAGAATAAAGG |
| 1507 | PIK3CA for primer | CGCATTTCCACAGCTACACCAT | 1508 | PIK3CA rev primer | TGTCTCGAATATTTACATTCACGTAGGTTG |
| 1509 | PIK3CA for primer | AGTGTGTGCATATGTGTATGTTGA | 1510 | PIK3CA rev primer | ACAAGGTACTCTTTGAGTGTTCACATT |
| 1511 | PIK3CA for primer | AAAATTTTACATAGGTGGAATGAATGGCT | 1512 | PIK3CA rev primer | TTAGCACCCTTTCGGCCTTTAA |
| 1513 | PIK3CA for primer | TTAAGGAACACTGTCCATTGGCA | 1514 | PIK3CA rev primer | AGTAACACCAATAGGGTTCAGCAAA |
| 1515 | PIK3CA for primer | TTTTATGGCAGTCAAACCTTCTCTC | 1516 | PIK3CA rev primer | CTTCAATCACTGACATATCTGGGAACT |
| 1517 | PIK3CA for primer | TTACAGAGTAACAGACTAGCTAGAGAC | 1518 | PIK3CA rev primer | AGCACTTACCTGTGACTCCATAGA |
| 1519 | PIK3CA for primer | ATTCTACCCAAATTGCTTCTGTCTGTTA | 1520 | PIK3CA rev primer | CAATTTAAGTGACATACCAATTTGTACAACA |
| 1521 | PIK3CA for primer | CCAGAGGTTTGGCCTGCTTT | 1522 | PIK3CA rev primer | TGTTTCATCCTTCTTCTCCTGTTTGAG |
| 1523 | PIK3CA for primer | TATTAAGTCAGTTTCTTACTGTGACTATCCTTT | 1524 | PIK3CA rev primer | CCCTGTAGAGCATCCATGAAATCTG |
| 1525 | PIK3CA for primer | ACTATTTTAAAGGCTTGAAGAGTGTCGAA | 1526 | PIK3CA rev primer | TCCCCATTTTTAAAGATGATCTCATTGTTCT |
| 1527 | PIK3CA for primer | GCGTGATCCCCAAATTTGCAT | 1528 | PIK3CA rev primer | CAAGACCTTGATTTTGCCAGATATTTTCC |
| 1529 | PIK3CA for primer | TTCCATCATTTAATTGTAAACGTGTTACTC | 1530 | PIK3CA rev primer | GCCGCCTTTGCACTGAATT |
| 1531 | PIK3CA for primer | ACTGCAGTTCAACAGCCACA | 1532 | PIK3CA rev primer | CCGACAGACTCATCTAACAAAAACT |
| 1533 | PIK3CA for primer | GATGCAGCCATTGACCTGTTTAC | 1534 | PIK3CA rev primer | ACATTTTAAACAGAGAAAACCATTACTTGTCC |
| 1535 | PIK3CA for primer | TCAAGACATTTTGTATCTGCATATATCAAACTA | 1536 | PIK3CA rev primer | GCACACGTTCTCGTTTATAACCAAATT |
| 1537 | PIK3CA for primer | AGTAAAGGAGCCCAAGAATGCAC | 1538 | PIK3CA rev primer | GATTGTTTCTAATAGAGCAGCCAGA |
| 1539 | PIK3CA for primer | TATTACTTATAGGTTTCAGGAGATGTGTTACA | 1540 | PIK3CA rev primer | TGTATGCAATGTCATCAAAAGATTGTAGTT |
| 1541 | PTEN for primer | GCATCAGCTACCGCCAAGT | 1542 | PTEN rev primer | CGATCTCTTTGATGATGGCTGTCA |
| 1543 | PTEN for primer | ATTCTTTTAGTTTGATTGCTGCATATTTCA | 1544 | PTEN rev primer | ATCAATATTGTTCCTGTATACGCCTTCAA |
| 1545 | PTEN for primer | TTTTTCTTATTCTGAGGTTATCTTTTTACCACA | 1546 | PTEN rev primer | TGGTCAAGATCTTCACAAAAGGGTTT |
| 1547 | PTEN for primer | TTTTTCTGTCCACCAGGGAGTAAC | 1548 | PTEN rev primer | CATTGGAATAGTTTCAAACATCATCTTGTGA |
| 1549 | PTEN for primer | AAAATCGTTTTTGACAGTTTGACAGT | 1550 | PTEN rev primer | GTCGTGTGGGTCCTGAATTG |
| 1551 | PTEN for primer | AGTTCCCTCAGCCGTTACCT | 1552 | PTEN rev primer | TCTGTCCTTATTTTGGATATTTCTCCCAATG |
| 1553 | PTEN for primer | ATAGTTTAAGATGAGTCATATTTGTGGGTT | 1554 | PTEN rev primer | CTGGTGTTACAGAAGTTGAACTGCTA |
| 1555 | SMARCA4 for primer | GCCACCTCACGTTCCACAT | 1556 | SMARCA4 rev primer | CCGCATCCCCATTCCTTTCAT |
| 1557 | SMARCA4 for primer | GGCTGTAAAAATCACAGACATATGC | 1558 | SMARCA4 rev primer | ACTGGCTGGAACTGGACTAGA |
| 1559 | SMARCA4 for primer | CACCAGCTCAGAGCTCAGATC | 1560 | SMARCA4 rev primer | GGAGGTAGCGTTGGCATCT |
| 1561 | SMARCA4 for primer | CCTCGGTGTCCGCAACAG | 1562 | SMARCA4 rev primer | CATTCGCTCTGAGACACCTGA |
| 1563 | SMARCA4 for primer | CAACCCTCTCACCGCCTTT | 1564 | SMARCA4 rev primer | TGAGCTCAATGGTCGCTTTG |
| 1565 | SMARCA4 for primer | TGTCATTGTGGATGCCACAG | 1566 | SMARCA4 rev primer | CGCTTGTAGGCCTTAGCATTG |
| 1567 | SMARCA4 for primer | GGGAGATGTGTCCACCATGC | 1568 | SMARCA4 rev primer | AGCTTCTGGATTTTGCCTGTGA |
| 1569 | SMARCA4 for primer | GCCACGTACCATGCCAACA | 1570 | SMARCA4 rev primer | CCGCAGTACCAGCTACACAT |
| 1571 | SMARCA4 for primer | CCCCCATGCTTTTGTAGGCT | 1572 | SMARCA4 rev primer | CACCAGCTCCGTGAGGTTA |
| 1573 | SMARCA4 for primer | GCAACCTCAGTGTCACACG | 1574 | SMARCA4 rev primer | CATCTGTGCCTGTGAGGATCT |
| 1575 | SMARCA4 for primer | GAGCAGATTTGTATGAAAGCCCTTAC | 1576 | SMARCA4 rev primer | CACTGAGAGCCTTGAAAGCC |
| 1577 | SMARCA4 for primer | CCGTGGAGGAGAAGAAGAAGATTC | 1578 | SMARCA4 rev primer | GCTGGCACAGAACAACCTGT |
| 1579 | SMARCA4 for primer | GATGATGAATATGGCGTGTCCCA | 1580 | SMARCA4 rev primer | CTCACCTGGTACTGTTTGAGGA |
| 1581 | SMARCA4 for primer | ACCGGCACTTGACTCTCATTT | 1582 | SMARCA4 rev primer | CGTAGCAAGAGAGGAAACCTCAG |
| 1583 | SMARCA4 for primer | TGTGCATCCTGCTTCCCTTG | 1584 | SMARCA4 rev primer | CGTTACCTTGGCGAGGATGT |
| 1585 | SMARCA4 for primer | TGCTGCCCACCATCTTCAAG | 1586 | SMARCA4 rev primer | CACCTGGAACCGTCAGCAT |
| 1587 | SMARCA4 for primer | GGGCCCTCGTGAGCATTA | 1588 | SMARCA4 rev primer | TCTTGTCCTTCTCGGAGCCA |
| 1589 | SMARCA4 for primer | CCATTTGGGTCCCTCTCATCT | 1590 | SMARCA4 rev primer | GGCTCACCTCGATGTGCTG |
| 1591 | SMARCA4 for primer | CCTGTCACTGACCCCTCTCT | 1592 | SMARCA4 rev primer | ACAGAAAACCTCTGGGTGGAC |
| 1593 | SMARCA4 for primer | TTGAGCTTCTTGATAGAATTCTTCCCAAA | 1594 | SMARCA4 rev primer | CATCAAGCCTGAGGTATTTAAAGCC |
| 1595 | SMARCA4 for primer | GGCATGCTGCTGAAAACCTTC | 1596 | SMARCA4 rev primer | GGTGAGGATTCCAGTCGCT |
| 1597 | SMARCA4 for primer | GGGCTCCTTTGGGACTGAC | 1598 | SMARCA4 rev primer | CAGCTAGGATCTTCTCCTCCAC |
| 1599 | SMARCA4 for primer | CAGCGGCACTGACAGTTTG | 1600 | SMARCA4 rev primer | CCTGCAGCGCTTACCATGAA |
| 1601 | SMARCA4 for primer | CGAGCTCCCCTCGTGGAT | 1602 | SMARCA4 rev primer | CTGCTTCTCCGTCAGTGAGTC |
| 1603 | SMARCA4 for primer | GGTGATAGCCGCCGGTTC | 1604 | SMARCA4 rev primer | GCTTGCGGATGAGCTCGTA |
| 1605 | SMARCA4 for primer | GGTCCTGAGGTAAGACCTGCT | 1606 | SMARCA4 rev primer | CCCTCCAGGTTGAAGGTCTG |
| 1607 | SMARCA4 for primer | CATCGTCTTGCAGTCGGTCTT | 1608 | SMARCA4 rev primer | CCCCGGGACTCACATTCG |
| 1609 | SMARCA4 for primer | AAGATCAAGCTTGGCCGGAA | 1610 | SMARCA4 rev primer | GCCTCACCTCCTCTTGTTCCT |
| 1611 | TP53 for primer | ACTTGATAAGAGGTCCCAAGACTTAGTA | 1612 | TP53 rev primer | CTTTTATCACCTTTCCTTGCCTCTTTC |
| 1613 | TP53 for primer | gtagcatctgTATCAGGCAAAGTCA | 1614 | TP53 rev primer | GTTGTAGCTAACTAACTTCAGAACACCAA |
| 1615 | TP53 for primer | GGAACAAGAAGTGGAGAATGTCAGT | 1616 | TP53 rev primer | ATGTCATCTCTCCTCCCTGCT |
| 1617 | TP53 for primer | cagcccAACCCTTGTCCTTA | 1618 | TP53 rev primer | CCTAGCAGAGACCTGTGGGA |
| 1619 | TP53 for primer | GGGCTCGACGCTAGGATCT | 1620 | TP53 rev primer | GACCCAGGGTTGGAAGTGTC |
| 1621 | CNTNAP5 for primer | CAGCGTTTTGACTTTGCTGTTCT | 1622 | CNTNAP5 rev primer | AGTACGCTTAGATAGTTGATTGTCTTTC |
| 1623 | CNTNAP5 for primer | CCACTAGCATCCCTGCTCTCT | 1624 | CNTNAP5 rev primer | CACGTAATTATTTAGTGCTTGGCATTAGG |
| 1625 | CNTNAP5 for primer | AGTGACACAGGACGCAACTG | 1626 | CNTNAP5 rev primer | TATTGGAAAATGACGGAAATATACTCTGG |
| 1627 | CNTNAP5 for primer | TCTGCAGACCTTTGCAGGAAA | 1628 | CNTNAP5 rev primer | AGACCTCGACTCTCATGCCA |
| 1629 | CNTNAP5 for primer | TGAAATAAGATGGGAAACAGTAACAGCA | 1630 | CNTNAP5 rev primer | GATTGAACCTGTACAGAAGTGAGCTT |
| 1631 | CNTNAP5 for primer | CCTGAAGTTCAAGAGCATGCAA | 1632 | CNTNAP5 rev primer | TGGAAGAGGAAGTCTGAGCCT |
| 1633 | CNTNAP5 for primer | CTGCCCTTGGACACAGACAT | 1634 | CNTNAP5 rev primer | GCTCAATGAGGACCGAGTGC |
| 1635 | CNTNAP5 for primer | CTTCACGGTGGACAAGCAC | 1636 | CNTNAP5 rev primer | TGATTTTCTTGCTGCGTTCATTGAA |
| 1637 | CNTNAP5 for primer | ACTTTTTCCTGCTCCGAACCA | 1638 | CNTNAP5 rev primer | TGTTCCATGTTCGAAACTGGAAACT |
| 1639 | CNTNAP5 for primer | GCTTCTGTCCACAGAGCTGT | 1640 | CNTNAP5 rev primer | TCCAGAGTGTCAGCAGACAGTA |
| 1641 | CNTNAP5 for primer | AGCAACTTGAATGATGGCCTGT | 1642 | CNTNAP5 rev primer | GCTATTTCCAGAATAAATCTGCACCCA |
| 1643 | CNTNAP5 for primer | CATCTTTATTGATAACCAGCCCAAGGA | 1644 | CNTNAP5 rev primer | ACAGAGGAGAAGAGACAATAATTACCTGT |
| 1645 | CNTNAP5 for primer | CTTCCATTAGGTGTTTGCCAAACTAC | 1646 | CNTNAP5 rev primer | GGGCAAATGATCAATCTACTCACA |
| 1647 | CNTNAP5 for primer | GCCCATCACACTGCTCACAT | 1648 | CNTNAP5 rev primer | CACTCGGGTCAGCTCTGTA |
| 1649 | CNTNAP5 for primer | CTATGCCATGGCCTTGGACT | 1650 | CNTNAP5 rev primer | CCTTACCCGGCGTGTTGA |
| 1651 | CNTNAP5 for primer | TTTCAGCTCAGTTTCTGTGCCA | 1652 | CNTNAP5 rev primer | CCCCAGTAAGGGTGCCTTT |
| 1653 | CNTNAP5 for primer | CGAGAGCTGCCTGGACATTC | 1654 | CNTNAP5 rev primer | GGGAATTTTATGCATCAATTAATGTGCT |
| 1655 | CNTNAP5 for primer | CCGACAGATCAAACTCAGAAGCC | 1656 | CNTNAP5 rev primer | GGAAAAGGGATAAGGGAGTAAGAACA |
| 1657 | CNTNAP5 for primer | CTTCTGGAACGCCGTCTCA | 1658 | CNTNAP5 rev primer | CCAAGATTTTCTAGGAAAACTCCGGAT |
| 1659 | CNTNAP5 for primer | ATCGTTTCTCCCACTCAAGCC | 1660 | CNTNAP5 rev primer | GGACTACAAGCTCCACAGGAC |
| 1661 | CNTNAP5 for primer | AACCTCAAGGAGACCTCCCT | 1662 | CNTNAP5 rev primer | AGCACAAAAGGAGCCTTAAGATGT |
| 1663 | CNTNAP5 for primer | TCATCCAGACAGAAAGGCTTCCTA | 1664 | CNTNAP5 rev primer | CCTGGCCTGACTCCAGATG |
| 1665 | CNTNAP5 for primer | TGATCATGTCTTTGCATTTTATAGGTGCT | 1666 | CNTNAP5 rev primer | GCAAAGTTATCTGCATCAATGGTGAA |
| 1667 | CNTNAP5 for primer | tttccagatgGGATCGTTTTATGCT | 1668 | CNTNAP5 rev primer | CCCTGAACTCTACTTCCGGAGA |
| 1669 | CNTNAP5 for primer | GGACTTCTGATTATCTTTTACATTGCAGAGA | 1670 | CNTNAP5 rev primer | GCAGCCTTCAGTGGTGCTA |
| 1671 | CNTNAP5 for primer | GCGGGAACCACTCACAAATG | 1672 | CNTNAP5 rev primer | AAGGAAAGCTTCAGATAACTTTCTCCAG |
| 1673 | CNTNAP5 for primer | GCAGTGGTGATATTCATCATCTTCTGT | 1674 | CNTNAP5 rev primer | ATTTCATTTCTGAAGGAACTGTCCAAATT |
| 1675 | CSMD3 for primer | GCTTCTGAAGAAGGCAAAGGTTG | 1676 | CSMD3 rev primer | CAATGGCCAAGCAGCTTTTGA |
| 1677 | CSMD3 for primer | TGAGCTAGAGCAGTAGAAATAACATGA | 1678 | CSMD3 rev primer | TTTTTGCACTTATATTTGCAGGATTTGGATT |
| 1679 | CSMD3 for primer | AAGTCATGAAGGTATATTTCGCTGCT | 1680 | CSMD3 rev primer | GAAACAACCCATGACCTTAACAGTTAC |
| 1681 | CSMD3 for primer | AGTCCAGGTGTTATCGGATCTACA | 1682 | CSMD3 rev primer | CAGAAACTCCTGCTCATGCAAA |
| 1683 | CSMD3 for primer | ATGCATATAAATTCTTACCCATGATAGCATT | 1684 | CSMD3 rev primer | GCACCTGCCTCCCTGATCTTA |
| 1685 | CSMD3 for primer | GTTGTAGACCCTTGGAGAAGGTG | 1686 | CSMD3 rev primer | CATTTTCTCACTCAAGCTCTGTTTG |
| 1687 | CSMD3 for primer | CACTTGAAATCCGAATGTATTGTTCTGA | 1688 | CSMD3 rev primer | ATATCTTTTTAATCTTCATTTTAGAGCCTACCC |
| 1689 | CSMD3 for primer | AAGTGCCATCTGCTTGACATATTCT | 1690 | CSMD3 rev primer | TTGTGGTGACCCTGGTATACCT |
| 1691 | CSMD3 for primer | AGGAAGCTTAACAATCTCATATTTGGT | 1692 | CSMD3 rev primer | TGCTGTTTTGACCTGTGTAGGG |
| 1693 | CSMD3 for primer | GCTAATACTAAAGCCCCAGTCGAA | 1694 | CSMD3 rev primer | TCTTTAAGGCTCAGGATATTTCAACAGC |
| 1695 | CSMD3 for primer | CCTCTACAAGTTGGCATTACTCCAC | 1696 | CSMD3 rev primer | GATATGGAGATGATTATGTGGTTGGACA |
| 1697 | CSMD3 for primer | CATATCTCAGTCCATTGGCTGGTA | 1698 | CSMD3 rev primer | AAGTATGCCATATGCTTACTACTCCT |
| 1699 | CSMD3 for primer | TCCAGAAAGGATGTATCCCTCCAT | 1700 | CSMD3 rev primer | AACACTTTGTTTTATAGCTGTGCAGTG |
| 1701 | CSMD3 for primer | CCAACTGCCATTAGCTAAACATGTTC | 1702 | CSMD3 rev primer | GTGGCGATCCAGGTACTCC |
| 1703 | CSMD3 for primer | GTTTTGTGTATACGTTGCTACTACTACT | 1704 | CSMD3 rev primer | AGTCATCAAGAACCTGCCAATTGA |
| 1705 | CSMD3 for primer | AAACATTCCTATGGTTTCCATGACA | 1706 | CSMD3 rev primer | TCAACCAAATGGACAATGGGACA |
| 1707 | CSMD3 for primer | AAGCTGTTTTAAGTGTGTCACTGA | 1708 | CSMD3 rev primer | AACAGACAGTGGAGCCATCC |
| 1709 | CSMD3 for primer | AAAAACTAGAATTATTCCAAAGATCAGAGGT | 1710 | CSMD3 rev primer | GGATATGTCAACAGGATCACAATTGGT |
| 1711 | CSMD3 for primer | CTGAAGAACCAATCAATCGAAAACCA | 1712 | CSMD3 rev primer | ACTTTATAACGTATTTCAGCGGGTCATT |
| 1713 | CSMD3 for primer | CAAAGACCTGAGGAAAGGCATTC | 1714 | CSMD3 rev primer | TGGAGAACTACCTACACCTCCAA |
| 1715 | CSMD3 for primer | AGGAACTGATTCAAACAATTCGATAACCA | 1716 | CSMD3 rev primer | GTCCTGCCTCCATCGAATGT |
| 1717 | CSMD3 for primer | TGGTTTTGTATTCATAATGGGAGCCAT | 1718 | CSMD3 rev primer | GTGATTTGTTAGCAATTACACCCTTTGG |
| 1719 | CSMD3 for primer | CTCCATGTTCCATCTGATTGGCATA | 1720 | CSMD3 rev primer | CCTAAAGCTCCAACAAATGGAGGA |
| 1721 | CSMD3 for primer | CGTTAAATGGTGCTAATCTCACATTC | 1722 | CSMD3 rev primer | GTTGGAAAAAGCAGTGCTGTGT |
| 1723 | CSMD3 for primer | CCAACAAGTCGGAATCCTCGAT | 1724 | CSMD3 rev primer | TTTTGAAAAGCTTTCTACTGTAGTACACCA |
| 1725 | CSMD3 for primer | TCTGGAAGAATTCTACAAACATGGTGAT | 1726 | CSMD3 rev primer | CAATGAATTACGGCTAGATTCTACTGGA |
| 1727 | CSMD3 for primer | ATAAATAATCATAGGAACCATTGAATACCTTGA | 1728 | CSMD3 rev primer | GTGTCTTCCAGGATTTACTTTAGTTGGT |
| 1729 | CSMD3 for primer | AGCAAATAGTTCTGCCAGCAGT | 1730 | CSMD3 rev primer | AACTAAGGGTGTGCCAACCTC |
| 1731 | CSMD3 for primer | TCCAGGACAAATTAAACAGTAGCAAATGA | 1732 | CSMD3 rev primer | TCAAATTCCACAGTGATTTCACAACAAG |
| 1733 | CSMD3 for primer | TCCAAAGCGGTATTGCCACT | 1734 | CSMD3 rev primer | ACGGTTCAAAACTACTTCCACAATCA |
| 1735 | CSMD3 for primer | AATCATATATTGGTTCTGTTTGAAGGACAGT | 1736 | CSMD3 rev primer | GGCACCATTTATTCTCCTGGGTAT |
| 1737 | CSMD3 for primer | GGTGCCATTCATTGCAGTTATATTCC | 1738 | CSMD3 rev primer | ATGTGAAATGCCTTTTTCTCC |
| 1739 | CSMD3 for primer | TTCACACCTTGGAAGTGGATGATT | 1740 | CSMD3 rev primer | TGTAATTGGTAATGATTTTACTGTGGGTCA |
| 1741 | CSMD3 for primer | CTTACCTTGGTATACAATATGAAACCCTTGT | 1742 | CSMD3 rev primer | ACTGTTATTGGCCGGCTTAGTG |
| 1743 | CSMD3 for primer | GAAAGCTCTTTCACGTGTTTTTAATACACA | 1744 | CSMD3 rev primer | CTATGTCAGACTTCAGTGGTGTGAT |
| 1745 | CSMD3 for primer | CGTGGCTTATCAATCATTGATTTGTAAA | 1746 | CSMD3 rev primer | GTTGGAGATGTAGTATCCTTTCAGTGT |
| 1747 | CSMD3 for primer | GTTCTTAACCTTTTTGAATGGCACATGA | 1748 | CSMD3 rev primer | CAATACCCCATCTTTTGAATAGTACGTCTA |
| 1749 | CSMD3 for primer | TCTCTGAAAAGATTAACTACACACATACAAT | 1750 | CSMD3 rev primer | ACCTGGATACCCTGAGCCTTA |
| 1751 | CSMD3 for primer | ACTCACCAATACAAGTAGGTAAGGAATCA | 1752 | CSMD3 rev primer | GAATTTGCAGTCGGTTCATCGG |
| 1753 | CSMD3 for primer | CATTCCTTAGTGTGTTTCTACTACAGG | 1754 | CSMD3 rev primer | AGAATCACTTCCACTGAGTTCAGGTA |
| 1755 | CSMD3 for primer | AAAGTCAGTCTTTAATCAGTAACTGATGCA | 1756 | CSMD3 rev primer | GCCAACTCAGCAATCTTCTCTGT |
| 1757 | CSMD3 for primer | GTTCCTTACCATGACAACTTGGC | 1758 | CSMD3 rev primer | ACCTATTACTGTGATGCTGGTTATGTTC |
| 1759 | CSMD3 for primer | CCATTCCAAGTCTGGTGCCATT | 1760 | CSMD3 rev primer | agtgccaactgTATTTTAAAGCAATTAATGT |
| 1761 | CSMD3 for primer | AACATGGAATGTTCATATTTACCTTTGTATTC | 1762 | CSMD3 rev primer | GAAGTTTTCAAGACAGCAAGTTACCA |
| 1763 | CSMD3 for primer | ATGAACGCCAAGGAGATAACATAGTC | 1764 | CSMD3 rev primer | GCACCCTGTGGAGGCAATTT |
| 1765 | CSMD3 for primer | ACATGACTATTTACATCACCTTCCTATTTT | 1766 | CSMD3 rev primer | AGCACCCTCAATATAGTAACCATCCA |
| 1767 | CSMD3 for primer | GGGTTGGTTAGGCACTTCAG | 1768 | CSMD3 rev primer | TCTCCTGGCTATCCTTTTCCATATGA |
| 1769 | CSMD3 for primer | AAAATCTATGACAGTATCCTACCAATACAGGA | 1770 | CSMD3 rev primer | GGCTACACTCTCCACGGAAGTA |
| 1771 | CSMD3 for primer | GTGGCCTTGGTCACTGATCT | 1772 | CSMD3 rev primer | AATCCTCCTCCTAAATTTTGCCTTTAATCA |
| 1773 | CSMD3 for primer | TTACTGGTATACACAAGTTGAAAGCCTT | 1774 | CSMD3 rev primer | ACGACTCATCTACTAGGTGCTTTTACT |
| 1775 | CSMD3 for primer | GAACAATATACAAGTATGTCTGAGTTCACCT | 1776 | CSMD3 rev primer | GTTCAAGCAGGAAAGGGAATCAATATTT |
| 1777 | CSMD3 for primer | TCATGGTTGTTTTCATAGΠGAGTGGAT | 1778 | CSMD3 rev primer | ACTTCAATATTCTGTTTTACCTGCAGCT |
| 1779 | CSMD3 for primer | GGTGCACTCCACACTCGT | 1780 | CSMD3 rev primer | CTCAATATGGTAGTCGAATCGGGTT |
| 1781 | CSMD3 for primer | CTCACCAGAGAATGTTATGTTAAATCCTTCA | 1782 | CSMD3 rev primer | TGACTGGTTCAGATCTTCCTCCAA |
| 1783 | CSMD3 for primer | GGGTAAAACTGCCATTCTCTGTGA | 1784 | CSMD3 rev primer | ACTCTCTGTGTTTTACTGATCTACCTGT |
| 1785 | CSMD3 for primer | AATAATACAAAAAGTAGGGCAAACAGAATG | 1786 | CSMD3 rev primer | ACAACAGACAACAGTCGTTCCAATA |
| 1787 | CSMD3 for primer | ATTTGGTAAATGCAAAAGAGAATGTCAA | 1788 | CSMD3 rev primer | ATTGTGAGTGGGTGATTGAAGCT |
| 1789 | CSMD3 for primer | GTAGGATAATGTGTTTCTTGTTGTGGT | 1790 | CSMD3 rev primer | TGGATGGAAAAGTAATGTGGAGTGG |
| 1791 | CSMD3 for primer | GAACCAATATACATGATCACTTTTCAACTC | 1792 | CSMD3 rev primer | GGAATTTCAGGCTGACCACTCA |
| 1793 | CSMD3 for primer | CAGCCCCAGTAAAGGTTCCA | 1794 | CSMD3 rev primer | GGACGATAATCTCTGATCCAGGGA |
| 1795 | CSMD3 for primer | AAGCAATTATATGCAAGGACAGGGT | 1796 | CSMD3 rev primer | TGGAGAGAAATCCATTGTTTGTCCCGA |
| 1797 | CSMD3 for primer | GGCATTCAAACCTTAAAACATCACGA | 1798 | CSMD3 rev primer | TGCAACCCTTGGAAGGTGAC |
| 1799 | CSMD3 for primer | GTTTGAAGGTGCAGCCACAT | 1800 | CSMD3 rev primer | AATTTGTATGCAGAGGATGTTTTAATAACC |
| 1801 | CSMD3 for primer | CACTGTCCTAGGATCTCCAACTTC | 1802 | CSMD3 rev primer | CACAACGCAGTATATTTAAGGTGCAT |
| 1803 | CSMD3 for primer | CCTTATTTGTATTCACTGCTGTGATGAC | 1804 | CSMD3 rev primer | TCTCTTTTAGTGAAAACGTGTGGCT |
| 1805 | CSMD3 for primer | CTAAAATCTGATCCGATTCTCTTCCCA | 1806 | CSMD3 rev primer | TCCCTGCTGTTGCAGTAAATGA |
| 1807 | CSMD3 for primer | ATTACCAAAATATAGCTTTGGCTGTAACT | 1808 | CSMD3 rev primer | GAAGACCAAGACATGCTGAACAGATA |
| 1809 | CSMD3 for primer | ACACCAAAATTATTTTACATATCAAAAGAGGCA | 1810 | CSMD3 rev primer | GCTCAGACTGCATTTTGTTACAGACA |
| 1811 | CSMD3 for primer | GCTTCAACAACATCAATGCAAGGT | 1812 | CSMD3 rev primer | GGGACACAATTTCACTCATATTTACTGA |
| 1813 | CSMD3 for primer | CCCCAGGCTCTGCTACAATG | 1814 | CSMD3 rev primer | TTTGTCATTTTTAACAGCTGAAGATGCTT |
| 1815 | CSMD3 for primer | ACAGGCACCAAACACATTTTTCAAATA | 1816 | CSMD3 rev primer | TCAGCTCACCTGCATAGCC |
| 1817 | CSMD3 for primer | CACAGCTGTAGCGGATCTTGT | 1818 | CSMD3 rev primer | GAAATAACTGTAAACTCTCCTTTTCCTCCTT |
| 1819 | CSMD3 for primer | ACCCAGGAAATATTTCTCTCCAGTCT | 1820 | CSMD3 rev primer | CAGTCATTTGCTCTAGAAGAAGAATACGA |
| 1821 | CSMD3 for primer | GCGCCATCATGGGAGTTGA | 1822 | CSMD3 rev primer | ATGTGGAGTTGGTTCCTTTGCT |
| 1823 | CSMD3 for primer | AAGTAAGCTGCCAGCCTCTC | 1824 | CSMD3 rev primer | TGGATTTACGTTTTGGAACCTCGT |
| 1825 | CSMD3 for primer | GAAGTCTAGGCGGCCACATTTA | 1826 | CSMD3 rev primer | GGGCTGAGGAGCAGGAATTAG |
| 1827 | LRP1B for primer | AGAAAGAGGTAATGCTGATGCCAA | 1828 | LRP1B rev primer | GAAATTGGTATAAGAGAGACAGTGGCATA |
| 1829 | LRP1B for primer | TGGTGAAATTCAGAACCATAAAACAGA | 1830 | LRP1B rev primer | ATCATGATCACAACGATGGAGGT |
| 1831 | LRP1B for primer | CAACAAACATTATGTTTCTGCACCTAGT | 1832 | LRP1B rev primer | CGTCCTCTTGGTGACTTTGATAACC |
| 1833 | LRP1B for primer | GAGAAACTTACTTGTGCTGATATGATCAGA | 1834 | LRP1B rev primer | GGGCTTTGTTATTCGCTGATCTGT |
| 1835 | LRP1B for primer | AGAACAGGGATTTCATGTACATACAAAGG | 1836 | LRP1B rev primer | AGTTATGTGATGGCTACTGTTACAATGG |
| 1837 | LRP1B for primer | ACTGAACTGCAATGTCCATTGGT | 1838 | LRP1B rev primer | TGTGAGGTTGACAAGTGTGTAAGG |
| 1839 | LRP1B for primer | GGTGCTGCTTTTACTTACAGTACTGA | 1840 | LRP1B rev primer | AAACTGTGGTAAGACAGTCTGTGAG |
| 1841 | LRP1B for primer | AAGGAAAAGTGAAAAAGATGTGAAATACAA | 1842 | LRP1B rev primer | GTTGGCCCAGTTATTCAGGAGAA |
| 1843 | LRP1B for primer | AATTTCACTCCATTTCACGAGGT | 1844 | LRP1B rev primer | GGAAAATATTTGATTAATGGCACCTGCAA |
| 1845 | LRP1B for primer | GTACTCTACTGAACCATGGCCAAA | 1846 | LRP1B rev primer | CAGGTTTATTACATCCACATAGGATCGA |
| 1847 | LRP1B for primer | AACACTCTCAGTAACAATTAAAAATGAAGACAA | 1848 | LRP1B rev primer | CAGTGTTCTGTATGATGGCTCTAATTCA |
| 1849 | LRP1B for primer | TGTTAACAGTAATATACTTGTCATTCTGCT | 1850 | LRP1B rev primer | GGATGGTACACTGAGAAGGATTTTAGT |
| 1851 | LRP1B for primer | GGTCCAGTGAGTAGTGTAGTAACTGA | 1852 | LRP1B rev primer | TGGTGTGTTGTTTTGTTTCCTTGTAG |
| 1853 | LRP1B for primer | CTTACAATCAAGCCACTGTTTGCT | 1854 | LRP1B rev primer | AATTCAAGAATAACAGGGATGGATGTATATT |
| 1855 | LRP1B for primer | TTGTAATAGGATTTGCAATAGTGGTTATAATCT | 1856 | LRP1B rev primer | GTTTGGCACATGTTCCCATCAAT |
| 1857 | LRP1B for primer | GTGATCTCACAATTTAGAAGTCTTGGG | 1858 | LRP1B rev primer | CTGTCGCTGTAAGCCTGGAT |
| 1859 | LRP1B for primer | CTATTCTGCATCCTTGCTCATCTGA | 1860 | LRP1B rev primer | AGGTAAGCTGACATATAAAGCAAGGC |
| 1861 | LRP1B for primer | AAATGTTTGTACTCCAAGTCATCAGAGAA | 1862 | LRP1B rev primer | CAGTCGGAGCAAATGTGCAA |
| 1863 | LRP1B for primer | CCTCCCCACTTCATTGATTCATG | 1864 | LRP1B rev primer | ACGACTGTGGAGACAACTCTGA |
| 1865 | LRP1B for primer | CCCAGAAATGTAGTTTGCAGAGAGAATT | 1866 | LRP1B rev primer | CAGAAGAATATGGATCCTGCTGACTG |
| 1867 | LRP1B for primer | TTCTCTTCATCACTGCCATCCAC | 1868 | LRP1B rev primer | TTGTATTTGAACAGATGGACTGTGTGA |
| 1869 | LRP1B for primer | TGTTTGCCCAAACCTAATTCATGTAATTC | 1870 | LRP1B rev primer | GCCAGTGATGGATGTATTTCAGCA |
| 1871 | LRP1B for primer | AAATGATATATTACTTCAATCCTTTTACCTGGC | 1872 | LRP1B rev primer | GTGTATACCAGCAAAATGGAAATGTGAT |
| 1873 | LRP1B for primer | ACCATTGGAACACCGGAACTG | 1874 | LRP1B rev primer | ACATAAGTTTTCTTGTCTTTTGAGTGTGT |
| 1875 | LRP1B for primer | CAGAACTAAAAATTGCCATCCTGCT | 1876 | LRP1B rev primer | TGGCGGTGTGATAGCCAAA |
| 1877 | LRP1B for primer | TCGGGAATACAGTTGTTGTTTTTACACT | 1878 | LRP1B rev primer | GATTTGGCATGGCAGTGAGG |
| 1879 | LRP1B for primer | AGTTGGCCTCGTCTGATGC | 1880 | LRP1B rev primer | ACAGCTGTTCTCCAGACTATTTCC |
| 1881 | LRP1B for primer | ACTTATGTTCATTAGTTAGGAATTTAATCACCT | 1882 | LRP1B rev primer | GCTCTACCAGCTTTCATCTGTGA |
| 1883 | LRP1B for primer | ACAGAGTCCAGTCCCACACT | 1884 | LRP1B rev primer | TATGTTTAAGAAGTTGTGAGGTAAGATTACCA |
| 1885 | LRP1B for primer | GGTTCATCAGATCCATCACCACA | 1886 | LRP1B rev primer | TCCATTATATATGCACTGGGTTCTTCAAC |
| 1887 | LRP1B for primer | GTGCAGTTGGATAAGCAAGTCCTAT | 1888 | LRP1B rev primer | GTCTCCAAACATCTCTGCATGATAAATAATG |
| 1889 | LRP1B for primer | ATGGAAAGAATAATTACCATCAGGTTGTCT | 1890 | LRP1B rev primer | CACTCAGCCGTGCCCATA |
| 1891 | LRP1B for primer | AGATGTAGTCTTCAAACAATGTTAGTGCAA | 1892 | LRP1B rev primer | TCTGTCAGCCCATGTAGTATTGA |
| 1893 | LRP1B for primer | CGGCCCAGTAGAGTCTACGA | 1894 | LRP1B rev primer | TTGGATTGACTGCTGCGAGT |
| 1895 | LRP1B for primer | AGTTTTTACAAACCCAGCTTGAGGA | 1896 | LRP1B rev primer | AGTATCCAAACTCAATGGCTTGTACC |
| 1897 | LRP1B for primer | TCCAATCCAATCGACAGCAAGT | 1898 | LRP1B rev primer | GCTCACTTCTTATTTTAGGGATTTACGC |
| 1899 | LRP1B for primer | CGGCTAGAATCGATCCAATAGATGAAT | 1900 | LRP1B rev primer | TTCTATGAACTATCATTGACAACATAGTCTTTT |
| 1901 | LRP1B for primer | TTAAATGTTACGGTGTCATCTTGGGA | 1902 | LRP1B rev primer | TTGTGTTGGTGTTTAGATGAAGAACCT |
| 1903 | LRP1B for primer | CGATTTGCAGCCATTTGGGTTA | 1904 | LRP1B rev primer | CAAAACATGTGTAGACATTGATGAATGC |
| 1905 | LRP1B for primer | GCTTTCTTAACCTCCAATTCTAATGTATCGT | 1906 | LRP1B rev primer | TGTTTGAGTAAGAAAGTCAGTGGATGTT |
| 1907 | LRP1B for primer | CACAGTCATCCTTATTGTCGCAAA | 1908 | LRP1B rev primer | GTATAAAACAATGGCATGCCCTTTATGT |
| 1909 | LRP1B for primer | CATTGAAAACACTTTTAGAGTAAATGCGGTAT | 1910 | LRP1B rev primer | CTAAATACTCAATGGCAGTGTGATGGA |
| 1911 | LRP1B for primer | CCGCCCATCAGCACAACTA | 1912 | LRP1B rev primer | GGTGTAGTGTACATTGAGGAGATGA |
| 1913 | LRP1B for primer | AAAACAAATAACCAAGGATGCTGCAA | 1914 | LRP1B rev primer | CCCCGACATTGGCTTTGTG |
| 1915 | LRP1B for primer | CCTGGCAGCTGAACATGTCA | 1916 | LRP1B rev primer | TGGTAGACATTTTCCAGTGGACA |
| 1917 | LRP1B for primer | AATGTCTCACCACAGTGGAATTCAT | 1918 | LRP1B rev primer | CACAGTTCAAAACAAACACAAATGTGA |
| 1919 | LRP1B for primer | GCACTTTAATTCATCTGAATAGTCTCCACA | 1920 | LRP1B rev primer | ACTGCACACATTTCTATAAGCTTGGA |
| 1921 | LRP1B for primer | TCATCTGAAGCATCTGCACAATCT | 1922 | LRP1B rev primer | CCATCTTTGCATTTGTTTATGTACCTGT |
| 1923 | LRP1B for primer | ACTTACCACAGTAGAGCAGTTTTTCATC | 1924 | LRP1B rev primer | GCAGCTAAAAATTCCTCCTGCAA |
| 1925 | LRP1B for primer | TCTAGCAATATTCGGTCCCCTCT | 1926 | LRP1B rev primer | CCAATACACCCTCTTTATTGTAGGTGAA |
| 1927 | LRP1B for primer | AATAAAACTACACTGTTTCTTACTTTTGACATT | 1928 | LRP1B rev primer | CGGCCTGGAGCATTTGACA |
| 1929 | LRP1B for primer | GTAGAGCTTGTCCAGTACAGTGTATC | 1930 | LRP1B rev primer | AATGCTTTTTCTGTCCAGAAGTCATTT |
| 1931 | LRP1B for primer | ATTACTGTACTTACTTTCAACAATTACTTGTCT | 1932 | LRP1B rev primer | GGTACCAACCGAATCTTTTACAGTGAT |
| 1933 | LRP1B for primer | ATGGTAAATCCTACAAGGGTAAAGCA | 1934 | LRP1B rev primer | AATGTGGATATGTTTTCAGTTGCAGTC |
| 1935 | LRP1B for primer | GCTTCCTGACAGCACCATCT | 1936 | LRP1B rev primer | TCGATATTTAGGAAAATAAATTGTACTGGTGTG |
| 1937 | LRP1B for primer | CTCTGAGCCATCCAAGCGA | 1938 | LRP1B rev primer | TGTCTTTTTGCATAGCTCTGCATACT |
| 1939 | LRP1B for primer | TCCAGGCCTTGGGAAATAATTACATAAC | 1940 | LRP1B rev primer | ATAAACACCTCTATTATTTCTCCTAGGTAACAT |
| 1941 | LRP1B for primer | CCAGCAATCCAGTCAACAGC | 1942 | LRP1B rev primer | GAAAATGATACCATCTACTGGACAGACA |
| 1943 | LRP1B for primer | AGGAAAAGATTGTATTCCCATCATGTT | 1944 | LRP1B rev primer | GGATGCTTTGATGCCTATATCAGGA |
| 1945 | LRP1B for primer | ACATGACATACGGTTCTTTTGGAGAT | 1946 | LRP1B rev primer | CATTTTCATTCTATGCAGGCAGCAA |
| 1947 | LRP1B for primer | CATAATAAATCAACCCCCAATCAATACAAAG | 1948 | LRP1B rev primer | CAACCTGGATGGTGGTAATTTAGAAGT |
| 1949 | LRP1B for primer | GTTCCATTCCCTGAACTGATCCAA | 1950 | LRP1B rev primer | CCCTCAAATCCAGTGGTGCT |
| 1951 | LRP1B for primer | CTTACCAACTGGCTCCTTCTGAT | 1952 | LRP1B rev primer | TTTTGAATTAAGAAAACTCTACTGGACCGA |
| 1953 | LRP1B for primer | GCCTTGCCACATTAATTTGCGTTT | 1954 | LRP1B rev primer | TCATTCACCTTTAAATCTTCTCTTTCCTGTT |
| 1955 | LRP1B for primer | CCGTTAATAAAAGCTCGTTTAATGGTTTGT | 1956 | LRP1B rev primer | TCCATACTTTAACTTCATCACGGCATT |
| 1957 | LRP1B for primer | GGTCTTCTTGTCTGAAGAAAGCTTCAT | 1958 | LRP1B rev primer | GTAATTACAGCTCCCAATCCTTGT |
| 1959 | LRP1B for primer | GGGATGGGAAAGGTATTCATGACC | 1960 | LRP1B rev primer | GTCTCTGGACTAGTTGAATGAGTTTTGA |
| 1961 | LRP1B for primer | GACATGTTAGGACACTTTCCAATGT | 1962 | LRP1B rev primer | CCTAATGGACTAACTGTGGACCACT |
| 1963 | LRP1B for primer | TGCCAAAATCAATCTATAAAAGGCAAACTTC | 1964 | LRP1B rev primer | AAACTAGATGGCTCCCTAAGAACTACA |
| 1965 | LRP1B for primer | TCCAGTATATGTTTCCTGCTATCCAGT | 1966 | LRP1B rev primer | ACAATCCTTGAATGGGTGCACA |
| 1967 | LRP1B for primer | GATTGAAGTGAAAATCAAGTGCTATTGTGT | 1968 | LRP1B rev primer | tCAGATCCTTTTGAAGCATTCATCATCT |
| 1969 | LRP1B for primer | GCAACACACACAAAATAGTTACTTACCA | 1970 | LRP1B rev primer | AGCAATCATCTAAAGTGCAGCCA |
| 1971 | LRP1B for primer | ACATGTTTTATTGTCTTTGTTGAGTTGAAGT | 1972 | LRP1B rev primer | CGCTGAACAATGGAGGCTGTA |
| 1973 | LRP1B for primer | TGGAGAAGTCAGTACAATTAAACATGCAAT | 1974 | LRP1B rev primer | CACCCAAGCATCCTTGTGCTA |
| 1975 | LRP1B for primer | GGTCCACACAAGAAACTGTCACA | 1976 | LRP1B rev primer | GACCTAATCACAAAAATCAAACTGACACT |
| 1977 | LRP1B for primer | ACCTTCTTTAGTACAATTGATCTGGGC | 1978 | LRP1B rev primer | TGTGTTCACTCTTGCTTTGATAATCAGT |
| 1979 | LRP1B for primer | TCTGAAGAAAGCCAGGCAGTC | 1980 | LRP1B rev primer | GCAAAAGTGGAAGATGCATTAGCA |
| 1981 | LRP1B for primer | AGCCCTTTAAGGAGGGTGT | 1982 | LRP1B rev primer | GAAGACGACTGTGGTGACCAG |
| 1983 | LRP1B for primer | CTTCCCTGTCACACAGCCAT | 1984 | LRP1B rev primer | ATATCCTTGATGTTCAAAATGCCTCT |
| 1985 | LRP1B for primer | CTCCATCACAAAGCCATCTCTTG | 1986 | LRP1B rev primer | CCAATTTTAAATAATTAATCTTGCTTCCCCCT |
| 1987 | LRP1B for primer | TGAATCCTCATCGCTTCCGTCTA | 1988 | LRP1B rev primer | ACTCTAGTTAATCCTGGAGAAGCACT |
| 1989 | LRP1B for primer | TTAATTCCTTTAGTTTCTTCTGCTTCTAGTC | 1990 | LRP1B rev primer | GGTGTGTGCTTGTGCCGATA |
| 1991 | LRP1B for primer | CTCCTGGGATAGCCAAGCAA | 1992 | LRP1B rev primer | CACCTCCTAGAAAGAACGTGAATC |
| 1993 | LRP1B for primer | AGCAATGTCACCTCACTTGTTATCAA | 1994 | LRP1B rev primer | ATTTTACAGATTGTTTACAGTGGGAGAGAG |
| 1995 | LRP1B for primer | AGAGTTAAACCGTTTGGCCACA | 1996 | LRP1B rev primer | CTTAGTTGGATGTATTGGACAGACTGG |
| 1997 | LRP1B for primer | CATCTATCTTCTGCCGGCCAAT | 1998 | LRP1B rev primer | CCAAGATAGCTGATGAATACATGATCCC |
| 1999 | LRP1B for primer | ACAAGTCCGAGTTTTGTAACTGCT | 2000 | LRP1B rev primer | TTTGAACTTGAATTTCACGCATGCT |
| 2001 | LRP1B for primer | TGATAAATTCGGATTCCCCAAGCA | 2002 | LRP1B rev primer | ACTGTGTTTGAAGATTATTTGTATGCAACC |
| 2003 | LRP1B for primer | GCTCCAGATCAATCAGGGTGAC | 2004 | LRP1B rev primer | GTGTATGTGTTTTTCAGATGTGCAACA |
| 2005 | LRP1B for primer | CCTCCTGCTTTTGTTATCTGGATACA | 2006 | LRP1B rev primer | AAAATGGCAACTCTAAGCTCAGTCA |
| 2007 | LRP1B for primer | GTCATAACTTACCAATTTTAGCCTTGCA | 2008 | LRP1B rev primer | TCAAGATGAATGTGCTGTTTATGGTACA |
| 2009 | LRP1B for primer | ACATTTCTGTTAACTAAGCCTCTATAAACAA | 2010 | LRP1B rev primer | GATGTTACTGTGAGGATGGATTCGAAA |
| 2011 | LRP1B for primer | AAAATTTAATATTTCAGTTGCATTGTTCCACAA | 2012 | LRP1B rev primer | CCAGCTGTGCAATGGTGTCT |
| 2013 | LRP1B for primer | CCAAGGCAAGCAATGTGATTCAA | 2014 | LRP1B rev primer | TAACAGTTTTACCATTTAGAAAAGCTAACAC |
| 2015 | LRP1B for primer | GTCTGAATCATCAGGGCAGTCA | 2016 | LRP1B rev primer | CGAAACATTTTCATTTTCAGATCAGCAGT |
| 2017 | LRP1B for primer | GTGAGTAAGGCGAGGAGAAACTC | 2018 | LRP1B rev primer | GCTCGATTCCAGGCTCCAA |
| 2019 | CNTN6 for primer | GACTGTTTTGTTCCACCTGATTGTATG | 2020 | CNTN6 rev primer | TATAATAATAGAACACTTTACCTGCAGAAGAGT |
| 2021 | CNTN6 for primer | CCACATGATGTCATTTTTCCTTTGGA | 2022 | CNTN6 rev primer | ggttcaGTGAGGAAGAACAAAATAGTGT |
| 2023 | CNTN6 for primer | TGAAGACTTTGAAACTAAAACAAGAAGCAC | 2024 | CNTN6 rev primer | TCCAAGTGACCGCATTGATGA |
| 2025 | CNTN6 for primer | AATGAATGCAGTAAGGACAGTATGg | 2026 | CNTN6 rev primer | CGTCTCTTGAGATACAAATCGCCTA |
| 2027 | CNTN6 for primer | GCTGCTAGCTCTTTTGATATTTAACAGG | 2028 | CNTN6 rev primer | acCTTACTTTCCAAGGGCAAAACA |
| 2029 | CNTN6 for primer | GGAACAGAAAATCCAAAATACACACCT | 2030 | CNTN6 rev primer | AACATAGTTGCTTACCTCTGGGTTG |
| 2031 | CNTN6 for primer | cgTACAAGCATCTTTATATGCCTTTTCC | 2032 | CNTN6 rev primer | ATATTTGTTTTCTGCAGCACATTGGT |
| 2033 | CNTN6 for primer | CTGGAGTCACTACATGTTAACTGAGT | 2034 | CNTN6 rev primer | CATCCGATAACAATATCCCCACCA |
| 2035 | CNTN6 for primer | CTTATGATTTACCAGTGTTGAAGAGCCT | 2036 | CNTN6 rev primer | GGCTATGCATGTATATGATCCAGCA |
| 2037 | CNTN6 for primer | ACCATAGGCTAGCATTTCATAAGCTTT | 2038 | CNTN6 rev primer | GTCATGGGACACCTGGCAT |
| 2039 | CNTN6 for primer | CCCCTCCATTGAAGTGGTATTTGTAT | 2040 | CNTN6 rev primer | AAGACTTGATTCATTTTATTAAGCACTTAACAA |
| 2041 | CNTN6 for primer | CCTTTCTGTCTGCAGGAATCTGT | 2042 | CNTN6 rev primer | TGCTTACCTCTAACAATGATATCGGCTA |
| 2043 | CNTN6 for primer | GAGGATGTGCAAGTGGAAGACA | 2044 | CNTN6 rev primer | GTAGCAACAGCCTGCCAAC |
| 2045 | CNTN6 for primer | ACAATTATTTGTTTTTGAGTAGTCCCAAC | 2046 | CNTN6 rev primer | TCATATTCCACCCAAGGACTCAAAC |
| 2047 | CNTN6 for primer | GCAACAGCATTGGGATTGGA | 2048 | CNTN6 rev primer | AGAACTGTATCATACGAAATCTTCCACAC |
| 2049 | CNTN6 for primer | TCAAGGTTTGTCTACAGAAATGAAAGCA | 2050 | CNTN6 rev primer | GACAACTTACCATCTTCCCCAGA |
| 2051 | CNTN6 for primer | TCTCCAGAGTTTTTCTGCTTCTGAAA | 2052 | CNTN6 rev primer | TGCGAAGTCCATTGGCACA |
| 2053 | CNTN6 for primer | AGTCAGTGGAAATGTCACAACCA | 2054 | CNTN6 rev primer | ACGAGACTTTTTGGTGGTAACATTGA |
| 2055 | CNTN6 for primer | GATGCATCATACTTTGACCATGAGC | 2056 | CNTN6 rev primer | AGTTTGTCAGCTTCCAGGCAAT |
| 2057 | CNTN6 for primer | GGCAAAACAGACAGAGTAAAACTCA | 2058 | CNTN6 rev primer | AATTTCCTCACTGCTGCTTCCA |
| 2059 | CNTN6 for primer | GGGTTTTGAAAAATTAATCTAACAGGCA | 2060 | CNTN6 rev primer | ATCACAATGACAATGGAAAGAAAATGGG |
| 2061 | CDH11 for primer | GTTCTTAAGGCCAAATTTGTATCGTTATTGT | 2062 | CDH11 rev primer | CCACCACAGATTCAGACTTGGA |
| 2063 | CDH11 for primer | CCCTTACCCAGGAGAATGACGAT | 2064 | CDH11 rev primer | CACTGCTCTCCTGCAACG |
| 2065 | CDH11 for primer | AAATACTTGTGCTATGCAATGTAAACG | 2066 | CDH11 rev primer | CCCCTGAAATCATTCACAATCCAAA |
| 2067 | CDH11 for primer | TAGAGTCTGAAGCGATAATACTTGGT | 2068 | CDH11 rev primer | AAACTACAAAACCTCTGGATAGAGAGGA |
| 2069 | CDH11 for primer | CGTGGTTCCTACAGGGCTTT | 2070 | CDH11 rev primer | TGCATGCCAAAGACCCTGAT |
| 2071 | CDH11 for primer | CATTTTCTTGGACTTCGTGGATGTAA | 2072 | CDH11 rev primer | AGCCAACGTGCACATCGA |
| 2073 | CDH11 for primer | GCTGCCTCTACCTTCAAGCTATA | 2074 | CDH11 rev primer | CCTATAAAACAATCGGGTCTTTCTCTTCC |
| 2075 | CDH11 for primer | TTACCCTAACTCACCTTTTTCAGCTTT | 2076 | CDH11 rev primer | GCTAAAGATCCAGACATTGGAGAAAATG |
| 2077 | CDH11 for primer | GCAGGAATAGGTTAGAGGAGGGA | 2078 | CDH11 rev primer | ACAACCAAAGTGACGATCACACT |
| 2079 | CDH11 for primer | AGAGTCCGCCCATATGTCCA | 2080 | CDH11 rev primer | CGAATATCTGTGATGTTGCCTGCT |
| 2081 | CDH11 for primer | GTCAGGCCTGGGACTTCTTA | 2082 | CDH11 rev primer | CCCACTTATGGAAATAGCGCCAA |
| 2083 | CDH11 for primer | CGTACGCACCCACATTGGA | 2084 | CDH11 rev primer | GAGCCACCGTCGGAATTCA |
| 2085 | CDH11 for primer | GTACTGGGCTCTCTCTTCTCGAT | 2086 | CDH11 rev primer | TCAGATATTGACTCTGGTGATGGGA |
| 2087 | CDH11 for primer | CTTTTGCTTCTTTCTGGCCACA | 2088 | CDH11 rev primer | GAACCAGTTCTTCGTGATAGAGGA |
| 2089 | CDH11 for primer | CTCCTTGCCCTTCTCATGGT | 2090 | CDH11 rev primer | ACCAAAAATGAAGGAGAACTACTGTTTACA |
| 2091 | AR for primer | AGCTAGCTGCAGCGACTAC | 2092 | AR rev primer | CCCAGCCCTAACTGCACTTC |
| 2093 | AR for primer | GTGAGGATGGTTCTCCCCAA | 2094 | AR rev primer | CTCTGGGACGCAACCTCTC |
| 2095 | AR for primer | CCTCCGGACGAGGATGAC | 2096 | AR rev primer | GCCTCGCTCAGGATGTCTTTA |
| 2097 | AR for primer | CATGCAACTCCTTCAGCAACA | 2098 | AR rev primer | GGCGTTGTCAGAAATGGTCGA |
| 2099 | AR for primer | ACAACGCCAAGGAGTTGTGTA | 2100 | AR rev primer | GCGGGTGGAACTCCCAAAA |
| 2101 | AR for primer | CGAAGAAGGCCAGTTGTATGGA | 2102 | AR rev primer | GGCCGAGTGTAGCCGTA |
| 2103 | AR for primer | TGGTGAGCAGAGTGCCCTAT | 2104 | AR rev primer | CGAAAGGCGACATTTCTGGAA |
| 2105 | AR for primer | CATTGACTATTACTTTCCACCCCAGA | 2106 | AR rev primer | ATGTGCAAGACCCTTTACCTTCA |
| 2107 | AR for primer | AGAGACTCTGGAAACTCATTATCAGGT | 2108 | AR rev primer | CGAAGACGACAAGATGGACAATTTTTC |
| 2109 | AR for primer | GTTGCATTGTGTGTTTTTGACCA | 2110 | AR rev primer | GTGCTGGAAGCCTCTCCTTC |
| 2111 | AR for primer | ACAACCCAGAAGCTGACAGTG | 2112 | AR rev primer | CAAAGGAGTCGGGCTGGTT |
| 2113 | AR for primer | ACGACCAGATGGCTGTCATTC | 2114 | AR rev primer | CCCCAGGAGCACTTACTCATT |
| 2115 | AR for primer | CTAATGCTCCTTCGTGGGCAT | 2116 | AR rev primer | TGCAATGATACGATCGAGTTCCTTG |
| 2117 | AR for primer | ACATCCTGCTCAAGACGCTT | 2118 | AR rev primer | TATTATCATTGGCTCTATCAGGCTGT |
| 2119 | AR for primer | CATCAGTTCACTTTTGACCTGCT | 2120 | AR rev primer | CACTGGGTGTGGAAATAGATGGG |
| 2121 | AR for primer | AAACGAATGCAGAGTGCTCCT | 2122 | AR rev primer | ACAACACTCACTACCCTAGAAACTGA |
| 2123 | ELTD1 for primer | AATTTGGCAACTGATGTTACATGAATATAC | 2124 | ELTD1 rev primer | GCTTACCTCTTCACAGTCAGCAA |
| 2125 | ELTD1 for primer | ACCCTGCAGTGTGACGAAAA | 2126 | ELTD1 rev primer | TGTATCCAATTTAAGATGAAAACTATCTCTTCT |
| 2127 | ELTD1 for primer | AGAAATGCAAAGCACATTCAAAATCG | 2128 | ELTD1 rev primer | TCTAAGCCCAGCCGTGGTA |
| 2129 | ELTD1 for primer | CTTGTGCAAAAATCCCTTGTTGTAGA | 2130 | ELTD1 rev primer | TCTAGCTCTTCTGTTCAATCATTGCC |
| 2131 | ELTD1 for primer | CATAGGACAAAAATAGAGATTAGGATGCCT | 2132 | ELTD1 rev primer | GTGAAATTCAAAGCACCAGGACAA |
| 2133 | ELTD1 for primer | AATTTAAGTGACACAACAAAGAGTCCA | 2134 | ELTD1 rev primer | TCAAACCCACCCACATTATATGAACTT |
| 2135 | ELTD1 for primer | ACATGTACATATGACATAATCAAATGCATG | 2136 | ELTD1 rev primer | CCAAAGAGAAAAGCTGCATATGATTCAA |
| 2137 | ELTD1 for primer | GGACCCTTGTTTCTTACCTATATCCG | 2138 | ELTD1 rev primer | CACATCTTACAAAACTCATGCACACT |
| 2139 | ELTD1 for primer | TCCTATGATTCACAGATAACTTGTCCCA | 2140 | ELTD1 rev primer | CTTTCATTTGTGAATAGTGTAGGATTCCA |
| 2141 | ELTD1 for primer | GACGGTTCCATCATTAGTGATAAACCT | 2142 | ELTD1 rev primer | TGAATGTGGAAATTTAACTCAGTCCTGT |
| 2143 | ELTD1 for primer | AGGACAGAAAAGCAGTCCCATC | 2144 | ELTD1 rev primer | GCAATGGAATTGAAGCCTGCTA |
| 2145 | DPP10 for primer | CACTGTCCAGGGTCCTGAAA | 2146 | DPP10 rev primer | GAATGAAGACACATGTGCAGAAGC |
| 2147 | DPP10 for primer | TTTTCTATCTCGGTAGGAACTGGGA | 2148 | DPP10 rev primer | GGGTTAAGAGGATGACTGACATAGT |
| 2149 | DPP10 for primer | AAAAACAAGCATAAGATAGGCATCTAACCT | 2150 | DPP10 rev primer | TCTGGATCGTGAAGCACAAAGTC |
| 2151 | DPP10 for primer | TTTTGCACGTTTTCTCCCCTAC | 2152 | DPP10 rev primer | AGTTTAATGACATGTCCATTCTCGCT |
| 2153 | DPP10 for primer | TCTTTTTCTAGGTAACCTTCAAAGCATCA AGGTAACCTTCAAAGCATCA | 2154 | DPP10 rev primer | GAAGATCACTCCTTTACCTGTTTGACA |
| 2155 | DPP10 for primer | TACTAAATTGTTGGTGTAGATATCAGTTTGT | 2156 | DPP10 rev primer | ACTGCAAGACGGAGTCCTCTA |
| 2157 | DPP10 for primer | AGTTTCAAAAGGCATCCTAACGT | 2158 | DPP10 rev primer | TTTCCAGAAGATGTCAGTCGCAAT |
| 2159 | DPP10 for primer | GGTGTACAATGGAGATTTCAAGGACA | 2160 | DPP10 rev primer | CACCAGTGGGCGATGTGA |
| 2161 | DPP10 for primer | TCATTTTCTCAAAGGCAGGTCAAGT | 2162 | DPP10 rev primer | TGAAATTGCATACCTTGATTTAAAGCTGTC |
| 2163 | DPP10 for primer | CCATGCAGGAATTAACAGTAGTAGG | 2164 | DPP10 rev primer | TCATAAAGAATTTGCTGCCGTCTCTA |
| 2165 | DPP10 for primer | GTGGAGAATTTCACCACGTAGCTAT | 2166 | DPP10 rev primer | AGGTTTAGAATGCCACAAGAACTG |
| 2167 | DPP10 for primer | GCAAATTACCGTGCGGCAT | 2168 | DPP10 rev primer | AAGATGGCATTTGACAACGCTAAC |
| 2169 | DPP10 for primer | CTGTGTCTAATGCTTGTGTTGTTTTCTTT | 2170 | DPP10 rev primer | GGAGATCCTTGCACACTGCTT |
| 2171 | DPP10 for primer | CCTTTTTCTTTCTCCAGTGCTTCTACT | 2172 | DPP10 rev primer | ATAATAAGAAATGTTGATTCATGGGACTAAAAC |
| 2173 | DPP10 for primer | CACATATTTTTAAATTCCAGGTCCAAGGG | 2174 | DPP10 rev primer | CGATGTCTAAGACTAATGCCAACCA |
| 2175 | DPP10 for primer | TTTGTTGTGTCACACTGATTCTGTTTAATG | 2176 | DPP10 rev primer | TCAGGATAGCTTCCTTCAGCATAGA |
| 2177 | DPP10 for primer | TTCTACCAGATTTTCAGTTGCTCTTGT | 2178 | DPP10 rev primer | GACATTATCCATGTCAATGAGTACGGA |
| 2179 | DPP10 for primer | GCAAGATTTGATGGCAGAGGAAGT | 2180 | DPP10 rev primer | GAAAACAGAGAAGAAAAATGTGCATCCA |
| 2181 | DPP10 for primer | TTGTATTTTCCTTTATAGGCAGCCAGT | 2182 | DPP10 rev primer | AAAGGTCATACCCTATCAAACAGCAG |
| 2183 | DPP10 for primer | TGAAAAAGTTCTCATACAAGCAGTGT | 2184 | DPP10 rev primer | GCTTACCTGCATAGTATAATTCACTCCA |
| 2185 | DPP10 for primer | CTACCACAGGAACCAGAAGAAGATG | 2186 | DPP10 rev primer | ATCTCCGTAAGCTGCCCTTG |
| 2187 | COL11A1 for primer | GCAGCGTTGTTTTCTATACCATCC | 2188 | COL11A1 rev primer | TGGTCCTGTTTGTTTTCTTGGCTA |
| 2189 | COL11A1 for primer | CCAACTTCAAATCCGAACTTCTGATTC | 2190 | COL11A1 rev primer | TGCTTTATTTTACAGTCCAGAAAAGGCT |
| 2191 | COL11A1 for primer | ACTCACCGCACAACCATCAT | 2192 | COL11A1 rev primer | CATCAGTCAGCAGCCTGGTA |
| 2193 | COL11A1 for primer | AGGTAATAACATACCAGTTTTCCCCTCT | 2194 | COL11A1 rev primer | TCACCTACTCACTAACTTTTCTGTTCCT |
| 2195 | COL11A1 for primer | AACAGATTGGTACTTACTCCCTCAGA | 2196 | COL11A1 rev primer | ACCTTCTAGGTGAATATTGGATTGATCCTAA |
| 2197 | COL11A1 for primer | GATGCAAACTGTCAGAACATCACTC | 2198 | COL11A1 rev primer | TGCCTCTTCTTTATTTATGTCCTCTCTCT |
| 2199 | COL11A1 for primer | AGGGAAAAGTACTTACAGTAGAGCCTT | 2200 | COL11A1 rev primer | ATATAACAGAGACACAAATGCTCCAG |
| 2201 | COL11A1 for primer | gtgtgtgtgtgtATTATTGGAAATGAGTAGA | 2202 | COL11A1 rev primer | AGGGCTCCCTGGAACTCAA |
| 2203 | COL11A1 for primer | GATTTTAATCTGCAAAAGCAGGGCTA | 2204 | COL11A1 rev primer | AGGGACCTCCTGGCTTACC |
| 2205 | COL11A1 for primer | AAAATGGCATCTTTTTAACACAGATGCT | 2206 | COL11A1 rev primer | GCAGAAGGTCCTCCTGGAAAA |
| 2207 | COL11A1 for primer | GAAACTTACTCGTTTTCCAGGAGGA | 2208 | COL11A1 rev primer | ATTTATGTGGGCCACAAAATAATATCCA |
| 2209 | COL11A1 for primer | GGACCAGCTTCCCCTTTCTC | 2210 | COL11A1 rev primer | AAAACTTGTAAGATAACTCTTCTTTCCT |
| 2211 | COL11A1 for primer | TTCAGAGTTCTTACATCAGCTCCATTG | 2212 | COL11A1 rev primer | ATGTTGGAAACACATTTGAATTTGGTCTT |
| 2213 | COL11A1 for primer | TGACTGCCATGCATATAACACATACC | 2214 | COL11A1 rev primer | TATTTTTCTATTATATTTTCTTAGGGTCTGCCA |
| 2215 | COL11A1 for primer | CCTGAAGACCTATTGGACCAGGA | 2216 | COL11A1 rev primer | ATGTTTCTTTCTCTTCTCTAGGGAGGT |
| 2217 | COL11A1 for primer | TGTTCTCTCAGTAAGAAGTAGGAAACTCA | 2218 | COL11A1 rev primer | ACAATGCAATAGCATGTTAGAGATAAACTCT |
| 2219 | COL11A1 for primer | GAGACCAACAGGACCTTGAACTC | 2220 | COL11A1 rev primer | GCTAGATTATTACTCTTGTAGCTGCACT |
| 2221 | COL11A1 for primer | ACATTACTTACAGGAGCACCTTTCTC | 2222 | COL11A1 rev primer | ACTTCTTTTCCTGTATACTAGGGCTCA |
| 2223 | COL11A1 for primer | CATCATACTTACAACTGGACCTGGTG | 2224 | COL11A1 rev primer | TCCTCCTATTGGTATTCAATTCATATGGA |
| 2225 | COL11A1 for primer | TGAGCTCCAGGAAGACCTCTT | 2226 | COL11A1 rev primer | ATGTGTAGAAATTAATCTGTCTTGACGATGT |
| 2227 | COL11A1 for primer | CAAAGGAACATACCTGTGGTCCAA | 2228 | COL11A1 rev primer | TGTAGCTTTTACAAATAATACTTTGCCTCCT |
| 2229 | COL11A1 for primer | TTTCATTTTATATTATGTGGCTGTATCATACGT | 2230 | COL11A1 rev primer | GCAGATGCTATGAGAAAGAATTCAAACT |
| 2231 | COL11A1 for primer | TATGAAAAATGTACATCATTTTAAATCAAGGCA | 2232 | COL11A1 rev primer | AAATTGAACTGTCTTTGCTAGGGAGTAG |
| 2233 | COL11A1 for primer | ATCATACCCGTGCACCTTTCTC | 2234 | COL11A1 rev primer | TTGTGACTGAAAATGTTAATTGCCATGT |
| 2235 | COL11A1 for primer | TGATAATGAAACATCAGCTGCAAGAGT | 2236 | COL11A1 rev primer | CTGAAGGACCCAAAGGTCGAG |
| 2237 | COL11A1 for primer | ACTTACTCTGTCACCTTTTAGACCCA | 2238 | COL11A1 rev primer | GGTTACTCCAAAGGACTGATTAAACTCC |
| 2239 | COL11A1 for primer | TGACCAAATTAAGGCATTTTCTCTTGGA | 2240 | COL11A1 rev primer | GGGTCATCCTGGGAAAGAAGG |
| 2241 | COL11A1 for primer | GGCAGACCTTATACCTGCCT | 2242 | COL11A1 rev primer | AGGGTCTTCCTGGTCCACAA |
| 2243 | COL11A1 for primer | CCCCCATTATTTCAAAGGAGCTG | 2244 | COL11A1 rev primer | AACTTTTTATATTTTCCTCTGGCAGGGT |
| 2245 | COL11A1 for primer | ATGGTTTCTTAGGGCTTATATTCAAATATGAGT | 2246 | COL11A1 rev primer | CAGGGTATGGCAGGTGTAGATG |
| 2247 | COL11A1 for primer | CAACAAGCTTTCCCTAGAAAATCTTCA | 2248 | COL11A1 rev primer | GGCGTGCTTTTTCTTTTTCTCCT |
| 2249 | COL11A1 for primer | GCTTCACCTGGAAGACCTCTT | 2250 | COL11A1 rev primer | TCATGTTGAAATGATACATTGTCCAAATG |
| 2251 | COL11A1 for primer | GGAATAGATGTATCTTACCCTGTGACCT | 2252 | COL11A1 rev primer | ACATATCTAGACTGGAATTGtgatgatga |
| 2253 | COL11A1 for primer | GAGTGAACTGACACAGGATGTGAATAT | 2254 | COL11A1 rev primer | CGCTTAATCATGCTGTTGGCATTATTAG |
| 2255 | COL11A1 for primer | CCTTTTTCCAGGTTTTCCCGTT | 2256 | COL11A1 rev primer | CTGAAACCATCTCTCTTGATGCTATT |
| 2257 | COL11A1 for primer | AGCATCTTCCGTATGTACACACA | 2258 | COL11A1 rev primer | AAAGGACCAACCATCTCTGCTC |
| 2259 | COL11A1 for primer | AAGTCTGTATGACATGCATTTGTAATGAGA | 2260 | COL11A1 rev primer | GGTATTATGGGTCCTCCAGGTCTA |
| 2261 | COL11A1 for primer | ATCTTAAAACATTTACATACTGCAGGTCCT | 2262 | COL11A1 rev primer | GGGTTATATTTCCCTGGTAGAGATGT |
| 2263 | COL11A1 for primer | ACACTACTACTTACAGGCTCAACCA | 2264 | COL11A1 rev primer | aaaTGGTCTTACAGACATTTAGGCTGT |
| 2265 | COL11A1 for primer | AATCCTCTCCCGTTAGATATTCTTCAGT | 2266 | COL11A1 rev primer | TACCTTGCTTTACTAACAATAAACTTCATGT |
| 2267 | COL11A1 for primer | GCCTAGGAGCTTCTGTCTGG | 2268 | COL11A1 rev primer | ATCATGATTTCCATACATTTGGGATCA |
| 2269 | COL11A1 for primer | CCTCCGTCTGTGCTATTGTCT | 2270 | COL11A1 rev primer | TCCTTTGCAATGTAGTATGCACCA |
| 2271 | COL11A1 for primer | TTTTACTGTTGTTAACCCAGTTCATCTGA | 2272 | COL11A1 rev primer | AATGGAATCACGGTTTTTGGAACAAG |
| 2273 | COL11A1 for primer | GTTACTTACTTCCCGTCAGCGA | 2274 | COL11A1 rev primer | GCATGGTATTCAGCAAATTGGTGT |
| 2275 | COL11A1 for primer | GCACTGAGTTGTGCTTGCTT | 2276 | COL11A1 rev primer | GCACTAGATTTTCACAATTCTCCAGAGG |
| 2277 | COL11A1 for primer | CAGTAGGACCGACGGCAATC | 2278 | COL11A1 rev primer | AAACGGTGGCTCTGGGATTT |
| 2279 | COL11A1 for primer | CGTTTCGTTTTCCACCTAGAGGAC | 2280 | COL11A1 rev primer | TGGCTGTGTAGCAAACATCCC |

## Claims

1. A method for determining a subject's risk for developing esophageal and gastroesophageal adenocarcinoma (EAC), said method comprising:
(a) obtaining a non-endoscopic biological sample from the esophagus of a subject having gastroesophageal cells;
(b) performing cytology of the obtained biological sample of (a) to identify presence or absence of cellular changes, implicated in development of EAC; the cellular changes comprise presence of columnar cell metaplasia; optionally wherein the cytology is carried out by Papanicolaou stain, Haematoxylin-eosin staining, Gene chip arrays and Fluorescence *in situ* hybridization (FISH);
(c) determining from the obtained biological sample from (b) showing presence of cellular changes, implicated in development of EAC, the nucleic acid sequence of each gene in a panel of genes implicated in the development of EAC; optionally wherein the sequencing is carried out by next generation sequencing;
(d) detecting a change in at least one of the genes implicated in the development of EAC, the change being detection of presence of one or more genetic mutations or change in gene copy number in the panel of genes sequenced from the obtained biological sample in (c), wherein the panel of genes comprises one or more genes selected from the group consisting of CDKN2A, ERBB2, SMARCA4, and LRP1B, wherein the change is:
i) a threonine to methionine change at position 910 in the SMARCA4 amino acid sequence (NP_001122316),
ii) a glycine to serine change at position 1162 in the SMARCA4 amino acid sequence,
iii) a valine to alanine change at position 2091 in the LRP1B amino acid sequence (NP_061027),
iv) a serine to cysteine change at position 4003 in the LRP1B amino acid sequence,
v) a threonine to alanine change at position 4410 in the LRP1B amino acid sequence,
vi) a serine to phenylalanine change at position 280 in the ERBB2 amino acid sequence (NP_001005862),
vii) a loss of the CDKN2A sequence (NP_000068),
or a combination thereof; and
(e) determining the subject's risk for developing EAC, wherein detecting the presence of cellular changes implicated in development of EAC in (b) and change in at least one of the genes implicated in the development of EAC in (d), indicates that said subject is at risk of developing EAC.

2. The method according to claim 1, wherein:
the method further comprises repeating steps (a)-(e) after a period of time for subjects determined to be at low risk for developing EAC.

3. The method according to claim 1, wherein at least two of the one or more genetic mutations is present in the biological sample.

4. The method of claim 1, wherein
said change in at least one of the genes implicated in the development of EAC is detected using a method selected from the group consisting of PCR, an array, a massarray, a beadarray, a microarray, a genechip, pyrosequencing, nanopore sequencing, nanoballs, sequencing by synthesis, sequencing by expansion, single molecule real time technology, true single molecule sequencing technology, sequencing by ligation, microfluidics, infrared fluorescence, next generation sequencing, and combinations thereof.

5. The method according to claim 1, wherein at least three of the one or more genetic mutations is present in the biological sample.

6. The method according to claim 1, wherein at least four of the one or more genetic mutations is present in the biological sample.

7. The method according to claim 1, wherein at least five of the one or more genetic mutations is present in the biological sample.

## Patentansprüche

1. Verfahren zur Bestimmung des Risikos eines Individuums Esophagus- und Gastroesophagus-Adenocarzinom (EAC) zu entwickeln, wobei das Verfahren umfasst:
(a) Erhalten einer nicht-endoskopischen biologischen Probe aus dem Esophagus eines Individuums mit gastroesophagealen Zellen;
(b) Durchführen einer Zytologie an der erhaltenen biologischen Probe von (a), um die Vorhandensein oder das Fehlen zellulärer Änderungen, die an der Entwicklung von EAC beteiligt sind, zu identifizieren; worin die zellulären Änderungen die Anwesenheit von säulenförmiger Zellmetaplasie umfassen; wahlweise worin die Zytologie mittels Papanicoiaou-Färbung, Haematoxylin-Eosin Färbung, Genchip-Arrays und Fluoreszenz in situ Hybridisierung (FISH) durchgeführt wird;
(c) Bestimmen aus der aus (b) erhaltenen biologischen Probe, die die Anwesenheit zellulärer Änderungen, die an der Entwicklung von EAC beteiligt sind, zeigt, der Nukleinsäuresequenz eines jeden Gens in einem Panel von Genen, die an der Entwicklung von EAC beteiligt sind; wahlweise worin die Sequenzierung mittels Sequenzierung der nächsten Generation durchgeführt wird;
(d) Erfassen einer Änderung in mindestens einem der Gene, die an der Entwicklung von EAC beteiligt sind, worin die Änderung die Erfassung der Anwesenheit von ein oder mehreren genetischen Mutationen oder Änderung der Gen-Kopieanzahl in dem Panel von Genen ist, die aus der erhaltenen biologischen Probe in (c) sequenziert wurden, worin das Panel von Genen ein oder mehrere Gene umfasst ausgewählt aus der Gruppe bestehend aus CDKN2A, ERBB2, SMARCA4, und LRP1B, worin die Änderung ist:
i) eine Threonin zu Methionin-Änderung an Position 910 in der SMARCA4 Aminosäuresequenz (NP_001122316),
ii) eine Glycin zu Serin-Änderung an Position 1162 in der SMARCA4 Aminosäuresequenz,
iii) eine Valin zu Alanin-Änderung an Position 2091 in der LRP1B Aminosäuresequenz (NP _061027),
iv) eine Serin zu Cystein-Änderung an Position 4003 in der LRP1B Aminosäuresequenz,
v) eine Threonin zu Alanin-Änderung an Position 4410 in der LRP1B Aminosäuresequenz,
vi) eine Serin zu Phenylalanin-Änderung an Position 280 in der ERBB2 Aminosäuresequenz (NP_001005862),
vii) ein Verlust der CDKN2A-Sequenz (NP _000068), oder eine Kombination davon; und
(e) Bestimmen des Risikos des Individuums EAC zu entwickeln, worin Erfassen der Anwesenheit zellulärer Änderungen, die an der Entwicklung von EAC beteiligt sind in (b) und Änderung in mindestens einem der Gene, die an der Entwicklung von EAC beteiligt sind in (d) anzeigt, dass das Individuum ein Risiko zur Entwicklung von EAC aufweist.

2. Verfahren nach Anspruch 1, wobei das Verfahren weiter umfasst, Wiederholen der Schritte (a)-(e) nach einer Zeitspanne für Individuen, die ein geringes Risiko für die Entwicklung von EAC aufweisen.

3. Verfahren nach Anspruch 1, wobei mindestens zwei der einen oder mehreren genetischen Mutationen in der biologischen Probe vorhanden sind.

4. Verfahren nach Anspruch 1, wobei die Änderung in mindestens einem der Gene, die an der Entwicklung von beteiligt sind, unter Verwendung eines Verfahrens nachgewiesen wird, ausgewählt aus der Gruppe bestehend aus PCR, einem Array, einem Massenarray, einem Kügelchen-Array, einem Mikroarray, einem Genchip, Pyro-Sequenzierung, Nanopore-Sequenzierung, Nanokügelchen, Sequenzierung durch Synthese, Sequenzierung durch Expansion, Einzelmolekül-Echtzeit-Technology, echte Einzelmolekül-Sequenzierungs-Technologie, Sequenzierung durch Ligation, Mikrofluidie, Infrarot-Fluoreszenz, Sequenzierung der nächsten Generation, und Kombinationen davon.

5. Verfahren nach Anspruch 1, worin mindestens drei der einen oder mehreren genetischen Mutationen in der biologischen Probe vorhanden sind.

6. Verfahren nach Anspruch 1, worin mindestens vier der einen oder mehreren genetischen Mutationen in der biologischen Probe vorhanden sind.

7. Verfahren nach Anspruch 1, worin mindestens fünf der einen oder mehreren genetischen Mutationen in der biologischen Probe vorhanden sind.

## Revendications

1. Procédé pour déterminer le risque d'un sujet de développer un adénocarcinome œsophagien et gastro-œsophagien (EAC), ledit procédé comprenant :
(a) obtenir un échantillon biologique non endoscopique à partir de l'œsophage d'un sujet ayant des cellules gastro-œsophagiennes ;
(b) réaliser une cytologie de l'échantillon biologique obtenu de (a) pour identifier la présence ou l'absence de changements cellulaires, impliqués dans le développement de l'EAC ; les changements cellulaires comprennent la présence de métaplasie de cellules cylindriques ; facultativement dans lequel la cytologie est réalisée par coloration de Papanicolaou, coloration à l'hématoxyline et à l'éosine, réseaux de puces à gènes et hybridation in situ en fluorescence (FISH) ;
(c) déterminer à partir de l'échantillon biologique obtenu de (b) montrant la présence de changements cellulaires, impliqués dans le développement de l'EAC, la séquence d'acide nucléique de chaque gène dans un panel de gènes impliqués dans le développement de l'EAC ; facultativement dans lequel le séquençage est réalisé par un séquençage de nouvelle génération ;
(d) détecter une modification dans au moins l'un des gènes impliqués dans le développement de l'EAC, la modification étant la détection de la présence d'une ou plusieurs mutations génétiques ou modification dans le nombre de copies de gènes dans le panel de gènes séquencés à partir de l'échantillon biologique obtenu dans (c), dans lequel le panel de gènes comprend un ou plusieurs gènes choisis dans le groupe consistant en CDKN2A, ERBB2, SMARCA4 et LRP1B, dans lequel la modification est :
i) une modification de thréonine en méthionine à la position 910 dans la séquence d'acides aminés de SMARCA4 (NP_001122316),
ii) une modification de glycine en sérine à la position 1162 dans la séquence d'acides aminés de SMARCA4,
iii) une modification de valine en alanine à la position 2091 dans la séquence d'acides aminés de LRP1B (NP_061027),
iv) une modification de sérine en cystéine à la position 4003 dans la séquence d'acides aminés de LRP1B,
v) une modification de thréonine en alanine à la position 4410 dans la séquence d'acides aminés de LRP1B,
vi) une modification de sérine en phénylalanine à la position 280 dans la séquence d'acides aminés de ERBB2 (NP_001005862),
vii) une perte de la séquence CDKN2a (NP_000068),
ou une combinaison de celles-ci ; et
(e) déterminer le risque du sujet de développer l'EAC, dans lequel la détection de la présence de changements cellulaires impliqués dans le développement de l'EAC dans (b) et une modification dans au moins l'un des gènes impliqués dans le développement de l'EAC dans (d), indique que ledit sujet présente un risque de développer l'EAC.

2. Procédé selon la revendication 1, dans lequel:
le procédé comprend en outre répéter les étapes (a) à (e) après une période de temps pour les sujets déterminés comme étant à faible risque de développer l'EAC.

3. Procédé selon la revendication 1, dans lequel au moins deux de la ou des mutations génétiques sont présentes dans l'échantillon biologique.

4. Procédé selon la revendication 1, dans lequel Ladite modification dans au moins l'un des gènes impliqués dans le développement de l'EAC est détectée à l'aide d'un procédé choisi dans le groupe consistant en une PCR, un réseau, un massARRAY, un réseau de billes, un microréseau, une puce à gènes, un pyroséquençage, un séquençage par nanopores, des nanobilles, un séquençage par synthèse, un séquençage par expansion, une technologie en temps réel sur molécule unique, une véritable technologie de séquençage sur molécule unique, un séquençage par ligature, un microfluidique, une fluorescence infrarouge, un séquençage de nouvelle génération et les combinaisons de ceux-ci.

5. Procédé selon la revendication 1, dans lequel au moins trois de la ou des mutations génétiques sont présentes dans l'échantillon biologique.

6. Procédé selon la revendication 1, dans lequel au moins quatre de la ou des mutations génétiques sont présentes dans l'échantillon biologique.

7. Procédé selon la revendication 1, dans lequel au moins cinq de la ou des mutations génétiques sont présentes dans l'échantillon biologique.
